(19) Europäisches Patentamt
European Patent Office
Office européen des brevets

(11) EP 4 086 253 A1

(12) # EUROPEAN PATENT APPLICATION
published in accordance with Art. 153(4) EPC

(43) Date of publication:
**09.11.2022 Bulletin 2022/45**

(21) Application number: **20909531.4**

(22) Date of filing: **30.12.2020**

(51) International Patent Classification (IPC):
***C07D 471/04*** *(2006.01)* ***A61P 35/00*** *(2006.01)*
***A61P 37/00*** *(2006.01)* ***A61K 31/395*** *(2006.01)*

(52) Cooperative Patent Classification (CPC):
**A61K 31/395; A61K 31/437; A61K 31/4985; A61P 35/00; A61P 37/00; C07D 471/04; C07D 487/04; C07D 519/00**

(86) International application number:
**PCT/CN2020/141307**

(87) International publication number:
**WO 2021/136354 (08.07.2021 Gazette 2021/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.01.2020 CN 202010006873**
**28.05.2020 CN 202010470293**
**04.09.2020 CN 202010922161**

(71) Applicants:
- **Shanghai Hansoh Biomedical Co., Ltd.**
**Shanghai 201203 (CN)**
- **Jiangsu Hansoh Pharmaceutical Group Co., Ltd.**
**Lianyungang, Jiangsu 222047 (CN)**

(72) Inventors:
- **ZENG, Mi**
**Shanghai 201203 (CN)**
- **GAO, Peng**
**Shanghai 201203 (CN)**
- **QI, Wenjie**
**Shanghai 201203 (CN)**
- **LI, Jian**
**Shanghai 201203 (CN)**
- **CHENG, Yu**
**Shanghai 201203 (CN)**
- **WANG, Ting**
**Shanghai 201203 (CN)**
- **BAO, Rudi**
**Shanghai 201203 (CN)**

(74) Representative: **Hoffmann Eitle**
**Patent- und Rechtsanwälte PartmbB**
**Arabellastraße 30**
**81925 München (DE)**

(54) **BIPHENYL DERIVATIVE INHIBITOR, PREPARATION METHOD THEREFOR AND USE THEREOF**

(57) A biphenyl derivative inhibitor represented by general formula (Ib) and use in the preparation of medicaments for treating cancer, infectious diseases, and autoimmune diseases.

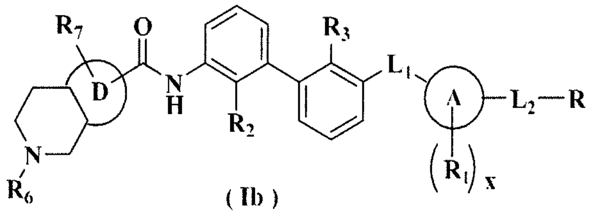

(Ib)

EP 4 086 253 A1

## Description

This application claims the priorities of Chinese patent application CN202010006873.9 filed on January 3, 2020, the Chinese patent application CN202010470293.5 filed on May 28, 2020, and the Chinese patent application CN202010922161.1 filed on September 4, 2020. The present disclosure refers to the full text of the above Chinese patent application.

Technical field

The present disclosure belongs to the field of drug synthesis, specifically related to a biphenyl derivative inhibitor, a preparation method therefor and a use thereof.

Background

The immune system plays an important role in the control of many diseases such as cancer. However, tumor cells evade immune attack or suppress the activation of the immune system through various pathways. Blocking the signal transmission at immunosuppressive checkpoints, such as programmed cell death protein 1 (PD-1), has proven to be a potential therapeutic modality.

PD-1, a member of the CD28 superfamily, is an immunosuppressive receptor on the surface of immune cells, especially cytotoxic T cells. PD-1 has two ligands, PD-L1 and PD-L2, wherein PD-L1, programmed cell death receptor ligand 1, is expressed in a variety of cells, such as macrophages and dendritic cells, and is generally highly expressed on tumor cells. PD-L1 plays an immunosuppressive effect by combining with PD-1 and enables tumor cells to escape the killing of T cells, inhibits the activation of T cells and the production of corresponding cytokines, attenuates infectious immunity and tumor immunity, and promotes the progress of infectious diseases and tumors. The use of PD-L1 inhibitors such as antibodies or small molecule inhibitors can relieve the immunosuppressive effect of PD-L1 and promote the immune clearance of tumors, thereby achieving the effect of tumor treatment.

PD-1/PD-L1 is a hot spot in tumor immunotherapy research in recent years, and the breadth, depth and durability of its monoclonal antibody drug response are very rare. At present, several PD-1/PD-L1 monoclonal antibodies have been marketed clinically and achieved great success. PD-L1 inhibitors can be used to treat almost all major cancers including non-small cell lung cancer, liver cancer, gastric cancer, bowel cancer, kidney cancer, etc., and have great clinical application value.

PD-L1 inhibitors from macromolecules to small molecules are becoming a new research and development trend and hotspot. Small molecule inhibitors have many natural advantages from administration methods to production costs, and have the potential to replace antibody macromolecules, currently foreign pharmaceutical companies including BMS and Incyte are actively developing PD-L1 small molecule inhibitors.

The oral small molecule inhibitor developed by BMS is currently in the preclinical research stage, and several patents have been published continuously. The small molecule inhibitor INCB086550 developed by Incyte is in phase I clinical research, and the small molecule inhibitor CA-170 developed by Aurigene/Curis is already in phase II clinical research.

International application WO2015034820, WO2015160641, WO2014151634, WO2017066227, WO2017070089, WO2017106634, WO2017112730, WO2017192961, WO2017222976, WO2018013789, WO2018044783, WO2018119224, WO2018119236, WO2018119263, WO2018119266 and WO2018119286, and several other patents reported PD-1 or PD-L1 small molecule compound inhibitors. Furthermore, international applications WO2014151634, WO2011161699, WO2012168944, WO2013132317, WO2013144704, WO2015033299, WO2015033301, WO2015033303 and WO2015036927 have reported macrocyclic and peptidic compounds PD-1 or PD-1 inhibitors. However, there is still a great need for PD-L1 small-molecule inhibitors with more effective, better pharmacokinetic properties and druggability of the PD-1/PD-L1 pathway.

PD-L1 small molecule inhibitors have good application prospects in the pharmaceutical industry as drugs. First, PD-L1 small molecule inhibitors can be administered orally, which has the advantage of greater compliance than intravenous administration of antibody drugs, and can avoid serious side effects such as colitis caused by long-term residence of antibody in the body. Second, small-molecule inhibitors of PD-L1 have a unique mechanism of action that binds PD-L1 and makes it endocytosed, and may show different efficacy from antibodies in clinical practice. Finally, the production and quality control costs of PD-L1 small molecule inhibitors are lower, with a price advantage far lower than the cost of macromolecular drugs, and like PD-L1 antibody inhibitors, they can be applied to a variety of major tumors and have a huge market potential.

Content of the present invention

An object of the present disclosure is to provide a compound represented by general formula (Ib), a stereoisomer thereof or a pharmaceutically acceptable salt thereof, the specific structure of the compound is as follows:

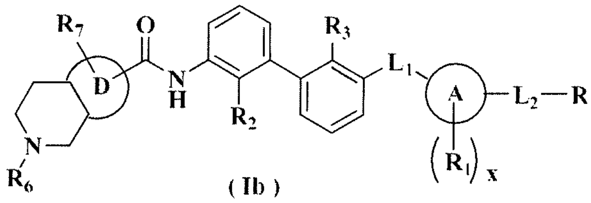

(Ib)

wherein:

$L_1$ is selected from a bond, $-(CH_2)_{n1}-$, $-(CH_2)_{n1}NR_{aa}C(O)(CR_{aa}R_{bb})_{n2}-$, $-(CH_2)_{n1}(CR_{aa}R_{bb})_{n2}-$,$-(CR_{aa}R_{bb})_{n1}O(CH_2)_{n2}-$, $-(CH_2)_{n1}O(CRaaR_{bb})_{n2}-$, $-(CR_{aa}R_{bb})_{n1}S(CH_2)_{n2}-$, $-(CH_2)_{n1}S(CR_{aa}R_{bb})_{n2}-$,$-(CR_{aa}R_{bb})_{n1}(CH_2)_{n2}NR_{cc}-$, $-(CH_2)_{n1}NR_{aa}(CR_{bb}R_{cc})_{n2}-$, $-(CH_2)_{n1}C(O)(CR_{aa}R_{bb})_{n2}-$, $-(CH_2)_{n1}P(O)R_{aa}-$,$-(CH_2)_{n1}S(O)_{n2}-$, $-(CH_2)_{n1}S(O)_{n2}NR_{aa}-$, $-(CH_2)_{n1}NR_{aa}S(O)_{n2}-$ or $-(CH_2)_{n1}C(O)NR_{aa}-$;

$R_{aa}$, $R_{bb}$ and $R_{cc}$ are each independently selected from hydrogen, deuterium, halogen, amino, nitro, hydroxyl, cyano, oxo, thioxo, carboxyl, alkyl, deuterated alkyl, haloalkyl, hydroxyalkyl, alkoxy, haloalkoxy, alkenyl, alkynyl, heterocyclylalkyl, cycloalkyl, heterocyclyl, aryl or heteroaryl, and the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl, and heteroaryl are optionally further substituted;

or, any two of $R_{aa}$, $R_{bb}$ and $R_{cc}$ are connected to form a cycloalkyl, heterocyclyl, aryl or heteroaryl, and the cycloalkyl, heterocyclyl, aryl and heteroaryl are optionally further substituted;

$L_2$ is selected from a bond, $-(CH_2)_{n3}-$, $-(CR_{dd}R_{ee})_{n3}-$, $-(CR_{dd}R_{ee})_{n3}(CH_2)_{n4}NR_{ff}-$,$-(CH_2)_{n3}NR_{dd}(CR_{ee}R_{ff})_{n4}-$, $-(CH_2)_{n3}(CR_{dd}R_{ee})_{n4}-$, $-(CR_{dd}R_{ee})_{n3}O(CH_2)_{n4}-$, $-(CH_2)_{n3}O(CR_{dd}R_{ee})_{n4}-$,$-(CR_{aa}R_{bb})_{n3}S(CH_2)_{n4}-$, $-(CH_2)_{n3}S(CR_{aa}R_{bb})_{n4}-$, $-(CH_2)_{n3}C(O)(CR_{dd}R_{ee})_{n4}-$, $-(CH_2)_{n3}NR_{dd}C(O)(CR_{ee}R_{ff})_{n4}-$,$-(CH_2)_{n3}P(O)R_{dd}-$, $-(CH_2)_{n3}S(O)_{n4}-$, $-(CH_2)_{n3}S(O)_{n4}NR_{dd}-$, $-(CH_2)_{n3}NR_{dd}S(O)_{n4}-$ or $-(CH_2)_{n3}C(O)NR_{dd}-$;

$R_{dd}$, $R_{ee}$ and $R_{ff}$ are each independently selected from hydrogen, deuterium, halogen, amino, nitro, hydroxyl, cyano, oxo, thioxo, carboxyl, alkyl, deuterated alkyl, haloalkyl, hydroxyalkyl, alkoxy, haloalkoxy, alkenyl, alkynyl, heterocyclylalkyl, cycloalkyl, heterocyclyl, aryl or heteroaryl, and the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl, and heteroaryl are optionally further substituted;

or, any two of $R_{dd}$, $R_{ee}$ and $R_{ff}$ are connected to form a cycloalkyl, heterocyclyl, aryl or heteroaryl, wherein the cycloalkyl, heterocyclyl, aryl and heteroaryl are optionally further substituted;

ring A is selected from cycloalkyl, heterocyclyl, aryl or heteroaryl;

R is selected from hydrogen, deuterium, halogen, amino, hydroxyl, mercapto, cyano, nitro, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloalkyl, alkoxy, alkylthio, haloalkoxy, hydroxyalkyl, cycloalkyl, heterocyclyl, aryl and heteroaryl are optionally further substituted;

$R_1$ is selected from hydrogen, deuterium, halogen, amino, hydroxyl, mercapto, cyano, nitro, oxo, thioxo, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloalkyl, alkoxy, alkylthio, haloalkoxy, hydroxyalkyl, cycloalkyl, heterocyclyl, aryl and heteroaryl are optionally further substituted;

$R_2$ is selected from hydrogen, deuterium, halogen, amino, hydroxyl, mercapto, cyano, nitro, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloalkyl, alkoxy, alkylthio, haloalkoxy, hydroxyalkyl, cycloalkyl, heterocyclyl, aryl and heteroaryl are optionally further substituted;

$R_3$ is selected from hydrogen, deuterium, halogen, amino, hydroxyl, mercapto, cyano, nitro, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloalkyl, alkoxy, alkylthio, haloalkoxy, hydroxyalkyl, cycloalkyl, heterocyclyl, aryl and heteroaryl are optionally further substituted;

ring D is selected from heterocyclyl or heteroaryl, preferably five- or six-membered heteroaryl, more preferably imidazolyl, thiazolyl or pyridyl;

$R_6$ is selected from hydrogen, deuterium, halogen, amino, hydroxyl, mercapto, cyano, nitro, oxo, thioxo, alkyl, alkenyl, alkynyl, deuterated alkyl, haloalkyl, alkoxy, alkylthio, haloalkoxy, hydroxyalkyl, cycloalkyl, heterocyclyl, aryl,

heteroaryl, $-(CH_2)_{m3}R_d$, $-(CH_2)_{m3}(CR_dR_e)_{m4}R_f$, $-(CH_2)_{m3}NR_dCHR_eR_f$, $(CH_2)_{m3}(CR_dR_e)_{m4}C(O)OR_f$, $-(CH_2)_{m3}C(O)O(CR_dR_e)_{m4}OC(O)R_f$, $-(CH_2)_{m3}CR_d = CR_eR_f$, $-(CH_2)_{m3}OR_d$,$-(CH_2)_{m3}NR_dR_e$, $-(CH_2)_{m3}SR_d$, $-(CH_2)_{m3}C(O)R_d$, $-(CH_2)_{m3}C(O)OR_d$, $-(CH_2)_{m3}S(O)_{m4}R_d$, $-(CH_2)_{m3}NR_dC(O)R_e$ or $-(CH_2)_{m3}C(O)NR_dR_e$, and the amino, alkyl, alkenyl, alkynyl, deuterated alkyl, haloalkyl, alkoxy, alkylthio, haloalkoxy, hydroxyalkyl, cycloalkyl, heterocyclyl, aryl and heteroaryl are optionally further substituted;

preferably hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl, 5- to 14-membered heteroaryl, $-(CH_2)_{m3}R_d$, $-(CH_2)_{m3}(CR_dR_e)_{m4}R_f$, $-(CH_2)_{m3}OR_{d,}$ $-(CH_2)_{m3}NR_dR_e$, $-(CH_2)_{m3}NR_dCHR_eR_f$, $(CH_2)_{m3}(CR_dR_e)_{m4}C(O)$, $-(CH_2)_{m3}C(O)O(CR_dR_e)_{m4}OC(O)R_f$ or $-(CH_2)_{m3}CR_d=CR_eR_f$;

more preferably hydrogen, methyl, ethyl, isopropyl, methoxy, cyclopropyl,

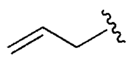,

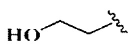, 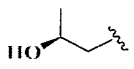, 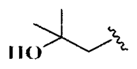, 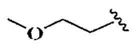, 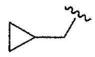, 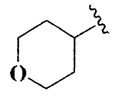, 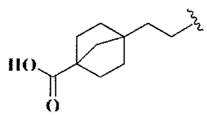,

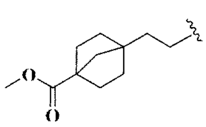, 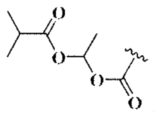, 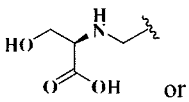 or 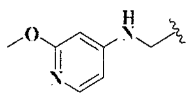;

$R_d$, $R_e$ and $R_f$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl, 5- to 14-membered heteroaryl, $-(CH_2)_{m5}R_h$, $(CH_2)_{m5}C(O)OR_h$, $-(CH_2)_{m5}OR_h$,$-(CH_2)_{m5}NR_hR_i$, $-(CH_2)_{m5}SR_h$, $-(CH_2)_{m5}C(O)R_h$, $-(CH_2)_{m5}NR_hC(O)R_i$ and $-(CH_2)_{m5}C(O)NR_hR_i$;

or, any two of $R_d$, $R_e$ and $R_f$ are connected to form a cycloalkyl, heterocyclyl, aryl or heteroaryl, and the cycloalkyl, heterocyclyl, aryl and heteroaryl are optionally further substituted;

$R_h$ and $R_i$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

$R_7$ is selected from hydrogen, deuterium, halogen, amino, hydroxyl, mercapto, cyano, nitro, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloaclkyl, alkoxy, alkylthio, haloalkoxy, hydroxyalkyl, cycloalkyl, heterocyclyl, aryl and heteroaryl are optionally further substituted;

preferably hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl;

more preferably hydrogen, fluorine, chlorine, bromine, methyl, ethyl or isopropyl; m3 is an integer of 0 to 3;

m4 is an integer from 0 to 3; and

m5 is an integer from 0 to 3.

In a further preferred embodiment of the present disclosure, the present disclosure provides a compound represented by general formula (I), a stereoisomer thereof or a pharmaceutically acceptable salt thereof, wherein the structure of the compound represented by general formula (I) is as follows:

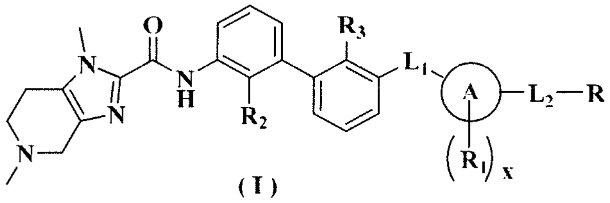

(I)

.

In a preferred embodiment of the present disclosure, $L_1$ is selected from a bond, $-NR_{aa}C(O)-$,$-(CH_2)_{n1}-$, $-C(O)-$, $-O(CH_2)_{n2}-$, $-(CH_2)_{n1}O-$, $-S(CH_2)_{n2}-$, $-(CH_2)_{n1}S-$ or $-(CH_2)_{n1}NR_{aa}-$;

more preferably a bond, -NHC(O)-, $-CH_2-$, -C(O)-, -O-, $-OCH_2-$, $-CH_2O-$, -S-, $-SCH_2-$, $-CH_2S-$,-NH- or $-CH_2NH-$;

$R_{aa}$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

n1 is an integer from 0 to 3.

In a preferred embodiment of the present disclosure, $L_2$ is selected from a bond, $-(CH_2)_{n3}-$,$-(CR_{dd}R_{ee})_{n3}-$, $-(CR_{dd}R_{ee})_{n3}(CH_2)_{n4}NR_{ff}-$, $-(CH_2)_{n3}NR_{dd}(CR_{ee}R_{ff})_{n4}-$, $-(CH_2)_{n3}(CR_{dd}R_{ee})_{n4}-$,$-(CR_{dd}R_{ee})_{n3}O(CH_2)_{n4}-$, $-(CH_2)_{n3}O(CR_{dd}R_{ee})_{n4}-$, $-(CR_{aa}R_{bb})_{n3}S(CH_2)_{n4}-$, $-(CH_2)_{n3}S(CR_{aa}R_{bb})_{n4}-$,$-(CH_2)_{n3}C(O)(CR_{dd}R_{ee})_{n4}-$, $-(CH_2)_{n3}NR_{dd}C(O)(CR_{ee}R_{ff})_{n4}-$, $-(CH_2)_{n3}P(O)R_{dd}-$, $-(CH_2)_{n1}S(O)_{n4}-$,$-(CH_2)_{n3}S(O)_{n4}NR_{dd}-$, $-(CH_2)_{n3}NR_{dd}S(O)_{n4}-$ or $-(CH_2)_{n3}C(O)NR_{dd}-$;

preferably a bond, $-(CH_2)_{n3}-$, $-(CR_{dd}R_{ee})_{n3}-$, $-(CR_{dd}R_{ee})_{n3}(CH_2)_{n4}NR_{ff}-$ or $-(CH_2)_{n3}NR_{dd}(CR_{ee}R_{ff})_{n4}-$;

more preferably a bond,

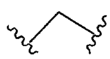, 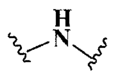, 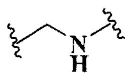, 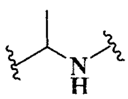, 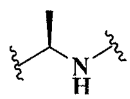,

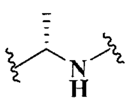, 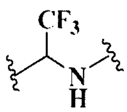, 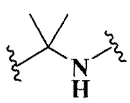, 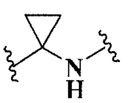, 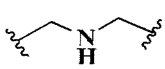, 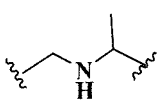, 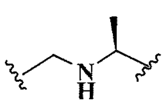,

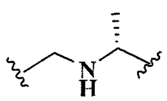, 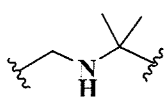, 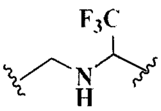, 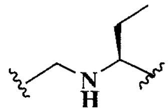, 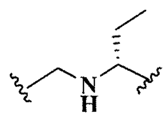, 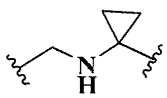,

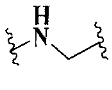 or 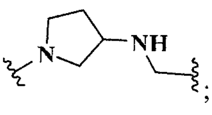;

$R_{dd}$, $R_{ee}$ and $R_{ff}$ are each independently selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio,

$C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

or, any two of $R_{dd}$, $R_{ee}$ and $R_{ff}$ are connected to form a $C_{3-12}$ cycloalkyl, 3- to 12-membered heterocyclyl, $C_{6-14}$ aryl or 5- to 14-membered heteroaryl, and the $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

n3 is an integer from 0 to 3; and

n4 is an integer from 0 to 3;

or, $L_2$ is selected from $-(CR_{dd}R_{ee})_{n3}-$, $-(CH_2)_{n3}NR_{dd}(CR_{ee}R_{ff})_{n4}-$ or $-(CH_2)_{n3}O(CR_{ee}R_{ff})_{n4}-$;

preferably

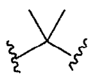, 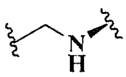, 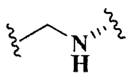, 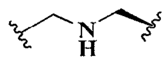, 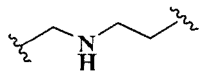,

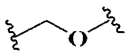, 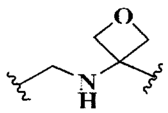 or 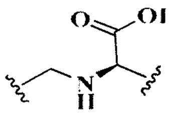;

$R_{dd}$, $R_{ee}$ and $R_{ff}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

or, any two of $R_{dd}$, $R_{ee}$ and $R_{ff}$ are connected to form a $C_{3-12}$ cycloalkyl, 3- to 12-membered heterocyclyl, $C_{6-14}$ aryl or 5- to 14-membered heteroaryl, and the $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

n3 is an integer from 0 to 3; and

n4 is an integer from 0 to 3.

In a preferred embodiment of the present disclosure, ring A is selected from $C_{3-8}$ cycloalkyl, 3- to 10-membered heterocyclyl, $C_{6-10}$ aryl or 5- to 12-membered heteroaryl,

preferably, 5- to 10-membered nitrogen-containing heteroaryl,

more preferably, 5- to 6-membered nitrogen-containing heteroaryl,

most preferred are the following groups:

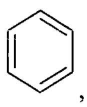, 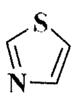, 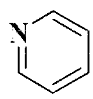, 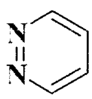, 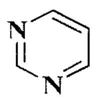 or 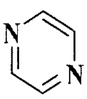.

In a further preferred embodiment of the present disclosure, R is selected from hydrogen, halogen, amino,

hydroxyl, cyano, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl or $-(CH_2)_{n5}NR_{gg}C(O)R_{hh}$, and the amino, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl and 5- to 12-membered heteroaryl are optionally further substituted by one or more substituents of hydrogen, halogen, amino, hydroxyl, oxo, $C_{1-6}$ alkyl, $C_{1-6}$ haloalkyl, $C_{1-6}$ hydroxyalkyl, $C_{1-6}$ alkoxy, $C_{3-8}$ cycloalkyl, $-(CH_2)_{n6}OR_{ii}$, $-(CH_2)_{n6}C(O)R_{ii}$ or $-(CH_2)_{n6}C(O)OR_{ii}$;
preferably fluorine, chlorine, bromine, hydroxyl, vinyl, ethynyl,

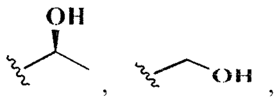
, ,

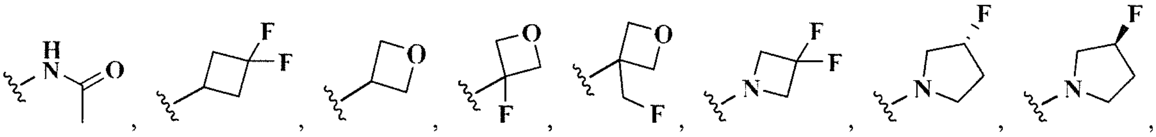
, , , , , , , ,

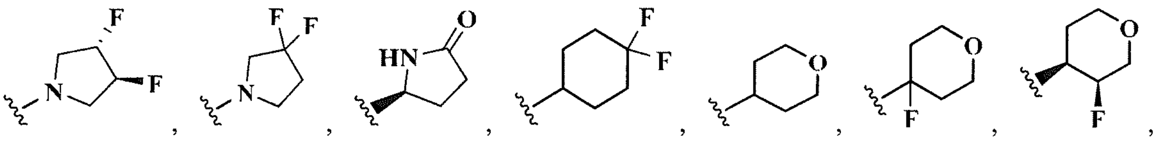
, , , , , , ,

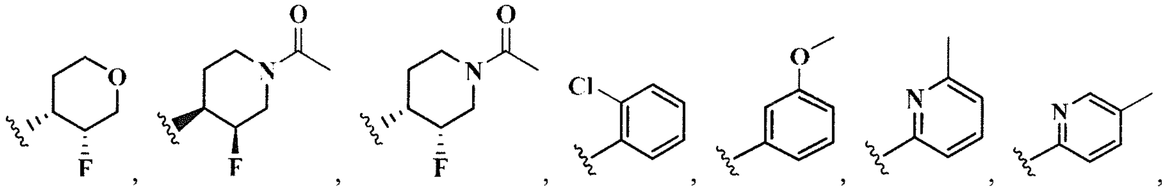
, , , , , , ,

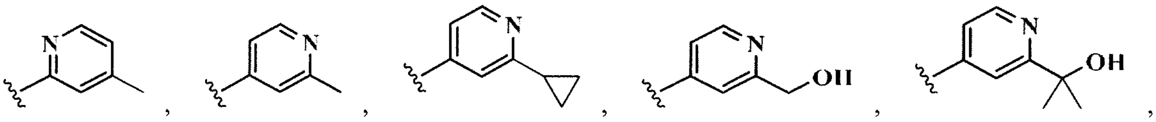
, , , , ,

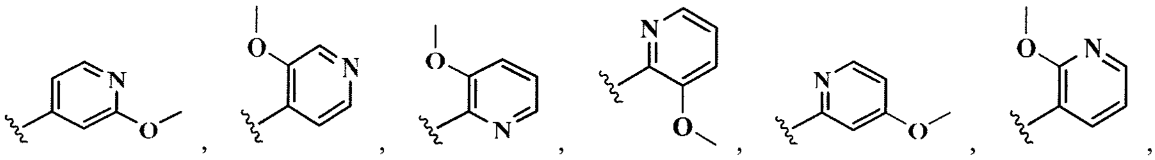
, , , , , ,

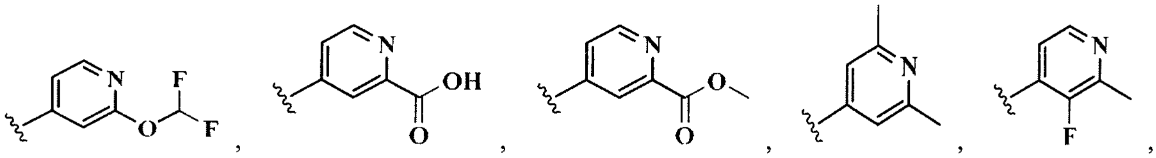
, , , , ,

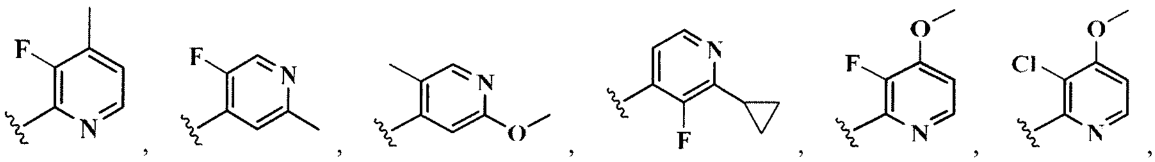
, , , , , ,

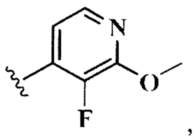, 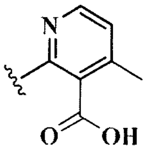, 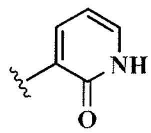, 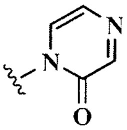, 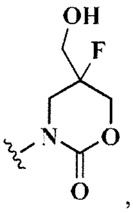, 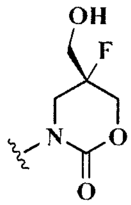, 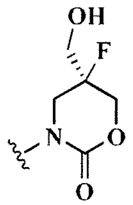 or

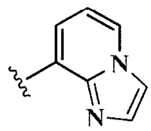;

$R_{gg}$, $R_{hh}$ and $R_{ii}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

n5 is an integer from 0 to 3; and

n6 is an integer from 0 to 3;

or R is selected from hydrogen, halogen, amino, hydroxyl, cyano, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $(CH_2)_{n5}C(O)OR_{gg}$, $(CH_2)_{n5}C(O)NR_{gg}R_{hh}$ or $-(CH_2)_{n5}NR_{gg}C(O)R_{hh}$, and the amino, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl and 5- to 12-membered heteroaryl are optionally further substituted by one or more substituents of hydrogen, deuterium, halogen, amino, hydroxyl, oxo, $C_{1-6}$ alkyl, $C_{1-6}$ haloalkyl, $C_{1-6}$ hydroxyalkyl, $C_{1-6}$ alkoxy, $C_{3-8}$ cycloalkyl, $-(CH_2)_{n6}OR_{ii}$, $-(CH_2)_{n6}C(O)R_{ii}$, $-(CH_2)_{n6}OC(O)R_{ii}$ or $-(CH_2)_{n6}C(O)OR_{ii}$,

preferably methyl, difluoromethyl, $-C(O)OH$, $-C(O)OCH_2CH_3$, $-C(O)NHCH_3$,

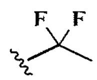,

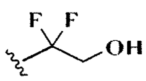, 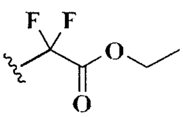, 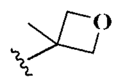, 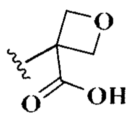, 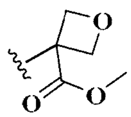, 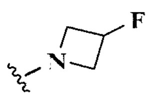, 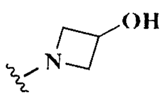,

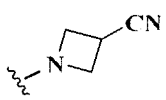, 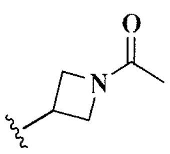, 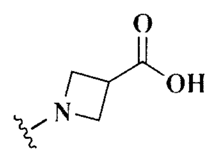, 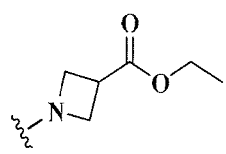, 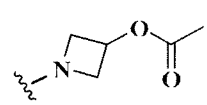,

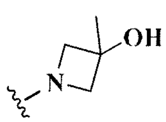, 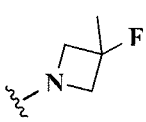, 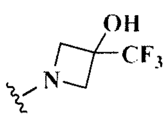, 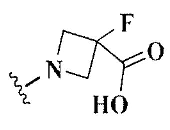, 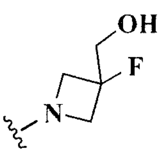, 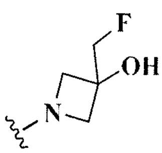,

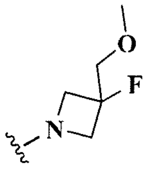, 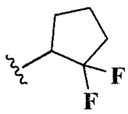, 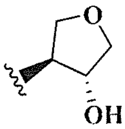, 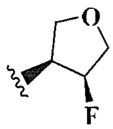, 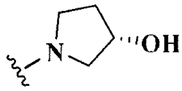, 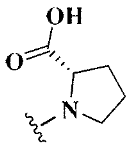, 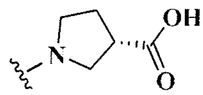,

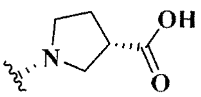, 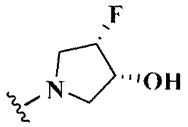, 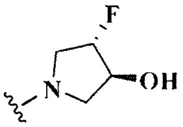, 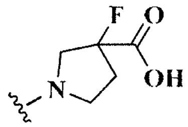, 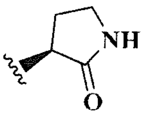,

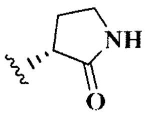, 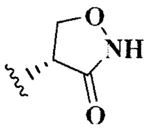, 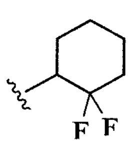, 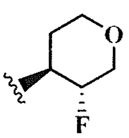, 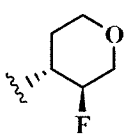, 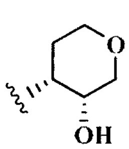, 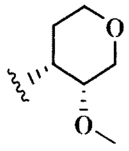,

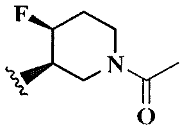, 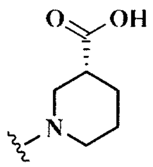, 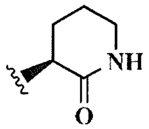, 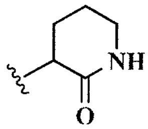, 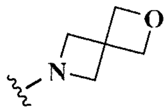, 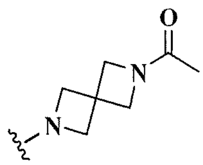,

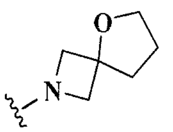 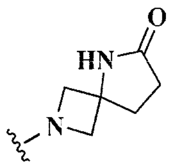, 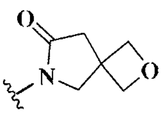, 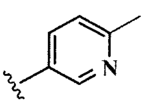, 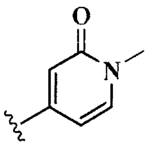, 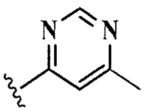,

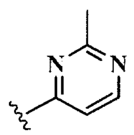 or 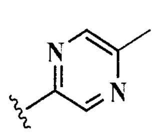 ;

$R_{gg}$, $R_{hh}$ and $R_{ii}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n5 is an integer from 0 to 3; and
n6 is an integer from 0 to 3;
or R is selected from hydrogen, halogen, amino, hydroxyl, cyano, $C_{1-3}$ alkyl, $C_{1-3}$ haloalkyl, $C_{2-4}$ alkenyl, $C_{2-4}$ alkynyl, $C_{3-8}$ cycloalkyl, 3- to 10-membered heterocyclyl, $C_{6-10}$ aryl, 5- to 12-membered heteroaryl, $(CH_2)_{n5}C(O)OR_{gg}$, $(CH_2)_{n5}C(O)NR_{gg}R_{hh}$ or $-(CH_2)_{n5}NR_{gg}C(O)R_{hh}$, and the amino, $C_{1-3}$ alkyl, $C_{1-3}$ haloalkyl, $C_{2-4}$ alkenyl, $C_{2-4}$ alkynyl, $C_{3-8}$ cycloalkyl, 3- to 10-membered heterocyclyl, $C_{6-10}$ aryl and 5- to 12-membered heteroaryl are optionally further substituted by one or more substituents of deuterium, halogen, amino, hydroxyl, cyano, carboxyl, oxo, $C_{1-6}$ alkyl, $C_{1-6}$ haloalkyl, $C_{1-6}$ hydroxyalkyl, $C_{1-6}$ alkoxy, $C_{3-8}$ cycloalkyl, $(CH_2)_{n6}R_{ii}$, $-(CH_2)_{n6}OR_{ii}$, $-(CH_2)_{n6}C(O)R_{ii}$, $-(CH_2)_{n6}OC(O)R_{ii}$, $-(CH_2)_{n6}C(O)OR_{ii}$, $-(CH_2)_{n6}C(O)NR_{ii}R_{i}$, $-(CH_2)_{n6}NR_{ii}R_{i}$, $-(CH_2)_{n6}NR_{ii}C(O)R_{i}$ or $-(CH_2)_{n6}S(O)_{m6}R_{ii}$;

preferably $-CH(CH_2OH)_2$, $-CH(CH_2OH)(COOCH_3)$,

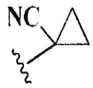 , 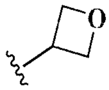 , 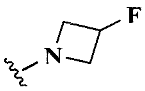 ,

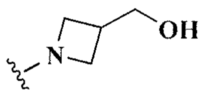 , 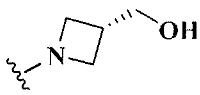 , 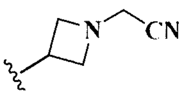 , 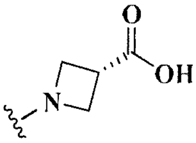 , 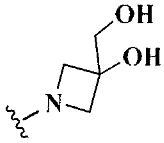 ,

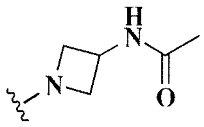 , 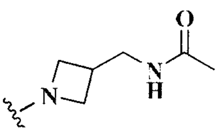 , 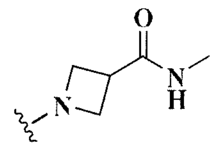 , 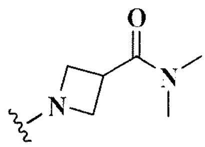 , 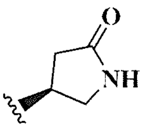 ,

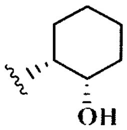 , 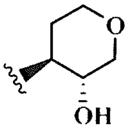 , 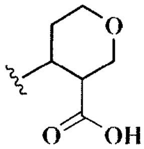 , 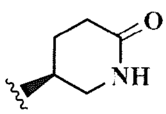 , 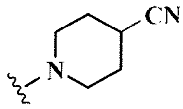 , 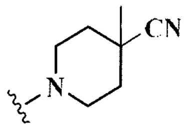 ,

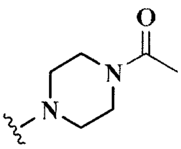 , 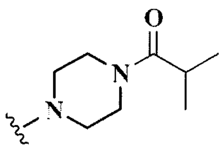 , 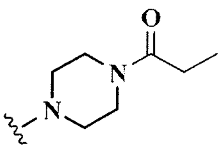 , 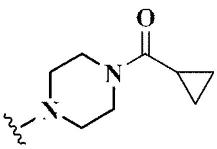 , 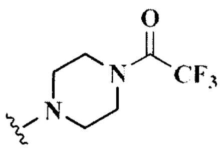 ,

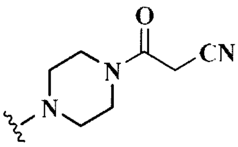 , 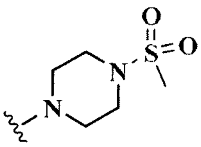 , 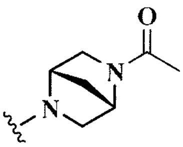 , 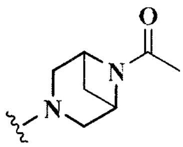 , 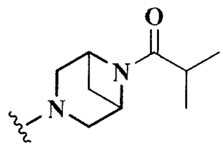 ,

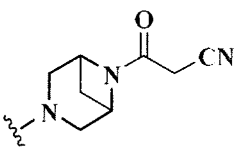 , 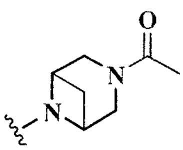 , 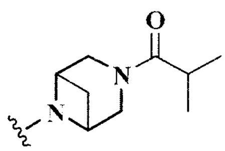 , 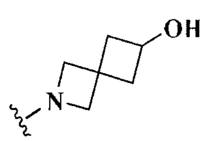 , 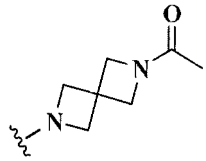 ,

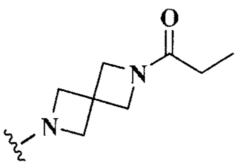 , 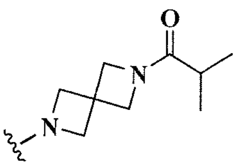 , 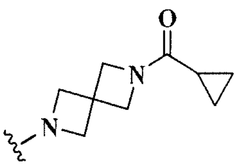 , 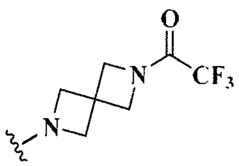 ,

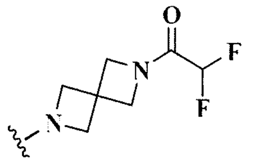, 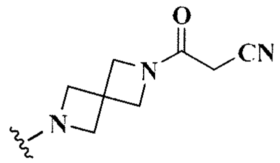, 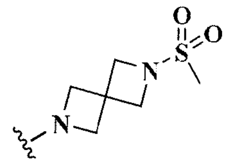, 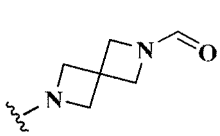,

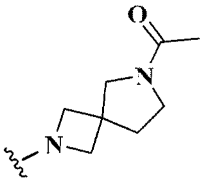, 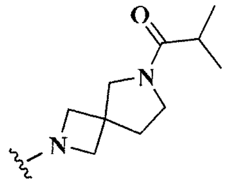, 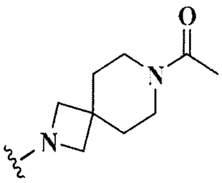 or 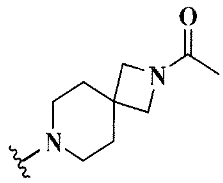;

$R_{gg}$, $R_{hh}$, $R_{ii}$ and $R_i$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl , $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of deuterium, halogen, amino, hydroxyl, mercapto, cyano, nitro, carboxyl , $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n5 is an integer from 0 to 3;
n6 is an integer from 0 to 3; and
m6 is an integer from 0 to 2.

In a preferred embodiment of the present disclosure, $R_1$ is selected from hydrogen, halogen, amino, hydroxyl, cyano, oxo, $C_{1-3}$ alkyl, $C_{1-3}$ haloalkyl, $C_{1-3}$ alkoxy, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $-CH_2)_{n7}OR_{jj}$, $-(CH_2)_{n7}C(O)NR_{jj}R_{pp}$ or$-O(CH_2)_{n7}R_{jj}$;

preferably hydrogen, fluorine, chlorine, bromine, methyl, ethyl, isopropyl, trifluoromethyl, methoxy, cyano, oxo, cyclopropyl, $-OCH_2CN$ or $-C(O)NH_2$;
$R_{jj}$ and $R_{pp}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl; and
n7 is an integer from 0 to 3.

In a preferred embodiment of the present disclosure, $R_2$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

In a further preferred embodiment of the present disclosure, $R_2$ is selected from hydrogen, halogen or $C_{1-3}$ alkyl;

In a further preferred embodiment of the present disclosure, $R_2$ is selected from chlorine or methyl.

In a further preferred embodiment of the present disclosure, $R_2$ is selected from hydrogen, fluorine, chlorine, bromine, methyl, ethyl or isopropyl.

In a preferred embodiment of the present disclosure, $R_3$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

In a further preferred embodiment of the present disclosure, $R_3$ is selected from hydrogen, halogen or $C_{1-3}$ alkyl;

In a further preferred embodiment of the present disclosure, $R_3$ is selected from chlorine or methyl.

In a further preferred embodiment of the present disclosure, $R_3$ is selected from hydrogen, fluorine, chlorine, bromine, methyl, ethyl or isopropyl.

In a further preferred embodiment of the present disclosure, the present disclosure provides a compound represented by formula (II), a stereoisomer thereof or a pharmaceutically acceptable salt thereof, wherein the specific general structure is as follows:

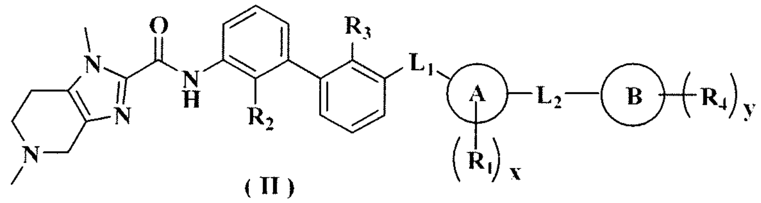

(II)

wherein:

ring B is selected from $C_{3-12}$ cycloalkyl, 3- to 12-membered heterocyclyl, $C_{6-14}$ aryl or 5- to 14-membered heteroaryl;
$R_4$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
preferably substituted by one or more substituents of hydrogen, halogen, amino, hydroxyl, oxo, $C_{1-6}$ alkyl, $C_{1-6}$ haloalkyl, $C_{1-6}$ alkoxy, $C_{3-8}$ cycloalkyl;
y is an integer from 0 to 6.

In a further preferred embodiment of the present disclosure, the present disclosure provides a compound represented by formula (IV-A), a stereoisomer thereof or a pharmaceutically acceptable salt thereof, wherein the specific general structure is as follows:

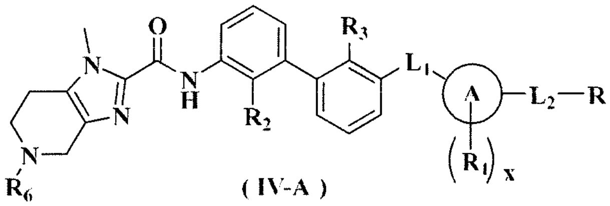

(IV-A)

wherein:

$L_1$ is selected from a bond or -NHC(O)-;
$L_2$ is selected from a bond, $-(CH_2)_{n3}-$, $-(CR_{dd}R_{ee})_{n3}-$, $-(CR_{dd}R_{ee})_{n3}O-$, $-(CR_{dd}R_{ee})_{n3}(CH_2)_{n4}NR_{ff}-$ or $-(CH_2)_{n3}NR_{dd}(CR_{ee}R_{ff})_{n4}-$;

ring A is selected from

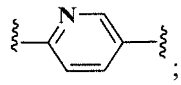;

$R_1$ is selected from hydrogen, halogen, amino, hydroxyl, cyano, oxo, $C_{1-3}$ alkyl, $C_{1-3}$ haloalkyl, $C_{1-3}$ alkoxy, $C_{1-6}$ hydroxyalkyl, $C_{1-6}$ alkyl, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $-(CH_2)_{n7}OR_{jj}$, $-(CH_2)_{n7}C(O)NR_{jj}R_{pp}$ or $-O(CH_2)_{n7}R_{jj}$;

$R_2$ is selected from hydrogen, halogen or $C_{1-3}$ alkyl; preferably methyl or chlorine;

$R_3$ is selected from hydrogen, halogen or $C_{1-3}$ alkyl; preferably methyl or chlorine;

R is selected from hydrogen, halogen, amino, hydroxyl, cyano, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $(CH_2)_{n5}C(O)OR_{gg}$, $(CH_2)_{n5}C(O)NR_{gg}R_{hh}$ or $-(CH_2)_{n5}NR_{gg}C(O)R_{hh}$, and the amino, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl and 5- to 12-membered heteroaryl are optionally further substituted by one or more substituents of hydrogen, deuterium, halogen, amino, hydroxyl, oxo, $C_{1-6}$ alkyl, $C_{1-6}$ haloalkyl, $C_{1-6}$ hydroxyalkyl, $C_{1-6}$ alkoxy, $C_{3-8}$ cycloalkyl, $-(CH_2)_{n6}OR_{ii}$, $-(CH_2)_{n6}C(O)R_{ii}$, $-(CH_2)_{n6}OC(O)R_{ii}$ or $-(CH_2)_{n6}C(O)OR_{ii}$;

$R_6$ is selected from hydrogen, methyl, ethyl, isopropyl, methoxy, cyclopropyl,

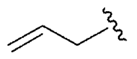,

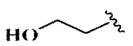, 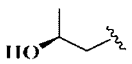, 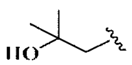, 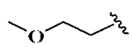, 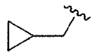, 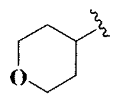, 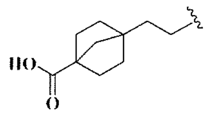,

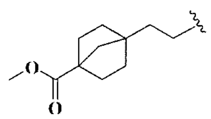, 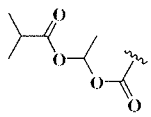, 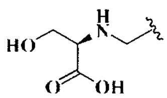 or 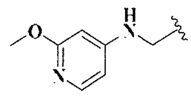;

; preferably methyl;

$R_{dd}$, $R_{ee}$, $R_{ff}$, $R_{gg}$, $R_{jj}$, $R_{pp}$, $R_{hh}$ and $R_{ii}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

n3, n4, n6 and n7 are each independently an integer selected from 0 to 3; and

x is an integer from 0 to 6.

In a further preferred embodiment of the present disclosure, the present disclosure provides a compound represented by formula (IV), a stereoisomer thereof or a pharmaceutically acceptable salt thereof, wherein the specific structure is as follows:

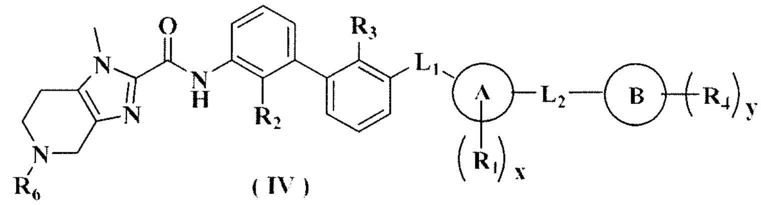

(IV)

wherein:

$L_1$ is selected from a bond or -NHC(O)-;
ring A is selected from

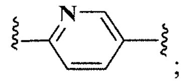
;

ring B is selected from $C_{3-12}$ cycloalkyl, 3- to 12-membered heterocyclyl, $C_{6-14}$ aryl or 5- to 14-membered heteroaryl,
$R_4$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, $-(CH_2)_{n8}R_{kk}$, $-(CH_2)_{n8}OR_{kk}$, $-(CH_2)_{n8}C(O)R_{kk}$ or $-(CH_2)_{n8}C(O)OR_{kk}$, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14- membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
$R_{kk}$ is selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
y is an integer from 0 to 6.

In a preferred embodiment of the present disclosure, ring B is selected from $C_{3-8}$ cycloalkyl, 3- to 10-membered heterocyclyl, $C_{6-10}$ aryl or 5- to 10-membered heteroaryl;
In a preferred embodiment of the present disclosure, ring B is selected from $C_{6-10}$ aryl, 3- to 8-membered nitrogen-containing heterocyclyl or 5- to 10-membered nitrogen-containing heteroaryl;
In a further preferred embodiment of the present disclosure, ring B is selected from the following groups:

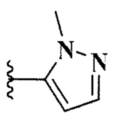 , 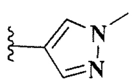 , 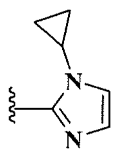 , 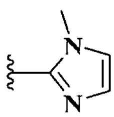 , 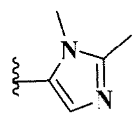 , 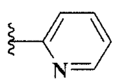 , 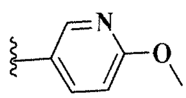 ,

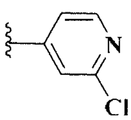 , 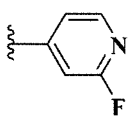 , 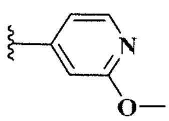 , 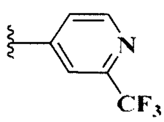 , 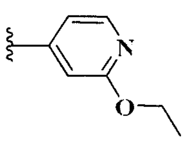 , 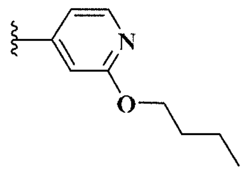 ,

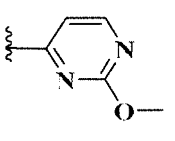 , 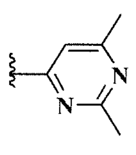 , 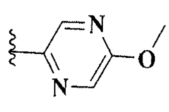 , 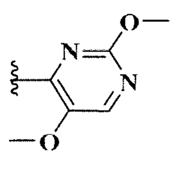 , 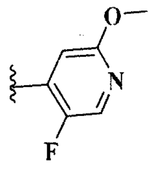 , 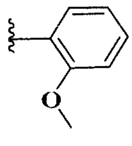 ,

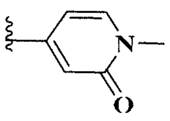, 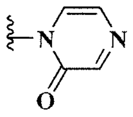, 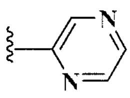, 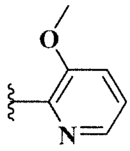, 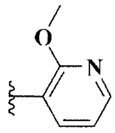, 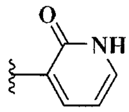, 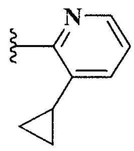,

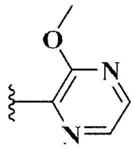 or 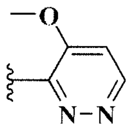.

In a further preferred embodiment of the present disclosure, ring B is selected from $C_{3-8}$ cycloalkyl, 3- to 8-membered heterocyclyl, $C_{6-10}$ aryl or 5- to 10-membered heteroaryl;
preferably $C_{3-8}$ cycloalkyl, 3- to 8-membered nitrogen-containing heterocyclyl, 3- to 8-membered oxygen-containing heterocyclyl, $C_{6-10}$ aryl or 5- to 10-membered nitrogen-containing heteroaryl;
more preferred are the following groups:

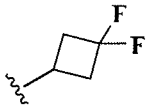, 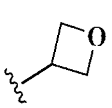, 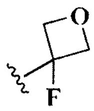, 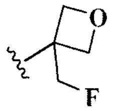, 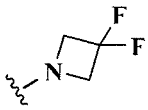, 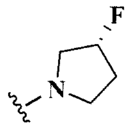, 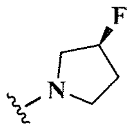,

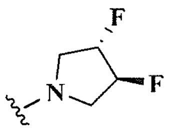, 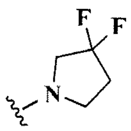, 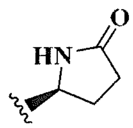, 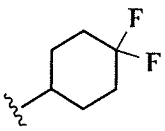, 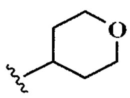, 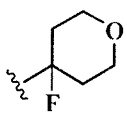, 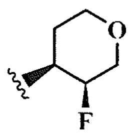,

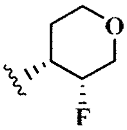, 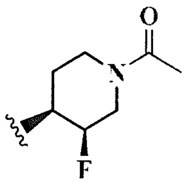, 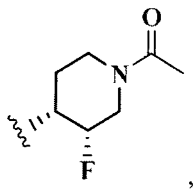, 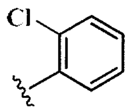, 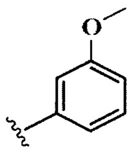, 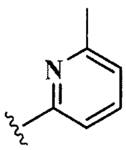, 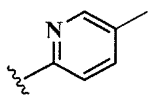,

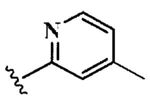, 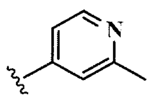, 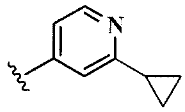, 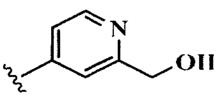, 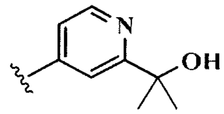,

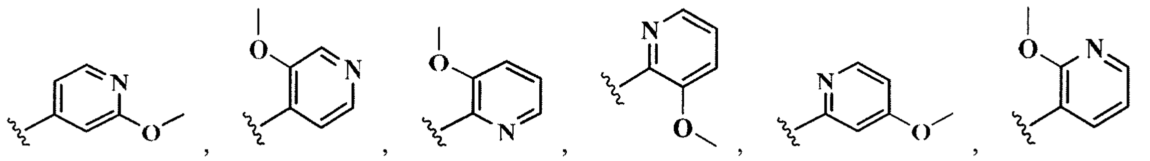, , , , , ,

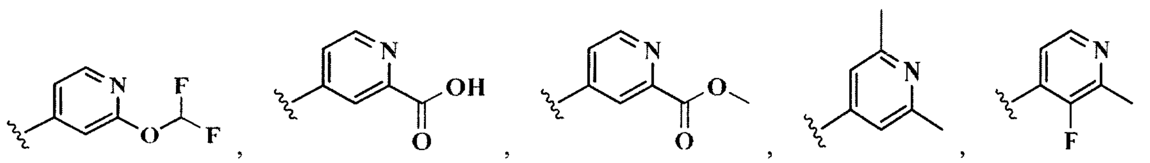, , , , ,

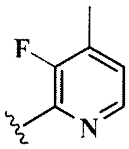, 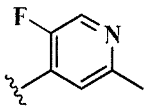, 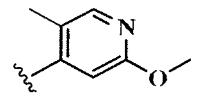, 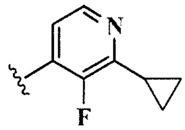, 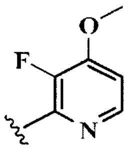, 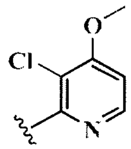,

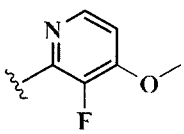, 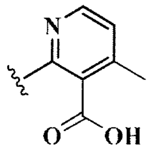, 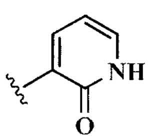, 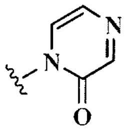, 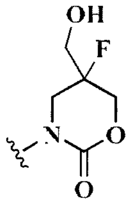, 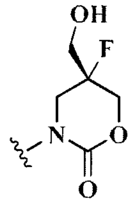, 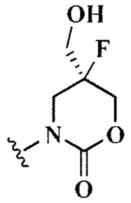 or

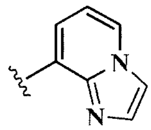.

In a preferred embodiment of the present disclosure, ring B is selected from $C_{3-8}$ cycloalkyl, 3- to 8-membered heterocyclyl, $C_{6-10}$ aryl or 5- to 10-membered heteroaryl;
preferable 3- to 8-membered nitrogen-containing heterocyclyl, 3- to 8-membered oxygen-containing heterocyclyl or 5- to 10-membered nitrogen-containing heterocyclyl;
more preferred are the following groups:

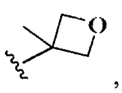, 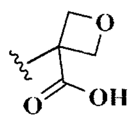, 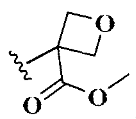, 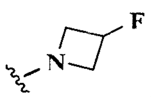, 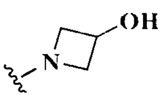, 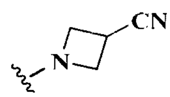, 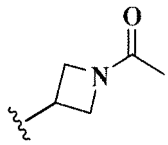,

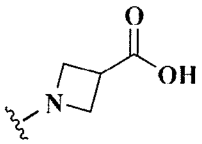, 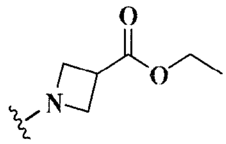, 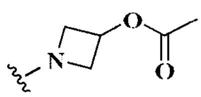, 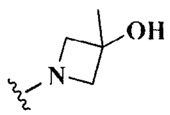, 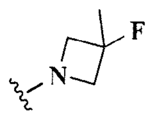,

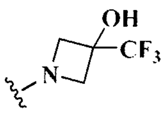, 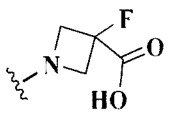, 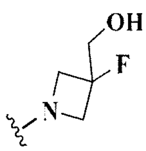, 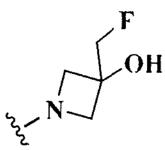, 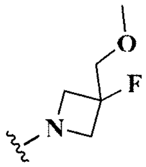, 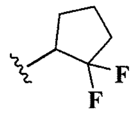,

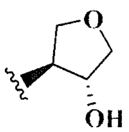, 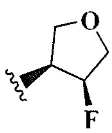, 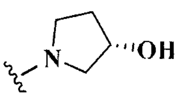, 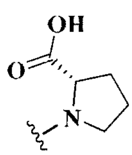, 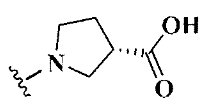, 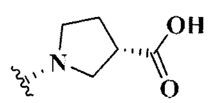,

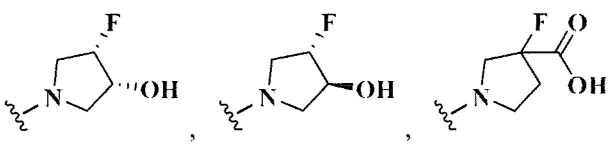 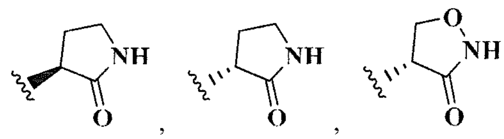,

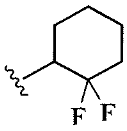, 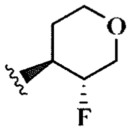, 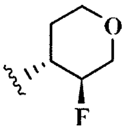, 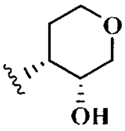, 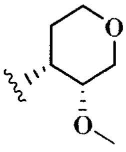, 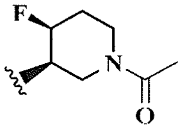, 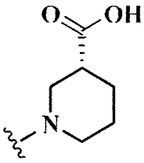,

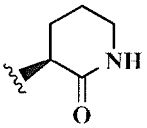, 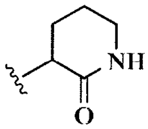, 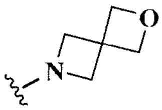, 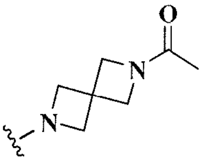, 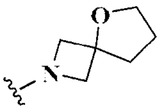, 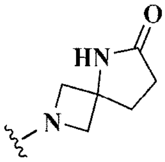,

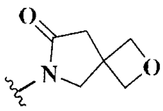, 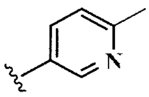, 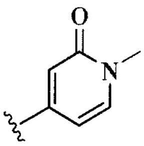, 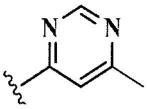, 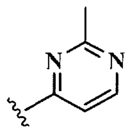 or 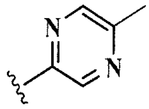.

In a preferred embodiment of the present disclosure, ring B is selected from $C_{3-8}$ cycloalkyl, 3- to 10-membered heterocyclyl, $C_{6-10}$ aryl or 5- to 10-membered heteroaryl;
preferably $C_{3-6}$ cycloalkyl, 3- to 6-membered oxygen-containing heterocyclyl or 3- to 10-membered nitrogen-containing heterocyclyl;
more preferred are the following groups:

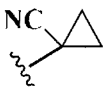, 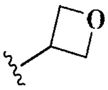, 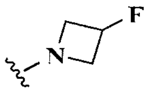, 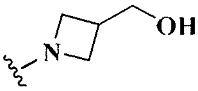, 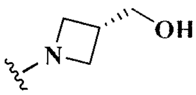, 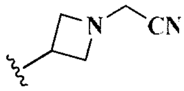,

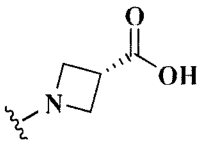, 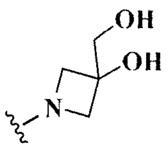, 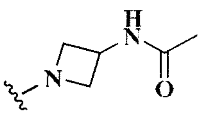, 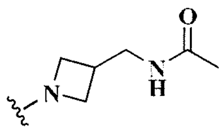, 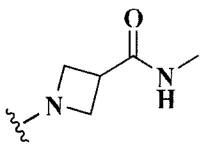,

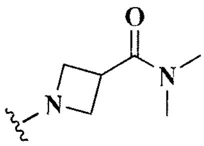, 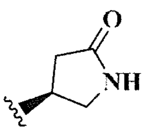, 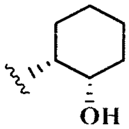, 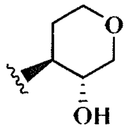, 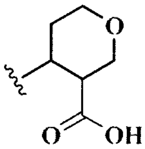, 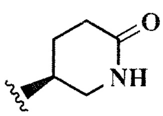,

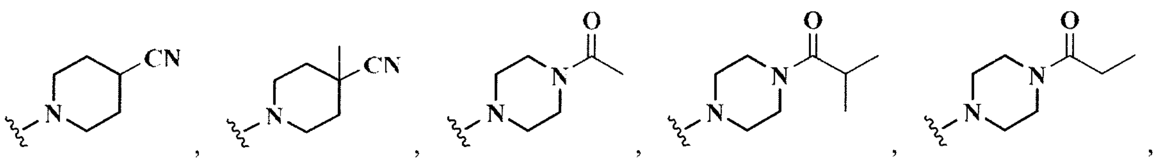, , , , ,

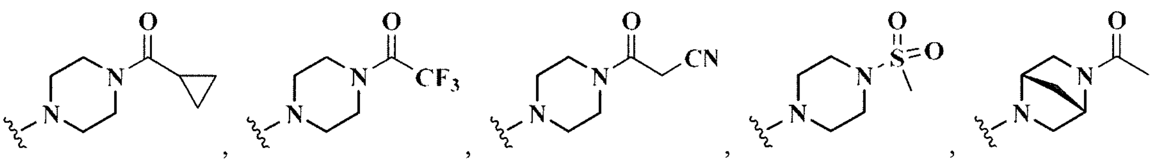, , , , ,

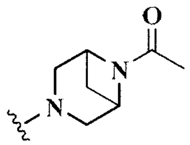, 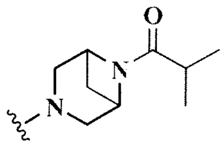, 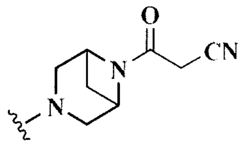, 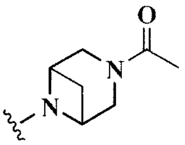, 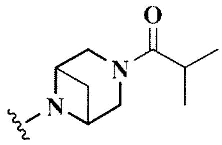,

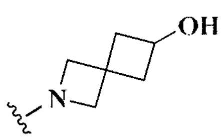, 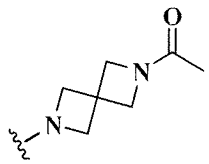, 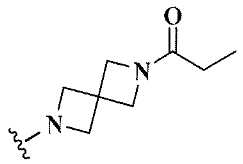, 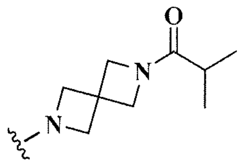,

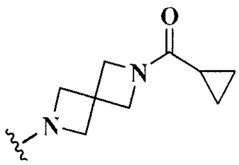, 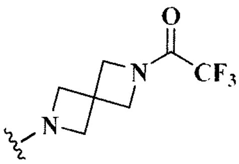, 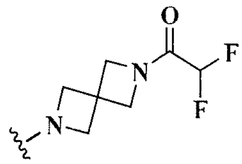, 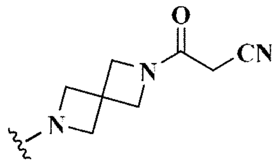,

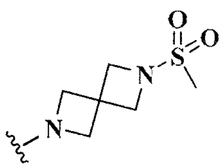, 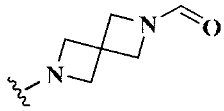, 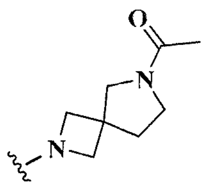, 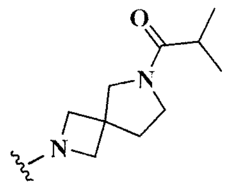,

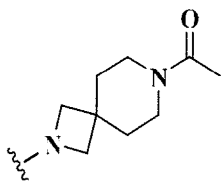 or 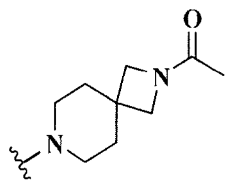.

In a preferred embodiment of the present disclosure, $R_4$ is selected from hydrogen, halogen, amino, hydroxyl, cyano, carboxyl, oxo, $C_{1-3}$ alkyl, $C_{1-3}$ haloalkyl, $C_{1-3}$ hydroxyalkyl, $C_{1-3}$ alkoxy, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $-(CH_2)_{n8}R_{kk}$, $-(CH_2)_{n8}OR_{kk}$, $-(CH_2)_{n8}OC(O)R_{kk}$, $-(CH_2)_{n8}C(O)R_{kk}$, $-(CH_2)_{n8}C(O)OR_{kk}$, $-(CH_2)_{n8}C(O)NR_{kk}R_k$, $-(CH_2)_{n8}NR_{kk}R_k$, $-(CH_2)_{n8}NR_{kk}C(O)R_k$ or $-(CH_2)_{n8}S(O)_{m8}R_{kk}$;

preferably hydrogen, fluorine, chlorine, bromine, methyl, ethyl, isopropyl, methoxy, hydroxyl, cyano, carboxyl, oxo, difluoromethyl, trifluoromethyl, cyclopropyl, $-CH_2F$, $-CH_2OH$, $-CH_2CN$, $-C(CH_3)_2OH$, $-C(O)CH_3$, $-C(O)OCH_3$, $-OCHF_2$, $-C(CH_3)_2OH$, $-CH_2OCH_3$, $-C(O)OCH_2CH_3$, $-OC(O)CH_3$, $-NHC(O)CH_3$, $-CH_2NHC(O)CH_3$, $-C(O)NHCH_3$, $-C(O)N(CH_3)_2$, $-C(O)CH_2CH_3$, $-C(O)CH(CH_3)_2$, $-C(O)CF_3$, $-C(O)CHF_2$, $-C(O)CH_2CN$, -CH(O)-, $S(O)_2CH_3$ or

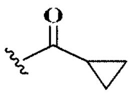;

$R_{kk}$ and $R_k$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium,

halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n8 is an integer from 0 to 3; and
m8 is an integer from 0 to 2.

In a further preferred embodiment of the present disclosure, the present disclosure provides a compound represented by formula (V), a stereoisomer thereof or a pharmaceutically acceptable salt thereof, wherein the specific structure is as follows:

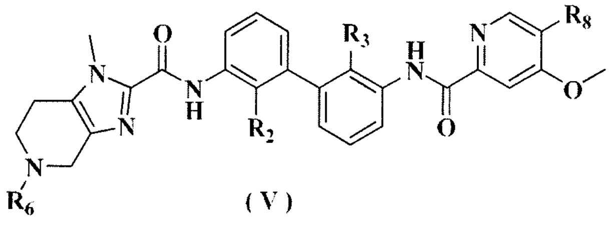

(V)

wherein:

$R_8$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ haloalkoxy, $C_{1-6}$ alkylthio, $C_{1-6}$ hydroxyalkyl, $C_{3-12}$ cycloalkyl, 3- to 12-membered heterocyclyl, $C_{6-14}$ aryl or 5- to 14-membered heteroaryl,$-(CH_2)_{n9}R_{ll}$, $-(CH_2)_{n9}NR_{ll}(CR_{mm}R_{nn})_{n10}R_{oo}$ or $-(CH_2)_{n9}NR_{ll}(CH_2)_{n10}NR_{mm}C(O)R_{nn}$, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
preferably hydrogen, halogen, amino, hydroxyl, $C_{1-6}$ alkyl, $C_{1-6}$ haloalkyl, $C_{1-6}$ alkoxy, $C_{1-6}$ hydroxyalkyl, $C_{2-6}$ alkenyl, $C_{2-6}$ alkynyl, $-(CH_2)_{n9}R_{ll}$, $-(CH_2)_{n9}NR_{ll}(CR_{mm}R_{nn})_{n10}R_{oo}$ or - $(CH_2)_{n9}NR_{ll}(CH_2)_{n10}NR_{mm}C(O)R_{nn}$;
more preferably $-CH_2OH$, $-C(CH_3)_2OH$, $-CH_2NHCH_2CH_2F$, $-CH_2NHCH_2CH_2OH$, - $CH_2NHCH_2CH(OH)CH_3$, $-CH_2NHCH_2CH_2NHC(O)CH_3$, $-CH_2NHCH_2CH=CH_2$ or $-CH_2NHCH_2C\equiv CH$;
$R_{ll}$, $R_{mm}$, $R_{nn}$ and $R_{oo}$ are each independently selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n9 is an integer from 0 to 3; and
n10 is an integer from 0 to 3.

In a further preferred embodiment of the present disclosure, the present disclosure provides a compound represented by formula (VI), a stereoisomer thereof or a pharmaceutically acceptable salt thereof:

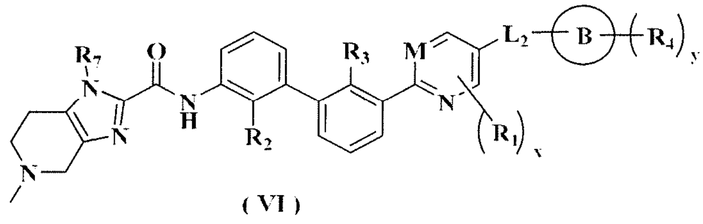

(VI)

wherein:

$R_1$ is selected from hydrogen, halogen, cyano, $C_{1-3}$ alkyl, $C_{1-3}$ haloalkyl or $C_{1-3}$ alkoxy, preferably hydrogen, fluorine, chlorine, cyano, methyl, trifluoromethyl or methoxy;

$R_2$ is selected from hydrogen, halogen or $C_{1-3}$ alkyl, preferably chlorine or methyl;

$R_3$ is selected from hydrogen, halogen or $C_{1-3}$ alkyl; preferably fluorine, chlorine or methyl;

M is -CH- or -N-;

$L_2$ is selected from a bond, $-(CH_2)_{n3}-$ or $-(CH_2)_{n3}NR_{dd}(CR_{ee}R_{ff})_{n4}-$, preferably a bond,

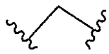,

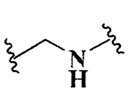, 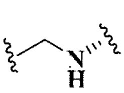, 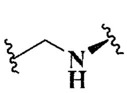, 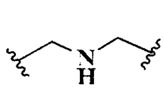, 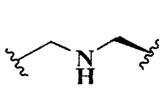, 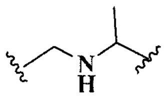 or

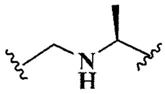;

ring B is selected from $C_{3-8}$ cycloalkyl, 3- to 10- membered heterocyclyl, $C_{6-10}$ aryl or 5- to 10-membered heteroaryl, preferably $C_{3-6}$ cycloalkyl, 3- to 10- membered heterocyclyl containing 1 to 3 heteroatoms selected from N or O, or 5- to 6-membered nitrogen-containing heteroaryl, and more preferably the following groups:

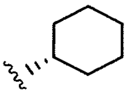, 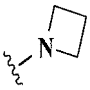, 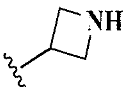, 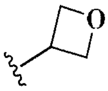, 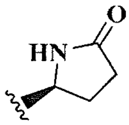, 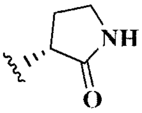, 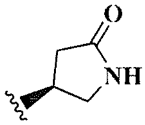, 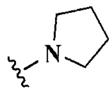,

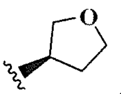, 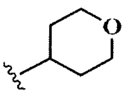, 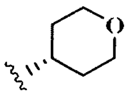, 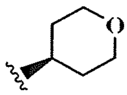, 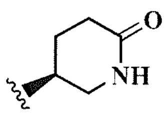, 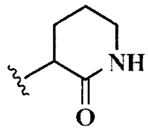, 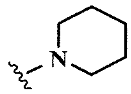,

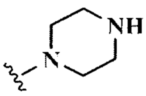, 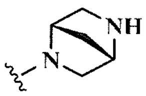, 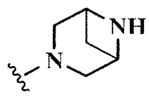, 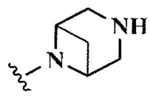, 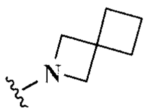, 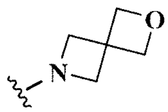,

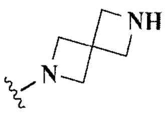, 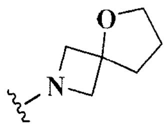, 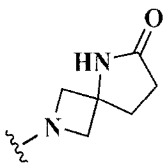, 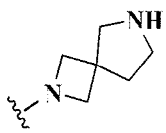, 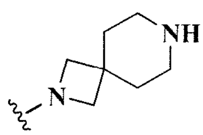,

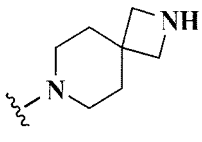 or 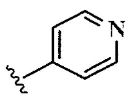;

$R_4$ is selected from hydrogen, deuterium, halogen, hydroxyl, cyano, carboxyl, $C_{1-3}$ alkyl, $C_{1-3}$ deuterated alkyl, $C_{1-3}$

haloalkyl, $C_{1-3}$ alkoxy, $C_{1-3}$ hydroxyalkyl, $-(CH_2)_{n8}R_{kk}$, $-(CH_2)_{n8}OR_{kk}$, $-(CH_2)_{n8}OC(O)R_{kk}$, $-(CH_2)_{n8}C(O)R_{kk}$, $-(CH_2)_{n8}C(O)OR_{kk}$, $-(CH_2)_{n8}C(O)NR_{kk}R_k$, $-(CH_2)_{n8}NR_{kk}C(O)R_k$ or $-(CH_2)_{n8}S(O)_{m8}R_{kk}$, preferably hydrogen, fluorine, chlorine, bromine, methyl, ethyl, propyl, isopropyl, hydroxyl, cyano, carboxyl, oxo, trifluoromethyl, hydroxymethyl, methoxy, $-CH_2F$, $-CH_2OH$, $-CH_2CN$, $-OC(O)CH_3$, $-C(O)OCH_3$, $-C(O)OCH_2CH_3$, $-NHC(O)CH_3$, $-CH_2NHC(O)CH_3$, $-C(O)N(CH_3)_2$, $-C(O)NHCH_3$, $-C(O)CH_3$, $-C(O)CH_2CH_3$, $-C(O)CH(CH_3)_2$, $-C(O)CF_3$, $-C(O)CHF_2$, $-C(O)CH_2CN$, $-CH(O)$, $-S(O)_2CH_3$ or

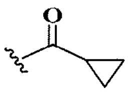;

$R_{dd}$, $R_{ee}$, $R_{ff}$, $R_{kk}$ and $R_k$ are each independently selected from hydrogen, deuterium, halogen, amino, hydroxyl, mercapto, cyano, nitro, carboxyl, $C_{1-3}$ alkyl, $C_{2-4}$ alkenyl, $C_{2-4}$ alkynyl, $C_{1-3}$ deuterated alkyl, $C_{1-3}$ haloalkyl, $C_{1-3}$ alkoxy, $C_{1-3}$ alkylthio, $C_{1-3}$ haloalkoxy, $C_{1-3}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

$R_7$ is selected from hydrogen, $C_{1-3}$ alkyl or $C_{3-6}$ cycloalkyl; preferably methyl, ethyl or cyclopropyl;

n3, n4, n8 and m8 are each independently selected from 0, 1 or 2;

x is 1 or 2; and

y is 0, 1, 2 or 3.

In a further preferred embodiment of the present disclosure, the present disclosure provides a method for preparing the compound represented by general formula (IV-A), the stereoisomer thereof or the pharmaceutically acceptable salt thereof, comprising the following steps:

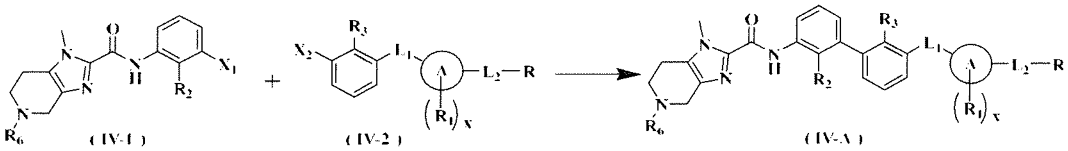
(IV-1) (IV-2) (IV-A)

reacting the compound represented by general formula (IV-1) with the compound represented by general formula (IV-2) to obtain the target compound represented by general formula (IV-A);

wherein:

$X_1$ is halogen, boronic acid or borate ester; preferably halogen; more preferably bromine;

$X_2$ is halogen, boronic acid or borate ester; preferably borate ester; more preferably

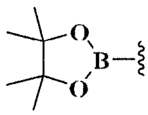.

In a further preferred embodiment of the present disclosure, the present disclosure provides a method for preparing the compound represented by general formula (IV), the stereoisomer thereof or the pharmaceutically acceptable salt thereof, comprising the following steps:

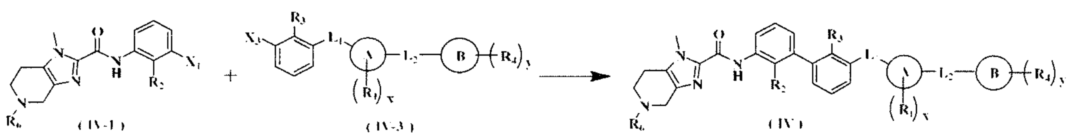
(IV-1) (IV-3) (IV)

reacting the compound represented by general formula (IV-1) with the compound represented by general formula (IV-3) to obtain the target compound represented by general formula (IV);
wherein:

$X_1$ is halogen, boronic acid or borate ester; preferably halogen; more preferably bromine;

$X_3$ is halogen, boronic acid or borate ester; preferably borate ester; more preferably

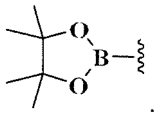

In a further preferred embodiment of the present disclosure, the present disclosure provides a method for preparing the compound represented by general formula (VI), the stereoisomer thereof or the pharmaceutically acceptable salt thereof, comprising the following steps:

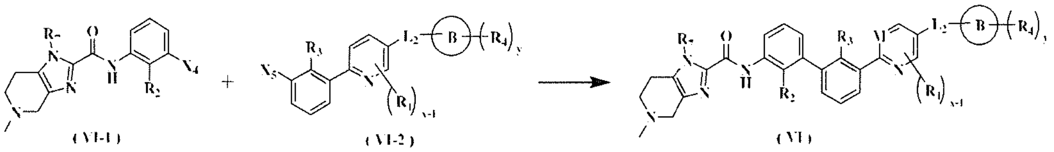

reacting the compound represented by general formula (VI-1) with the compound represented by general formula (VI-2) to obtain the target compound represented by general formula (VI);
wherein:

$X_4$ is halogen, boronic acid or borate ester; preferably halogen; more preferably bromine;
$X_5$ is halogen, boronic acid or borate ester; preferably borate ester; more preferably

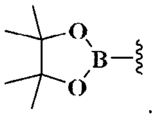

The present disclosure further relates to a pharmaceutical composition comprising a therapeutically effective amount of the compound represented by general formula (I), and the stereoisomer thereof or the pharmaceutically acceptable salt thereof, and one or more pharmaceutically acceptable carriers or excipients.

The present disclosure further relates to a use of the compound represented by any of the general formula, the stereoisomer thereof or the pharmaceutically acceptable salt thereof, or the pharmaceutical composition thereof in the manufacture of a PD-1 or PD-L1 inhibitor medicament.

The present disclosure further relates to a use of the compound represented by the general formula, the stereoisomer thereof or the pharmaceutically acceptable salt thereof, or the pharmaceutical composition thereof in the manufacture of a medicament for the treatment of cancer, infectious diseases and autoimmune diseases, wherein the cancer is selected from skin cancer, lung cancer, urological tumor, hematological tumor, breast cancer, glioma, digestive system tumor, reproductive system tumor, lymphoma, neurological tumor, brain tumor, head and neck cancer; the infectious disease is selected from bacterial infection or viral infection; the autoimmune disease is selected from organ-specific autoimmune disease, systemic autoimmune disease, wherein, the organ-specific autoimmune disease comprises chronic lymphocytic thyroiditis , hyperthyroidism, insulin-dependent diabetes mellitus, myasthenia gravis, ulcerative colitis, pernicious anemia with chronic atrophic gastritis, pulmonary hemorrhagic nephritic syndrome, primary biliary cirrhosis, multiple cerebral sclerosis, acute idiopathic polyneuritis, and the systemic autoimmune diseases comprises rheumatoid arthritis, systemic lupus erythematosus, systemic vasculitis, scleroderma, pemphigus, dermatomyositis, mixed connective tissue disease, autoimmune hemolytic anemia.

The present disclosure further relates to the compound represented by the general formula, the stereoisomer thereof or the pharmaceutically acceptable salt thereof, or the pharmaceutical composition thereof in the manufacture of a method for treating cancer, infectious diseases and autoimmune diseases.

The present disclosure also relates to a method for the treatment, prevention and/or pre-preparation of treatment for cancer, infectious diseases and autoimmune diseases, comprising administering a therapeutically effective dose of

the compound represented by the general formula, the stereoisomer thereof or the pharmaceutically acceptable salt thereof, or the pharmaceutical composition thereof to a patient.

The present disclosure also provides a method of treating disease conditions including, but not limited to, conditions associated with PD-1 or PD-L1 using the compounds or pharmaceutical compositions of the present disclosure.

The present disclosure also relates to a method of treating cancer, infectious disease, autoimmune disease in a mammal comprising administering a therapeutically effective amount of the compound or the pharmaceutically acceptable salt, ester, pro-drug, solvate, hydrate or derivative of the present disclosure to the mammal.

In some embodiments, the methods relate to the treatment of disorders such as cancer, infectious diseases, autoimmune diseases, and the like.

In some embodiments, the cancer involved in the method is selected from skin cancer, lung cancer, urological tumor, hematological tumor, breast cancer, glioma, digestive system tumor, reproductive system tumor, lymphoma, neurological system tumor, brain tumor, head and neck cancer.

In some embodiments, the infectious disease involved in the method is selected from bacterial infection, viral infection.

In some embodiments, the autoimmune disease involved in the method is selected from organ-specific autoimmune disease, systemic autoimmune disease, wherein, the organ-specific autoimmune disease includes chronic lymphocytic thyroiditis , hyperthyroidism, insulin-dependent diabetes mellitus, myasthenia gravis, ulcerative colitis, pernicious anemia with chronic atrophic gastritis, pulmonary hemorrhagic nephritic syndrome, primary biliary cirrhosis, multiple cerebral sclerosis, acute idiopathic polyneuritis, and the systemic autoimmune diseases including rheumatoid arthritis, systemic lupus erythematosus, systemic vasculitis, scleroderma, pemphigus, dermatomyositis, mixed connective tissue disease, autoimmune hemolytic anemia.

## Detailed description of the present disclosure

Unless the contrary is stated, the terms used in the description and claims have the following meanings.

The term "alkyl" refers to a saturated aliphatic hydrocarbon group, which is a straight or branched chain group containing 1 to 20 carbon atoms, preferably alkyl containing 1 to 8 carbon atoms, more preferably alkyl containing 1 to 6 carbon atoms, most preferably alkyl contianing 1 to 3 carbon atoms. Non-limiting examples include methyl, ethyl, *n*-propyl, isopropyl, *n*-butyl, isobutyl, tert-butyl, *sec*-butyl, n-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2- methylpropyl, 1,1,2-trimethylpropyl, 1,1-dimetbylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-metliylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers. More preferrably lower alkyl containing 1 to 6 carbon atoms, non-limiting examples include methyl, ethyl, *n*-propyl, isopropyl, *n*-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl may be substituted or unsubstituted, when substituted, the substituents may be substituted at any available attachment point, and the substituents are preferably one or more of the following groups, which are independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate, preferably alkyl substituted by methyl, ethyl, isopropyl, tert-butyl, haloalkyl, deuterated alkyl, alkyl substituted by alkoxy, and alkyl substituted by hydroxyl.

The term "alkylidene" refers to that one hydrogen atom of an alkyl is further substituted, for example: "methylene" refers to $-CH_2-$, "ethylene" refers to $-(CH_2)_2-$, and "propylene" refers to $-(CH_2)_3-$, "butylene" refers to $-(CH_2)_4-$, etc. The term "alkenyl" refers to an alkyl as defined above containing at least two carbon atoms and at least one carbon-carbon double bond, such as vinyl, 1 -propenyl, 2-propenyl, 1-, 2-, or 3-butenyl etc. The alkenyl may be substituted or unsubstituted, when substituted, the substituents are preferably one or more of the following groups, which are independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio.

The term "cycloalkyl" refers to a saturated or partially unsaturated monocyclic or polycyclic hydrocarbon substituent, and the cycloalkyl ring contains 3 to 20 carbon atoms, preferably 3 to 10 carbon atoms, more preferably 3 to 8 carbon atoms, further preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctanyl, etc., polycyclic cycloalkyl includes spiro, fused and bridged cycloalkyl, preferably cyclopropyl, cyclobutyl,

cyclopentyl, cyclohexyl and cycloheptyl.

The term "spirocycloalkyl" refers to polycyclyl that shares one carbon atom (called a spiro atom) between 5- to 20-membered monocyclic rings, which may contain one or more double bonds, but none of the rings have complete conjugate π electron system. Preferably 6- to 14-membered, more preferably 7- to 10-membered. According to the number of shared spiro atoms between the rings, the spirocycloalkyl is classified into single spirocycloalkyl, bispirocycloalkyl or polyspirocycloalkyl, preferably single spirocycloalkyl and bispirocycloalkyl. More preferably, 4-membered/4-membered, 4-membered/5-membered, 4-membered/6-membered, 5-membered/5-membered, or 5-membered/6-membered monospirocycloalkyl. Non-limiting examples of spirocycloalkyl include:

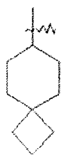 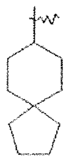 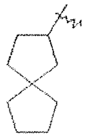 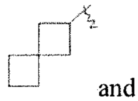 and 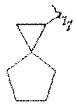 ;

spirocycloalkyl also contains single spirocycloalkyl and heterocycloalkyl sharing a spiro atom, non-limiting examples of which include:

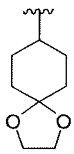 and 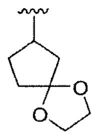 .

The term "fused cycloalkyl" refers to a 5- to 20-membered all-carbon polycyclic group in which each ring in the system shares an adjacent pair of carbon atoms with other rings in the system, wherein one or more of the rings may comprise one or multiple double bonds, but none of the rings have a fully conjugated π-electron system. Preferably 6- to 14-membered, more preferably 7- to 10-membered. According to the number of constituent rings, it can be classified into bicyclic, tricyclic, tetracyclic or polycyclic fused cycloalkyl, preferably bicyclic or tricyclic, and more preferably 5-membered/5-membered or 5-membered/6-membered bicyclic cycloalkyl. Non-limiting examples of fused cycloalkyl include:

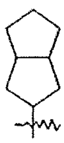 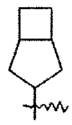 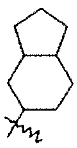 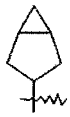 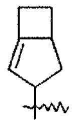 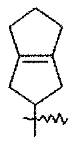 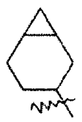 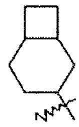 and 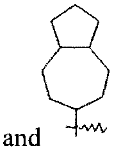 .

The term "bridged cycloalkyl" refers to 5- to 20-membered all-carbon polycyclic group, in which any two rings share two carbon atoms that are not directly connected, it may contain one or more double bonds, but no ring has a complete conjugated π electron system. Preferably 6- to 14-membered, more preferably 7- to 10-membered. According to the number of constituent rings, it can be classified into bicyclic, tricyclic, tetracyclic or polycyclic bridged cycloalkyl, preferably bicyclic, tricyclic, or tetracyclic, and more preferably bicyclic or tricyclic. Non-limiting examples of bridge cycloalkyl include:

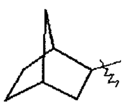 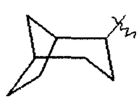 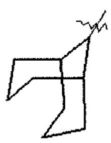 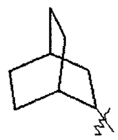 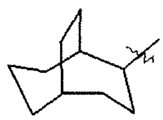

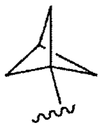 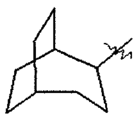 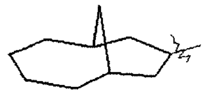 and 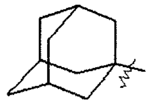

The cycloalkyl ring may be fused to an aryl, heteroaryl or heterocycloalkyl ring, wherein the ring connected to the parent structure is cycloalkyl, non-limiting examples include indanyl, tetrahydronaphthyl, benzocycloheptanyl, etc. The cycloalkyl may be substituted or unsubstituted, when substituted, the substituents are preferably one or more of the following groups, which are independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboylate.

The term "heterocyclyl" refers to saturated or partially unsaturated monocyclic or polycyclic hydrocarbon substituent containing 3 to 20 ring atoms, wherein one or more of the ring atoms are heteroatoms selected from nitrogen, oxygen or $S(O)_m$ (wherein m is an integer of 0 to 2), but not including the ring part of -O-O-, -O-S- or -S-S-, and the remaining ring atoms are carbon. It preferably contains 3 to 12 ring atoms, wherein 1 to 4 ring atoms are heteroatoms; more preferably contains 3 to 10 ring atoms; most preferably contains 3 to 8 ring atoms. Non-limiting examples of monocyclic heterocyclyl include oxazinonyl, pyrazinonyl, pyridonyl, pyrrolidinyl, tetrahydropyrrolyl, tetrahydropyrrolidonyl, azetidinyl, oxetanyl, oxanyl, imidazolidinyl, tetrahydrofuranyl, tetrahydrothienyl, dihydroimidazolyl, dihydrofuranyl, dihydropyrazolyl, dihydropyrrolyl, piperidinyl, piperidinonyl, piperazinyl, morpholinyl, thiomorpholinyl, homopiperazinyl, tetrahydropyranyl, pyranyl, etc.; preferably oxazinonyl, pyrazinonyl, pyridonyl, pyrrolidinyl, tetrahydropyrrolyl, tetrahydropyrrolidonyl, azetidinyl, oxetanyl, tetrahydrofuranyl, pyrazolidinyl, morpholinyl, piperazinyl, piperidinyl, piperidinonyl, tetrahydropyranyl and pyranyl; more preferably oxazinonyl, tetrahydrofuranyl, isoxazolidinonyl, tetrahydropyrrolyl, tetrahydropyrrolonyl, azetidinyl, oxetanyl, piperidinyl, piperidinonyl and tetrahydropyranyl. Polycyclic heterocyclyl includes spiro, fused and bridged heterocyclyl; the spiro, fused and bridged heterocyclyl are optionally connected to other groups through a single bond, or fused-connected to other cycloalkyl, heterocyclyl, aryl and heteroaryl through any two or more of ring atoms.

The term "spiroheterocyclyl" refers to polycyclic heterocyclyl sharing one atom (called a spiro atom) between 5- to 20-membered monocyclic ring, wherein one or more ring atoms are selected from nitrogen, oxygen or $S(O)_m$ (wherein m is an integer of 0 to 2) heteroatoms, and the remaining ring atoms are carbon. It may contain one or more double bonds, but none of the rings have complete conjugate π electron system. Preferably 6- to 14-membered, more preferably 7- to 10-membered. According to the number of spiro atoms shared between the rings, the spiroheterocyclyl is classified into single spiroheterocyclyl, dispiroheterocyclyl or polyspiroheterocyclyl, preferably single spiroheterocyclyl and dispiroheterocyclyl. More preferably, 4-membered/4-membered, 4-membered/5-membered, 4-membered/6-membered, 5-membered/5-membered, or 5-membered/6-membered monospiroheterocyclyl. Non-limiting examples of spiroheterocyclyl include:

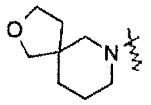 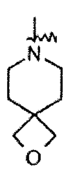 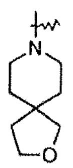 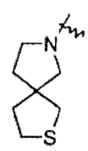 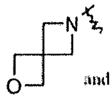 and 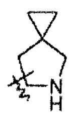 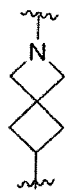

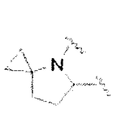 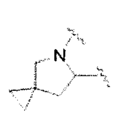 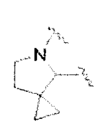 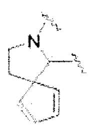 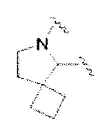 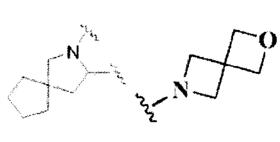

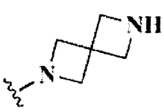 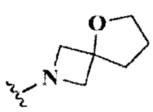 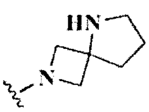 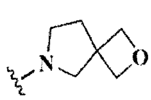

The term "fused heterocyclyl" refers to a 5- to 20-membered polycyclic heterocyclyl in which each ring in the system shares an adjacent pair of atoms with other rings in the system, one or more of the rings may comprise one or multiple double bonds, but none of the rings have a fully conjugated π-electron system, wherein one or more of the ring

atoms are heteroatoms selected from nitrogen, oxygen or $S(O)_m$ (wherein m is an integer of 0 to 2), the rest of the ring atoms are carbon. Preferably 6- to 14-membered, more preferably 7- to 10-membered. According to the number of constituent rings, it can be classified into bicyclic, tricyclic, tetracyclic or polycyclic fused heterocyclyl, preferably bicyclic or tricyclic, and more preferably 5-membered/5-membered or 5-membered/6-membered bicyclic fused heterocylyl. Non-limiting examples of fused heterocyclyl include:

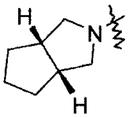 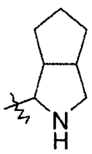 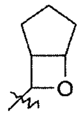 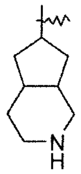 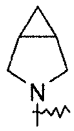 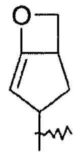 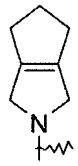 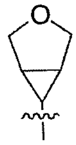 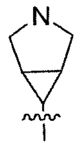

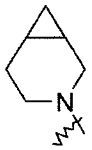 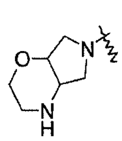 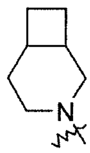 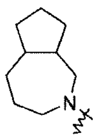 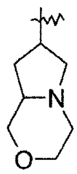 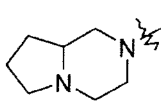 and 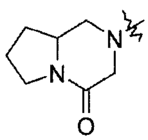 .

The term "bridged heterocyclyl" refers to a polycyclic heterocyclyl in which any two rings share two atoms that are not directly connected, it may contain one or multiple double bonds, but none of the rings have a fully conjugated $\pi$-electron system, wherein one or more of the ring atoms are heteroatoms selected from nitrogen, oxygen or $S(O)_m$ (wherein m is an integer of 0 to 2), the rest of the ring atoms are carbon. Preferably 6- to 14-membered, more preferably 7- to 10-membered. According to the number of constituent rings, it can be classified into bicyclic, tricyclic, tetracyclic or polycyclic bridged heterocyclyl, preferably bicyclic, tricyclic, or tetracyclic, and more preferably bicyclic or tricyclic. Non-limiting examples of bridged heterocyclyl include:

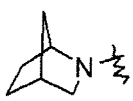 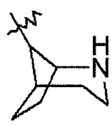 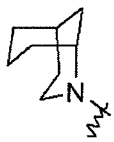 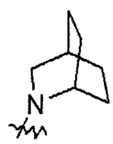

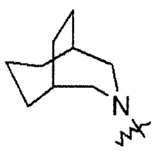 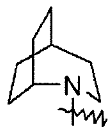 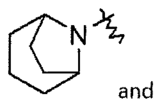 and 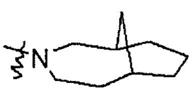 .

The heterocyclyl ring may be fused to an aryl, heteroaryl or cycloalkyl ring, wherein the ring connected to the parent structure is heterocyclyl, non-limiting examples of which include:

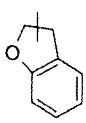 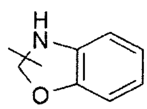 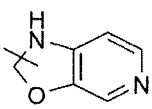 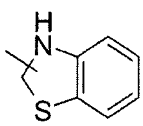

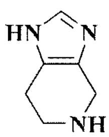 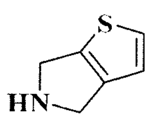 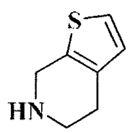 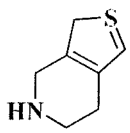 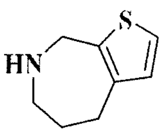 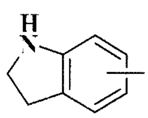 .

The heterocyclyl may be substituted or unsubstituted, when substituted, the substituents are preferably one or more of the following groups, which are independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy,

cycloalkylthio, heterocycloalkylthio, oxo, carboxyl or carboylate.

The term "aryl" refers to a 6- to 14-membered all-carbon monocyclic or fused polycyclic (that is, rings sharing adjacent pairs of carbon atoms) with conjugated $\pi$-electron system, preferably 6- to 10-membered, more preferably 6- to 8-membered, such as phenyl and naphthyl. More preferably phenyl. The aryl ring may be fused to heteroaryl, heterocyclyl or cycloalkyl ring, wherein the ring connected to the parent structure is aryl ring, non-limiting examples of which include:

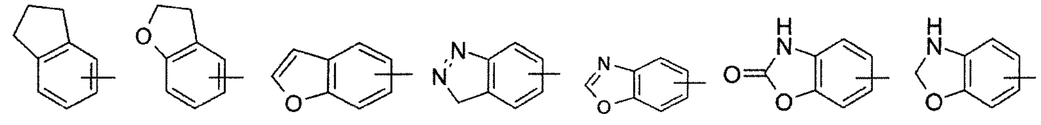

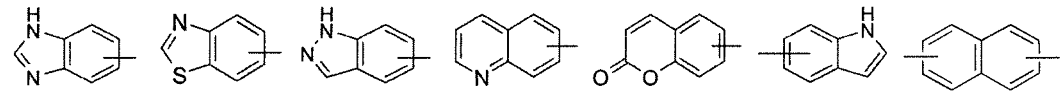

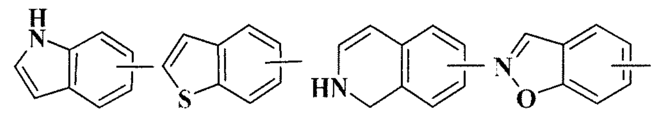

.

The aryl may be substituted or unsubstituted, when substituted, the substituents are preferably one or more of the following groups, which are independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

The term "heteroaryl" refers to heteroaromatic system containing 1 to 4 heteroatoms and 5 to 14 ring atoms, wherein the heteroatoms are selected from oxygen, sulfur, and nitrogen. The heteroaryl is preferably 5- to 10-membered, more preferably 5- or 8-membered, most preferably 5- or 6-membered, such as imidazolyl, furanyl, thiophenyl, thiazolyl, pyrazolyl, oxazolyl, pyrrolyl, triazolyl, tetrazolyl, pyridyl, pyrimidinyl, thiadiazolyl, pyridazinyl and pyrazinyl; preferably triazolyl, thiophenyl, thiazolyl, pyridinyl, imidazolyl, pyrazolyl, pyridazinyl, pyrazinyl or pyrimidinyl; more preferably, pyridinyl, imidazolyl, pyrazolyl, pyridazinyl, pyrazinyl or pyrimidinyl. The heteroaryl ring may be fused to an aryl, heterocyclyl or cycloalkyl ring, wherein the ring connected to the parent structure is heteroaryl ring, non-limiting examples of which include:

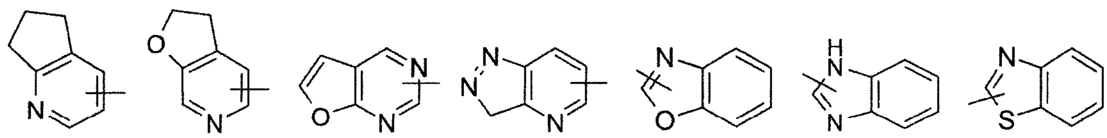

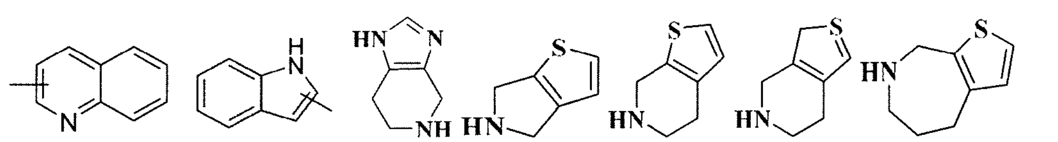

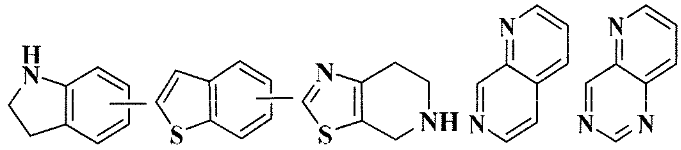

.

The heteroaryl may be optionally substituted or unsubstituted, when substituted, the substituents are preferably one or more of the following groups, which are independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

The term "fused cyclyl" refers to a polycyclyl formed by two or more carbocyclic rings or hetero rings sharing

a ring edge, and the fused cyclyl includes a fused cycloalkyl, a fused heteroaryl, a fused aryl and a fused heteroaryl, wherein the fused cycloalkyl is a polycyclyl formed by a cycloalkyl with a rings, an aryl or a heteroaryl sharing a ring edge; the fused heterocyclyl refers to a polycyclyl formed by a heterocyclyl with a cycloalkyl, an aryl or a heteroaryl sharing a ring edge; the fused aryl refers to a polycyclyl formed by an aryl with a cycloalkyl, a heterocyclyl or a heteroaryl sharing a ring edge; the fused heteroaryl refers to a polycyclyl formed by a heteroaryl with a cycloalkyl, a heterocyclyl or a hetero group sharing a ring edge; for example:

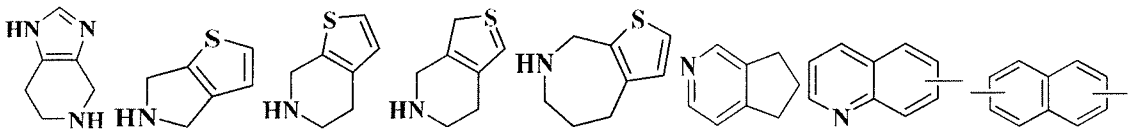

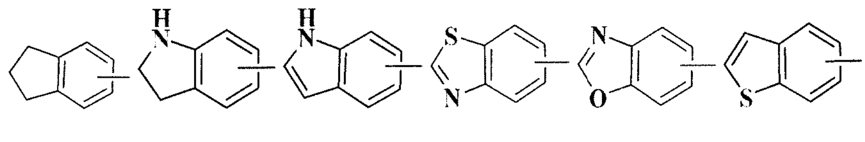

.

The term "alkoxy" refers to -O-(alkyl) and -O-(unsubstituted cycloalkyl), wherein the definition of alkyl is as described above, preferably alkyl containing 1 to 8 carbon atoms, more preferably alkyl containing 1 to 6 carbon atoms, most preferably alkyl contianing 1 to 3 carbon atoms. Non-limiting examples of alkoxy include: methoxy, ethoxy, propoxy, butoxy, cyclopropoxy, cyclobutoxy, cyclopentyloxy, cyclohexyloxy. The alkoxy may be optionally substituted or unsubstituted, when substituted, the substituents are preferably one or more of the following groups, which are independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

The term "alkylthio" refers to -S-(alkyl) and -S-(unsubstituted cycloalkyl), wherein alkyl is as defined above. Non-limiting examples of alkylthio include: methylthio, ethylthio, propylthio, butylthio, cyclopropylthio, cyclobutylthio, cyclopentylthio, cyclohexylthio. The alkylthio may be optionally substituted or unsubstituted, when substituted, the substituents are preferably one or more of the following groups, which are independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboylate.

"Haloalkyl" refers to alkyl substituted by one or more halogens, wherein the alkyl is as defined above.

"Haloalkoxy" refers to alkoxy substituted by one or more halogens, wherein the alkoxy is as defined above.

"Hydroxyalkyl" refers to alkyl substituted by one or more hydroxyl, wherein the alkyl is as defined above.

"Alkenyl" refers to chain alkenyl, also known as olefinic group, preferably alkyl containing 2 to 8 carbon atoms, more preferably alkyl containing 2 to 6 carbon atoms, most preferably alkyl containing 2 to 3 carbon atoms. Wherein the alkenyl may be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

"Alknyl" refers to (CH=C-), preferably alkyl containing 2 to 8 carbon atoms, more preferably alkyl containing 2 to 6 carbon atoms, most preferably alkyl containing 2 to 3 carbon atoms. Wherein the alkynyl may be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

"Haloalkyl" refers to alkyl substituted by one or more halogens, wherein the alkyl is as defined above.

"Haloalkoxy" refers to alkoxy substituted by one or more halogens, wherein the alkoxy is as defined above.

"Hydroxyalkyl" refers to alkyl substituted by one or more hydroxyl, wherein the alkyl is as defined above.

"Hydroxy" refers to the -OH group.

"Halogen" refers to fluorine, chlorine, bromine or iodine.

"Amino" refers to $-NH_2$.

"Cyano" refers to -CN.

"Nitro" refers to $-NO_2$.

"Carboxyl" refers to -C(O)OH.

"THF" refers to tetrahydrofuran.

"EtOAc" refers to ethyl acetate.

"MeOH" refers to methanol.

"DMF" refers to *N,N*-dimethylformamide.

"DIPEA" refers to diisopropylethylamine.

"TFA" refers to trifluoroacetic acid.

"MeCN" refers to acetonitrile.

"DMA" refers to *N,N*-dimethylacetamide.

"$Et_2O$" refers to diethyl ether.

"DCE" refers to 1,2-dichloroethane.

"DIPEA" refers to *N,N*-diisopropylethylamine.

"NBS" refers to *N*-bromosuccinimide.

"NIS" refers to *N*-iodosuccinimide.

"Cbz-Cl" refers to benzyl chloroformate.

$Pd_2(dba)_3$" refers to tris(dibenzylideneacetone)dipalladium.

"Dppf'refers to 1,1'-bis(diphenylphosphino)ferrocene.

"HATU" refers to 2-(7-aza-1*H*-benzotriazol-1-yl)-*N,N,N',N'*-tetramethyluronium hexafluorophosphate.

"KHMDS" refers to potassium hexamethyldisilazide.

"LiHMDS" refers to lithium bistrimethylsilylamide.

"MeLi" refers to methyl lithium.

"n-BuLi" refers to *n*-butyl lithium.

"$NaBH(OAc)_3$" refers to sodium triacetoxyborohydride.

"*t*-BuONO" refers to *tert*-butyl nitrite.

"EA" refers to ethyl acetate.

"PE" refers to petroleum ether.

"DCM" refers to dichloromethane.

"STAB" refers to sodium triacetoxyborohydride.

"$Pd(dcypf)Cl_2$" refers to dichloro[1,1'-bis(dicyclohexylphosphine)ferrocene]palladium.

"X is selected from A, B, or C", "X is selected from A, B and C", "X is A, B or C", "X is A, B and C" and other terms all express the same meaning, which means that X can be any one or more of A, B, and C.

The hydrogen described in the present disclosure can be replaced by its isotope deuterium, and any hydrogen in the embodiment compounds of the present disclosure can also be replaced by a deuterium atom.

"Optional" or "optionally" refers to that the event or environment described later can but does not have to occur, and the description includes occasions where the event or environment occurs or does not occur. For example, "heterocyclyl optionally substituted by alkyl" refers to that alkyl may but does not have to be present, and the description includes the case where the heterocyclyl is substituted by alkyl and the case where the heterocyclyl is not substituted by alkyl.

"Substituted" refers to one or more hydrogen atoms in the group, preferably up to 5, more preferably 1 to 3 hydrogen atoms, independently substituted by a corresponding number of substituents. It goes without saying that the substituents are only in their possible chemical positions, and those skilled in the art can determine (by experiment or theory) possible or impossible substitutions without too much effort. For example, amino or hydroxyl having free hydrogen may be unstable when combined with a carbon atom having an unsaturated (e.g., olefinic) bond.

"Pharmaceutical composition" refers to a mixture containing one or more of the compounds described herein or the physiologically/pharmaceutically acceptable salt or prodrug thereof and other chemical components, and the other component is, for example, physiologically/pharmaceutically acceptable carrier and excipient. The purpose of the pharmaceutical composition is to promote the administration to the organism, facilitate the absorption of the active ingredient and then exert the biological activity.

"Pharmaceutically acceptable salt" refers to the salt of the compound of the present disclosure, which is safe and effective when used in mammals, and has due biological activity.

Detailed description of the preferred embodiment

The present disclosure is further described in conjunction with the embodiments, but these embodiments do not limit the scope of the present disclosure.

## Embodiment

The structures of the compounds in the present disclosure were determined by nuclear magnetic resonance (NMR) or/and liquid chromatography-mass spectrometry (LC-MS). NMR chemical shifts ($\delta$) were given in parts per million (ppm). The NMR was measured with a Bruker AVANCE-400 nuclear magnetic instrument, and the measuring solvent was deuterated dimethyl sulfoxide (DMSO-$d_6$), deuterated methanol ($CD_3OD$) and deuterated chloroform ($CDCl_3$), and the internal standard was tetramethylsilane (TMS).

liquid chromatography-mass spectrometry LC-MS was measured with an Agilent 1200 Infinity Series mass spectrometer. HPLC was measured with an Agilent 1200DAD high pressure liquid chromatograph (Sunfire C18 150 × 4.6 mm chromatographic column) and a Waters 2695-2996 high pressure liquid chromatograph (Gimini C18 150 × 4.6 mm chromatographic column).

Yantai Huanghai HSGF254 or Qingdao GF254 silica gel plate was used as the thin layer chromatography silica gel plate, and the size used for TLC was 0.15 mm to 0.20 mm, and the size used for separation and purification of products by thin layer chromatography was 0.4 mm to 0.5 mm. Yantai Huanghai silica gel 200-300 mesh silica gel was usually used in column chromatography as the carrier.

The starting materials in the embodiments of the present disclosure were known and commercially available, or could be synthesized using or according to methods known in the art.

Unless otherwise specified, all the reactions of the present disclosure were carried out under continuous magnetic stirring, in a dry nitrogen or argon atmosphere, the solvent was dry solvent, and the unit of reaction temperature was Celsius.

Embodiment 1

*N*-(2-Chloro-3'-(4-methoxy-5-(((2-methoxypyridin-4-yl)amino)methyl)picolinamido)-2'-methyl-[1,1'-biphenyl]-3-yl)-1,5-dimethyl-4,5,6,7-tetrahydro-1*H*-imidazo[4,5-c]pyridine-2-carboxamide

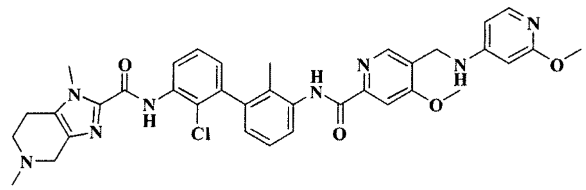

Step 1: Preparation of *tert*-butyl-2-((3-bromo-2-chlorophenyl)carbamoyl)-1-methyl-1,4,6,7-tetrahydro-5*H*-imidazo[4,5-*c*]pyridine-5-carboxylate

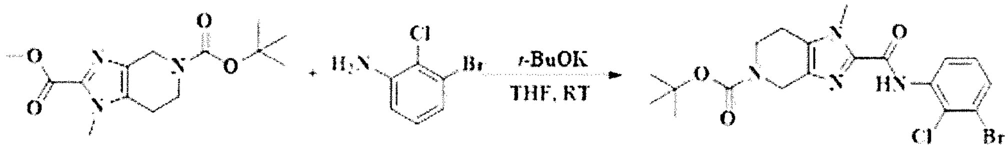


3-Bromo-2-chloroaniline (417 mg, 2.02 mmol), potassium *tert*-butoxide (378 mg, 3.38 mmol) were sequentially added to a solution of 5-(*tert*-butyl) 2-methyl-1-methyl-1,4,6,7-tetrahydro-5*H*-imidazo[4,5-c]pyridine-2,5-dicarboxylate (500 mg, 1.69 mmol) in tetrahydrofuran (10 mL), and then the reaction mixture was stirred at room temperature for 1 hour. After the reaction was completed, the reaction mixture was extracted with ethyl acetate (15 mL × 3), washed with saturated aqueous sodium chloride solution (15 mL × 3); the organic phase was collected, dried over anhydrous sodium sulfate. After filtration, the organic phase was concentrated under reduced pressure, then the obtained product was separated and purified by silica gel column chromatography (ethyl acetate: petroleum ether=1: 1) to obtain the title compound (680 mg, 86 %).

MS *m*/*z* (ESI): 469.1 $[M+H]^+$.

Step 2: Preparation of *N*-(3-bromo-2-chlorophenyl)-1,5-dimethyl-4,S,6, 7-tetrahydro-1*H*-imidazo[4,5-*c*]pyridine-2-carboxamide

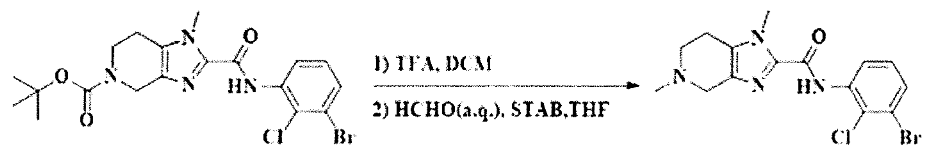


*tert*-Butyl-2-((3-bromo-2-chlorophenyl)carbamoyl)-1-methyl-1,4,6,7-tetrahydro-5*H*-imidazo[4,5-c]pyridine-5-carboxylate (300 mg, 0.64 mmol) was dissolved in dichloromethane (5 mL), then trifluoroacetic acid (1 mL) was added, and the reaction mixture was stirred at room temperature for 30 minutes and then concentrated; then tetrahydrofuran (10 mL) was added to dissolve, and aqueous formaldehyde solution (37 wt%, 0.48 mL, 6.39 mmol) and sodium triacetoxyborohydride (406 mg, 1.92 mmol) were added, and the mixture was stirred at room temperature for 1 hour. After the reaction was completed, the reaction was quenched with saturated sodium bicarbonate solution (20 mL), then the reaction mixture was extracted with ethyl acetate (15 mL × 3), washed with saturated aqueous sodium chloride solution (10 mL × 3), and the organic phase was collected, dried over anhydrous sodium sulfate, filtered and concentrated under reduced pressure. The obtained product was separated and purified by silica gel column chromatography (dichloromethane: methanol=95: 5) to obtain the title compound (223 mg, 91 %).

MS *m/z* (ESI): 383.0 $[M+H]^+$.

Step 3: Preparation of 2-methyl-3-(4,4,5,5-tetramethyl-1,3,2-dioxaborolan-2-yl)aniline

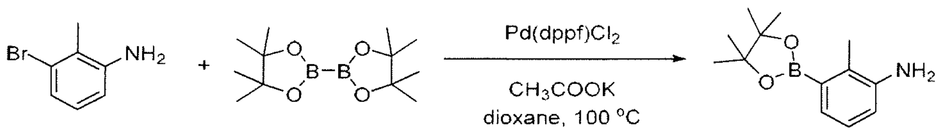


3-Bromo-2-methylaniline (1.8 g, 9.68 mmol), 4,4,4',4',5,5,5',5'-octamethyl-2,2'-bi(1,3,2-dioxaborolane) (2.96 g, 11.62 mmol), $Pd(dppf)Cl_2$ (702 mg, 0.96 mmol) and potassium acetate (2.86 g, 29.06 mmol) were added to a two-necked reaction flask, and dioxane (40 mL) was added; the reaction system was vacuumized and replaced with dry nitrogen, then the reaction system was heated to 100 °C and reacted for 8 hours, then the reaction mixture was concentrated under reduced pressure to remove the solvent, and the residue was separated by silica gel column chromatography to obtain the title compound as a light yellow solid compound (1.71 g , 76 %).

MS m/z (ESI): 234.1 $[M+H]^+$.

Step 4: Preparation of *N*-(3'-amino-2-chloro-2'-methyl-[1,1'-biphenyl]-3-yl)-1,5-dimethyl-4,5,6,7-tetrahydro-1*H*-imidazo[4,5-*c*]pyridine-2-carboxamide

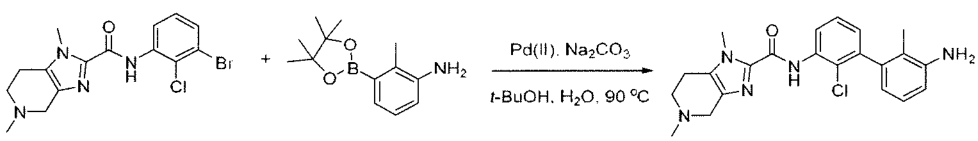


*N*-(3-Bromo-2-chlorophenyl)-1,5-dimethyl-4,5,6,7-tetrahydro-1*H*-imidazo[4,5-c]pyridine-2-carboxamide (683 mg, 1.78 mmol), 2-methyl-3-(4,4,5,5-tetramethyl-1,3,2-dioxaborolan-2-yl)aniline (623 mg, 2.67 mmol), dicyclohexylphosphine palladium dichloride (290 mg, 0.356 mmol) and sodium carbonate (566 mg, 5.34 mmol) were added to a mixed solvent of dioxane (5 mL) and water (5 mL), and the reaction system was protected by nitrogen, heated to 90 °C, stirred and reacted for 15 hours, then the reaction mixture was concentrated under reduced pressure to remove the solvent; the residue was dispersed in ethyl acetate, and the organic phase was washed with saturated aqueous sodium chloride solution, dried over anhydrous sodium sulfate; after filtration and concentration, the residue was separated by silica gel column chromatography to obtain the title compound (185 mg, 25 %).

MS *m/z* (ESI): 410.4 $[M+H]^+$.

Step 5: Preparation of methyl 5-formyl-4-methoxypicolinate

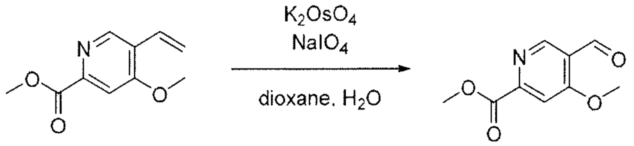


Methyl 4-methoxy-5-vinylpicolinate (10 g, 51.7 mmol) was dissolved in a mixed solvent of dioxane (500 mL) and water (200 mL), and the reaction mixture was cooled to 0 °C, then $K_2OsO_4$ .$2H_2O$ (953 mg, 2.58 mmol) and $NaIO_4$ (55 g, 258.5 mmol) were added successively under stirring conditions, and the reaction mixture was stirred and reacted at 0 °C for 4 hours. After the reaction was completed, the reaction mixture was concentrated under reduced pressure to remove most of organic solvent, and the residue was extracted with ethyl acetate; the organic phase was washed with saturated aqueous sodium chloride solution, dried over anhydrous sodium sulfate; after filtration and concentration, the residue was separated by silica gel column chromatography to obtain the title compound (6.8 g, 67 %).

MS *m/z* (ESI): 196.1 $[M+H]^+$.

Step 6: Preparation of methyl 4-methoxy-5-(((2-methoxypyridin-4-yl)amino)methyl)picolinate

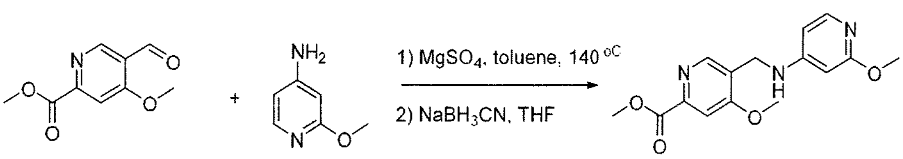


The crude product of methyl 5-formyl-4-methoxypicolinate and 2-methoxy-4-aminopyridine (2.0 g, 10.2 mmol) were dispersed in toluene (60 mL), and anhydrous magnesium sulfate (10 g) was added. The reaction system was protected with dry nitrogen, heated to 140 °C, stirred and reacted for 16 hours. The reaction mixture was concentrated under reduced pressure to remove the solvent, and the residue was dispersed in tetrahydrofuran (60 mL), then sodium cyanoborohydride (1.28 g, 20.4 mmol) was added; the reaction mixture was stirred at room temperature for 1.5 hours, then the reaction mixture was concentrated under reduced pressure to remove the solvent, and the residue was diluted with water, extracted with ethyl acetate; the organic phase was washed with saturated aqueous sodium chloride solution, dried over anhydrous sodium sulfate; after filtration and concentration, the residue was separated by silica gel column chromatography to obtain the title compound (1.48 g, 48 %).

MS *m/z* (ESI): 304.2 $[M+H]^+$.

Step 7: Preparation of *N*-(2-chloro-3'-(4-methoxy-5-(((2-methoxypyridin-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

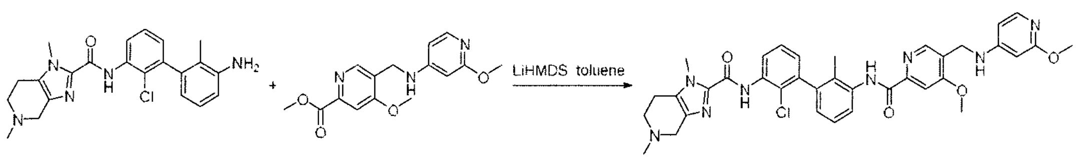


*N*-(3'-Amino-2-chloro-2'-methyl-[1,1'-biphenyl]-3-yl)-1,5-dimethy1-4,5,6, 7-tetrahydro-1*H*-imidazo[4,5-c]pyridine-2-carboxamide (600 mg, 1.467 mmol) and methyl 4-methoxy-5-(((2-methoxypyridin-4-yl)amino)methyl)picolinate (535 mg, 1.76 mmol) were dissolved in dry toluene (15 mL); the reaction system was protected with dry nitrogen, and LiHMDS (7.4 mL, 7.4 mmol) was added under stirring at room temperature, then the reaction mixture was continued to stir and react at room temperature for 16 hours; the reaction mixture was diluted with ethyl acetate, washed with saturated aqueous sodium chloride solution, dried over anhydrous sodium sulfate; after filtration and concentration, the residue was separated by prep-HPLC to obtain the title compound (537 mg, 54 %).

MS m/z (ESI): 681.1 [M+H]+.

$^1$H NMR (400 MHz, $CD_3OD$) $\delta$ 10.35 (s, 1H), 9.91 (s, 1H), 8.37 (s, 1H), 8.31 (dd, *J* = 8.1, 0.9 Hz, 1H), 7.92 (d, J= 7.6 Hz, 1H), 7.78 (s, 1H), 7.66 (d, *J*= 5.8 Hz, 1H), 7.46 (t, *J*= 7.9 Hz, 1H), 7.34 (t, *J*= 7.9 Hz, 1H), 7.08 (dd, *J*= 7.5, 1.3 Hz, 1H), 7.01 (dd, *J* = 14.8, 6.9 Hz, 2H), 6.29 (dd, *J*= 5.8, 1.7 Hz, 1H), 5.79 (d, *J*= 0.7 Hz, 1H), 4.34 (d, *J*= 6.0 Hz, 2H), 4.04 (s, 3H), 3.90 (s, 3H), 3.70 (s, 3H), 3.36 (s, 2H), 2.68 (s, 4H), 2.38 (s, 3H), 2.02 (s, 3H).

**Embodiment 2**

*N*-(2,2'-Dichloro-3'-(4-methoxy-5-(((2-methoxypyridin-4-yl)ainino)methyl)picolinamido)-[1,1'-biphenyl]-3-yl)-1,5-dimethyl-4,5,6,7-tetrahydro-1*H*-imidazo[4,5-*c*]pyridine-2-carboxamide

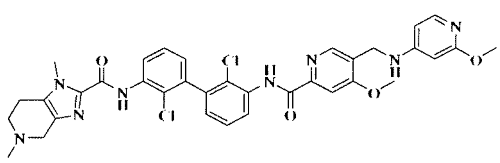

Step 1: Preparation of 2-chloro-3-(4,4,5,5-tetramethyl-1,3,2-dioxaborolan-2-yl)aniline

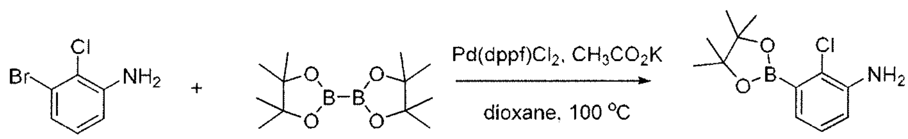


3-Bromo-2-chloroaniline (2.0 g, 9.68 mmol), 4,4,4',4',5,5,5',5'-octamethyl-2,2'-bi(1,3,2-dioxaborolane) (2.96 g, 11.62 mmol), $Pd(dppf)Cl_2$ (702 mg, 0.96 mmol) and potassium acetate (2.86 g, 29.06 mmol) were added to a two-necked reaction flask, and dioxane (40 mL) was added; the reaction system was vacuumized and replaced with dry nitrogen, then the reaction system was heated to 100 °C and reacted for 5 hours, then the reaction mixture was concentrated under reduced pressure to remove the solvent, and the residue was separated by silica gel column chromatography to obtain the title compound as a light yellow solid compound (1.78 g , 74 %).

MS m/z (ESI): 254.1 [M+H]+.

Step 2: Preparation of *N*-(3'-amino-2,2'-dichloro-[1,1'-biphenyl]-3-yl)-1,5-dimethyl-4,5,6,7-tetrahydro-1*H*-imidazo[4,5-*c*]pyridine-2-carboxamide

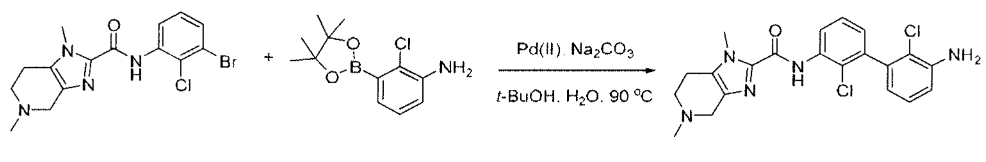


*N*-(3-Bromo-2-chlorophenyl)-1,5-dimethyl-4,5,6,7-tetrahydro-1*H*-imidazo[4,5-c]pyridine-2-carboxamide (683 mg, 1.78 mmol), 2-chloro-3-(4,4,5,5-tetramethyl-1,3,2-dioxaborolan-2-yl)aniline (676 mg, 2.67 mmol), dicyclohexylphosphine palladium dichloride (290 mg, 0.356 mmol) and sodium carbonate (566 mg, 5.34 mmol) were added to a mixed solvent of dioxane (5 mL) and water (5 mL); the reaction system was protected by nitrogen, heated to 90 °C, stirred and reacted for 15 hours, and the reaction mixture was concentrated under reduced pressure to remove the solvent; the residue was dispersed in ethyl acetate, and the organic phase was washed with saturated aqueous sodium chloride solution, dried over anhydrous sodium sulfate; after filtration and concentration, the residue was separated by silica gel column chromatography to obtain the title compound (178 mg, 23 %).

MS *m/z* (ESI): 430.1 [M+H]+.

Step 3: Preparation of *N*-(2,2'-dichloro-3'-(4-methoxy-5-(((2-methoxypyridin-4-yl)amino)methy))picolinamido)-[1,1'-biphenyl]-3-yl)-1,5-dimethyl-4,5,6,7-tetrahydro-1*H*-imidazo[4,5-*c*]pyridine-2-carboxamide

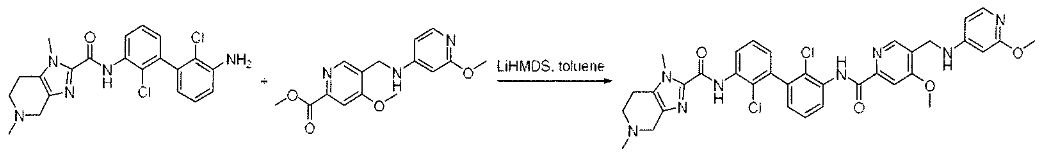


*N*-(3'-amino-2,2'-dichloro-[1,1'-biphenyl]-3-yl)-1,5-dimethyl-4,5,6,7-tetrahydro-1*H*-imidazo[4,5-c]pyridine-2-carboxamide (178 mg, 414 mmol) and methyl 4-methoxy-5-(((2-methoxypyridin-4-yl)amino)methyl)picolinate (151 mg, 497 mmol) were dissolved in dry toluene (5 mL); the reaction system was protected with dry nitrogen, and LiHMDS (2.1 mL, 2.1 mmol) was added under stirring at room temperature, then the reaction mixture was continued to stir and react at room temperature for 16 hours; the reaction mixture was diluted with ethyl acetate, washed with saturated aqueous sodium chloride solution, dried over anhydrous sodium sulfate; after filtration and concentration, the residue was separated by prep-HPLC to obtain the title compound (177 mg, 61 %).

MS m/z (ESI): 701.2 $[M+H]^+$.

$^1H$ NMR (400 MHz, $CD_3OD$) δ 8.60 (dd, J = 8.3, 1.1 Hz, 1H), 8.48-8.40 (m, 2H), 7.90 (s, 1H), 7.65 (d, J = 6.4 Hz, 1H), 7.45 (dt, J = 11.0, 8.0 Hz, 2H), 7.11 (ddd, J = 7.5, 4.2, 1.2 Hz, 2H), 6.42 (dd, J = 6.5, 1.7 Hz, 1H), 6.07 (d, J = 1.0 Hz, 1H), 4.52 (s, 2H), 4.08 (s, 3H), 3.99 (d, J = 4.5 Hz, 5H), 3.89 (s, 3H), 3.34 (d, J = 5.6 Hz, 2H), 2.96 (t, J =5.6 Hz, 2H), 2.83 (s, 3H).

## Embodiment 3

*N*-(2-Chloro-2'-fluoro-3'-(4-metboxy-5-(((2-methoxypyridin-4-yl)amino)niethyl)picolinamido)-[1,1'-biphenyl]-3-yl)-1,5-dimethyl-4,5,6,7-tetrahydro-1*H*-imidazo[4,5-*c*]pyridine-2-carboxamide

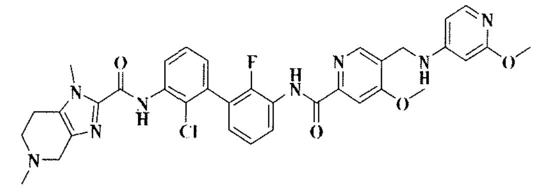

Preparation of *N*-(2-chloro-2'-fluoro-3'-(4-methoxy-5-(((2-methoxypyridin-4-yl)amino)methy])picolinamido)-[1,1'-biphenyl]-3-yl)-1,5-dimethyl-4,5,6,7-tetrahydro-1*H*-imidazo[4,5-c]pyridine-2-carboxamide was referred to Embodiment 1.

MS m/z (ESI): 685.2 $[M+H]^+$.

## Embodiment 4

*N*-(2-Fluoro-3'-(4-methoxy-5-(((2-methoxypyridin-4-yl)amino)methyl)picolinamido)-2'-methyl-[1,1'-biphenyl]-3-yl)-1,5-dimethyl-4,5,6,7-tetrahydro-1*H*-imidazo[4,5-c]pyridine-2-carboxamide

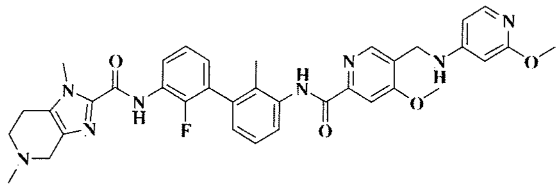

Preparation of *N*-(2-fluoro-3'-(4-methoxy-5-(((2-methoxypyridin-4-yl)amino)methyl)picolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 1.

MS *m*/*z* (ESI): 665.2 [M+H]$^+$.

### Embodiment 5

*N*-(2'-Chtoro-2-fluoro-3'-(4-methoxy-5-(((2-methoxypyridm-4-yl)amino)methyl)picotmamido)-[1,1'-biphenyl]-3-yl)-1,5-dimethyl-4,5,6,7-tetrahydro-1*H*-imidazo[4,5-c]pyridine-2-carboxainide

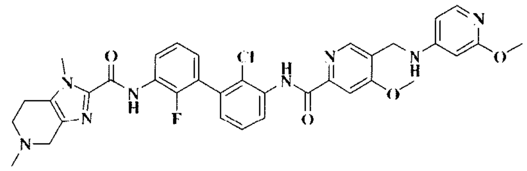

Preparation of *N*-(2'-chloro-2-fluoro-3'-(4-methoxy-5-(((2-methoxypyridin-4-yl)amino)methyl)picolinamido)-[1,1'-biphenyl]-3-yl)-1,5-dimethyl-4,5,6,7-tetraliydro-1*H*-imidazo[4,5-*c*]pyridine-2-carboxamide was referred to Embodiment 2.

MS *m*/*z* (ESI): 685.2 [M+H]$^+$.

### Embodiment 6

*N*-(2-Chloro-3'-(5-(((3-fluoro-2-methylpyridin-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-c]pyridine-2-carboxainide

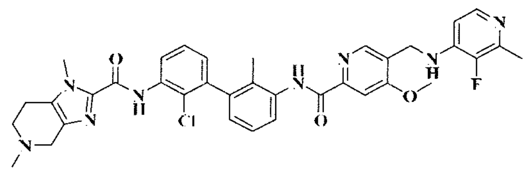

Preparation of *N*-(2-chloro-3'-(5-(((3-fluoro-2-methylpyridin-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS *m*/*z* (ESI): 683.2 [M+H]$^+$.

### Embodiment 7

*N*-(2-Fluoro-3'-(5-(((3-fluoro-2-methylpyridin-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

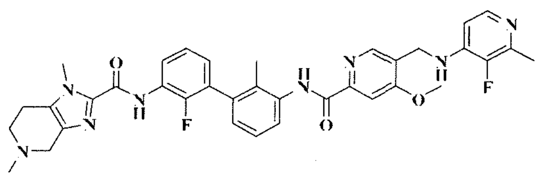

Preparation of *N*-(2-ftuoro-3'-(5-(((3-fluoro-2-methylpyridin-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS m/z (ESI): 667.2 $[M+H]^+$.

## Embodiment 8

*N*-(2-Chloro-3'-(5-(((3-fluoro-2-methoxypyridin-4-yl)amino)methyl)-4-methoxypicolinamido)-2'-methyl-[1,1-biphenyt]-3-yt)-1,5-dimethyl-4,5,6,7-tetrahydro-1*H*-imidazo[4,5-*c*]pyridine-2-carboxamide

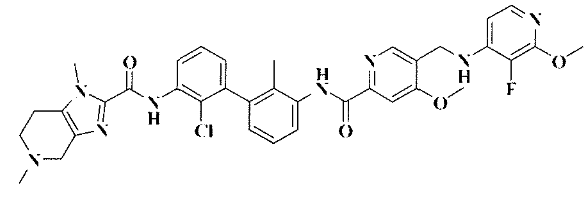

Preparation of *N*-(2-chloro-3'-(5-(((3-fluoro-2-methoxypyridin-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS *m/z* (ESI): 699.2 $[M+H]^+$.

## Embodiment 9

4-(((6-((2'-Chloro-3'-(1,5-dimethyl-4,5,6,7-tetrahydro-1*H*-imidazo[4,5-*c*]pyridine-2-carboxamido)-2-methyl-[1,1'-biphenyl]-3-yl)carbamoyl)-4-methoxypyridin-3-yl)methyl)amino)picolinic acid

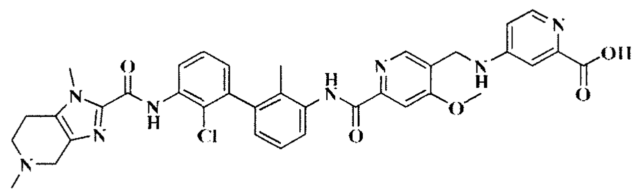

Preparation of 4-(((6-((2'-chloro-3'-(1,5-dimethyl-4,5,6,7-tetrahydro-1*H*-imidazo[4,5-*c*]pyridine-2-carboxamido)-2-methyl-[1,1'-biphenyl]-3-yl)carbamoyl)-4-methoxypyridin-3-yl)methyl)amino)picolinic acid was referred to Embodiment 1.

MS *m/z* (ESI): 695.2 $[M+H]^+$.

## Embodiment 10

*N*-(2-Chloro-3'-(5-(((2-(2-hydroxypropan-2-yl)pyridin-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

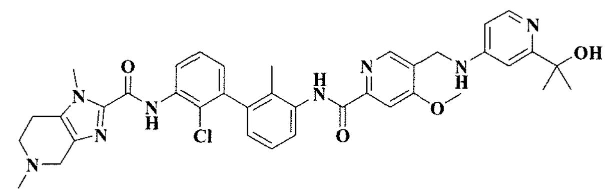

Preparation of *N*-(2-chloro-3'-(5-(((2-(2-hydroxypropan-2-yl)pyridin-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 1.

MS *m/z* (ESI): 709.2 [M+H]+.

**Embodiment 11**

*N*-(2-Chloro-3'-(4-methoxy-5-(((4-methylpyridin-2-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

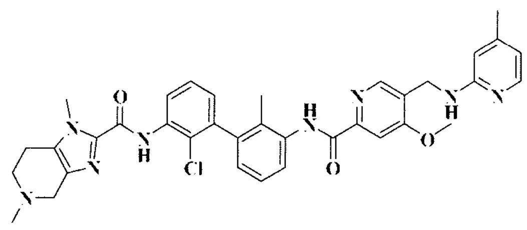

Step 1: Preparation of methyl 4-methoxy-5-(((4-methylpyridin-2-yl)amino)methyl)picolinate

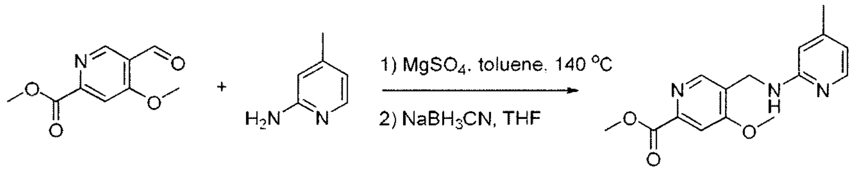


Methyl 5-formyl-4-methoxypicolinate (500 mg, 2.59 mmol) and 4-methylpyridin-2-amine (96 mg, 0.26 mmol) were dispersed in anhydrous methanol (30 mL), then anhydrous magnesium sulfate (1.5 g) was added, and the reaction system was heated to 90 °C under the protection of dry nitrogen, and the reaction mixture was stirred and reacted for 16 hours, then the reaction mixture was concentrated under reduced pressure to remove the solvent, and the residue was dispersed in tetrahydrofuran (30 mL). Sodium cyanoborohydride (814 mg, 12.95 mmol) was added under stirring at room temperature, then the reaction mixture was continued to stir and react at room temperature for 1 hour; the reaction mixture was concentrated under reduced pressure to remove the solvent, and the residue was diluted with water, extracted with ethyl acetate, then the ethyl acetate layer was washed with saturated aqueous sodium chloride solution, dried over anhydrous sodium sulfate; after filtration and concentration, the residue was separated by prep-HPLC to obtain the title compound (84 mg, 11 %).

MS *m/z* (ESI): 288.2 [M+H]+.

Step 2: Preparation of *N*-(2-chloro-3'-(4-methoxy-5-(((4-methylpyridin-2-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

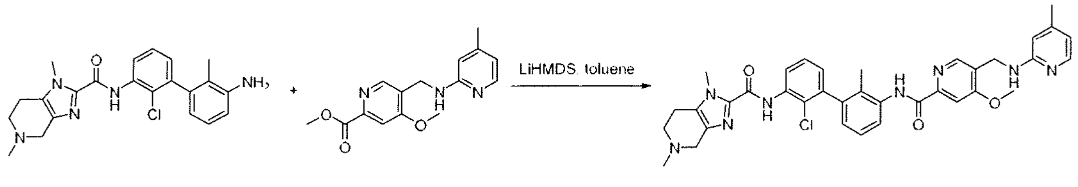


*N*-(3'-Amino-2-chloro-2'-methyl-[1,1'-biphenyl]-3-yl)-1,5-dimethyl-4,5,6,7-tetrahydro-1*H*-imidazo[4,5-c]pyridine-2-carboxamide (50 mg, 0.1008 mmol) and methyl 4-methoxy-5-(((4-methylpyridin-2-yl)amino)methyl)picolinate (30.4

mg, 0.105 mmol) were dispersed in dry toluene (2.5 mL); the reaction system was protected with dry nitrogen, and LiHMDS (0.454 mL, 0.454 mmol) was added, and the reaction mixture was continued to stir and react at room temperature for 16 hours, then the reaction mixture was diluted with ethyl acetate, washed with saturated aqueous sodium chloride solution, dried over anhydrous sodium sulfate; after filtration and concentration, the residue was separated by prep-HPLC to obtain the title compound (26.7 mg, 40 %).

MS m/z (ESI): 665.2 $[M+H]^+$.

$^1H$ NMR (400 MHz, $CD_3OD$) δ 8.42 (dd, J = 8.2, 1.3 Hz, 1H), 8.39 (s, 1H), 7.97 (d, J =8.1 Hz, 1H), 7.84 (s, 1H), 7.77 (d, J = 6.0 Hz, 1H), 7.41 (t, J = 7.9 Hz, 1H), 7.33 (t, J = 7.9 Hz, 1H), 7.08 (dd, J = 7.5, 1.3 Hz, 1H), 7.04 (d, J = 7.0 Hz, 1H), 6.47 (d, J = 4.6 Hz, 2H), 4.57 (s, 2H), 4.06 (s, 3H), 3.99 (s, 3H), 3.78 (s, 2H), 3.16-3.10 (m, 2H), 2.89 (t, J = 5.7 Hz, 2H), 2.70 (s, 3H), 2.23 (s, 3H), 2.09 (s, 3H).

**Embodiment 12**

*N*-(2-Chloro-3'-(4-methoxy-5-(((4-methoxypyridin-2-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

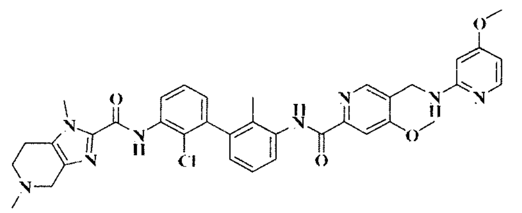

Preparation of *N*-(2-chloro-3'-(4-methoxy-5-(((4-methoxypyridin-2-yl)amino)methyl)picolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 11.

MS *m*/*z* (ESI): 681.2 $[M+H]^+$.

Embodiment 13

*N*-(2-Chloro-3'-(4-methoxy-5-((((3-methoxypyridin-2-yl)methyl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

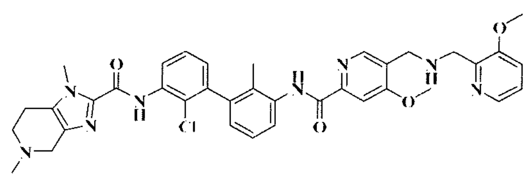

Step 1: Preparation of *N*-(2-chloro-3'-(4-methoxy-5-vinylpicolinamido)-2'-methyl-[1,1'-biphenyl]-3-yl)-1,5-dimethyl-4,5,6,7-tetrahydro-1*H*-imidazo[4,5-*c*]pyridine-2-carboxamide

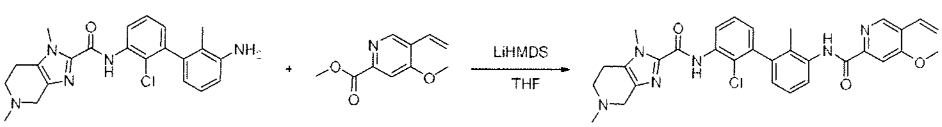


LiHMDS (1.15 mL, 1.3 M in THF) was added dropwise to a solution of *N*-(3'-amino-2-chloro-2'-methyl-[1,1'-biphenyl]-3-y])-15-dimethyl-4,5,6,7-tetrahydro-1*H*-imidazo[4,5-*c*]pyhdine-2-carboxamide (153 mg, 0.374 mmol) and methyl 4-methoxy-5-vinylpicolinate (79 mg, 0.411 mmol) in tetrahydrofuran (4 mL), and the mixture was stirred at room

temperature for 3 hours. The reaction mixture was quenched with water (5 mL) and extracted with ethyl acetate (10 mL). The organic phase was washed with saturated aqueous sodium chloride solution (5 mL), dried over anhydrous sodium sulfate, then the mixture was filtered, and concentrated under reduced pressure to obtain the title compound as a brown solid (237 mg, crude product).

MS m/z (ESI):571.2[M+H]$^+$.

Step 2: Preparation of *N*-(2-chloro-3'-(5-formyl-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

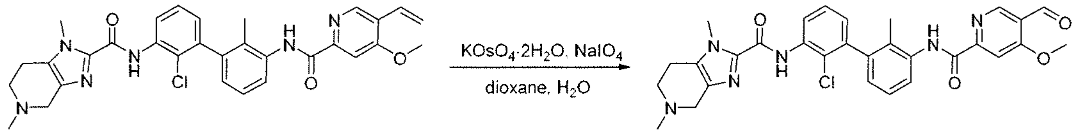


Under an ice bath, sodium periodate (267 mg, 1.25 mmol) was added to a mixed solution of *N*-(2-chloro-3'-(4-methoxy-5-vinylpicolinamido)-2'-methyl-[1,1'-biphenyl]-3-yl)-1,5-dimethyl-4,5,6,7-tetrahydro-1*H*-imidazo[4,5-*c*]pyridine-2-carboxamide (237 mg, crude product, about 0.374 mmol) and potassium osmate dihydrate (15 mg, 0.0416 mmol) in dioxane (6 mL) and water (2 mL), and the mixture was stirred at room temperature for 2 hours. The reaction mixture was quenched with water (10 mL) and extracted with dichloromethane (30 mL). The organic phase was washed with saturated aqueous sodium chloride solution (10 mL), dried over anhydrous sodium sulfate, and the mixture was filtered, concentrated under reduced pressure, and the residue was purified by silica gel column chromatography to obtain the title compound as a brown solid (66 mg, the yield in two steps was 31 %).

MS *m/z* (ESI):573.2[M+H]$^+$.

Step 3: Preparation of *N*-(2-chloro-3'-(4-methoxy-5-((((3-methoxypyridin-2-yl)methyl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

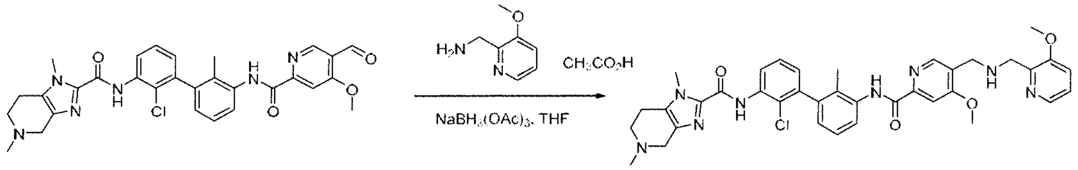


*N*-(2-Chloro-3'-(5-formyl-4-methoxypicolinamido)-2'-methyl-[1,1'-biphenyl]-3-yl)-1,5-dimethyl-4,5,6,7-tetrahydro-1*H*-imidazo[4,5-*c*)pyridine-2-carboxamide (280 mg, 0.489 mmol) and (3-methoxypyridin-2-yl)methanamine (337 mg, 2.445 mmol) were dissolved in a mixed solvent of tetrahydrofuran (6 mL) and water (1 mL), then acetic acid (0.5 mL) and sodium triacetoxyborohydride (518 mg, 2.445 mmol) were added successively under stirring at room temperature; the reaction mixture was stirred at room temperature for 5 hours, and the reaction mixture was diluted with saturated aqueous sodium carbonate solution, and extracted with dichloromethane. The organic phase was dried over anhydrous sodium sulfate; after filtration and concentration, the residue was separated by prep-HPLC to obtain the title compound (10.5 mg, 3 %).

MS *m/z* (ESI): 695.2 [M+H]$^+$.

$^1$H NMR (400 MHz, $CD_3OD$) δ 8.49 (s, 1H), 8.43 (dd, J = 8.1, 0.8 Hz, 1H), 8.12 (d, J = 4.5 Hz, 1H), 7.96 (d, J = 8.1 Hz, 1H), 7.84 (s, 1H), 7.46-7.38 (m, 2H), 7.34 (dd, J = 13.8, 8.2 Hz, 2H), 7.08 (dd, J = 12.5, 4.6 Hz, 2H), 4.16 (s, 4H), 4.05 (s, 3H), 3.98 (s, 3H), 3.90 (s, 3H), 3.58 (s, 2H), 2.93 (t, J = 5.6 Hz, 2H), 2.82 (t, J = 5.4 Hz, 2H), 2.56 (s, 3H), 2.11 (s, 3H).

**Embodiment 14**

*N*-(2-Chloro-3'-(5-((((3-fluoro-4-methoxypyridin-2-yl)methyl)amino)metliyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

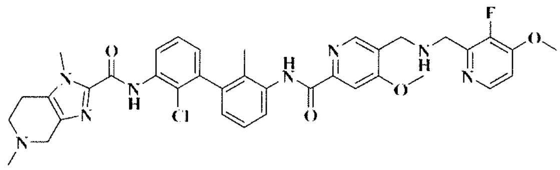

Preparation of *N*-(2-chloro-3'-(5-((((3-fluoro-4-methoxypyridin-2-yl)methyl)amino)methyl)-4-metlioxypicolinamido)-2'-methyl-[1,1'-biphenyl]-3-yl)-1,5-dimethy1-4,5,6,7-tetrahydro-1*H*-imidazo[4,5-c]pyridine-2-carboxamide was referred to Embodiment 13.

MS *m*/*z* (ESI): 713.2 $[M+H]^+$.

**Embodiment 15**

*N*-(2-Chloro-3'-(5-((((3-chloro-4-methoxypyridin-2-yl)methyl)amino)methyl)-4-methoxypicolinamido)-2'-methyl-[1,1'-biphenyl]-3-yl)-1,5-dimethyl-4,5,6, 7-tetrahydro-1*H*-imidazo[4,5-*c*]pyridine-2-carboxamide

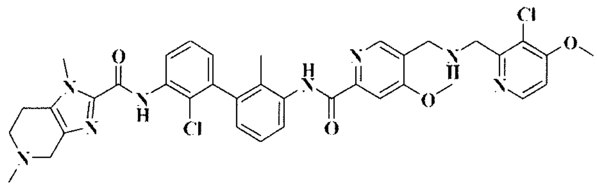

Preparation of *N*-(2-chloro-3'-(5-((((3-chloro-4-methoxypyridin-2-yl)methyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 13.

MS *m*/*z* (ESI): 729.2 $[M+H]^+$.

**Embodiment 16**

*N*-(2-Chloro-3'-(4-methoxy-5-((((2-carbonyl-1,2-dihydropyridin-3-yl)methyl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

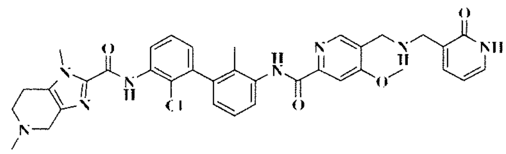

Preparation of *N*-(2-chloro-3'-(4-methoxy-5-((((2-carbonyl-1,2-dihydropyridin-3-yl)methyl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamidee was referred to Embodiment 13.

MS *m*/*z* (ESI): 681.2 $[M+H]^+$.

**Embodiment 17**

*N*-(2-Chloro-3'-(4-methoxy-5-((2-carbonylpyrazin-1(2*H*)-yl)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

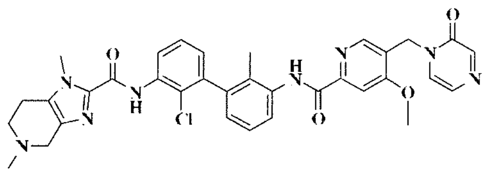

Preparation of *N*-(2-chloro-3'-(4-methoxy-5-((2-carbonylpyrazin-1(2*H*)-yl)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS *m/z* (ESI): 653.2 [M+H]$^+$.

**Embodiment** 18

*N*-(2-Chloro-3'-(5-(((2-fluoroethyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

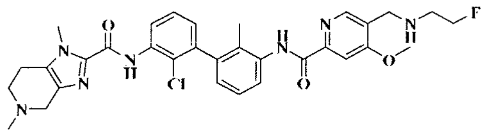

Step 1: Preparation of methyl 5-(((*tert*-butoxycarbonyl)(2-fluoroethyl)amino)methyl)-4-methoxypicolinate

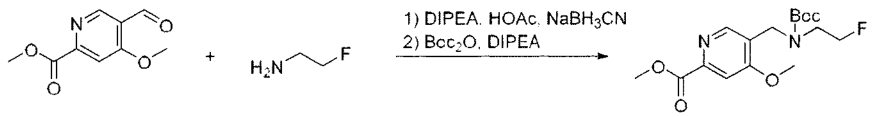


Methyl 5-formyl-4-methoxypicolinate (200 mg, 1.025 mmol) and 2-fluoroethan-1-amine (122 mg, 1.23 mmol) were dissolved in tetrahydrofuran (10 mL), then DIPEA (0.253 mL, 1.54 mmol) and 5 drops of acetic acid were added, and the mixture was stirred evenly at room temperature, then sodium cyanoborohydride (129 mg, 2.05 mmol) was added, and the reaction mixture was stirred and reacted at room temperature for 1 hour, then DIPEA (0.5 mL, 3.0 mmol) and $Boc_2O$ (336 mg, 1.54 mmol) were added; the reaction mixture was continued to stir and react at room temperature for 2 hours, then the reaction mixture was concentrated under reduced pressure to remove the solvent, and the residue was dispersed in ethyl acetate, washed with saturated sodium bicarbonate and saturated aqueous sodium chloride solution, dried over anhydrous sodium sulfate; after filtration and concentration, the residue was separated by silica gel column chromatography to obtain the title compound (81 mg, 23 %).

MS *m/z* (ESI): 343.1 [M+H]$^+$.

Step 2: Preparation of *N*-(2-chloro-3'-(5-(((2-fluoroethyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

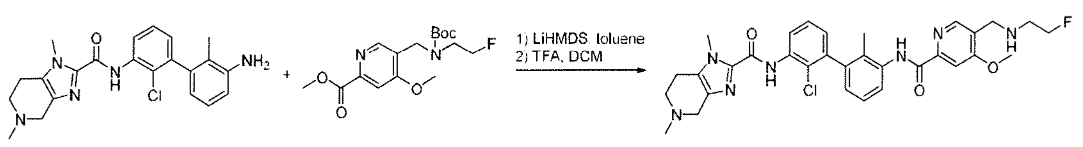


*N*-(3'-Amino-2-chloro-2'-methyl-[1,1'-biphenyl]-3-yl)-1,5-dimethyl-4,5,6,7-tetrahydro-1*H*-imidazo[4,5-c]pyridine-2-carboxamide (50 mg, 0.121 mmol) and methyl 5-(((*tert*-butoxycarbonyl)(2-fluoroethyl)amino)methyl)-4-methoxypicolinate (50 mg, 0.146 mmol) were dispersed in dry toluene (4 mL); the reaction system was protected by dry nitrogen, and LiHMDS (0.426 mL, 0.426 mmol) was added, then the reaction mixture was stirred and reacted at room temperature for 30 hours. The reaction mixture was diluted with ethyl acetate, washed with saturated aqueous sodium chloride solution, dried over anhydrous sodium sulfate; the mixture was filtered, and concentrated. The residue was dissolved in dichloromethane (2 mL), and trifluoroacetic acid (2 mL) was added; the reaction mixture was stirred and reacted at room temperature for 0.5 hours, then the reaction mixture was concentrated under reduced pressure to remove the solvent, and the residue was separated by prep-HPLC to obtain the title compound (19.3 mg, 26 %).

MS *m/z* (ESI): 620.2 $[M+H]^+$.

$^1H$ NMR (400 MHz, DMSO-$d_6$) δ 10.41 (s, 1H), 9.94 (s, 1H), 8.58 (s, 1H), 8.28 (dd, J = 8.2, 1.1 Hz, 1H), 7.89 (d, J = 7.9 Hz, 1H), 7.80 (s, 1H), 7.48 (t, J = 7.9 Hz, 1H), 7.36 (t, J = 7.8 Hz, 1H), 7.11 (dd, J =7.6, 1.3 Hz, 1H), 7.05 (d, J =7.5 Hz, 1H), 4.73-4.54 (m, 2H), 4.09 (s, 2H), 4.03 (s, 3H), 3.92 (s, 3H), 3.81 (s, 2H), 3.20-3.03 (m, 4H), 2.84 (d, J = 5.3 Hz, 2H), 2.64 (s, 3H), 2.04 (s, 3H).

## Embodiment 19

-(2-Chloro-3'-(5-((((4-fluorotetrahydro-2*H*-pyran-4-yl)methyl)amino)methyl)-4-methoxypicolinamido)-2'-methyl-[1,1'-biphenyl]-3-yl)-1 ,5-dimethyl-4,5,6,7-tetrahydro-1*H*-imidazo[4,5-*c*]pyridine-2-carboxamide

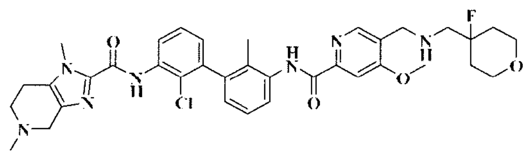

Preparation of *N*-(2-chloro-3'-(5-((((4-fluorotetrahydro-2*H*-pyran-4-yl)methyl)amino)methyl)-4-methoxypicolinamido)-2'-methyl-(1,1'-biphenyl]-3-yl)-1,5-dimethyl-4,5,6,7-tetrahydro-*1*H-imidazo(4,5-c]pyridine-2-carboxamide was referred to Embodiment 18.

MS *m/z* (ESI): 690.2 $[M+H]^+$.

## Embodiment 20

*N*-(2-Chloro-3'-(5-((((3-fluorooxetan-3-yl)methyl)amino)methyl)-4-methoxypicolinamido)-2'-methyl-[1,1'-biphenyl]-3-yl)-1,5-dimethyl-4,5,6,7-tetrahydro- 1*H*-imidazo[4,5-*c*]pyridine-2-carboxamide

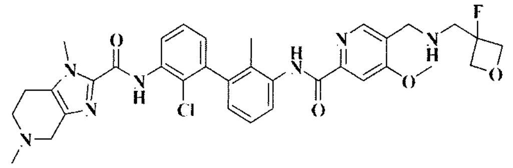

Preparation of *N*-(2-chloro-3'-(5-((((3-fluorooxetan-3-yl)metliyl)amino)methyl)-4-methoxypicolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 18.

MS *m/z* (ESI): 662.2 $[M+H]^+$.

**Embodiment 21**

*N*-(2-Chloro-3'-(5-(((3-(fluoromethyl)oxetan-3-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

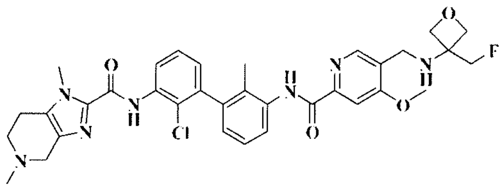

Preparation of *N*-(2-chloro-3'-(5-(((3-(fluoromethyl)oxetan-3-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 18.

MS *m/z* (ESI): 662.2 $[M+H]^+$.

**Embodiment 22**

*N*-(2-Chloro-3'-(5-((5-fluoro-5-(hydroxymethyl)-2-carbonyl-1,3-oxazinan-3-yl)methyl)-4-methoxypicolinamido)-2'-methyl-[1,1'-biphenyt]-3-yl)-1,5-dimethyl-4,5,6,7-tetrahydro-1*H*-imidazo[4,5-*c*]pyridine-2-carboxamide

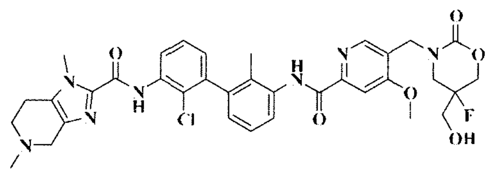

Step 1: Preparation of methyl 5-(((*tert*-butoxycarbonyl)((3-fluorooxetan-3-yl)methyl)amino)methyl)-4-methoxypicolinate

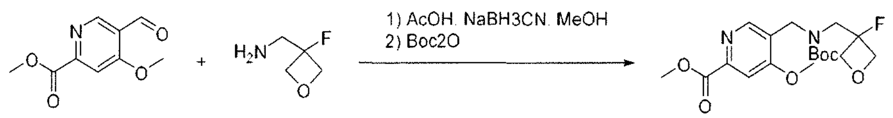


Methyl 5-fonnyl-4-methoxy-2-picolinate (30 mg, 0.153 mmol), 3-fluoro-3-oxetanemethanamine (24 mg, 0.229 mmol), AcOH (23 mg , 0.383 mmol), $NaBH_3CN$ (28 mg, 0.459 mmol) were added to MeOH (4 mL), and the mixture was stirred at room temperature for 16 hours. DIPEA (99 mg, 0.765 mmol) and $Boc_2O$ (37 mg, 0.168 mmol) were added to the reaction mixture, and the mixture was stirred at room temperature for 1 hour. The reaction mixture was concentrated under reduced pressure, and the residue was separated and purified by silica gel column chromatography to obtain the title compound as a colorless oil (11 mg, 19 %).

MS m/z (ESI):385.2$[M+H]^+$.

Step 2: Preparation of *N*-(2-chloro-3'-(5-((5-fluoro-5-(hydroxymethyl)-2-carbonyl-1,3-oxazidin-3-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

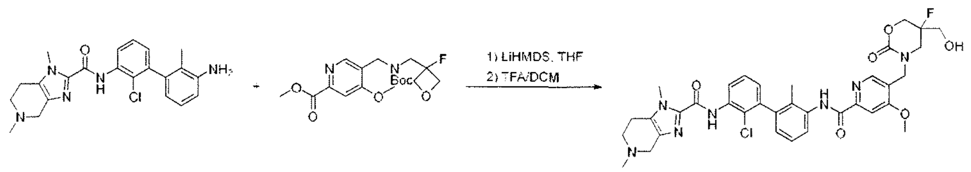


Under ice bath, LiHMDS (0.114 mL, 1.0 M in THF) was added dropwise to a solution of *N*-(3'-amino-2-chloro-2'-methyl-[1,1'-biphenyl]-3-yl)-1,5-dimethyl-4,5,6,7-tetrahydro-1*H*-imidazo[4,5-*c*]pyridine-2-carboxamide (12 mg, 0.0286 mmol) and methyl 5-(((*tert*-butoxycarbonyl)((3-fluorooxetan-3-yl)methyl)amino)methyl)-4-methoxypicolinate (11 mg, 0.0286 mmol) in tetrahydrofuran (0.5 mL), then the mixture was warmed to room temperature and stirred for 16 hours. The reaction mixture was quenched with water (5 mL) and extracted with ethyl acetate (10 mL). The organic phase was washed with saturated aqueous sodium chloride solution (5 mL), dried over anhydrous sodium sulfate, then the mixture was filtered, and concentrated under reduced pressure. The residue was dissolved in dichloromethane (1 mL), and trifluoroacetic acid (0.25 mL) was added, and the mixture was stirred at room temperature for 1 hour. The reaction mixture was concentrated under reduced pressure, and the residue was diluted with MeOH (0.5 mL), then DIPEA was added to adjust the pH value to 8, and then the mixture was separated and purified by prep-HPLC to obtain the title compound (8.0 mg, 40 %).

MS *m/z* (ESI):706.2[M+H]$^+$.

## Embodiment 23

(*S*)-*N*-(2-Chloro-3'-(5-((5-fluoro-5-(hydroxymethyl)-2-carbonyl-1,3-oxazinan-3-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

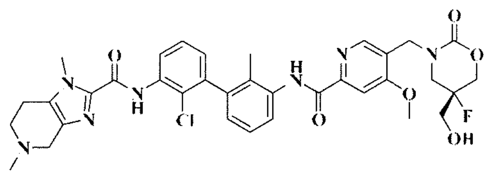

(*S*)-*N*-(2-Chloro-3'-(5-((5-fluoro-5-(hydroxymethiyl)-2-carbonyl-1,3-oxazinan-3-yl)methyl)-4-methoxypicolinamido)-2'-methyl-[1,1'-biphenyl]-3-yl)-1,S-dimethyl-4,S,6,7-tetrahydro-1*H*-imidazo[4,5-*c*]pyridine-2-carboxamide was prepared by chiral resolution of Embodiment 22.

MS *m/z* (ESI): 706.2 [M+H]$^+$.

## Embodiment 24

(*R*)-*N*-(2-Chloro-3'-(5-((5-fluoro-5-(hydroxymethyl)-2-carbonyl-1,3-oxazinan-3-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

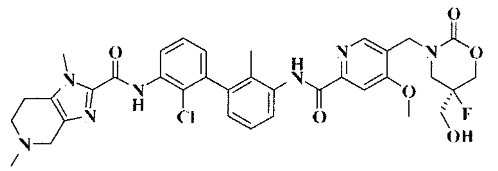

(*R*)-*N*-(2-Chloro-3'-(S-((5-fluoro-5-(hydroxymethyl)-2-carbonyl-1,3-oxazinan-3-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was prepared by chiral resolution of Embodiment 22.

MS *m/z* (ESI): 706.2 [M+H]$^+$.

**Embodiment 25**

(*R*)-*N*-(2-Chloro-3'-(5-((3-fluoropyrrolidin-1-yl)methyl)-4-methoxypicolinamido)-2'-methyl-[1,1'-biphenyl]-3-yl)-1,5-dimethyl-4,5,6,7-tetrahydro-1*H*-imidazo[4,5-c]pyridine-2-carboxamide

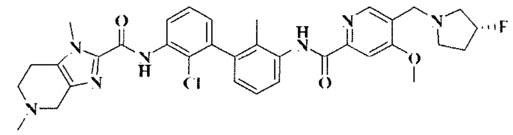

Preparation of (*R*)-*N*-(2-chloro-3'-(5-((3-fluoropyrrolidin-1-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 18.

MS *m/z* (ESI): 646.2 [M+H]+.

**Embodiment 26**

(*S*)-*N*-(2-Chloro-3'-(5-((3-fluoropyrrolidin-1-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

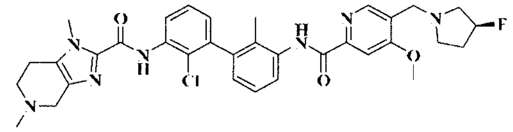

Preparation of (*S*)-*N*-(2-chloro-3'-(5-((3-fluoropyrrolidin-1-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 18.

MS *m/z* (ESI): 646.2 [M+H]+.

**Embodiment 27**

*N*-(2-Chloro-3'-(5-(((3*S*,4*S*)-3,4-difluoropyrrolidin-1-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

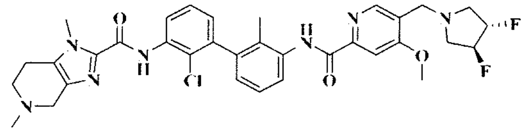

Preparation of *N*-(2-chloro-3'-(5-(((3*S*,4*S*)-3,4-difluoropyrrolidin-1-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 18.

MS *m/z* (ESI): 664.2 [M+H]+.

**Embodiment 28**

*N*-(2-Chloro-3'-(5-((3,3-difluoropyrrolidin-1-yl)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

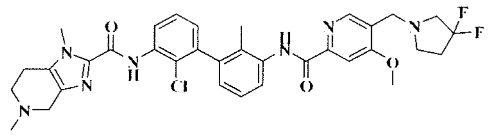

Preparation of *N*-(2-chloro-3'-(5-((3,3-difluoropyrrolidin-1-yl)methyl)-4-methoxypicolinamido)-2'-methyl-[1,1'-biphenyl]-3-yl)-1,5-dimethyl-4,5,6,7-tetrahydro-1H-imidazo[4,5-*c*]pyridine-2-carboxamide was referred to Embodiment 18.

MS *m/z* (ESI): 664.2 [M+H]$^+$.

**Embodiment 29**

*N*-(2-Chloro-3'-(5-((((3S,4S)-3-fluorotetrahydro-2*H*-pyran-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

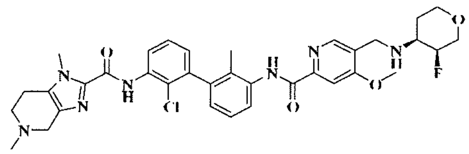

Preparation of *N*-(2-chloro-3'-(5-((((3*S*,4*S*)-3-fluorotetrahydro-2*H*-pyran-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 18.

MS *m/z* (ESI): 676.2 [M+H]$^+$.

**Embodiment 30**

*N*-(2-Chloro-3'-(5-((((3*R*,4*R*)-3-fluorotetrahydro-2*H*-pyran-4-yl)ai-nino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

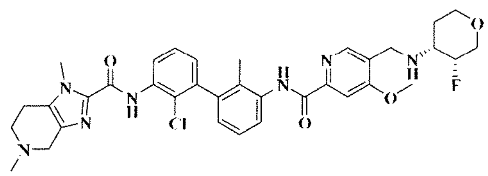

Preparation of *N*-(2-chloro-3'-(5-((((3*R*,4*R*)-3-fluorotetrahydro-2*H*-pyran-4-yl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 18.

MS *m/z* (ESI): 676.2 [M+H]$^+$.

$^1$H NMR (400 MHz, DMSO-$d_6$) δ 10.38 (s, 1H), 9.92 (s, 1H), 8.57 (s, 1H), 8.32 (d, J = 8.3 Hz, 1H), 7.95 (d, J = 8.1 Hz, 1H), 7.74 (s, 1H), 7.47 (t, J = 8.0 Hz, 1H), 7.35 (t, J = 7.8 Hz, 1H), 7.09 (d, J = 7.5 Hz, 1H), 7.03 (d, J = 7.7 Hz, 1H), 4.72 (d, J = 49.1 Hz, 1H), 3.99 (s, 3H), 3.97-3.88 (m, 4H), 3.88-3.79 (m, 3H), 3.51-3.42 (m, 2H), 3.41-3.38 (m, 2H), 2.82-2.72 (m, 1H), 2.71-2.67 (m, 4H), 2.38 (s, 3H), 2.05 (s, 3H), 1.78-1.54 (m, 2H).

**Embodiment 31**

*N*-(3'-(5-((((3*R*,4*S*)-1-Acetyl-3-fluoropiperidin-4-yl)amino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide

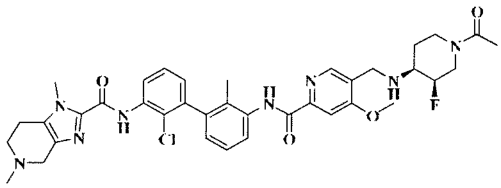

Preparation of *N*-(3'-(5-((((3*R*,4*S*)-1-acetyl-3-fluoropiperidin-4-yl)amino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 18.
MS *m*/*z* (ESI): 717.2 $[M+H]^+$.

**Embodiment 32**

*N*-(3'-(5-((((3*S*,4*R*)-1-Acetyl-3-fluoropiperidin-4-yl)amino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide

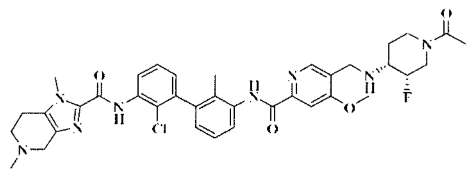

Preparation of *N*-(3'-(5-((((3*R*,4*S*)-1-acetyl-3-fluoropiperidin-4-yl)amino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 18.
MS *m*/*z* (ESI): 717.2 $[M+H]^+$.

**Embodiment 33**

*N*-(2-Chloro-3'-(5-(((3,3-difluorocyclobutyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

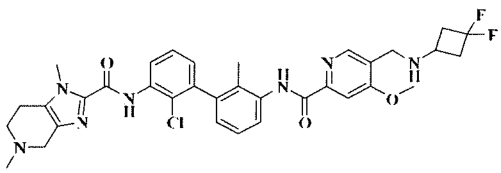

Preparation of *N*-(2-chloro-3'-(5-(((3,3-difluorocyclobutyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 13.
MS *m*/*z* (ESI): 664.2 $[M+H]^+$.

**Embodiment 34**

*N*-(2-Chloro-3'-(5-(((4,4-difluorocyclohexyl)amino)methyl)-4-methoxypicolinamido)-2'-methyl-[1,1'-biphenyl]-3-yl)-1,5-dimethyl-4,5,6,7-tetrahydro-1*H*-imidazo[4,5-c]pyridine-2-carboxainide

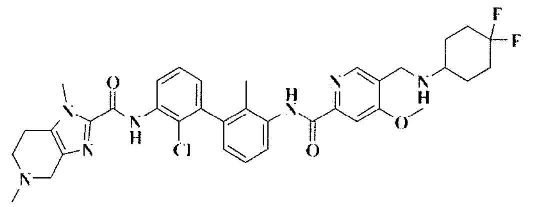

Preparation of *N*-(2-chloro-3'-(5-(((4,4-difluorocyclohexyl)amino)methyl)-4-methoxypicolinaniido)-2'-methyl-[1,1'-biphenyl]-3-yl)-1,5-dimethyl-4.5,6,7-tetrahydro-1*H*-imidazo[4,5-*c*]pyridine-2-carboxamide was referred to Embodiment 13.

MS *m/z* (ESI): 692.2 $[M+H]^+$.

**Embodiment 35**

*N*-(2-Chloro-3'-(4-methoxy-5-(((tetrahydro-2*H*-pyran-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

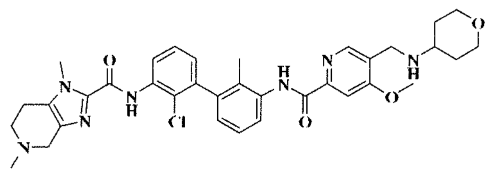

Preparation of *N*-(2-chloro-3'-(4-methoxy-5-(((tetrahydro-2*H*-pyran-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 13.

MS *m/z* (ESI): 658.2 $[M+H]^+$.

**Embodiment 36**

*N*-(2-Chloro-3'-(4-methoxy-5-((oxetan-3-ylamino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

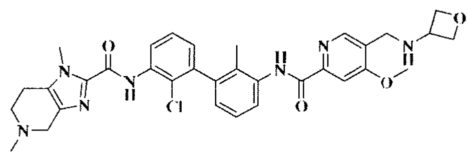

Preparation of *N*-(2-chloro-3'-(4-methoxy-5-((oxetan-3-ylamino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 13.

MS *m/z* (ESI): 630.2 $[M+H]^+$.

**Embodiment 37**

*N*-(2-Chloro-3'-(5-(((2-hydroxyethyl)amino)methyl)-4-methoxypicolinamido)-2'-methyl-[1,1'-biphenyl]-3-yl)-1,5-dimethyl-4,5,6,7-tetrahydro-1*H*-imidazo[4,5-c]pyridine-2-carboxamide

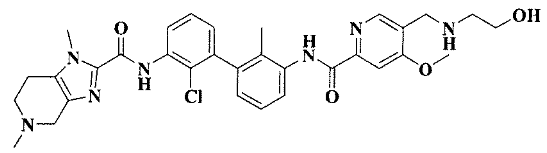

Preparation of *N*-(2-chloro-3'-(5-(((2-hydroxyethyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 13.
MS *m*/*z* (ESI): 618.2 $[M+H]^+$.

**Embodiment 38**

(*S*)-*N*-(2-Chloro-3'-(5-(((2-hydroxypropyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

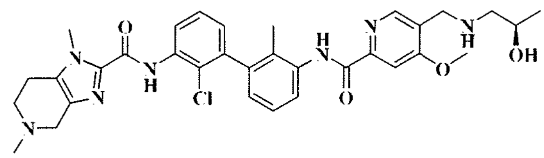

Preparation of (*S*)-*N*-(2-chloro-3'-(5-(((2-hydroxypropyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 13.
MS *m*/*z* (ESI): 632.2 $[M+H]^+$.

**Embodiment 39**

(*S*)-*N*-(2-Chloro-3'-(4-methoxy-5-((((5-carbonylpyrrolidin-2-yl)methyl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

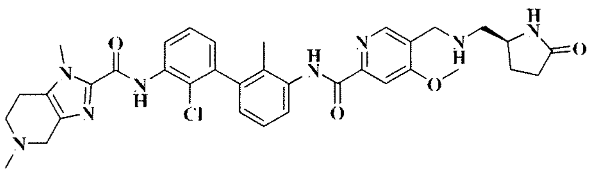

Preparation of (*S*)-*N*-(2-chloro-3'-(4-methoxy-5-((((5-carbonylpyrrolidin-2-yl)methyl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 13.
MS *m*/*z* (ESI): 671.2 $[M+H]^+$.

**Embodiment 40**

*N*-(3'-(5-(((2-Acetylaminoethyl)amino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide

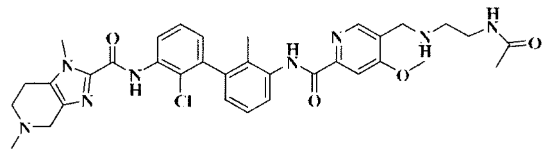

Preparation of *N*-(3'-(5-(((2-acetylaminoethyl)amino)metliyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 13.

MS *m/z* (ESI): 659.2 [M+H]+.

**Embodiment 41**

*N*-(3'-(5-((Allylamino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide

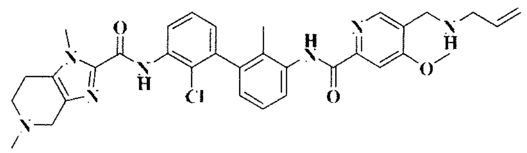

Step 1: Preparation of methyl 5-((allyl(*tert*-butoxycarbonyl)amino)methyl)-4-methoxypicolinate

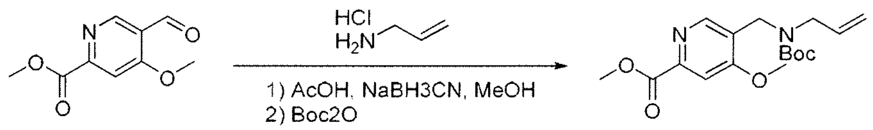


Methyl 5-formyl-4-methoxy-2-picolinate (30 mg, 0.153 mmol), allylamine hydrochloride (21 mg, 0.229 mmol), AcOH (23 mg, 0.383 mmol), $NaBH_3CN$ (28 mg, 0.459 mmol) were added to MeOH (4 mL), and the mixture was stirred at room temperature for 16 hours. DIPEA (99 mg, 0.765 mmol) and $Boc_2O$ (37 mg, 0.168 mmol) were added to the reaction mixture, and the mixture was stirred at room temperature for 1 hour. The reaction mixture was concentrated under reduced pressure, and the residue was separated and purified by silica gel column chromatography to obtain the title compound as a colorless oil (35 mg, 67 %).

MS *m/z* (ESI):337.1[M+H]+.

Step 2: Preparation of *N*-(3'-(5-((allylamino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide

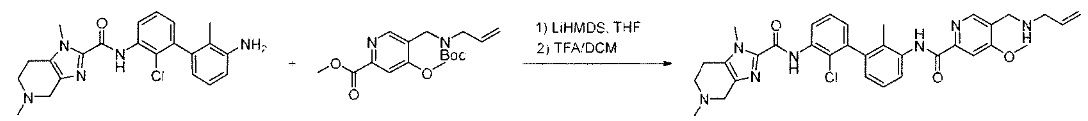

Under ice bath, LiHMDS (0.21 mL, 1.0 M in THF) was added dropwise to a solution of *N*-(3'-amino-2-chloro-2'-methyl-[1,1'-biphenyl]-3-yl)-1,5-dimethyl-4,5,6,7-tetrahydro-1*H*-imidazo[4,5-*c*]pyridine-2-carboxamide (43 mg, 0.105 mmol) and methyl 5-((allyl(*tert*-butoxycarbonyl)amino)methyl)-4-methoxypicolinate (35 mg, 0.105 mmol) in tetrahydrofuran (1 mL), then the mixture was warmed to room temperature and stirred for 16 hours. The reaction mixture was quenched with water (5 mL) and extracted with ethyl acetate (10 mL). The organic phase was washed with saturated aqueous sodium chloride solution (5 mL), dried over anhydrous sodium sulfate, then the mixture was filtered, and concentrated under reduced pressure. The residue was dissolved in dichloromethane (1 mL), then trifluoroacetic acid (0.25 mL) was added, and the mixture was stirred at room temperature for 0.5 hours. The reaction mixture was concentrated under reduced pressure, and the residue was separated and purified by prep-HPLC to obtain the title compound (3.9 mg, 6 %).
MS *m/z* (ESI):614.1[M+H]+.

**Embodiment 42**

*N*-(2-Chloro-3'-(4-methoxy-5-((prop-2-yn-1-ylamino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

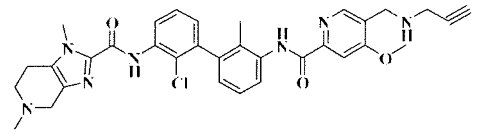

Preparation of *N*-(2-chloro-3'-(4-methoxy-5-(-ylamino)methyl)picolinamido)-2'-methyt-[1,1'-biphenyl]-3-yl)-1,5-dimethyl-4,5,6,7-tetrahydro-1*H*-imidazo[4,5-*c*]pyridine-2-carboxamide was referred to Embodiment 41.
MS *m/z* (ESI): 612.2 [M+H]+.

**Embodiment 43**

(*S*)-*N*-(2-Chloro-3'-(5-(((1-hydroxypropan-2-yl)amino)methyl)-6-methoxypicolinamido)-2'-methyl-[1,1'-biphenyl]-3-yl)-1,5-dimetbyl-4,5,6,7-tetrahydro-1*H*-imidazo[4,5-*c*]pyridine-2-carboxamide

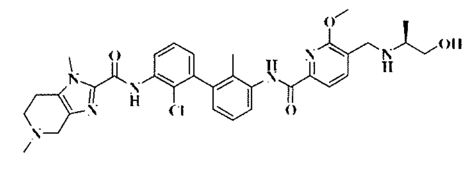

Step 1: Preparation of methyl 5-formyl-6-methoxypicolinate

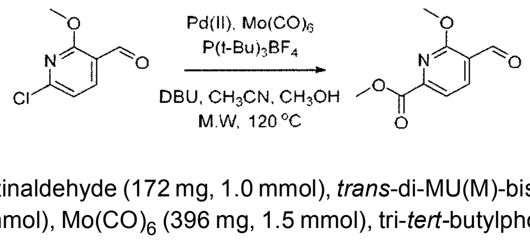


6-Chloro-2-methoxynicotinaldehyde (172 mg, 1.0 mmol), *trans*-di-MU(M)-bis[2-(di-*o*-tolylphosphino)benzyl]acetate dipalladium(II) (94 mg, 0.1 mmol), $Mo(CO)_6$ (396 mg, 1.5 mmol), tri-*tert*-butylphosphine tetrafluoroborate (116 mg, 0.4 mmol) and DBU (228 mg, 1.5 mmol) were added to a mixed solvent of acetonitrile (1 mL) and methanol (4 mL), and the reaction system was transferred to a microwave synthesizer under the protection of dry nitrogen, and heated to 120 °C and reacted for 5 hours. After the reaction was completed, the reaction mixture was concentrated under reduced pressure to remove the solvent, and the residue was separated by silica gel column chromatography to obtain the title

compound as a light yellow solid compound (28.7 mg, 15 %).

MS *m/z* (ESI): 196.1 [M+H]+.

Step 2: Preparation of methyl (*S*)-5-(((*tert*-butoxycarbonyl)(1-((*tert*-butyldimethylsilyl)oxy)propan-2-yl)amino)methyl)-6-methoxypicolinate

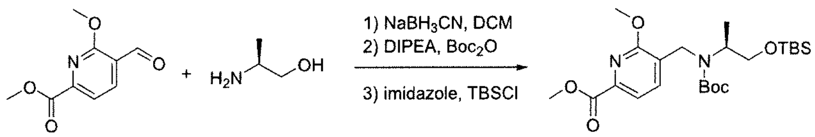


Methyl 5-formyl-6-methoxypicolinate (150 mg, 0.773 mmol) and (*S*)-2-aminopropan-1-ol (70 mg, 0.928 mmol) were dissolved in dichloromethane (10 mL), then 1 drop of acetic acid and sodium cyanoborohydride (97 mg, 1.546 mmol) were added under stirring at room temperature, then the reaction mixture was stirred and reacted at room temperature for 1 hour, and DIPEA (0.216 mL, 1.436 mmol) and $Boc_2O$ (240 mg, 1.1 mmol) were added. The reaction mixture was stirred at room temperature for 2 hours, then the reaction mixture was concentrated under reduced pressure to remove the solvent; the residue was dispersed in ethyl acetate, washed with saturated aqueous sodium chloride solution, dried over anhydrous sodium sulfate; the mixture was filtered, concentrated, and the residue was dispersed in dichloromethane (10 mL), then DIPEA (0.5 mL, 3.0 mmol), imidazole (53 mg, 0.773 mmol) and TBSCl (233 mg, 1.546 mmol) were added, and the reaction mixture was stirred and reacted at room temperature for 4 hours. The reaction mixture was concentrated under reduced pressure to remove the solvent, and the residue was separated by silica gel column chromatography to obtain the title compound (139 mg, 38 %).

MS *m/z* (ESI): 469.1 [M+H]+.

Step 3: Preparation of (*S*)-*N*-(2-chloro-3'-(5-(((1-hydroxypropan-2-yl)amino)methy1)-6-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

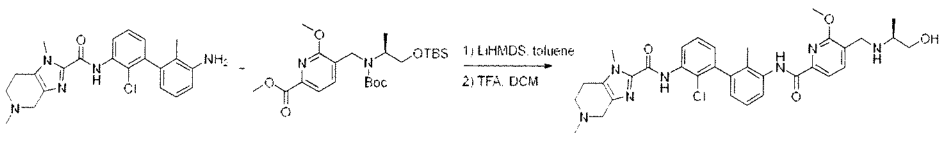


*N*-(3'-Amino-2-chloro-2'-methyl-[1,1'-biphenyl]-3-yl)-1,5-dimethyl-4,5,6,7-tetrahydro-1*H*-imidazo[4,5-*c*]pyridine-2-carboxamide (50 mg, 0.122 mmol) and methyl (*S*)-5-(((*tert*-butoxycarbonyl)(1-((*tert*-butyldimethylsilyl)oxy)propan-2-yl)amino)methyl)-6-methoxypicolinate (69 mg, 0.146 mmol) were dispersed in dry toluene (5 mL), and the reaction system was protected with dry nitrogen, and LiHMDS (0.427 mL, 0.427 mmol) was added under stirring at room temperature; the reaction mixture was diluted with ethyl acetate, washed with saturated aqueous sodium chloride solution, dried over anhydrous sodium sulfate, and then the reaction mixture was concentrated under reduced pressure. The residue was redissolved in dichloromethane (4 mL), and trifluoroacetic acid (2 mL) was added, then the reaction mixture was stirred and reacted at room temperature for 1.5 hours; the reaction mixture was concentrated under reduced pressure to remove the solvent, and the residue was separated by prep-HPLC to obtain the title compound (30.2 mg, 39 %).

MS *m/z* (ESI): 632.2 [M+H]+.

$^1H$ NMR (400 MHz, $CD_3OD$) δ 8.43 (dd, J = 8.2, 1.2 Hz, 1H), 8.03 (t, J = 8.8 Hz, 2H), 7.90 (d, J = 7.5 Hz, 1H), 7.43 (t, J = 7.9 Hz, 1H), 7.36 (t, J = 7.9 Hz, 1H), 7.08 (t, J = 7.7 Hz, 2H), 4.30 (s, 2H), 4.17 (s, 3H), 3.99 (s, 3H), 3.84 (dd, J = 11.8, 3.8 Hz, 1H), 3.77 (s, 2H), 3.60 (dd, J = 11.9, 6.5 Hz, 1H), 3.38-3.35 (m, 1H), 3.12 (t, J = 5.7 Hz, 2H), 2.93-2.85 (m, 2H), 2.69 (s, 3H), 2.15 (s, 3H), 1.36 (d, J = 6.7 Hz, 3H).

**Embodiment 44**

*N*-(2-Chloro-3'-(4-cyclopropyl-5-(((2-methoxypyridin-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

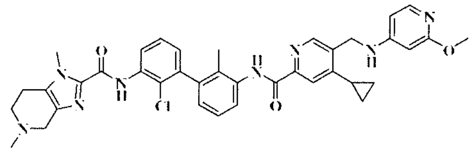

Step 1: Preparation of methyl 6-((2'-chloro-3'-(1,5-dimethyl-4,5,6,7-*tetrahydro*-1*H*-imidazo[4,S-*c*]pyridine-2-carboxamido)-2-methyl-[1,1 '-biphenyl]-3-yl)carbamoyl)-4-cyclopropylnicotinate

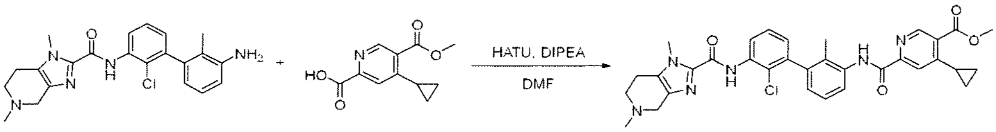


*N*-(3'-Amino-2-chloro-2'-methyl-[1,1'-biphenyl]-3-yl)-1,5-dimethyl-4,5,6,7-tetrahydro-1*H*-imidazo[4,5-c]pyridine-2-carboxamide (150 mg, 0.302 mmol), 4-cyclopropyl-5-(carbomethoxy<methoxycarbonyl>)o-picolinic acid (100 mg, 0.454 mmol) were dissolved in dry DMF (4 mL); DIPEA (0.1 mL, 0.604 mmol) and HATU (207 mg, 0.544 mmol) were successively added under stirring at room temperature, and the reaction mixture was stirred and reacted at room temperature for 5 hours, then the reaction mixture was concentrated under reduced pressure to remove the solvent; the residue was dispersed in ethyl acetate, washed with saturated aqueous sodium chloride solution, dried over anhydrous sodium sulfate, then the mixture was filtered, concentrated under reduced pressure, and the residue was directly used in the subsequent reaction.

MS *m/z* (ESI): 613.1 $[M+H]^+$.

Step 2: Preparation of *N*-(2-chloro-3'-(4-cyclopropyl-5-(hydroxymethyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

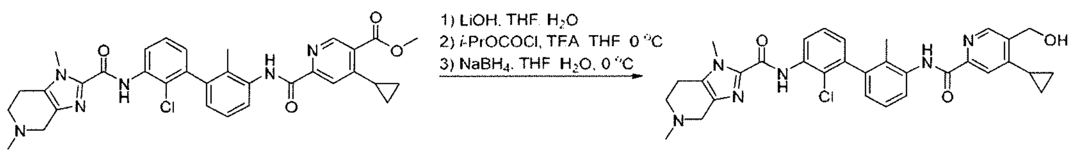


The crude product of methyl 6-((2'-chloro-3'-(1,5-dimethyl-4,5,6,7-tetrahydro-1*H*-imidazo[4,5-*c*]pyridine-2-carboxamido)-2-methyl-[1,1 '-biphenyl]-3-yl)carbamoyl)-4-cyclopropylnicotinate was dissolved in a mixed solvent of tetrahydrofuran (6 mL) and water (2 mL); $LiOH.H_2O$ (38 mg, 0.908 mmol) was added under stirring at room temperature, and the reaction mixture was stirred at room temperature for 2 hours, and the reaction mixture was concentrated under reduced pressure to remove the solvent, and the residue was dispersed in water; 0.5 N aqueous hydrochloric acid solution (2 mL) was added, and the reaction mixture was extracted with ethyl acetate, dried over anhydrous sodium sulfate, then the mixture was filtered and concentrated; the residue was dissolved in tetrahydrofuran (30 mL), cooled to 0 °C, and triethylamine (0.19 mL, 1.362 mmol) and isopropyl chloroformate (67 mg, 0.545 mmol) were added successively under stirring; the reaction mixture was continued to stir and react at 0 °C for 0.5 hours, and aqueous solution (10 mL, pre-cooled to 0 °C) of sodium borohydride (34 mg, 0.908 mmol) was added; the reaction mixture was stirred for 0.5 hours, and the reaction mixture was concentrated under reduced pressure to remove the organic solvent; the residue was extracted with ethyl acetate, washed with saturated aqueous sodium chloride solution, dried over anhydrous sodium sulfate, then the mixture was filtered and concentrated, and the residue was directly used in the next step.

MS *m/z* (ESI): 585.1 [M+H]+.

Step 3: Preparation of *N*-(2-chloro-3'-(4-cyclopropyl-5-(((2-methoxypyridin-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

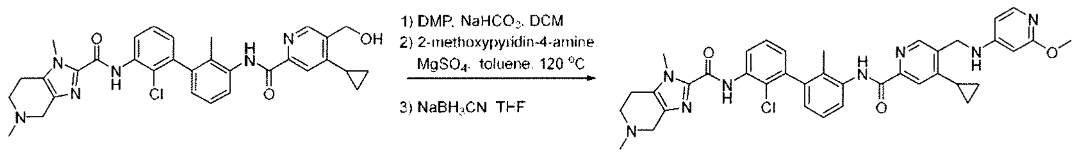


The crude product of *N*-(2-chloro-3'-(4-cyclopropyl-5-(hydroxymethyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide was dissolved in dichloromethane (10 mL), then sodium bicarbonate (254 mg, 3.02 mmol) and Dess-Martin oxidant (256 mg, 0.604 mmol) were sequentially added under stirring at room temperature; the reaction mixture was stirred and reacted at room temperature for 1.5 hours, then the reaction mixture was filtered, and the filter residue was washed with dichloromethane, and the filtrate was combined and concentrated under reduced pressure; the residue was dispersed in toluene (20 mL), and 2-methoxypyridin-4-amine (75 mg, 0.604 mmol) and anhydrous magnesium sulfate (2.0 g) were added, and the reaction system was heated to 120 °C under the protection of dry nitrogen, stirred and reacted for 16 hours, then the reaction mixture was concentrated under reduced pressure to remove the solvent; the residue was dispersed in tetrahydrofuran (20 mL), and sodium cyanoborohydride (40 mg, 0.604 mmol) was added under stirring at room temperature, and the reaction mixture was stirred and reacted at room temperature for 0.5 hours, reaction mixtureconcentrated under reduced pressure to remove the solvent. The residue was dispersed in ethyl acetate, washed with saturated aqueous sodium chloride solution, dried over anhydrous sodium sulfate, and then the mixture was filtered and concentrated. The residue was dissolved in dichloromethane (2 mL), and trifluoroacetic acid (2 mL) was added, then the reaction mixture was stirred and reacted at room temperature for 0.5 hours, reaction mixtureconcentrated under reduced pressure to remove the solvent; the residue was dispersed in tetrahydrofuran (6 mL), and 37 % w/w aqueous formaldehyde solution (2 mL) was added, then sodium cyanoborohydride (40 mg, 0.604 mmol) was added to the reaction mixture under stirring at room temperature; the reaction was continued to stir at room temperature for 0.5 hours, and the reaction mixture was concentrated under reduced pressure to remove the solvent. The residue was separated by prep-HPLC to obtain the title compound as a white solid compound (17 mg, 8 %).

MS *m/z* (ESI): 691.2 [M+H]+.

$^1$H NMR (400 MHz, $CD_3OD$) $\delta$ 8.51 (s, 1H), 8.42 (dd, J = 8.3, 1.1 Hz, 1H), 7.94 (d, J = 7.7 Hz, 1H), 7.78 (s, 1H), 7.67 (d, J = 6.3 Hz, 1H), 7.42 (t, J = 7.9 Hz, 1H), 7.33 (t, J = 7.8 Hz, 1H), 7.08 (dd, J = 7.7, 1.0 Hz, 1H), 7.04 (d, J = 7.4 Hz, 1H), 6.37 (dd, J = 6.2, 1.6 Hz, 1H), 5.99 (d, J = 1.5 Hz, 1H), 4.67 (s, 2H), 3.99 (s, 3H), 3.83 (s, 3H), 3.80 (s, 2H), 3.15 (t, J = 5.8 Hz, 2H), 2.90 (t, J = 6.0 Hz, 2H), 2.71 (s, 3H), 2.20-2.13 (m, 1H), 2.09 (s, 3H), 1.24-1.16 (m, 2H), 0.97-0.90 (m, 2H).

**Embodiment 45**

*N*-(2-Chloro-3'-(4-(cyanomethoxy)-5-(((2-methoxypyridin-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

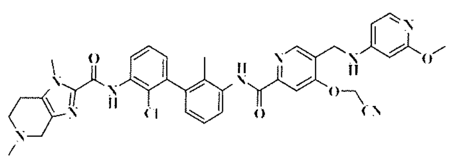

Preparation of *N*-(2-chloro-3'-(4-(cyanometlioxy)-5-(((2-methoxypyridin-4-yl)amino)methyl)picolinamido)-2'-methyl-[1,1'-biphenyl]-3-yl)-1,5-dimethyl-4,S,6,7-tetrahydro-1*H*-imidazo[4,5-*c*]pyridine-2-carboxamide was referred to Embodiment 44.

MS *m/z* (ESI): 706.2 [M+H]+.

**Embodiment 46**

*N*-(2-Chloro-3'-(6-methoxy-5-(((2-methoxypyridin-4-yl)amino)methyl)pyrazine-2-carboxamido)-2'-methyl-[1,1'-biphenyl]-3-yl)-1,5-dimethyl-4,5,6,7-tetrahydro-1*H*-imidazo[4,5-*c*]pyridine-2-carboxamide

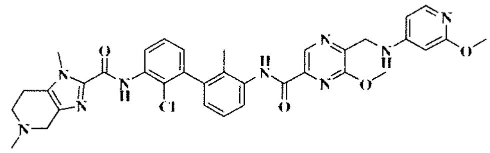

Preparation of *N*-(2-chloro-3'-(6-methoxy-5-(((2-methoxypyridin-4-yl)amino)methyl)pyrazine-2-carboxamido)-2'-methyl-[1,1'-biphenyl]-3-yl)-1,5-dimethy1-4,5,6,7-tetrahydro-1*H*-imidazo[4,5-*c*]pyridine-2-carboxamide was referred to Embodiment 43.

MS *m/z* (ESI): 682.2 [M+H]+.

**Embodiment 47**

*N*-(2-Chloro-3'-(5-methoxy-6-(((2-methoxypyridin-4-yl)amino)methyl)pyridazine-3-carboxamido)-2'-methyl-[1,1'-biphenyl]-3-yl)-1,5-dimethyl-4,5,6,7-tetrahydro-1*H*-imidazo[4,5-*c*]pyridine-2-carboxamide

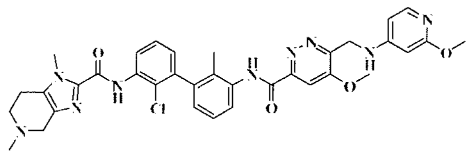

Preparation of *N*-(2-chloro-3'-(5-methoxy-6-(((2-methoxypyridin-4-yl)amino)methyl)pyridazine-3-carboxamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 43.

MS *m/z* (ESI): 682.2 [M+H]+.

**Embodiment 48**

*N*-(2-Chloro-3'-(4,6-dimethoxy-5-(((tetrahydro-2*H*-pyran-4-yl)amino)methyl)pyrimidine-2-carboxamido)-2'-methyl-[1,1'-biphenyl]-3-yl)-1,5-dimethyl-4,5,6,7-tetrahydro-1*H*-imidazo[4,5-*c*]pyridine-2-carboxamide

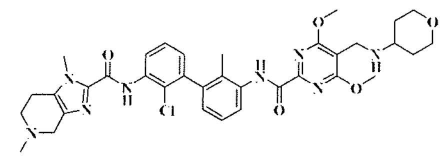

Preparation of *N*-(2-chloro-3'-(4,6-dimethoxy-5-(((tetrahydro-2*H*-pyran-4-yl)amino)methyl)pyrimidine-2-carboxamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 43.

MS *m/z* (ESI): 689.2 [M+H]+.

**Embodiment 49**

*N*-(2-Chloro-3'-(5-(2-hydroxymethyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

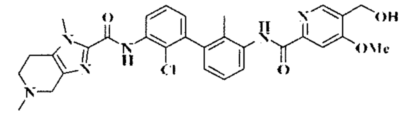

*N*-(3'-Amino-2-chloro-2'-methyl-[1,1'-biphenyl]-3-yl)-1,5-dimethyl-4,5,6,7-tetrahydro-1*H*-imidazo[4,5-*c*]pyridine-2-carboxamide (200 mg, 0.489 mmol) and methyl 4-methoxy-5-vinylpicolinate (142 mg, 0.733 mmol) were dispersed in toluene (10 mL), and the reaction system was protected with dry nitrogen, and LiHMDS (2.2 mL, 2.2 mmol) was added dropwise under stirring at room temperature; the reaction mixture was continued to stir and react at room temperature for 24 hours. The reaction mixture was diluted with ethyl acetate, washed with saturated aqueous sodium chloride solution, dried over anhydrous sodium sulfate, then the mixture was filtered, and concentrated. The crude product was dissolved in a mixed solution of dioxane (20 mL) and water (8 mL), and then the solution was cooled in an ice-water bath to 0 °C; $K_2O_2O_4.2H_2O$ (18 mg, 0.049 mmol) and sodium periodate (524 mg, 2.45 mmol) were added with stirring, and the reaction mixture was stirred and reacted for 8 hours, then the reaction mixture was extracted with ethyl acetate, washed with saturated aqueous sodium chloride solution, dried over sodium sulfate, then the mixture was filtered, and concentrated under reduced pressure. The residue was dissolved in tetrahydrofuran, and sodium cyanoborohydride (308 mg, 4.89 mmol) was added under stirring at room temperature; the reaction was continued to stir and react at room temperature for 14 hours, and then the reaction mixture was concentrated under reduced pressure to remove the solvent. The residue was separated by prep-HPLC to obtain the title compound as a white solid (9.3 mg, 3 %).

MS *m/z* (ESI): 575.2 $[M+H]^+$.

$^1$H NMR (400 MHz, $CD_3OD$) δ 8.54 (s, 1H), 8.43 (dd, J = 8.0, 0.8 Hz, 1H), 8.36 (s, 1H), 7.98 (d, J = 8.3 Hz, 1H), 7.83 (s, 1H), 7.45-7.40 (m, 1H), 7.34 (t, J = 7.6 Hz, 1H), 7.10 (dd, J = 7.4, 1.2 Hz, 1H), 7.05 (d, J = 6.9 Hz, 1H), 4.72 (s, 2H), 4.02 (s, 3H), 4.00 (s, 3H), 3.87 (s, 2H), 3.24-3.18 (m, 2H), 2.95-2.90 (m, 2H), 2.76 (s, 3H), 2.12 (s, 3H).

**Embodiment 50**

*N*-(2-Chloro-3'-(5-(2-hydroxypropan-2-yl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

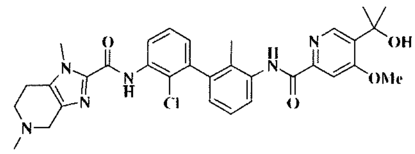

Step 1: Preparation of methyl 4-methoxy-5-(prop-1-en-2-yl)picolinate

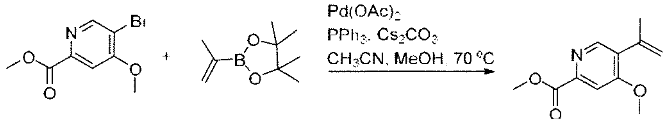


Methyl 5-bromo-4-methoxypicolinate (500 mg, 2.04 mmol), 4,4,5,5-tetramethyl-2-(prop-1-en-2-yl)-1,3,2-dioxaborolane (515 mg, 3.06 mmol), palladium acetate (45 mg, 0.2 mmol), triphenylphosphine (105 mg, 0.4 mmol) and potassium carbonate (564 mg, 4.08 mmol) were added to a mixed solvent of acetonitrile (20 mL) and methanol (10 mL), and the reaction system was vacuumized and protected with dry nitrogen, then the reaction system was heated to 70 °C, stirred and reacted for 16.5 hours; the reaction mixture was concentrated under reduced pressure to remove the

organic solvent, and the residue was dispersed in ethyl acetate, then washed with saturated aqueous sodium chloride solution, dried over anhydrous sodium sulfate, then the mixture was filtered, concentrated, and the residue was separated by silica gel column chromatography to obtain the title compound (185 mg, 44 %).

MS *m/z* (ESI): 208.2 $[M+H]^+$.

Step 2: Preparation of *N*-(2-chloro-3'-(4-methoxy-5-(prop-1-en-2-yl)picolinamido)-2'-methyl-[1,1'-biphenyl]-3-yl)-1 ,5-dimethyl-4,5,6,7-tetrahydro-1*H*-imidazo[4,5-*c*]pyridine-2-carboxamide

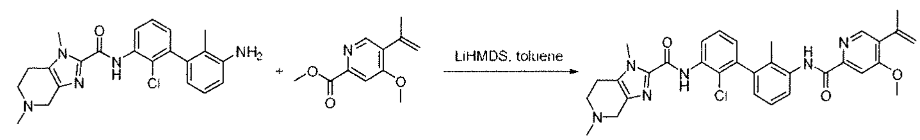


*N*-(3'-Amino-2-chloro-2'-methyl-[1,1'-biphenyl]-3-yl)-1,5-dimethyl-4,5,6,7-tetrahydro-1*H*-imidazo[4,5-*c*]pyridine-2-carboxamide (100 mg, 0.244 mmol) and methyl 4-methoxy-5-(prop-1-en-2-yl)picolinate (185 mg, 0.893 mmol) were dispersed in dry toluene (5 mL), and the reaction system was protected with dry nitrogen, and LiHMDS (1.0 mL, 0.976 mmol) was added; the reaction mixture was continued to stir and react at room temperature for 24 hours, and the reaction mixture was diluted with ethyl acetate, washed with saturated aqueous sodium chloride solution, dried over anhydrous sodium sulfate; after filtration and concentration, the residue was directly used in the next step.

MS *m/z* (ESI): 585.1 $[M+H]^+$.

Step 3: Preparation of *N*-(2-chloro-3'-(5-(2-hydroxypropan-2-yl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

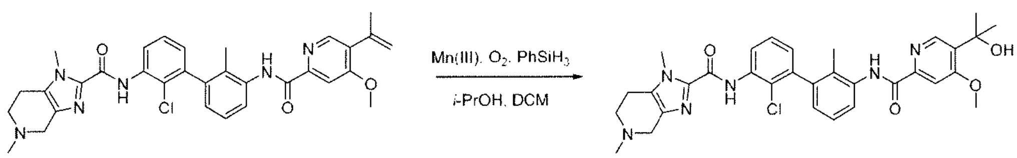


*N*-(2-Chloro-3'-(4-methoxy-5-(prop-1-en-2-yl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide (168 mg, 0.244 mmol) was dissolved in a mixed solvent of isopropanol (10 mL) and dichloromethane (2 mL), then the reaction mixture was cooled to 0 °C in an ice-water bath; the reaction system was replaced with oxygen, and tris(2,2,6,6-tetramethyl-3,5-heptenoic acid) manganese (30 mg, 0.049 mmol) and phenylsilane (53 mg, 0.49 mmol) were added successively, the resulting reaction mixture was further stirred and reacted at 0 °C for 6 hours, then the reaction mixture was concentrated under reduced pressure to remove the solvent; the residue was separated by silica gel column chromatography and prep-HPLC successively to obtain the title product as a white solid compound (20.3 mg, 14 %).

MS *m/z* (ESI): 603.1 $[M+H]^+$.

$^1H$ NMR (400 MHz, $CD_3OD$) δ 8.74 (s, 1H), 8.43 (dd, J = 8.3, 1.3 Hz, 1H), 8.00-7.95 (m, 1H), 7.83 (s, 1H), 7.45-7.38 (m, 1H), 7.34 (t, J = 7.8 Hz, 1H), 7.08 (dd, J = 7.6, 1.4 Hz, 1H), 7.05 (d, J = 7.2 Hz, 1H), 4.03 (s, 3H), 3.98 (s, 3H), 3.59 (s, 2H), 2.94 (t, J = 5.8 Hz, 2H), 2.82 (t, J = 5.8 Hz, 2H), 2.57 (s, 3H), 2.12 (s, 3H), 1.61 (s, 6H).

**Embodiment 51**

*N*-(2-Chloro-3'-(4-cyclopropyl-5-(hydroxymethyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

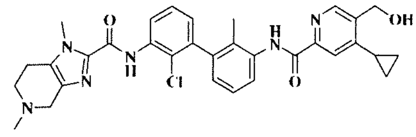

Preparation of *N*-(2-chloro-3'-(4-cyclopropy)-5-(hydroxymethyl)picolinamido)-2'-methyl-1,1'-biphenyl]-3-yl)-1,5-dimethyl-4,5,6,7-tetrahydro-1*H*-imidazo[4,5-c]pyridine-2-carboxamide was referred to Embodiment 49.

MS *m/z* (ESI): 585.1 $[M+H]^+$.

$^1H$ NMR (400 MHz, $CD_3OD$) δ 8.60 (s, 1H), 8.45-8.41 (m, 1H), 7.95 (d, J = 7.9 Hz, 1H), 7.72 (s, 1H), 7.42 (t, J = 7.9 Hz, 1H), 7.34 (t, J = 7.8 Hz, 1H), 7.09 (dd, J = 7.5, 1.3 Hz, 1H), 7.05 (d, J = 7.8 Hz, 1H), 4.91 (s, 2H), 3.99 (s, 3H), 3.85 (s, 2H), 3.20 (t, J = 5.7 Hz, 2H), 2.92 (t, J = 5.8 Hz, 2H), 2.75 (s, 3H), 2.23-2.16 (m, 1H), 2.11 (s, 3H), 1.19 (dt, J = 6.3, 4.5 Hz, 2H), 0.90 (dt, J = 6.6, 5.0 Hz, 2H).

**Embodiment 52**

*N*-(2-Chloro-3'-(4-(cyanomethoxy)-5-(2-hydroxypropan-2-yl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

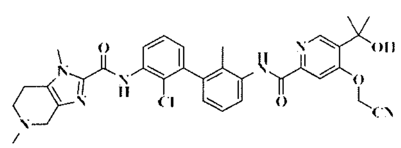

Preparation of *N*-(2-chloro-3'-(4-(cyanomethoxy)-5-(2-hydroxypropan-2-yl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 50.

MS *m/z* (ESI): 628.2 $[M+H]^+$.

**Embodiment 53**

*N*-(2-Chloro-3'-(4-methoxy-5-(((2-methylpyridin-4-yl)oxy)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

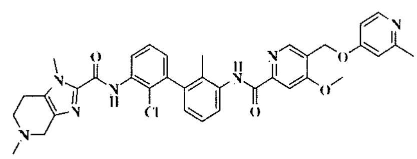

Preparation of *N*-(2-chloro-3'-(4-methoxy-5-(((2-methylpyridin-4-yl)oxy)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS *m/z* (ESI): 666.2 $[M+H]^+$.

**Embodiment 54**

*N*-(2-Chloro-3'-(5-(((2,6-dimethylpyridin-4-yl)oxy)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

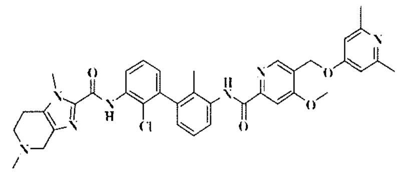

Preparation of *N*-(2-chloro-3'-(5-(((2,6-dimethylpyridin-4-yl)oxy)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS *m*/*z* (ESI): 680.2 $[M+H]^+$.

**Embodiment 55**

*N*-(2,2'-Dichloro-3'-(6-methoxy-S-(2-(methylamino)propan-2-yl)pyrazine-2-carboxamido-[1,1'-biphenyl]-3-yl)-1,5-dimetliyl-4,5,6,7-tetrahydro-1*H*-imidazo[4,5-*c*]pyridine-2-carboxamide

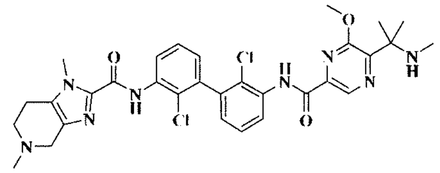

Preparation of *N*-(2,2'-dichloro-3'-(6-methoxy-5-(2-(methylamino)propan-2-yl)pyrazine-2-carboxamido)-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS *m*/*z* (ESI): 637.2 $[M+H]^+$.

**Embodiment 56**

(*S*)-*N*-(2,2'-Dichloro-3'-(4-methoxy-5-((((5-carbonylpyrrolidin-2-yl)methyl)amino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

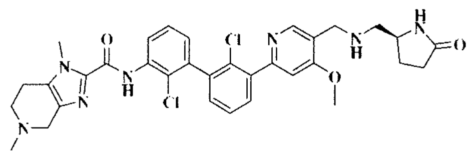

Step 1: Preparation of (3-bromo-2-chlorophenyl) boronic acid

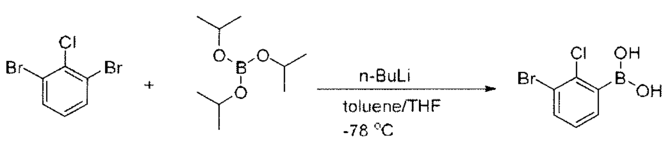


1,3-Dibromo-2-chlorobenzene (2.7 g, 10 mmol) and triisopropyl borate (2.3 g, 12 mmol) were added to a mixed solvent of tetrahydrofuran (7 mL) and toluene (28 mL); under the protection of nitrogen, a solution of 1.6 M *n*-butyllithium in *n*-hexane (7.5 mL, 12 mmol) was added dropwise at -78 °C, and the reaction mixture was continued to stir at this temperature for 2 hours, gradually warmed to room temperature, and continued to for 2 hours. After the reaction was

quenched with 2 M hydrochloric acid solution, the reaction mixture was continued to stir at room temperature for 30 minutes. Ethyl acetate was added to the mixture for extraction, and the organic phase was dried over anhydrous sodium sulfate, then the mixture was filtered, and the filtrate was concentrated to obtain the title compound as a crude product, which was directly used in the next step.

Step 2: Preparation of 6-(3-bromo-2-chlorophenyl)-4-methoxynicotinaldehyde

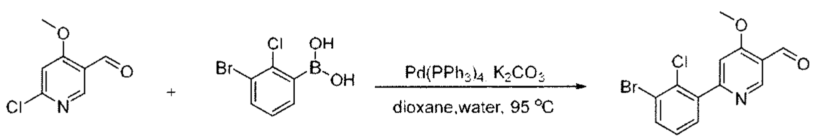


6-Chloro-4-methoxynicotinaldehyde (654 mg, 3.83 mmol), (3-bromo-2-chlorophenyl)boronic acid (900 mg, 3.83 mmol), $Pd(PPh_3)_4$ (266 mg, 0.23 mmol) and potassium carbonate (1.06 g, 7.66 mmol) were dissolved in a mixed solvent of dioxane (20 mL) and water (2 mL), then the reaction system was vacuumized and replaced with nitrogen, and after nitrogen protection, the reaction mixture was heated to 95 °C and stirred overnight. After the reaction was completed, the reaction mixture was cooled, filtered, and the filtrate was concentrated, and the residue was separated by flash silica gel column chromatography to obtain the title compound (520 mg, 40 %).
MS *m/z* (ESI): 326.2 $[M+H]^+$.

Step 3: Preparation of 6-(2-chloro-3-(4,4,5,5-tetramethyl-1,3,2-dioxaborolan-2-yl)phenyl)-4-methoxynicotinaldehyde

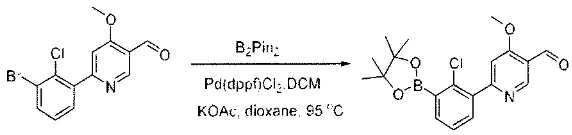


6-(3-Bromo-2-chlorophenyl)-4-methoxynicotinaldehyde (520 mg, 1.59 mmol), bis(pinacolato)diboron (484 mg, 1.91 mol), complex (132 mg, 0.16 mmol) of $Pd(dppf)Cl_2$ and DCM, and potassium acetate (467 mg, 4.77 mmol) were dissolved in dioxane (15 mL), and the reaction system was vacuumized and replaced with nitrogen; under the protection of nitrogen, the reaction mixture was heated and stirred at 95 °C overnight. After the reaction was completed, the reaction mixture was cooled and concentrated. The residue was separated by flash silica gel column chromatography to obtain the title compound (460 mg, 77 %).
MS *m/z* (ESI): 375.2 $[M+H]^+$.

Step 4: *N*-(2,2'-dichloro-3'-(5-fonnyl-4-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

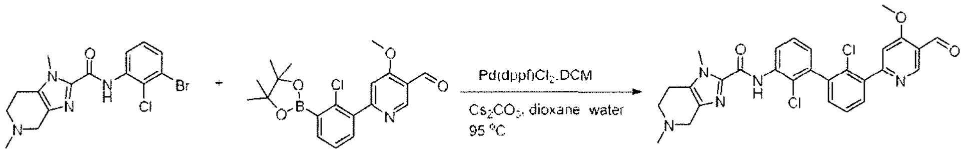


*N*-(3-Bromo-2-chlorophenyl)-1,5-dimethyl-4,5,6,7-tetraliydro-1*H*-imidazo[4,5-*c*]pyridine-2-carboxamide (342 mg, 0.898 mmol), 6-(2-chloro-3-(4,4,5,5-tetramethyl-1,3,2-dioxaborolan-2-yl)phenyl)-4-methoxynicotinaldehyde (420 mg, 1.12 mol), complex (91 mg, 0.112 mmol) of $Pd(dppf)Cl_2$ and DCM, and cesium carbonate (728 mg, 2.24 mmol) were dissolved in a mixed solvent of dioxane (10 mL) and water (1.5 mL), and the reaction system was vacuumized and replaced with nitrogen; under the protection of nitrogen, the reaction mixture was heated and stirred at 100 °C overnight. After the reaction was completed, the reaction mixture was cooled and concentrated. The residue was separated by

reverse phase to obtain the title compound (110 mg, 23 %).

MS *m/z* (ESI): 550.2 [M+H]+.

Step 5: (*S*)-*N*-(2,2'-dichloro-3'-(4-methoxy-5-((((5-carbonylpyrrolidin-2-yl)methyl)amino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

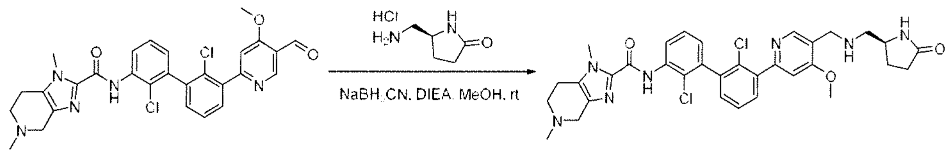


*N*-(2,2'-Dichloro-3'-(5-formyl-4-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (30 mg, 0.0545 mmol) and (*S*)-5-(aminomethyl)pyrrolidin-2-one hydrochloric acid (8.2 mg, 0.545 mmol) were dissolved in methanol (3 mL), and 2 drops of DIPEA were added, and the reaction mixture was stirred for 3 minutes, then 3 drops of acetic acid were added, and the mixture was stirred at room temperature for 3 hours. After sodium cyanoborohydride (6.7 mg, 0.109 mmol) was added, the reaction mixture was continued to stir at room temperature overnight. After the reaction was completed, the reaction mixture was concentrated, and the residue was purified by prep-HPLC to obtain the title compound (6.8 mg, 19 %).

MS *m/z* (ESI): 648.2 [M+H]+.

**Embodiment 57**

*N*-(2,2'-Dichloro-3'-(4-methoxy-5-(((tetrahydro-2*H*-pyran-4-yl)amino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

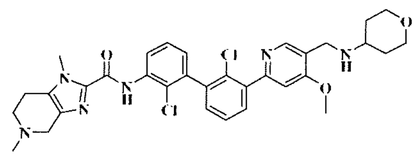

Preparation of *N*-(2,2'-dichloro-3'-(4-methoxy-5-(((tetrahydro-2*H*-pyran-4-yl)amino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 56.

MS *m/z* (ESI): 635.2 [M+H]+.

**Embodiment 58**

(*S*)-*N*-(2-Chloro-3'-(4-methoxy-5-((((5-carbonylpyrrolidin-2-yl)methyl)amino)methyl)pyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

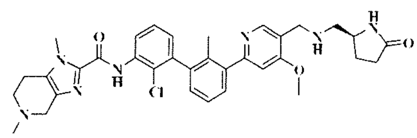

Step 1: Preparation of (3-bromo-2-methylphenyl)boronic acid

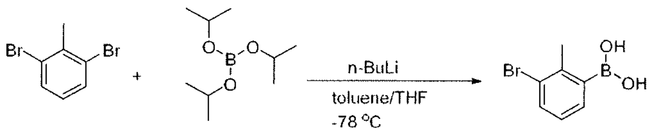


1,3-Dibromo-2-methylbenzene (2 g, 8 mmol) and triisopropyl borate (1.8 g, 8 mmol) were added to a mixed solvent of tetrahydrofuran (7 mL) and toluene (28 mL), under the protection of nitrogen, a solution of 1.6 M *n*-butyllithium in *n*-hexane (6 mL, 9.6 mmol) was added dropwise at -78 °C; the reaction mixture was continued to stir at this temperature for 2 hours, gradually warmed to room temperature, and then continued to stir for 2 hours. After the reaction was quenched with 2 M hydrochloric acid solution, the reaction mixture was continued to stir at room temperature for 30 minutes. Ethyl acetate was added to the mixture for extraction, and the organic phase was dried over anhydrous sodium sulfate, then the mixture was filtered, and the filtrate was concentrated to obtain the title compound as a crude product, which was directly used in the next step.

Step 2: Preparation of 6-(3-bromo-2-methylphenyl)-4-methoxynicotinaldehyde

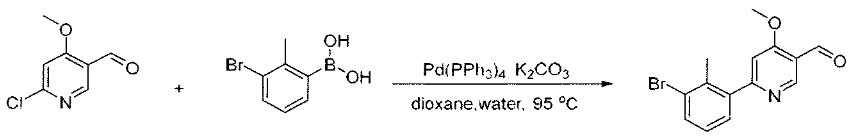


6-Chloro-4-methoxynicotinaldehyde (820 mg, 4.79 mmol), (3-bromo-2-methylphenyl)boronic acid (1.23 g, 5.75 mmol), $Pd(PPh_3)_4$ (0.55 g, 0.48 mmol) and potassium carbonate (1.32 g, 9.58 mmol) were dissolved in a mixed solvent of dioxane (20 mL) and water (2 mL), then the reaction system was vacuumized and replaced with nitrogen, and after nitrogen protection, the reaction mixture was heated to 95 °C and stirred overnight. After the reaction was completed, the reaction mixture was cooled, filtered, and the filtrate was concentrated, and then the residue was separated by flash silica gel column chromatography to obtain the title compound (760 mg, 52 %).

MS *m/z* (ESI): 305.2 $[M+H]^+$.

Step 3: Preparation of 4-methoxy-6-(2-methyl-3-(4,4,5,5-tetramethyl-1,3,2-dioxaborolan-2-yl)phenyl)nicotinealdehyde

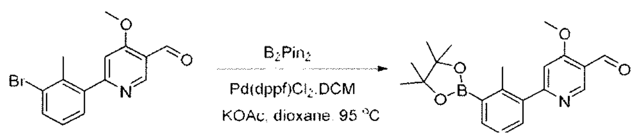


6-(3-Bromo-2-methylphenyl)-4-methoxynicotinaldehyde (760 mg, 2.48 mmol), bis(pinacolato)diboron (757 mg, 2.98 mol), complex (207 mg, 0.25 mmol) of $Pd(dppf)Cl_2$ and DCM, and potassium acetate (729 mg, 7.44 mmol) were dissolved in dioxane (20 mL), and the reaction system was vacuumized and replaced with nitrogen, under the protection of nitrogen, the reaction mixture was heated and stirred at 95 °C overnight. After the reaction was completed, the reaction mixture was cooled and concentrated. The residue was separated by flash silica gel column chromatography to obtain the title compound (750 mg, 86 %).

MS *m/z* (ESI): 305.2 $[M+H]^+$.

Step 4: Preparation of *N*-(2-chloro-3'-(5-formyl-4-methoxypyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

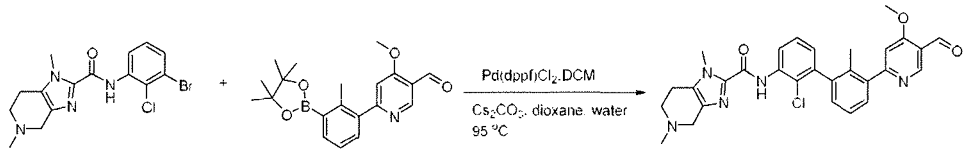


*N*-(3-Bromo-2-chlorophenyl)-1,5-dimethyl-4,5,6,7-tetrahydro-1*H*-imidazo[4,5-*c*]pyridine-2-carboxamide (676 mg, 1.77 mmol), 4-methoxy-6-(2-methyl-3-(4,4,5,5-tetramethyl-1,3,2-dioxaborolan-2-yl)phenyl)nicotinaldehyde (750 mg, 2.12 mol), complex (145 mg, 0.177 mmol) of $Pd(dppf)Cl_2$ and DCM, and cesium carbonate (1.15 g, 3.54 mmol) were dissolved in a mixed solvent of dioxane (25 mL) and water (2 mL), and the reaction system was vacuumized and replaced with nitrogen, under the protection of nitrogen, the reaction mixture was heated and stirred at 95 °C overnight. After the reaction was completed, the reaction mixture was cooled and concentrated. The residue was separated by flash silica gel column chromatography to obtain the title compound (560 mg, 60 %).

MS *m*/*z* (ESI): 529.2 $[M+H]^+$.

Step 5: Preparation of (*S*)-*N*-(2-chloro-3'-(4-methoxy-5-((((5-carbonylpyrrolidin-2-yl)methyl)amino)methyl)pyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

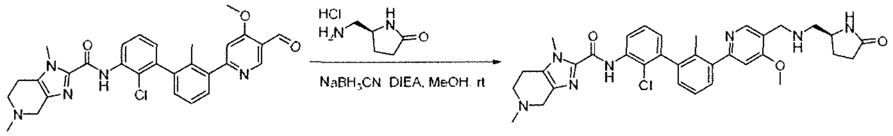


*N*-(2-Chloro-3'-(5-formyl-4-methoxypyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide (40 mg, 0.075 mmol) and (*S*)-5-(aminomethyl)pyrrolidin-2-one hydrochloric acid (18 mg, 0.12 mmol) were dissolved in methanol (2 mL), after DIPEA (15.4 mg, 0.12 mmol) was added, 3 drops of acetic acid were added, and the mixture was stirred at room temperature for 1 hour. After sodium cyanoborohydride (11.6 mg, 0.189 mmol) was added, the reaction mixture was continued to stir at room temperature overnight. After the reaction was completed, the reaction mixture was concentrated, and the residue was purified by reverse phase to obtain the title compound (10.8 mg, 23 %).

MS *m*/*z* (ESI): 628.2 $[M+H]^+$.

**Embodiment 59**

*N*-(2-Chloro-3'-(4-methoxy-5-(((tetrahydro-2*H*-pyran-4-yl)amino)methyl)pyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

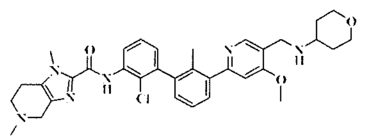

Preparation of *N*-(2-chloro-3'-(4-methoxy-5-(((tetrahydro-2*H*-pyran-4-yl)amino)methyl)pyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 58.

MS *m*/*z* (ESI): 615.2 $[M+H]^+$.

**Embodiment 60**

(*R*)-*N*-(2-Chloro-3'-(5-((3-fluoropyrrolidin-1-yl)methyl)-4-methoxypyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

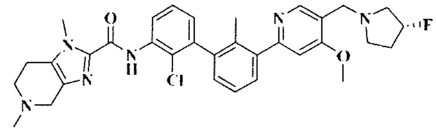

Preparation of (*R*)-*N*-(2-chloro-3'-(5-((3-fluoropyrrolidin-1-yl)methyl)-4-methoxypyridin-2-yl)-2'-methyl-[1,1'-biphenyl]-3-yl)-1,5-dimethyl-4,5,6,7-tetrahydro-1*H* imidazo[4,5-*c*]pyridine-2-carboxamide was referred to Embodiment 58.
MS *m*/*z* (ESI): 603.2 $[M+H]^+$.

**Embodiment 61**

*N*-(2-Chloro-3'-(4-methoxy-5-(((2-methoxypyridin-4-yl)amino)methyl)pyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

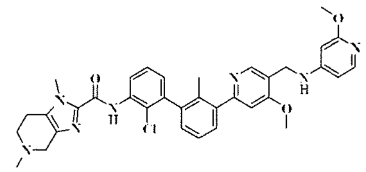

Preparation of *N*-(2-chloro-3'-(4-methoxy-5-(((2-methoxypyridin-4-yl)amino)methyl)pyridin-2-yl)-2'-methyl-[1,1'-biphenyl)-3-yl)-1,5-dimethyl-4,5,6,7-tetrahydro-1*H*-imidazo[4,5-*c*]pyridine-2-carboxamide was referred to Embodiment 58.
MS *m*/*z* (ESI): 638.2 $[M+H]^+$.

**Embodiment 62**

(*S*)-*N*-(2,2'-dichloro-3'-(6-methoxy-5-((((5-carbonylpyrrolidin-2-yl)methyl)amino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

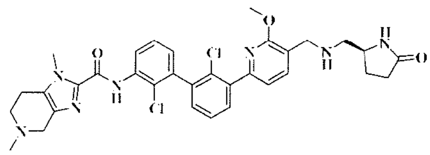

Preparation of (*S*)-*N*-(2,2'-dichloro-3'-(6-methoxy-5-((((5-carbonylpyrrolidin-2-yl)methyl)amino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 56.
MS *m*/*z* (ESI): 648.2 $[M+H]^+$.

**Embodiment 63**

(*R*)-*N*-(2,2'-Dichloro-3'-(5-((3-fluoropyrrolidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

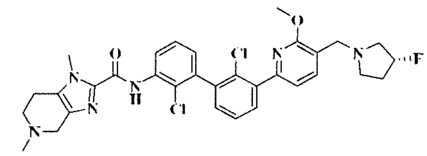

Preparation of (*R*)-*N*-(2,2'-dichloro-3'-(5-((3-fluoropyrrolidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 56.

MS *m*/*z* (ESI): 623.2 $[M+H]^+$.

**Embodiment 64**

*N*-(2,2'-Dichloro-3'-(6-methoxy-S-(((2-methoxypyridin-4-yl)amino)methyl)pyridin-2-yl)-[1,1'-biphenyl]-3-yl)-1,5-dimethyl-4,5,6,7-tetrahydro-1H-imidazo[4,5-*c*]pyridine-2-carboxamide

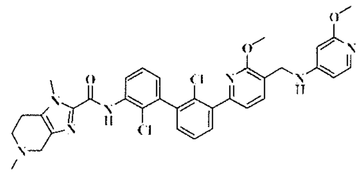

Preparation of *N*-(2,2'-dichloro-3'-(6-methoxy-5-(((2-methoxypyridin-4-yl)amino)methyl)pyridin-2-yl)-[1,1'-biphenyl]-3-yl)-1,5-dimethyl-4,5,6,7-tetraliydro-1*H*-imidazo[4,5-*c*]pyridine-2-carboxamide was referred to Embodiment 56.

MS *m*/*z* (ESI): 658.2 $[M+H]^+$.

**Embodiment 65**

*N*-(2,2'-Dichloro-3'-(5-(((3-fluoro-2-methylpyridin-4-yl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

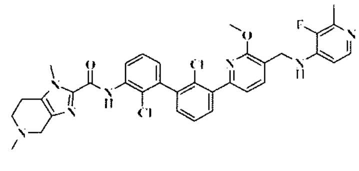

Preparation of *N*-(2,2'-dichloro-3'-(5-(((3-fluoro-2-methylpyridin-4-yl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 56.

MS *m*/*z* (ESI): 660.2 $[M+H]^+$.

### Embodiment 66

(*R*)-*N*-(2-Chloro-3'-(5-((3-fluoropyrrolidin-1-yl)methyl)-6-methoxypyridin-2-yl)-2'-methyl-[1,1'-biphenyl]-3-yl)-1,5-dimethyl-4,5,6,7-tetrahydro-1*H*-imidazo[4,5-c]pyridine-2-carboxamide

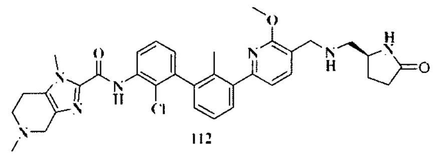

112

Preparation of (*R*)-*N*-(2-chloro-3'-(5-((3-fluoropyrrolidin-1-yl)methyl)-6-methoxypyridin-2-yl)-2'-methyl-[1,1'-biphenyl]-3-yl)-1 ,5-dimethyl-4,5,6,7-tetrahydro-1*H*-imidazo[4,5-*c*]pyridine-2-carboxamide was referred to Embodiment 56.

MS *m*/*z* (ESI): 603.2 $[M+H]^+$.

### Embodiment 67

*N*-(2-Chloro-3'-(5-(((3-fluoro-2-methylpyridin-4-yl)amino)methyl)-6-methoxypyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

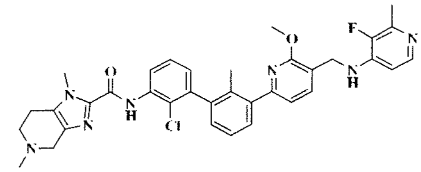

Preparation of *N*-(2-chloro-3'-(5-(((3-fluoro-2-metliylpyridin-4-yl)amino)methyl)-6-methoxypyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 56.

MS *m*/*z* (ESI): 640.2 $[M+H]^+$.

### Embodiment 68

*N*-(2-Chloro-3'-(4-methoxy-5-(((2-methoxypyridin-4-yl)amino)methyl)picolinamido)-2'-methyl-[1,1'-biphenyl]-3-yl)-1-methyl-4,5,6,7-tetrahydro-1*H*-imidazo[4,5-c]pyridine-2-carboxamide

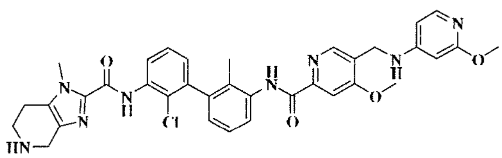

Step 1: Preparation of *tert*-butyl 2-((3'-amino-2-chloro-2'-methyl-[1,1'-biphenyl]-3-yl)carbamoyl)-1-methyl-1,4,6,7-tetrahydro-5*H*-imidazo[4,5-*c*]pyridine-5-carboxylate

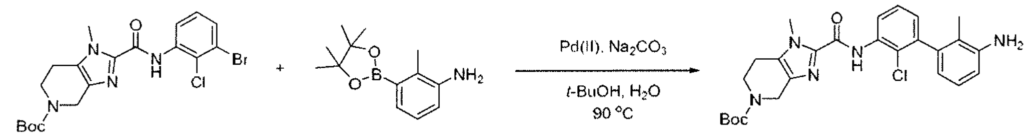


*tert*-Butyl 2-((3-bromo-2-chlorophenyl)carbamoyl)-1-methyl-1,4,6,7-tetrahydro-5*H*-imidazo[4,5-*c*]pyridine-5-carboxylate (500 mg, 1.06 mmol), 2-methyl-3-(4,4,5,5-tetramethyl-1,3,2-dioxaborolan-2-yl)aniline (298 mg, 1.28 mmol), biscyclohexylphosphine palladium dichloride (173 mg, 0.212 mmol) and sodium carbonate (337 mg, 3.18 mmol) were added to a mixed solvent of *tert*-butanol (20 mL) and water (2 mL); the reaction system was protected by nitrogen, heated to 90 °C and stirred for 16 hours, then the reaction mixture was concentrated under reduced pressure to remove the solvent, and the residue was dispersed in dichloromethane; the organic phase was washed with saturated aqueous sodium chloride solution, dried over anhydrous sodium sulfate; after filtration and concentration, and the residue was separated by silica gel column chromatography to obtain the title compound (254 mg, 48 %).

MS *m/z* (ESI): 496.2 $[M+H]^+$.

Step 2: Preparation of N-(2-chloro-3'-(4-methoxy-S-(((2-methoxypyridin-4-yl)amino)methyl)picolinamido)-2'-methyl-[1,1'-biphenyl]-3-yl)-1-methyl-4,5,6,7-tetrahydro-1*H*-imidazo[4,5-*c*]pyridine-2-carboxamide

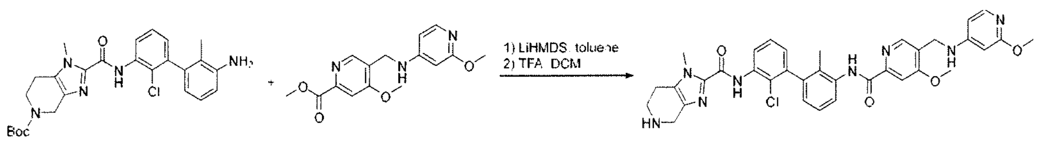


Methyl 5-(((*tert*-butoxycarbonyl)(2-methoxypyridin-4-yl)amino)methyl)-4-methoxypicolinate (50 mg, 0.101 mmol) and methyl 4-methoxy-5-(((2-methoxypyridin-4-yl)amino)methyl)picolinate (36.7 mg, 0.121 mmol) were dissolved in dry toluene (2.5 mL), and the reaction system was protected with dry nitrogen, then LiHMDS (0.353 mL, 0.353 mmol) was added under stirring at room temperature; the reaction mixture was continued to stir at room temperature for 16 hours, and the reaction mixture was diluted with ethyl acetate, washed with saturated aqueous sodium chloride solution, and dried over anhydrous sodium sulfate, then the mixture was filtered, and concentrated. The residue was redissolved in dichloromethane (2 mL), and trifluoroacetic acid (2 mL) was added under stirring at room temperature; the reaction mixture was stirred at room temperature for 0.5 hours, and then the reaction mixture was concentrated under reduced pressure to remove the solvent. The residue was separated by prep-HPLC to obtain the title compound (23 mg, 32 %).

MS *m/z* (ESI): 667.2 $[M+H]^+$.

**Embodiment 69**

*N*-(2-Chloro-3'-(4-methoxy-5-(((2-methoxypyridin-4-yl)amino)methyl)picolinamido)-2'-methyl-[1,1'-biphenyl]-3-yl)-5-ethyl-1-methyl-4,5,6,7-tetrahydro-1*H*-imidazo[4,5-*c*]pyridine-2-carboxainide

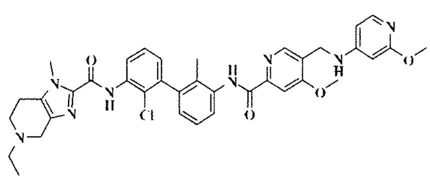

*N*-(2-Chloro-3'-(4-methoxy-5-(((2-methoxypyridin-4-yl)amino)methyl)picolinamido)-2'-methyl-[1,1-biphenyl]-3-yl)-1-methyl-4,5,6,7-tetrahydro-1*H*-imidazo[4,5-*c*]pyridine-2-carboxamide (67 mg, 0.101 mmol) was dissolved in tetrahydrofuran (6 mL) and water (1 mL); acetaldehyde (1 mL) and sodium cyanoborohydride (13 mg, 0.202 mmol) were added successively under stirring at room temperature, and then the reaction mixture was continued to stir at room temperature for 0.5 hours, then the reaction mixture was concentrated under reduced pressure to remove the organic solvent, and the residue was separated by prep-HPLC to obtain the title compound (15.5 mg, 22 %).

MS *m/z* (ESI): 695.1 [M+H]+.

$^1H$ NMR (400 MHz, $CD_3OD$) δ 8.43 (d, J = 4.2 Hz, 2H), 7.96 (d, J = 7.9 Hz, 1H), 7.87 (s, 1H), 7.65 (d, J = 6.3 Hz, 1H), 7.42 (t, J = 7.9 Hz, 1H), 7.33 (t, J = 7.9 Hz, 1H), 7.08 (d, J = 7.5 Hz, 1H), 7.04 (d, J = 7.5 Hz, 1H), 6.45-6.35 (m, 1H), 6.03 (s, 1H), 4.50 (s, 2H), 4.07 (s, 5H), 4.00 (s, 3H), 3.86 (s, 3H), 3.42 (t, J = 5.6 Hz, 2H), 3.15 (dd, J = 14.4, 7.2 Hz, 2H), 2.98 (t, J = 5.0 Hz, 2H), 2.08 (s, 3H), 1.35 (t, J = 7.2 Hz, 3H).

**Embodiment 70**

*N*-(2-Chloro-3'-(4-methoxy-5-(((2-methoxypyridin-4-yl)amino)methyl)picolinamido)-2'-methyl-[1,1'-biphenyl]-3-yl)-5-isopropyl-1-methyl-4,5,6,7-tetrahydro-1H-imidazo[4,5-*c*]pyridine-2-carboxamide

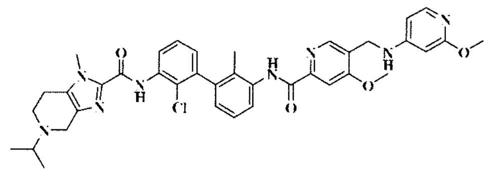

*N*-(2-Chloro-3'-(4-methoxy-5-(((2-methoxypyridin-4-yl)amino)methyl)picolinamido)-2'-methyl-[1,1'-biphenyl]-3-yl)-1-methyl-4,5,6,7-tetrahydro-1*H*-imidazo[4,5-c]pyridine-2-carboxamide (94 mg, 0.141 mmol) was dissolved in tetrahydrofuran (6 mL) and acetone (2 mL); sodium cyanoborohydride (18 mg, 0.282 mmol) was added under stirring at room temperature, and then the reaction mixture was continued to stir at room temperature for 2 hours, then the reaction mixture was concentrated under reduced pressure to remove the organic solvent, and the residue was separated by prep-HPLC to obtain the title compound (18.5 mg, 19 %).

MS *m/z* (ESI): 709.2 [M+H]+.

$^1H$ NMR (400 MHz, $CD_3OD$) δ 8.43 (dd, J = 8.1, 1.2 Hz, 1H), 8.40 (s, 1H), 7.97 (d, J = 7.8 Hz, 1H), 7.86 (s, 1H), 7.64 (d, J = 6.1 Hz, 1H), 7.42 (t, J = 7.9 Hz, 1H), 7.33 (t, J = 7.9 Hz, 1H), 7.08 (dd, J = 7.5, 1.3 Hz, 1H), 7.04 (d, J = 7.6 Hz, 1H), 6.31 (dd, J = 6.1, 1.9 Hz, 1H), 5.91 (d, J = 1.5 Hz, 1H), 4.45 (s, 2H), 4.07 (s, 3H), 3.99 (s, 3H), 3.86 (s, 2H), 3.79 (s, 3H), 3.26 (dd, J = 9.8, 4.2 Hz, 1H), 3.18 (t, J = 5.2 Hz, 2H), 2.86 (t, J = 5.5 Hz, 2H), 2.09 (s, 3H), 1.27 (d, J = 6.6 Hz, 6H).

**Embodiment 71**

*N*-(2-Chloro-3'-(4-methoxy-5-(((2-methoxypyridin-4-yl)amino)methyl)picolinamido)-2'-methyl-[1,1'-biphenyl]-3-yl)-5-cyclopropyl-1-methyl-4,5,6,7-tetrahydro-1*H*-imidazo[4,5-*c*]pyridine-2-carboxamide

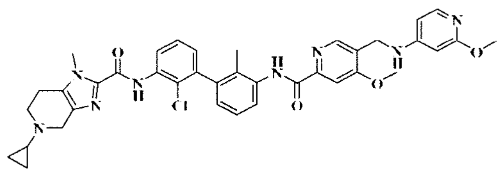

Preparation of *N*-(2-chloro-3'-(4-methoxy-5-(((2-methoxypyridin-4-yl)amino)methyl)picolinamido)-2'-methyl-[1,1'-biphenyl]-3-y)-5-cyclopropyl-1-methyl-4,5,6,7-tetrahydro-1*H*-imidazo[4,5-*c*]pyridine-2-carboxamide was referred to Embodiment 69.

MS *m/z* (ESI): 707.2 [M+H]+.

**Embodiment 72**

*N*-(2-Chloro-3'-(4-methoxy-5-(((2-methoxypyridin-4-yl)amino)methyl)picolinamido)-2'-methyl-[1,1'-biphenyl]-3-yl)-5-(2-hydroxyethyl)-1-methyl-4,5,6,7-tetraliydro-1*H*-imidazo[4,5-*c*]pyridine-2-carboxamide

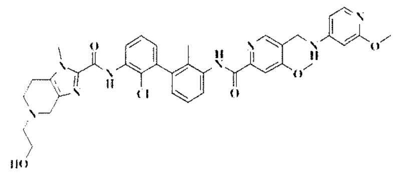

Preparation of *N*-(2-chloro-3'-(4-methoxy-5-(((2-methoxypyridin-4-yl)amino)methyl)picolinamido)-2'-methyl-[1,1'-biphenyl]-3-yl)-5-(2-hydroxyethyl)-1-methyl-4,5,6,7-tetrahydro-1*H*-imidazo[4,5-*c*]pyridine-2-carboxamide was referred to Embodiment 69.
MS *m/z* (ESI): 711.2 $[M+H]^+$.

**Embodiment 73**

(*R*)-*N*-(2-Chloro-3'-(4-methoxy-5-(((2-methoxypyridin-4-yl)amino)methyl)picolinamido)-2'-methyl-[1,1'-biphenyl]-3-yl)-5-(2-hydroxypropyl)-1-methyl-4,5,6,7-tetrahydro-1*H*-imidazo[4,5-*c*]pyridine-2-carboxamide

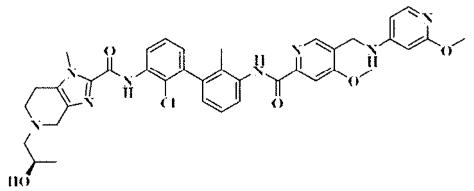

Preparation of (*R*)-*N*-(2-chloro-3'-(4-methoxy-5-(((2-methoxypyridin-4-yl)amino)methyl)picolinamido)-2'-methyl-[1,1'-biphenyl]-3-yl)-5-(2-hydroxypropyl)-1-methyl-4,5,6,7-tetrahydro-1*H*-imidazo[4,5-*c*]pyridine-2-carboxamide was referred to Embodiment 69.
MS *m/z* (ESI): 725.2 $[M+H]^+$.

**Embodiment 74**

*N*-(2-Chloro-3'-(4-methoxy-5-(((2-methoxypyridin-4-yl)amino)methyl)picolinamido)-2'-methyl-[1,1'-biphenyl]-3-yl)-5-(2-methoxyethyl)-1-methyl-4,5,6,7-tetrahydro-1*H*-imidazo[4,5-*c*]pyridine-2-carboxamide

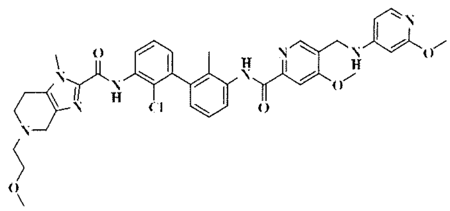

Preparation of *N*-(2-chloro-3'-(4-methoxy-5-(((2-methoxypyridin-4-yl)amino)methyl)picolinamido)-2'-methyl-[1,1'-biphenyl]-3-yl)-5-(2-methoxyethyl)-1-methyl-4,5,6,7-tetrahydro-1*H*-imidazo[4,5-*c*]pyridine-2-carboxamide was referred to Embodiment 69.
MS *m/z* (ESI): 725.2 $[M+H]^+$.

**Embodiment 75**

Methyl 4-(2-(2-((2-chloro-3'-(4-methoxy-5-(((2-methoxypyridin-4-yl)amino)methyl)picolinamido)-2'-methyl-[1,1'-biphenyl]-3-yl)carbamoyl)-1-methyl-1,4,6,7-tetrahydro-5*H*-imidazo [4,5-*c*]pyridin-5-yl)ethyl)bicyclo[2.2.1]heptane-1-carboxylate

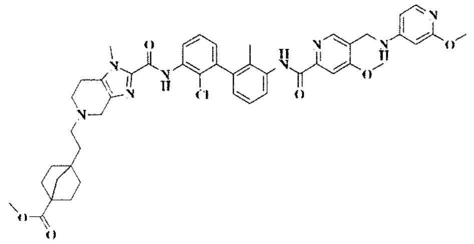

Preparation of methyl 4-(2-(2-((2-chloro-3'-(4-methoxy-5-(((2-methoxypyridin-4-yl)amino)methyl)picolinamido)-2'-methyl-[1,1'-biphenyl]-3-yt)carbamoyl)-1-methyl-1,4,6,7-tetrahydro-5*H*-imidazo[4,5-*c*]pyridin-5-yl)ethyl)bicyclo[2.2.1]heptane-1-carboxylate was referred to Embodiment 69.
MS *m*/*z* (ESI): 847.2 $[M+H]^+$.

**Embodiment 76**

4-(2-(2-((2-Chloro-3'-(4-methoxy-5-(((2-methoxypyridin-4-yl)amino)methyl)picolinamido)-2'-methyl-[1,1'-biphenyl]-3-yl)carbamoyl)-1-methyl-1,4,6,7-tetrahydro-5*H*-imidazo[4,5-*c*]pyridin-5-yl)ethyl)bicyclo[2.2.1]heptane-1-carboxylic acid

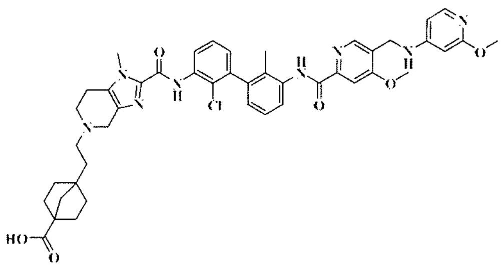

Preparation of 4-(2-(2-((2-chloro-3'-(4-methoxy-5-(((2-methoxypyridin-4-yl)amino)methyl)picolinamido)-2'-methyl-[1,1'-biphenyl]-3-yl)carbamoyl)-1-methyl-1,4,6,7-tetrahydro-5*H*-imidazo[4,5-*c*]pyridin-5-yl)etbyl)bicyclo[2.2.1]heptane-1-carboxylic acid was referred to Embodiment 69.
MS *m*/*z* (ESI): 833.2 $[M+H]^+$.

**Embodiment 77**

1-(Isobutoxy)ethyl 2-((2-chloro-3'-(4-methoxy-5-(((2-methoxypyridin-4-yl)amino)methyl)picolinamido)-2'-methyl-[1,1'-biphenyl]-3-yl)carbamoyl)-1-methyl-1,4,6,7-tetrahydro-5*H*-imidazo[4,5-*c*]pyridine-5-carboxylate

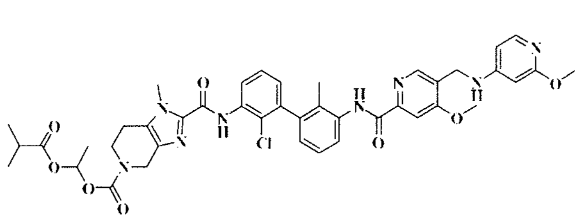

Preparation of 1-(isobutoxy)ethyl 2-((2-chloro-3'-(4-methoxy-5-(((2-methoxypyridin-4-yl)amino)methyl)picolinamido)-2'-methyl-[1,1'-biphenyl]-3-yl)carbamoyl)-1-methyl-1,4,6,7-tetrahydro-5*H*-imidazo[4,5-*c*]pyridine-5-carboxylate was referred to Embodiment 69.

MS *m/z* (ESI): 825.2 [M+H]+.

**Embodiment 78**

*N*-(2-Chloro-3'-(5-(((3-fluoro-2-methylpyridin-4-yl)amino)methyl)-4-methoxypicolinamido)-2'-methyl-[1,1'-biphenyl]-3-yl)-5-ethyl-1-methyl-4,5,6,7-tetrahydro-1*H*-imidazo[4,5-*c*]pyridine-2-carboxamide

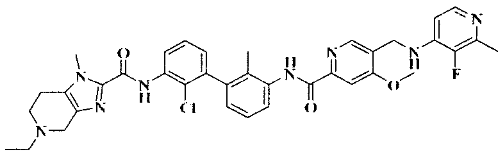

Preparation of *N*-(2-chloro-3'-(5-(((3-fluoro-2-methylpyridin-4-yl)amino)methyl)-4-methoxypicolinamido)-2'-methyl-[1,1'-biphenyl]-3-yl)-5-ethyl-1-methyl-4,5,6,7-tetrahydro-1*H*-imidazo[4,5-*c*]pyridine-2-carboxamide was referred to Embodiment 69.

MS *m/z* (ESI): 697.2 [M+H]+.

**Embodiment 79**

(*R*)-*N*-(2-Chloro-3'-(5-((3-fluoropyrrolidin-1-yl)methyl)-4-methoxypicolinamido)-2'-methyl-[1,1'-biphenyl]-3-yl)-5-ethyl-1-methyl-4,5,6,7-tetrahydro-1*H*-imidazo[4,5-*c*]pyridine-2-carboxamide

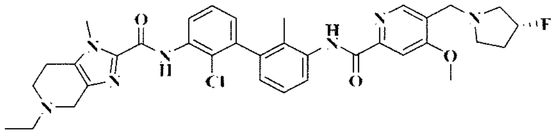

Preparation of (*R*)-*N*-(2-chloro-3'-(5-((3-fluoropyrrolidin-1-yl)methyl)-4-methoxypicolinamido)-2'-methyl-[1,1'-biphenyl]-3-yl)-5-ethyl-1-methyl-4,5,6,7-tetrahydro-1*H*-imidazo[4,5-*c*]pyridine-2-carboxamide was referred to Embodiment 69.

MS *m/z* (ESI): 660.2 [M+H]+.

**Embodiment 80**

4-(2-(2-((2-Chloro-3'-(5-(((3-fluoro-2-methylpyridin-4-yl)amino)methyl)-4-methoxypicolinamido)-2'-methyl-[1,1'-biphenyl]-3-yl)carbamoyl)-1-methyl-1,4,6,7-tetrahydro-5*H*-imidazo [4,5-*c*]pyridin-5-yl)ethyl)bicyclo[2.2.1]heptane-1-carboxylic acid

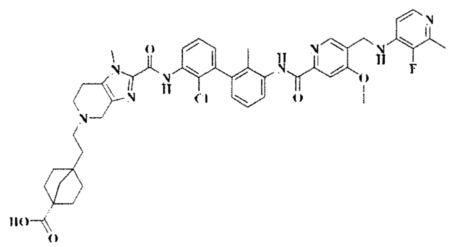

Preparation of 4-(2-(2-((2-chloro-3'-(5-(((3-fluoro-2-methylpyridin-4-yl)amino)methyl)-4-methoxypicolinamido)-2'-methyl-[1,1'-biphenyl]-3-yl)carbamoyl)-1-methyl-1,4,6,7-tetrahydro-5*H*-imidazo [4,5-*c*]pyridin-5-yl)ethyl)bicyclo[2.2.1]heptane-1-carboxylic acid was referred to Embodiment 69.

MS *m/z* (ESI): 835.2 $[M+H]^+$.

## Embodiment 81

N-(2,2'-Dichloro-3'-(4-methoxy-5-(((2-methoxypyridin-4-yl)amino)methyl)picotinamido)-[1,1'-biphenyl]-3-yl)-1-methyl-4,5,6,7-tetrahydro-1*H*-imidazo[4,5-*c*]pyridine-2-carboxamide

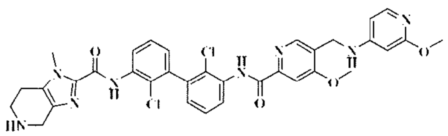

Step 1: Preparation of *tert*-butyl 2-((3'-amino-2,2'-dichloro-[1,1'-biphenyl]-3-yl)carbamoyl)-1-methyl-1,4,6,7-tetrahydro-5*H*-imidazo[4,5-*c*]pyridine-5-carboxylate

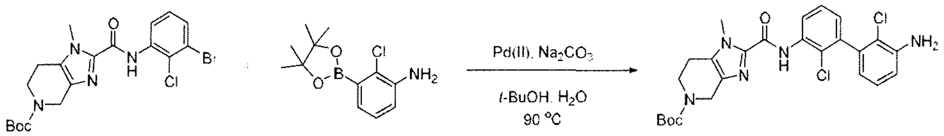


*Tert*-butyl 2-((3-bromo-2-chlorophenyl)carbamoyl)-1-metliyl-1,4,6,7-tetrahydro-5*H*-imidazo[4,5-*c*]pyridine-5-carboxylate (500 mg, 1.06 mmol), 2-chloro-3-(4,4,5,5-tetramethyl-1,3,2-dioxaborolan-2-yl)aniline (324 mg, 1.28 mmol), biscyclohexylphosphine palladium dichloride (173 mg, 0.212 mmol) and sodium carbonate (337 mg, 3.18 mmol) were added to a mixed solvent of *tert*-butanol (20 mL) and water (2 mL), and the reaction system was protected by nitrogen, heated to 90°C, stirred and reacted for 17 hours, then the reaction mixture was concentrated under reduced pressure to remove the solvent; the residue was dispersed in dichloromethane, and the organic phase was washed with saturated aqueous sodium chloride solution, dried over anhydrous sodium sulfate; after filtration and concentration, and the residue was separated by silica gel column chromatography to obtain the title compound (259 mg, 47 %).

MS *m/z* (ESI): 516.2 $[M+H]^+$.

Step 2: Preparation of *N*-(2,2'-dichloro-3'-(4-methoxy-5-(((2-methoxypyridin-4-yl)amino)methyl)picolinamido)-[1,1'-biphenyl]-3-yl)-1-methyl-4,5,6,7-tetrahydro-1*H*-imidazo[4,5-*c*]pyridine-2-carboxamide

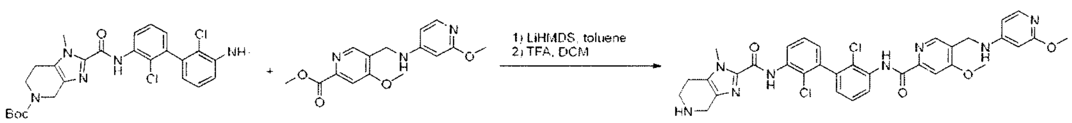


*tert*-Butyl 2-((3'-amino-2,2'-dichloro-[1,1'-biphenyl]-3-yl)carbamoyl)-1-methyl-1,4,6,7-tetrahydro-5*H*-imidazo[4,5-*c*]pyridine-5-carboxylate (20 mg, 0.0388 mmol) and methyl 4-methoxy-5-(((2-methoxypyridin-4-yl)amino)methyl)picolinate (15 mg, 0.0496 mmol) were dissolved in dry toluene (2 mL), and the reaction system was protected with dry nitrogen, then LiHMDS (0.136 mL, 0.136 mmol) was added under stirring at room temperature; the reaction mixture was continued to stir and react at room temperature for 18 hours, and the reaction mixture was diluted with ethyl acetate, washed with saturated aqueous sodium chloride solution, and dried over anhydrous sodium sulfate, then the mixture was filtered, and concentrated. The residue was redissolved in dichloromethane (2 mL), and trifluoroacetic acid (2 mL) was added under stirring at room temperature; the reaction mixture was stirred and reacted at room temperature for 0.5 hours, and then the reaction mixture was concentrated under reduced pressure to remove the solvent. The residue was

separated by prep-HPLC to obtain the title compound (2.7 mg, 10 %).

MS *m/z* (ESI): 687.1 [M+H]+.

$^1$H NMR (400 MHz, $CD_3OD$) $\delta$ 8.61 (dd, *J* = 8.4, 1.1 Hz, 1H), 8.47 (dd, *J* = 8.1, 0.9 Hz, 1H), 8.43 (s, 1H), 7.91 (s, 1H), 7.66 (d, *J* = 6.3 Hz, 1H), 7.46 (dd, *J* = 16.5, 8.3 Hz, 2H), 7.12 (dd, *J* = 7.5, 0.9 Hz, 2H), 6.40 (dd, *J* = 6.5, 1.9 Hz, 1H), 6.05 (s, 1H), 4.51 (s, 2H), 4.24 (s, 2H), 4.09 (s, 3H), 4.02 (s, 3H), 3.88 (s, 3H), 3.59 (t, *J* = 6.0 Hz, 2H), 3.03 (t, *J* = 6.3 Hz, 2H).

### Embodiment 82

*N*-(2,2'-Dichloro-3'-(4-methoxy-5-(((2-methoxypyridin-4-yl)amino)methyl)picolinamido)-[1,1'-biphenyl]-3-yl)-5-ethyl-1-methyl-4,5,6,7-tetrahydro-1*H*-imidazo[4,5-*c*]pyridine-2-carboxamide

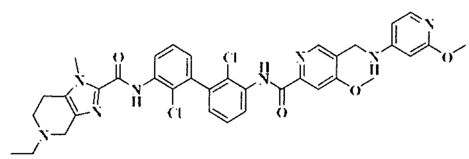

*N*-(2,2'-Dichloro-3'-(4-methoxy-5-(((2-methoxypyridin-4-yl)amino)methyl)picolinamido)-[1,1'-biphenyl]-3-yl)-1-methyl-4,5,6,7-tetrahydro-1*H*-imidazo[4,5-*c*]pyridine-2-carboxamide (75 mg, 0.101 mmol) was dissolved in tetrahydrofuran (6 mL), then acetaldehyde (45 mg, 1.01 mmol) and sodium cyanoborohydride (13 mg, 0.202 mmol) were added successively under stirring at room temperature, and then the reaction mixture was stirred and reacted at room temperature for 0.5 hours, then the reaction mixture was concentrated under reduced pressure to remove the organic solvent, and the residue was separated by prep-HPLC to obtain the title compound (38.7 mg, 54 %).

MS *m/z* (ESI): 715.2 [M+H]+.

$^1$H NMR (400 MHz, $CD_3OD$) $\delta$ 8.60 (dd, J = 8.2, 1.1 Hz, 1H), 8.46 (dd, J = 8.3, 1.2 Hz, 1H), 8.40 (s, 1H), 7.89 (s, 1H), 7.64 (d, J = 6.2 Hz, 1H), 7.44 (dt, J = 11.3, 8.0 Hz, 2H), 7.15-7.07 (m, 2H), 6.34 (dd, J = 6.2, 2.0 Hz, 1H), 5.96 (d, J = 1.8 Hz, 1H), 4.47 (s, 2H), 4.08 (s, 3H), 3.99 (s, 3H), 3.87 (s, 2H), 3.82 (s, 3H), 3.23 (t, J = 5.9 Hz, 2H), 2.98 (dd, J = 14.5, 7.2 Hz, 2H), 2.91 (t, J = 5.8 Hz, 2H), 1.29 (t, J = 7.2 Hz, 3H).

### Embodiment 83

5-(Cyclopropylmethyl)-*N*-(2,2'-dichloro-3'-(4-methoxy-5-(((2-methoxypyridin-4-yl)amino)methyl)picolinamido)-[1,1'-biphenyl]-3-yl)-1-methyl-4,5,6,7-tetrahydro-1*H*-imidazo[4,5-c]pyridine-2-carboxamide

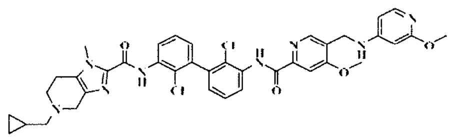

*N*-(2,2'-Dichloro-3'-(4-methoxy-5-(((2-methoxypyridin-4-yl)amino)methyl)picolinamido)-[1,1'-biphenyl]-3-yl)-1-methyl-4,5,6,7-tetrahydro-1*H*-imidazo[4,5-*c*]pyridine-2-carboxamide (75 mg, 0.101 mmol) was dissolved in tetrahydrofuran (6 mL), then cyclopropylcarbaldehyde (71 mg, 1.01 mmol) and sodium cyanoborohydride (13 mg, 0.202 mmol) were added successively under stirring at room temperature, and then the reaction mixture was stirred at room temperature for 0.5 hours, reaction mixtureconcentrated under reduced pressure to remove the organic solvent, and the residue was separated by prep-HPLC to obtain the title compound (36 mg, 48 %).

MS *m/z* (ESI): 741.2 [M+H]+.

$^1$H NMR (400 MHz, $CD_3OD$) $\delta$ 8.60 (dd, J = 8.3, 1.1 Hz, 1H), 8.46 (dd, J = 8.4, 1.1 Hz, 1H), 8.39 (s, 1H), 7.89 (s, 1H), 7.63 (d, J = 6.2 Hz, 1H), 7.44 (dt, J = 11.2, 8.0 Hz, 2H), 7.15-7.07 (m, 2H), 6.32 (dd, J = 6.2, 1.8 Hz, 1H), 5.94 (d, J = 1.5 Hz, 1H), 4.45 (s, 2H), 4.08 (s, 3H), 3.99 (s, 3H), 3.92 (s, 2H), 3.80 (s, 3H), 3.24 (t, J = 5.7 Hz, 2H), 2.90 (t, J = 5.6 Hz, 2H), 2.78 (d, J = 6.9 Hz, 2H), 1.11-1.02 (m, 1H), 0.71-0.63 (m, 2H), 0.31 (q, J = 5.0 Hz, 2H).

## Embodiment 84

1-(Isobutoxy)ethyl 2-((2,2'-dichloro-3'-(4-methoxy-5-(((2-methoxypyridin-4-yl)amino)methyl)picolinamido)-[1,1'-biphenyl]-3-yl)carbamoyl)-1-methyl-1,4,6,7-tetrahydro-5*H*-imidazo[4,5-*c*]pyridine-5-carboxylate

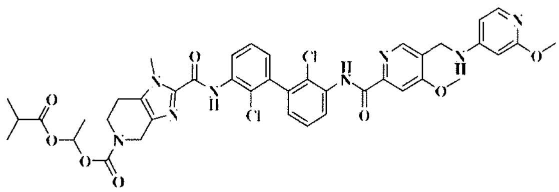

*N*-(2,2'-Dichloro-3'-(4-methoxy-5-(((2-methoxypyridin-4-yl)amino)methyl)picolinamido)-[1,1'-biphenyl]-3-yl)-1-methyl-4,5,6,7-tetrahydro-1*H*-imidazo[4,5-c]pyridine-2-carboxamide (75 mg, 0.101 mmol) was dissolved in dry tetrahydrofuran (5 mL), then DIPEA (0.05 mL, 0.302 mmol) and 1-(((4-nitrophenoxy)carbonyl)oxy)ethyl isobutyrate (45 mg, 0.151 mmol) were added successively under stirring at room temperature, and then the reaction mixture was stirred at room temperature for 4 hours, then the reaction mixture was concentrated under reduced pressure to remove the solvent, and the residue was separated by silica gel column chromatography to obtain the title compound (26.3 mg, 31 %).
MS *m*/*z* (ESI): 845.2 $[M+H]^+$.
$^1H$ NMR (400 MHz, $CD_3OD$) $\delta$ 8.63-8.58 (m, 1H), 8.46 (d, *J* = 7.5 Hz, 1H), 8.40 (s, 1H), 7.89 (s, 1H), 7.63 (d, *J* = 6.1 Hz, 1H), 7.45 (dt, *J* = 13.3, 8.0 Hz, 2H), 7.11 (td, *J* = 8.1, 1.2 Hz, 2H), 6.80-6.74 (m, 1H), 6.30 (dd, *J* = 6.0, 2.0 Hz, 1H), 5.91 (d, *J* = 1.7 Hz, 1H), 4.49 (s, 2H), 4.44 (s, 2H), 4.09 (s, 3H), 3.98 (s, 3H), 3.83 (s, 2H), 3.79 (s, 3H), 2.76 (s, 2H), 2.52 (dd, *J* = 14.0, 6.3 Hz, 1H), 1.49 (dd, *J* = 11.2, 5.6 Hz, 3H), 1.12 (dd, *J* = 13.8, 6.8 Hz, 6H).

## Embodiment 85

*N*-(2-Chloro-3'-(4-methoxy-5-(((2-methoxypyridin-4-yl)amino)methyl)picolinamido)-2'-methyl-[1,1'-biphenyl]-3-yl)-5-methyl-4,5,6,7-tetrahydrothiazolo[5,4-*c*]pyridine-2-carboxamide

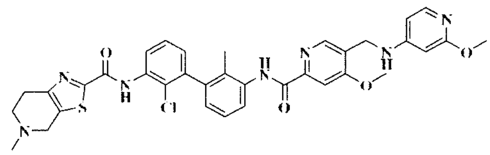

Preparation of *N*-(2-chloro-3'-(4-methoxy-5-(((2-methoxypyridin-4-yl)amino)methyl)picolinamido)-2'-methyl-[1,1'-biphenyl]-3-yl)-5-methyl-4,5,6,7-tetrahydrothiazolo[5,4-c]pyridine-2-carboxamide was referred to Embodiment 1.
MS *m*/*z* (ESI): 684.2 $[M+H]^+$.

## Embodiment 86

*N*-(2-Chloro-3'-(4-methoxy-5-(((2-methoxypyridin-4-yl)amino)methyl)picolinamido)-2'-methyl-[1,1'-biphenyl]-3-yl)-4,5,6,7-tetrahydropyrazolo[1,5-*a*]pyrazine-2-carboxamide

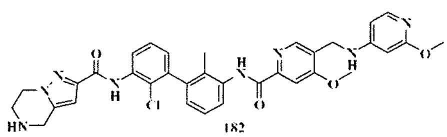

182

Step 1: Preparation of *tert*-butyl (3'-amino-2-chloro-2'-methyl-[1,1'-biphenyl]-3-yl)carbamate

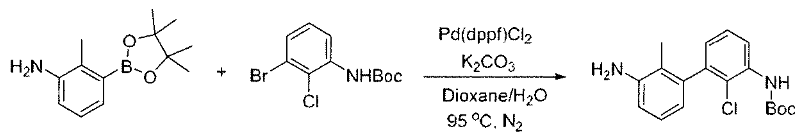


Compound 2-methyl-3-(4,4,5,5-tetramethyl-1,3,2-dioxaborolan-2-yl)aniline (2.2 g, 9.44 mmol), *tert*-butyl (3-bromo-2-chlorophenyl)carbamate (2.63 g, 8.59 mmol), Pd(dppf)$Cl_2$ (780 mg, 1.07 mmol) and potassium carbonate (3.55 g, 25.7 mmol) were dissolved in a mixed solvent of dioxane (50 mL) and water (12 mL), then the reaction system was vacuumized and replaced with nitrogen, and after nitrogen protection, the reaction mixture was heated to 95 °C and stirred overnight. After the reaction was completed, the reaction mixture was cooled to room temperature and concentrated, and the obtained residue was separated and purified by flash silica gel column chromatography (PE: EA = 3: 1) to obtain *tert-butyl* (3'-amino-2-chloro-2'-methyl-[1,1'-biphenyl]-3-yl)carbamate (2.5 g, 88 %).

$^1H$ NMR (400 MHz, $CDCl_3$) $\delta$ 8.18 (dd, *J* = 8.4, 1.5 Hz, 1H), 7.27 (t, *J* = 7.9 Hz, 1H), 7.16 (s, 1H), 7.09 (t, *J* = 7.7 Hz, 1H), 6.90 (dd, *J* = 7.6, 1.6 Hz, 1H), 6.79 (dd, *J* = 8.0, 1.2 Hz, 1H), 6.62 (dd, *J* = 7.6, 1.2 Hz, 1H), 4.25 (s, 2H), 1.91 (s, 3H), 1.54 (s, 9H).

Step 2: Preparation of *tert*-butyl ((6-((3'-((*tert*-butoxycarbonyl)amino)-2'-chloro-2-methyl-[1,1'-biphenyl]-3-yl)carbamoyl)-4-methoxypyridin-3-yl)methyl)(2-methoxypyridin-4-yl)carbamate

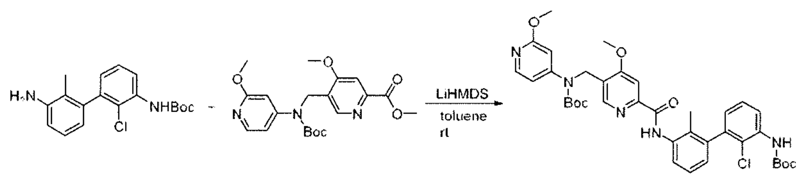


*tert*-Butyl (3'-amino-2-chloro-2'-methyl-[1,1'-biphenyl]-3-yl)carbamate (404 mg, 1.22 mmol) and methyl 5 -(((*tert*-butoxycarbonyl)(2-methoxypyridin-4-yl)amino)methyl)-4-methoxypicolinate (540 mg, 1.34 mmol) were dissolved in toluene (30 mL), then a solution of 1 M LiHMDS in toluene (5 mL, 5 mmol) was added dropwise with stirring at room temperature, and the resulting reaction mixture was stirred at room temperature overnight. The reaction was quenched with methanol, then the reaction mixture was concentrated. The obtained residue was diluted with ethyl acetate, washed with saturated brine, dried over anhydrous sodium sulfate, and the mixture was filtered. The filtrate was concentrated under reduced pressure to obtain the title compound (500 mg, 58 %).

Step 3: Preparation of *N*-(3'-amino-2'-chloro-2-methyl-[1,1'-biphenyl]-3-yl)-4-methoxy-5-(((2-methoxypyridin-4-yl)amino)methyl)picolinamide

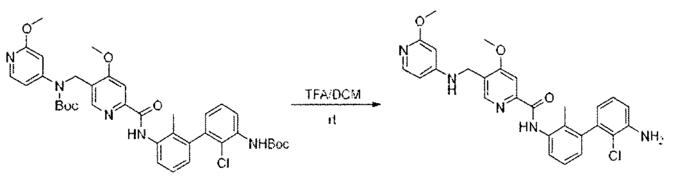


*tert-Butyl* ((6-((3'-((*tert*-butoxycarbonyl)amino)-2'-chloro-2-methyl-[1,1'-biphenyl]-3-yl)carbamoyl)-4-methoxypyridin-3-yl)methyl)(2-methoxypyridin-4-yl)carbamate (500 mg, 0.711 mmol) was dissolved in dichloromethane (3 mL), then trifluoroacetic acid (1.5 mL) was added at room temperature. The mixture was stirred at room temperature for 3 hours, and then concentrated under reduced pressure. The obtained crude product was diluted with ethyl acetate, then the mixture was washed successively with saturated aqueous sodium bicarbonate solution and saturated brine, dried

over anhydrous sodium sulfate, and then the mixture was filtered. The filtrate was concentrated to obtain the title compound (350 mg, 98 %).

MS *m/z* (ESI): 504.2 [M+H]+.

Step 4: Preparation of *tert*-butyl 2-((2-chloro-3'-(4-methoxy-5-(((2-methoxypyridin-4-yl)amino)methyl)picolinamide)-2'-methyl-[1,1'-biphenyl]-3-yl)carbamoyl)-6,7-dihydropyrazolo[1,5-a]pyrazine-5(4*H*)-carboxylate

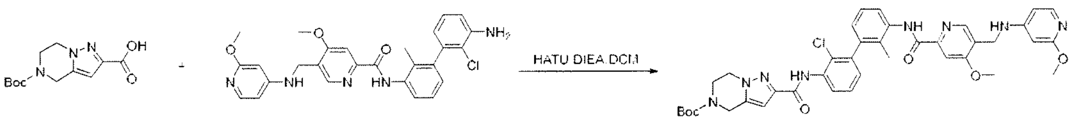


*N*-(3'-Amino-2'-chloro-2-methyl-[1,1'-biphenyl]-3-yl)-4-methoxy-5-(((2-methoxypyridin-4-yl)amino)methyl)picolinamide (250 mg, 0.496 mmol) and 5-(*tert*-butoxycarbonyl)-4,5,6,7-tetrahydropyrazolo[1,5-*a*]pyrazine-2-carboxylic acid (147 mg, 0.551 mmol) were dissolved in dichloromethane (5 mL), then DIPEA (200 mg, 1.56 mmol) and TATU (386 mg, 1.02 mmol) were added successively at room temperature. The mixture was stirred at room temperature for 3 days. The reaction mixture was concentrated under reduced pressure, and then the reaction mixture was purified by reverse phase to obtain the title compound (80 mg, 21 %).

MS *m/z* (ESI): 753.2 [M+H]+.

Step 5: Prepaaration of *N*-(2-chloro-3'-(4-methoxy-5-(((2-methoxypyridin-4-yt)amino)methyl)picolinamido)-2'-methyl-[1,1'-biphenyl]-3-yl)-4,5,6,7-tetrahydropyrazoto[1,5-*a*]pyrazme-2-carboxamide

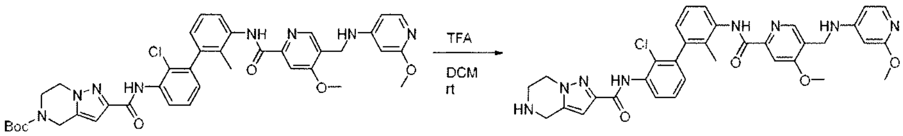


*tert-Butyl* 2-((2-chloro-3'-(4-methoxy-5-(((2-methoxypyridin-4-yl)amino)methyl)picolinamido)-2'-methyl-[1,1'-biphenyl]-3-yl)carbamoyl)-6,7-dihydropyrazolo[1,5-a]pyrazine-5(4*H*)-carboxylate (80 mg, 0.106 mmol) was dissolved in dichloromethane (3 mL), and trifluoroacetic acid (1.5 mL) was added at room temperature. The mixture was continued to stir at room temperature for 2 hours, and then the mixture was concentrated under reduced pressure. The obtained crude product was separated and purified by prep-HPLC to obtain the title compound (25 mg, 36 %).

MS *m/z* (ESI): 653.2 [M+H]+.

**Embodiment 87**

*N*-(2-Chloro-3'-(4-methoxy-5-(((2-methoxypyridin-4-yl)amino)methyl)picolinamido)-2'-methyl-[1,1'-biphenyl]-3-yl)-5-methyl-4,5,6,7-tetrahydropyrazolo[1,5-*a*]pyrazine-2-carboxamide

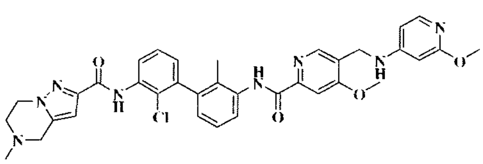

Preparation of *N*-(2-chloro-3'-(4-methoxy-5-(((2-methoxypyridin-4-yl)amino)methyl)picolinamido)-2'-methyl-[1,1'-biphenyl]-3-yl)-5-methyl-4,5,6,7-tetrahydropyrazolo[1,5-*a*]pyrazine-2-carboxamide was referred to Embodiment 86.

MS m/z (ESI): 667.2 [M+H]+.

Embodiment 88

*N*-(2-Chloro-3'-(5-(hydroxymethyl)-4-methoxypicolinamido)-2'-methyl-[1,1'-biphenyl]-3-yl)-5-ethyl-1-methyt-4,5,6,7-tetrahydro-1*H*-imidazo[4,5-*c*]pyridine-2-carboxamide

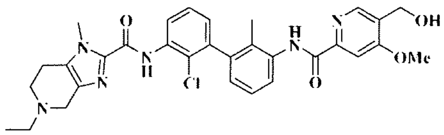

Preparation of *N*-(2-chloro-3'-(5-(hydroxymethyl)-4-methoxypicolinamido)-2'-methyl-[1,1'-biphenyl]-3-yl)-5-ethyl-1-methyl-4,5,6,7-tetrahydro-1*H*-imidazo[4,5-*c*]pyridine-2-carboxamide was referred to Embodiment 49.
MS *m*/*z* (ESI): 589.2 $[M+H]^+$.

**Embodiment 89**

*N*-(2-Chloro-3'-(5-(hydroxymethyl)-4-methoxypicolinamido)-2'-methyl-[1,1'-biphenyl]-3-yl)-5-isopropyl-1-methyl-4,5,6,7-tetrahydro-1*H*-imidazo[4,5-*c*]pyridine-2-carboxamide

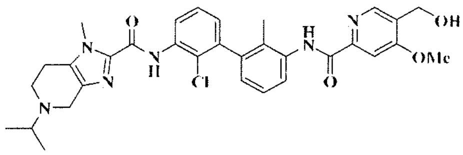

Preparation of *N*-(2-chloro-3'-(5-(hydroxymethyl)-4-methoxypicolinamido)-2'-methyl-[1,1'-biphenyl]-3-yl)-5-isopropyl-1-methyl-4,5,6,7-tetrahydro-1*H*-imidazo[4,5-*c*]pyridine-2-carboxamide was referred to Embodiment 49.
MS *m*/*z* (ESI): 603.2 $[M+H]^+$.

**Embodiment** 90

*N*-(2-Chloro-3'-(4-methoxy-5-(((2-methylpyrimidin-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

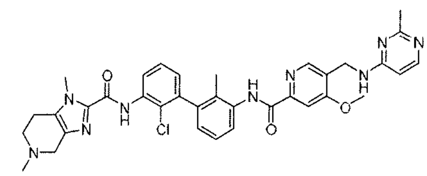

Preparation of *N*-(2-chloro-3'-(4-methoxy-5-(((2-methylpyrimidin-4-yl)amino)methyl)picolinamido)-2'-methyl-[1,1'-biphenyl]-3-yl)-1 ,5-dimethyl-4,5,6,7-tetrahydro-1H-imidazo[4,5-c]pyridine-2-carboxamide was referred to Embodiment 1.
MS *m*/*z* (ESI): 666.2 $[M+H]^+$.

**Embodiment 91**

*N*-(2-Chloro-3'-(4-methoxy-5-(((6-methylpyrimidin-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

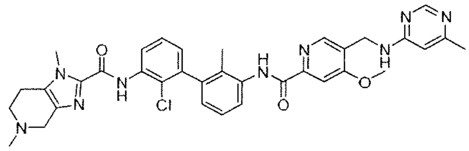

Preparation of *N*(2-chloro-3'-(4-methoxy-5-(((6-methylpyrimidin-4-y))amino)methyt)picolinamido)-2'-methyl-[1,1'-biphenyt]-3-yl)-1,5-dimethyl-4,5,6,7-tetrahydro-1*H*-imidazo[4,5-c]pyridine-2-carboxamide was referred to Embodiment 1.

MS *m/z* (ESI): 666.2 $[M+H]^+$.

**Embodiment 92**

*N*-(2-Chloro-3'-(4-methoxy-5-(((6-methylpyridin-3-yl)amino)methyl)picolinamido)-2'-methy)-[1,1'-biphenyl]-3-yl)-1,5-dimethyl-4,5,6,7-tetrahydro-1*H*-imidazo[4,5-*c*]pyridine-2-carboxamide

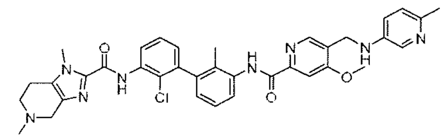

Preparation of *N*-(2-chloro-3'-(4-methoxy-5-(((6-methylpyridin-3-yl)amino)methyl)picolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 1.

MS *m/z* (ESI): 665.2 $[M+H]^+$.

**Embodiment 93**

*N*-(2-Chloro-3'-(4-methoxy-5-(((5-methylpyrazin-2-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

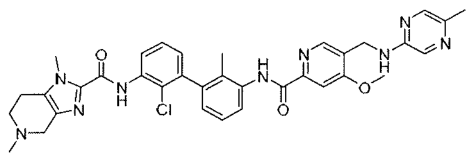

Preparation of *N*-(2-chloro-3'-(4-methoxy-5-(((5-methylpyrazin-2-yl)amino)methyl)picolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 1.

MS *m/z* (ESI): 666.2 $[M+H]^+$.

**Embodiment 94**

*N*-(2-Chloro-3'-(4-methoxy-5-(((1-methyl-2-carbonyl-1,2-dihydropyridin-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

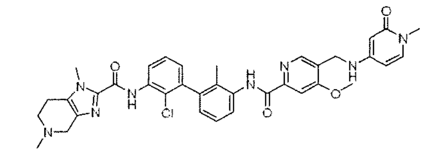

Preparation of *N*-(2-chloro-3'-(4-methoxy-5-(((1-methyl-2-carbonyl-1,2-dihydropyridin-4-yl)amino)methyl)picolinamido)-2'-methyl-[1,1'-biphenyl]-3-yl)-1 ,5-dimethyl-4,5,6,7-tetrahydro-1*H*-imidazo[4,5-*c*]pyridine-2-carboxamide was referred to Embodiment 1.

MS m/z (ESI): 681.2 $[M+H]^+$.

**Embodiment 95**

*N*-(2-Chloro-3'-(4-methoxy-5-(((2-(methylamino)-2-carbonylethyl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

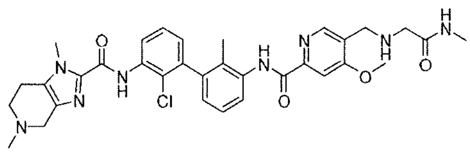

Step 1: Preparation of methyl 4-methoxy-5-(((2-(methylamino)-2-carbonylethyl)amino)methyl)picolinate

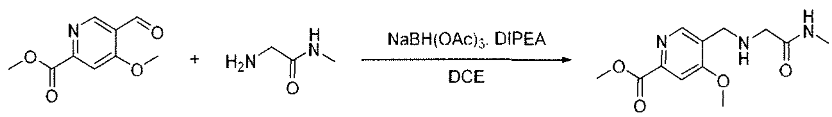


DIPEA (60 mg, 0.462 mmol) was added to a dichloroethane (0.5 mL) solution of methyl 5-formyl-4-methoxypicolinate (60 mg, 0.308 mmol) and 2-amino-*N*-methylacetamide hydrochloride (58 mg, 0.462 mmol), then the reaction mixture was stirred at room temperature for 10 minutes, and then sodium triacetoxyborohydride (131 mg, 0.616 mmol) was added; the mixture was warmed to 45 °C and stirred for 2 hours. The reaction mixture was diluted with DCM (10 mL), then the reaction mixture was washed with saturated aqueous sodium bicarbonate solution (5 mL), and the organic layer was concentrated under reduced pressure to obtain the title compound as a white solid (40 mg, 49 %).

MS *m/z* (ESI): 267.2 $[M+H]^+$.

Step 2: Preparation of N-(2-chloro-3'-(4-methoxy-5-(((2-(methylamino)-2-carbonylethyl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

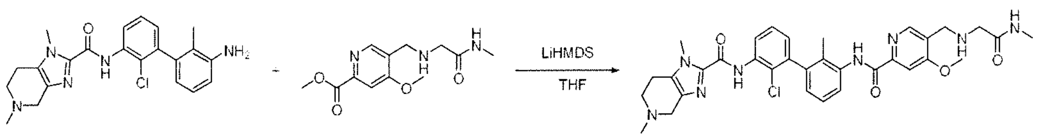

LiHMDS (0.15 mL, 1.3 M in THF) was added dropwise to a tetrahydrofuran (1 mL) solution of *N*-(3'-amino-2-chloro-2'-methyl-[1,1'-biphenyl]-3-yl)-1,5-dimethyl-4,5,6,7-tetrahydro-1*H*-imidazo[4,5-c]pyridine-2-carboxamide (20 mg, 0.049 mmol) and methyl 4-methoxy-5-(((2-(methylamino)-2-carbonylethyl)amino)methyl)picolinate (40 mg, 0.150 mmol), then the reaction mixture was stirred at room temperature for 16 hours. The reaction mixture was quenched with saturated aqueous ammonium chloride solution (2 mL) and then extracted with ethyl acetate (10 mL). The organic phase was washed with saturated aqueous sodium chloride solution (5 mL), dried over anhydrous sodium sulfate, and the mixture was filtered, concentrated under reduced pressure, and the residue was separated and purified by prep-HPLC to obtain the title compound as a white solid (5 mg, 16 %).
MS *m/z* (ESI): 645.2 $[M+H]^+$.
$^1H$ NMR (400 MHz, DMSO-$d_6$) $\delta$ 10.38 (s, 1H), 9.92 (s, 1H), 8.54 (s, 1H), 8.32 (d, J = 8.2 Hz, 1H), 7.96 (d, J = 8.1 Hz, 1H), 7.80 (s, 1H), 7.75 (s, 1H), 7.51-7.43 (m, 1H), 7.35 (t, J = 7.9 Hz, 1H), 7.09 (d, J = 7.6 Hz, 1H), 7.03 (d, J = 7.6 Hz, 1H), 3.99 (s, 3H), 3.90 (s, 3H), 3.73 (s, 2H), 3.36 (s, 2H), 3.10 (s, 2H), 2.74-2.65 (m, 4H), 2.61 (d, J = 4.7 Hz, 3H), 2.38 (s, 3H), 2.05 (s, 3H).

Embodiment 96

(*R*)-N-(2-Chloro-3'-(4-methoxy-5-(((3-carbonylisoxazolidin-4-yl)amino)methyl)picolinamido)-2'-methyl-[1,1'-biphenyl]-3-yl)-1 ,5-dimethyl-4,5,6,7-tetrahydro-1*H*-imidazo[4,5-c]pyridine-2-carboxamide

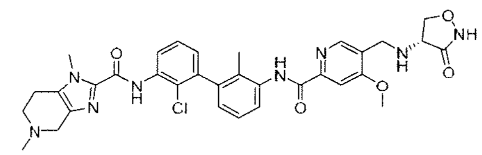

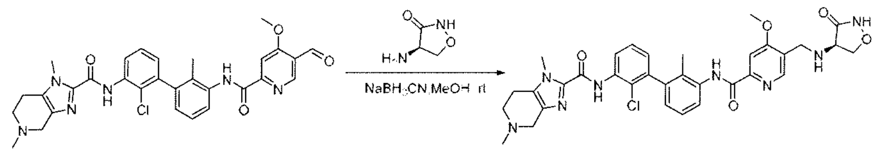


*N*-(2-Chloro-3'-(5-formyl-4-methoxypicolinamido)-2'-methyt-[1,1'-biphenyl]-3-yl)-1,5-dimethyl-4,5,6,7-tetrahydro-1*H*-imidazo[4,5-c]pyridine-2-carboxamide (37 mg, 0.065 mmol) and (R)-4-aminoisoxazolidin-3-one (13 mg, 0.129 mmol) were dissolved in methanol (3 mL), then 2 drops of acetic acid were added, and the mixture was stirred at room temperature for 2 hours. After sodium cyanoborohydride (10 mg, 0.163 mmol) was added, the reaction mixture was continued to stir at room temperature overnight. After the reaction was completed, the reaction mixture was concentrated, and the residue was purified by reverse phase to obtain the title compound (1.4 mg, 3 %).
MS m/z (ESI): 659.3 $[M+H]^+$.

**Embodiment 97**

(*R*)-*N*-(2-Chloro-3'-(4-methoxy-5-(((2-carbonylpyrrolidin-3-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

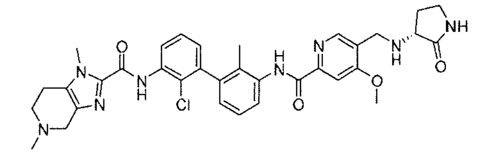

Preparation of (*R*)-*N*-(2-chloro-3'-(4-Methoxy-5-(((2-carbonylpyrrolidin-3-yl)amino)methyl)picolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 96.
MS *m/z* (ESI): 657.2 $[M+H]^+$.

**Embodiment 98**

(*S*)-*N*-(2-Chloro-3'-(4-methoxy-5-(((2-carbonylpiperidin-3-yl)amino)methyl)picolinamido)-2'-methyl-[1,1'-bipheny]]-3-y])-],5-dimethyl-4,5,6,7-tetrahydro-1*H*-imidazo[4,5-*c*]pyridine-2-carboxamide

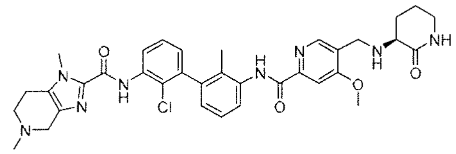

Preparation of (*S*)-*N*-(2-chloro-3'-(4-methoxy-5-(((2-carbonylpiperidin-3-yl)amino)methyl)picolinamido)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 96.

MS *m*/*z* (ESI): 671.2 $[M+H]^+$.

**Embodiment 99**

*N*-(2-Chloro-3'-(4-methoxy-5-(((3-methyloxetan-3-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

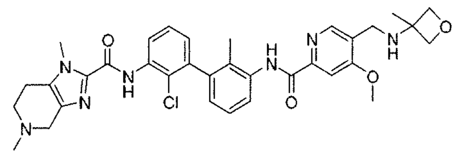

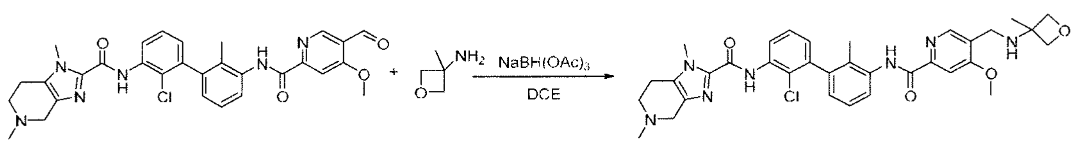


3-Amino-3-methyloxetane (7 mg, 0.0785 mmol) was added to a dichloroethane (1 mL) solution of *N*-(2-chloro-3'-(5-formyl-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide (30 mg, 0.052 mmol), then the mixture was stirred at room temperature for 1 hour, then sodium triacetoxyborohydride (33 mg, 0.156 mmol) was added, and the mixture was stirred at room temperature for 2 hours. The reaction mixture was quenched with saturated aqueous sodium bicarbonate solution (1 mL), then the reaction mixture was extracted with dichloromethane (5 mL); the organic layer was concentrated under reduced pressure, and the residue was separated and purified by prep-HPLC to obtain the title compound as a white solid (11 mg, 33 %).

MS *m*/*z* (ESI): 644.2 $[M+H]^+$.

**Embodiment 100**

*N*-(3'-(5-(((1-Acetylazetidin-3-yl)amino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-c]pyridine-2-carboxamide

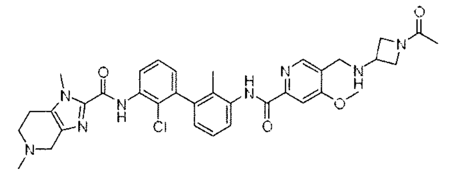

Preparation of *N*-(3'-(5-(((1-acetylazetidin-3-yl)amino)methyl)-4-methoxypicolinamido)-2-chloro-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 96.

MS *m*/*z* (ESI): 671.2 [M+H]+.

**Embodiment 101**

Methyl 3-(((6-((2'-chloro-3'-(1,5-dimethyl-4,5,6,7-tetrahydro-1*H*-imidazo[4,5-*c*]pyridine-2-carboxamido)-2-methyl-[1,1'-biphenyl]-3-yl)carbamoyl)-4-methoxypyridin-3-yl)methyl)amino)oxetane-3-carboxylate

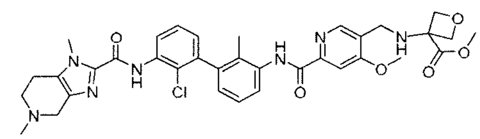

Preparation of methyl 3-(((6-((2'-chloro-3'-(1,5-dimethyl-4,5,6,7-tetrahydro-*1*H-imidazo[4,5-*c*]pyridine-2-carboxamido)-2-methyl-[1,1'-biphenyl]-3-yl)carbamoyl)-4-methoxypyridin-3-yl)methyl)amino)oxetane-3-carboxylate was referred to Embodiment 96.

MS *m*/*z* (ESI): 688.2 [M+H]+.

**Embodiment 102**

3-(((6-((2'-Chloro-3'-(1,5-dimethyl-4,5,6,7-tetrahydro-1*H*-imidazo[4,5-*c*]pyridine-2-carboxamido)-2-methyl-[1,1'-biphenyl]-3-yl)carbamoyl)-4-methoxypyridin-3-yl)methyl)amino)oxetane-3-carboxylic acid

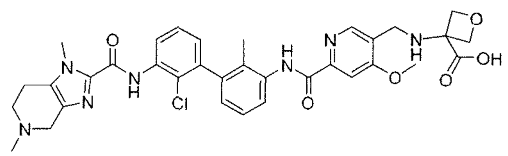

Preparation of 3-(((6-((2'-chloro-3'-(1,5-dimethyl-4,5,6,7-tetrahydro-1*H*-imidazo[4,5-*c*]pyridine-2-carboxamido)-2-methyl-[1,1'-biphenyl]-3-yl)carbamoyl)-4-methoxypyridin-3-yl)methyl)amino)oxetane-3-carboxylic acid was referred to Embodiment 96.

MS *m*/*z* (ESI): 674.2 [M+H]+.

**Embodiment 103**

*N*-(2-Chloro-3'-(5-(((2,2-difluoroethyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

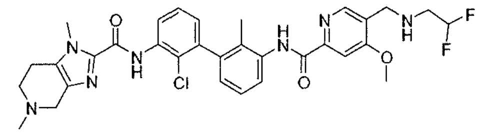

Preparation of *N*-(2-chloro-3'-(5-(((2,2-difluoroethyl)amino)methyl)-4-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 96.
MS *m/z* (ESI): 638.2 [M+H]+.

**Embodiment 104**

*N*-(2-Chloro-3'-(4-methoxy-5-((7-carbonyl-2-oxa-6-azaspiro[3.4]octan-6-yl)methy))picolinamido)-2'-methyl-[1,1'-biphenyl]-3-yl)-1,5-dimethyl-4,5,6,7-tetrahydro-1*H*-imidazo[4,5-*c*]pyridine-2-carboxamide

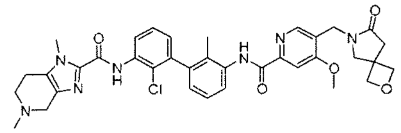

Preparation of *N*-(2-chloro-3'-(4-methoxy-5-((7-carbonyl-2-oxa-6-azaspiro[3.4]octan-6-yl)metliyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 96.
MS *m/z* (ESI): 684.2 [M+H]+.

**Embodiment 105**

*N*-(2-Chloro-3'-(5-(((3-fluoro-2-methylpyridin-4-yl)amino)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

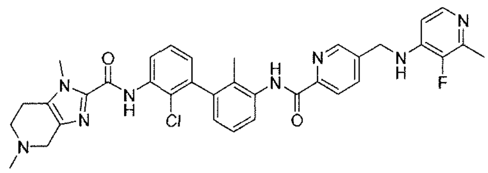

Preparation of *N*-(2-chloro-3'-(5-(((3-fluoro-2-methylpyridin-4-yl)amino)methyl)picolinamido)-2'-methy)-[1,1'-biphenyl]-3-yl)-1,5-dimethyl-4,5,6,7-tetrahydro-1*H*-imidazo[4,5-*c*]pyridine-2-carboxamide was referred to Embodiment 1.
MS *m/z* (ESI): 653.2 [M+H]+.

**Embodiment 106**

(*R*)-*N*-(2-Chloro-3'-(5-((3-fluoropyrrolidin-1-yl)methyl)picolinamido)-2'-methyl-[1,1'-biphenyl]-3-yl)-1,5-dimethyl-4,5,6,7-tetrahydro-1*H*-imidazo[4,5-*c*]pyridine-2-carboxamide

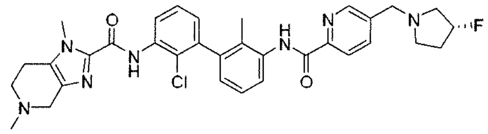

Preparation of (*R*)-*N*(2-chloro-3'-(5-((3-fluoropyrrolidin-1-yl)methyl)picolinamido)-2'-methyl-[1,1 '-biphenyl]-3-yl)-1,5-dimethyl-4,5,6,7-tetrahydro-1*H*-imidazo[4,5-*c*]pyridine-2-carboxamide was referred to Embodiment 1.

MS *m*/*z* (ESI): 616.2 [M+H]+.

**Embodiment 107**

(*R*)-*N*(2-Chloro-3'-(5-((3-fluoropyrrolidin-1-yl)methyl)-3-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

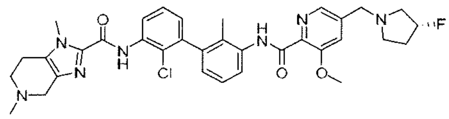

Preparation of (*R*)-*N*-(2-chloro-3'-(5-((3-fluoropyrrolidin-1-yl)methyl)-3-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS *m*/*z* (ESI): 646.2 [M+H]+.

**Embodiment 108**

*N*-(2-Chloro-3'-(5-((3-fluoroazetidin-1-yl)methyl)-3-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

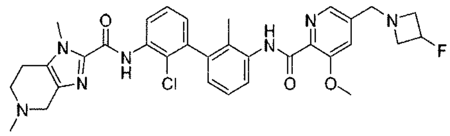

Preparation of *N*-(2-chloro-3'-(5-((3-fluoroazetidin-1-yl)methyl)-3-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 1.

MS *m*/*z* (ESI): 632.2 [M+H]+.

**Embodiment 109**

*N*-(2-Chloro-3'-(5-((((3*R*,4*R*)-3-fluorotetrahydro-2*H*-pyran-4-yl)amino)methyl)-3-methoxypicolinamido)-2'-methyl-[1,1'-biphenyl]-3-yl)-1,5-dimethyl-4,5,6,7-tetrahydro-1*H*-imidazo[4,5-*c*]pyridine-2-carboxamide

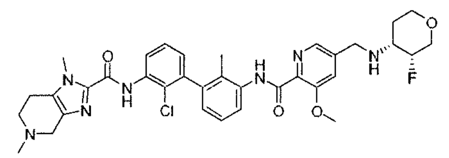

Preparation of *N*-(2-chloro-3'-(5-((((3*R*,4*R*)-3-fluorotetrahydro-2*H*-pyran-4-yl)amino)methyl)-3-methoxypicolinamido)-2'-metliyl-[1,1'-biphenyl]-3-yl)-1,5-dimethyl-4,5,6,7-tetrahydro-1*H*-imidazo[4,5-c]pyridine-2-carboxamide was referred to Embodiment 1.

MS *m/z* (ESI): 676.2 [M+H]+.

**Embodiment 110**

*N*-(2-Chloro-3'-(4-methoxy-5-(((2-carbonylpiperidin-3-yl)amino)methyl)pyridin-2-yl)-2'-methyl-[1,1'-biphenyt]-3-yl)-1,5-dimethyl-4,5,6,7-tetrahydro-1*H*-imidazo[4,5-*c*]pyridine-2-carboxamide

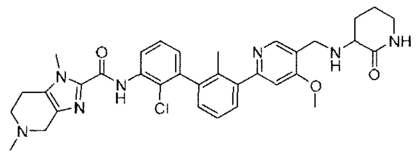

Preparation of *N*-(2-chloro-3'-(4-methoxy-5-(((2-carbonylpiperidin-3-yl)amino)methyl)pyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 58.

MS *m/z* (ESI): 628.2 [M+H]+.

**Embodiment 111**

(*R*)-*N*-(2-Chloro-3'-(4-methoxy-5-(((2-carbonylpyrrolidin-3-yl)amino)methyl)pyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

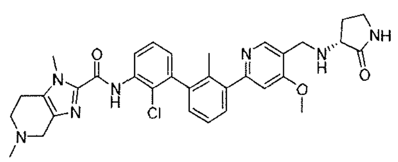

Preparation of (*R*)-*N*-(2-chloro-3'-(4-methoxy-5-(((2-carbonylpyrrolidin-3-yl)amino)methyl)pyridin-2-yl)-2'-methyl-[1,1'-biphenyl]-3-yl)-1,5-dimethyl-4,5,6,7-tetrahydro-1*H*-imidazo[4,5-c]pyridine-2-carboxamide was referred to Embodiment 58.

MS *m/z* (ESI): 614.2 [M+H]+.

**Embodiment 112**

*N*-(2-Chloro-3'-(6-methoxy-5-((oxetan-3-ylamino)methyl)pyridin-2-yl)-2'-methyl-[1,1'-biphenyl]-3-yl)-1,5-dimetliyl-4,5,6,7-tetrahydro-1*H*-imidazo[4,5-*c*]pyridine-2-carboxamide

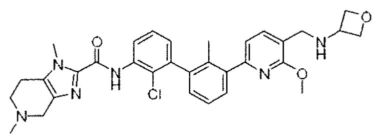

Step 1: Preparation of 6-(3-bromo-2-methylphenyl)-2-methoxynicotinaldehyde

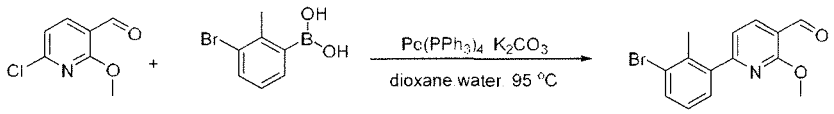


6-Chloro-2-methoxynicotinaldehyde (1 g, 5.88 mmol), (3-bromo-2-methylphenyl)boronic acid (1.38 g, 6.43 mmol), $Pd(PPh_3)_4$ (693 mg, 0.60 mmol) and potassium carbonate (1.62 g, 11.76 mmol) were dissolved in a mixed solvent of dioxane (20 mL) and water (2 mL), then the reaction system was vacuumized and replaced with nitrogen, and after nitrogen protection, the reaction mixture was heated to 95 °C and stirred overnight. After the reaction was completed, the reaction mixture was cooled, then the mixture was filtered, and the filtrate was concentrated, and the residue was separated by flash silica gel column chromatography (PE: EA= 4: 1) to obtain the title compound (616 mg, 34 %).
MS *m/z* (ESI): 305.7 $[M+H]^+$.

Step 2: Preparation of 2-methoxy-6-(2-methyl-3-(4,4,5,5-tetramethyl-1,3,2-dioxaborolan-2-yl)phenyl)nicotinealdehyde

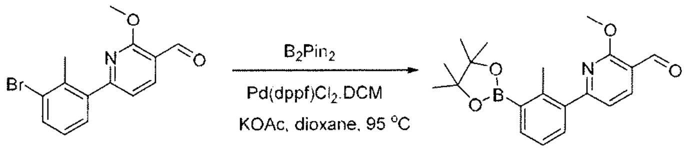


6-(3-Bromo-2-methylphenyl)-2-methoxynicotinaldehyde (616 mg, 2 mmol), bis(pinacolato)diboron (660 mg, 2.6 mol), complex (165 mg, 0.2 mmol) of $Pd(dppf)Cl_2$ and DCM, and potassium acetate (588 mg, 6 mmol) were dissolved in dioxane (17 mL), and the mixture was vacuumized and replaced with nitrogen; under the protection of nitrogen, the reaction mixture was heated and stirred at 95 °C overnight. After the reaction was completed, the reaction mixture was cooled and concentrated. The residue was separated by flash silica gel column chromatography (PE: EA = 3: 1) to obtain the title compound (600 mg, 86 %).
MS *m/z* (ESI): 305.7 $[M+H]^+$.

Step 3: Preparation of *N*-(2-chloro-3'-(5-formyl-6-methoxypyridin-2-yl)-2'-methyl-[1,1'-biphenyl]-3-yl)-1,5-dimethyl-4,5,6,7-tetrahydro-1*H*-imidazo[4,5-c]pyridine-2-carboxamide

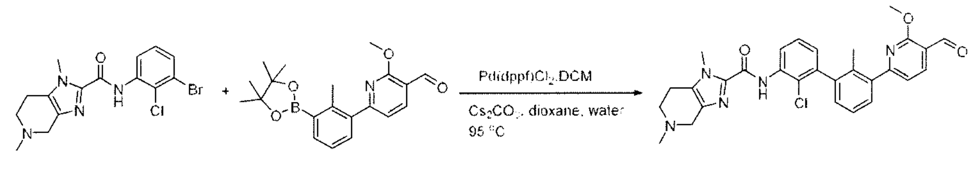


*N*-(3-Bromo-2-chlorophenyl)-1,5-dimethyl-4,5,6,7-tetrahydro-1*H*-imidazo[4,5-c]pyridine-2-carboxamide (200 mg, 0.522 mmol), 2-methoxy-6-(2-methyl-3-(4,4,5,5-tetramethyl-1,3,2-dioxaborolan-2-yl)phenyl)nicotinaldehyde (258 mg, 0.73 mol), complex (86 mg, 0.104 mmol) of $Pd(dppf)Cl_2$ and DCM, and cesium carbonate (424 mg, 1.31 mmol) were dissolved in a mixed solvent of dioxane (10 mL) and water (1.5 mL), and the mixture was vacuumized and replaced with nitrogen; under the protection of nitrogen, the reaction mixture was heated and stirred at 95 °C overnight. After the reaction was completed, the reaction mixture was cooled and concentrated. The residue was separated by flash silica gel column chromatography (DCM: MeOH = 10: 1) to obtain the title compound (170 mg, 61 %).
MS m/z (ESI): 529.7 $[M+H]^+$.

Step 4: Preparation of *N*-(2-chloro-3'-(6-methoxy-5-((oxetan-3-ylamino)methyl)pyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

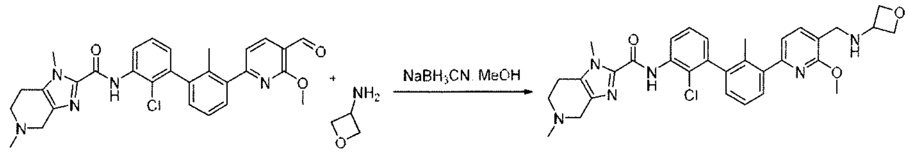


*N*-(2-Chloro-3'-(5-formyl-6-methoxypyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide (130 mg, 0.245 mmol) and oxetan-3-amine (72 mg, 0.981 mmol) were dissolved in methanol (3 mL), then 3 drops of acetic acid were added, and the reaction mixture was stirred at room temperature for 3 hours. After sodium cyanoborohydride (30 mg, 0.491 mmol) was added, the reaction mixture was continued to stir at room temperature overnight. After the reaction was completed, the reaction mixture was concentrated, and the residue was purified by reverse phase to obtain the title compound (9.1 mg, 7 %).

MS *m/z* (ESI): 587.2 $[M+H]^+$.

$^1$H NMR (400 MHz, DMSO-$d_6$) δ 10.04 (s, 1H), 8.45 (d, J = 8.3 Hz, 1H), 7.89 (d, J = 7.4 Hz, 1H), 7.59 (t, J = 6.9 Hz, 2H), 7.50 (t, J = 7.8 Hz, 1H), 7.32 (d, J = 7.5 Hz, 1H), 7.25 (d, J = 7.5 Hz, 2H), 4.71 (t, J = 6.6 Hz, 2H), 4.43 (t, J = 6.2 Hz, 2H), 4.02 (s, 7H), 3.76 (s, 2H), 3.50 (s, 2H), 2.82 (s, 5H), 2.51 (s, 3H), 2.19 (s, 3H).

## Embodiment 113

*N*-(2-Chloro-3'-(5-((3-fluoroazetidin-1-yl)methyl)-6-methoxypyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide

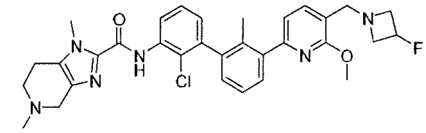

Preparation of *N*-(2-chloro-3'-(5-((3-fluoroazetidin-1-yl)methyl)-6-methoxypyridin-2-yl)-2'-methyl-[1,1'-biphenyl]-3-yl)-1,5-dimethyl-4,5,6,7-tetrahydro-1*H*-imidazo[4,5-*c*]pyridine-2-carboxamide was referred to Embodiment 112.

MS *m/z* (ESI): 589.2 $[M+H]^+$.

## Embodiment 114

*N*-(2,2'-Dichloro-3'-(6-methoxy-5-((oxetan-3-ylamino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

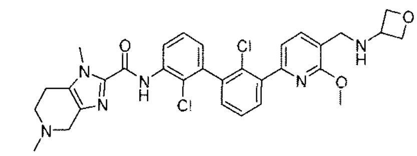

Step 1: Preparation of 6-(3-bromo-2-chlorophenyl)-2-methoxynicotinaldehyde

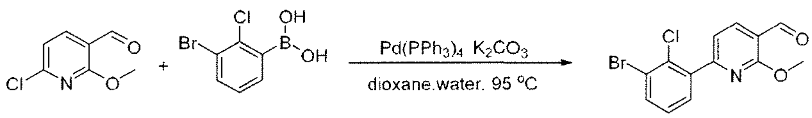


6-Chloro-2-methoxynicotinaldehyde (400 mg, 2.34 mmol), (3-bromo-2-chlorophenyl)boronic acid (600 mg, 2.57 mmol), $Pd(PPh_3)_4$ (266 mg, 0.23 mmol) and potassium carbonate (646 mg, 4.68 mmol) were dissolved in a mixed solvent

of dioxane (20 mL) and water (2 mL), then the reaction system was vacuumized and replaced with nitrogen, and after nitrogen protection, the reaction mixture was heated to 95 °C and stirred overnight. After the reaction was completed, the reaction mixture was cooled, then the mixture was filtered; the filtrate was concentrated, and the residue was separated by flash silica gel column chromatography (PE: EA = 4: 1) to obtain the title compound (600 mg, 78 %).

MS *m/z* (ESI): 326.7 [M+H]+.

Step 2: Preparation of 6-(2-chloro-3-(4,4,5,5-tetramethyl-1,3,2-dioxaborolan-2-yl)phenyl)-2-methoxynicotinaldehyde

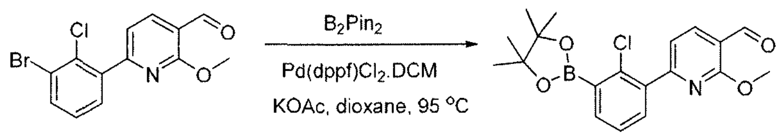


6-(3-Bromo-2-chlorophenyl)-2-methoxynicotinaldehyde (600 mg, 1.83 mmol), bis(pinacolato)diboron (607 mg, 2.39 mol), complex (149 mg, 0.18 mmol) of Pd(dppf)$Cl_2$ and DCM, and potassium acetate (538 mg, 5.49 mmol) were dissolved in dioxane (17 mL), and the mixture was vacuumized and replaced with nitrogen; under the protection of nitrogen, the reaction mixture was heated and stirred at 95 °C overnight. After the reaction was completed, the reaction mixture was cooled and concentrated. The residue was separated by flash silica gel column chromatography (PE: EA = 3: 1) to obtain the title compound (600 mg, 87 %).

MS *m/z* (ESI): 375.7 [M+H]+.

Step 3: Preparation of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

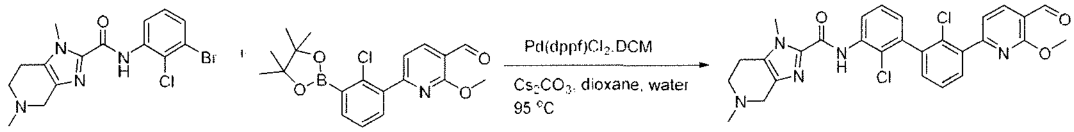


*N*-(3-Bromo-2-chlorophenyl)-1,5-dimethyl-4,5,6,7-tetrahydro-1*H*-imidazo[4,5-*c*]pyridine-2-carboxamide (170 mg, 0.444 mmol), 6-(2-chloro-3-(4,4,5,5-tetramethyl-1,3,2-dioxaborolan-2-yl)phenyl)-2-methoxynicotinaldehyde (216 mg, 0.577 mol), complex (73 mg, 0.09 mmol) of Pd(dppf)$Cl_2$ and DCM, and cesium carbonate (360 mg, 1.11 mmol) were dissolved in a mixed solvent of dioxane (10 mL) and water (2 mL), and the reaction system was vacuumized and replaced with nitrogen; under the protection of nitrogen, the reaction mixture was heated and stirred at 95 °C overnight. After the reaction was completed, the reaction mixture was cooled and concentrated. The residue was separated by reverse phase to obtain the title compound (200 mg, 82 %).

MS *m/z* (ESI): 550.7 [M+H]+.

Step 4: Preparation of *N*-(2,2'-dichloro-3'-(6-methoxy-5-((oxetan-3-ylamino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

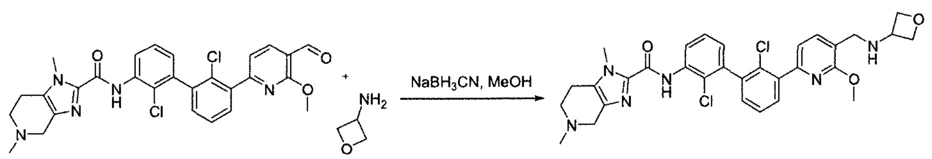


*N*-(2,2'-Chloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (100 mg, 0.181 mmol) and oxetan-3-amine (52 mg, 0.726 mmol) were dissolved in methanol (3 mL), then 4 drops of acetic acid were added, and the reaction mixture was stirred at room

temperature for 3 hours. After sodium cyanoborohydride (22 mg, 0.362 mmol) was added, the reaction mixture was continued to stir at room temperature overnight. After the reaction was completed, the reaction mixture was concentrated, and the residue was purified by reverse phase to obtain the title compound (10.3 mg, 9 %).

MS *m/z* (ESI): 607.2 $[M+H]^+$.

$^1$H NMR (400 MHz, DMSO-$d_6$) δ 9.91 (s, 1H), 8.35 (d, J = 8.3 Hz, 1H), 7.79 (d, J = 7.5 Hz, 1H), 7.67 (d, J = 7.6 Hz, 1H), 7.51 (dt, J = 25.0, 7.7 Hz, 2H), 7.41 (d, J = 7.6 Hz, 1H), 7.26 (d, J = 7.5 Hz, 1H), 7.18 (d, J = 7.6 Hz, 1H), 4.59 (t, J = 6.5 Hz, 2H), 4.32 (t, J = 6.2 Hz, 2H), 3.91 (d, J = 8.4 Hz, 7H), 3.63 (s, 2H), 3.37 (s, 2H), 2.69 (s, 4H), 2.38 (s, 3H).

**Embodiment 115**

*N*-(2,2'-Dichloro-3'-(5-((((3*R*,4*R*)-3-fluorotetrahydro-2*H*-pyran-4-yl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

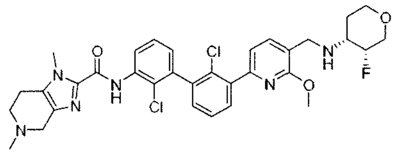

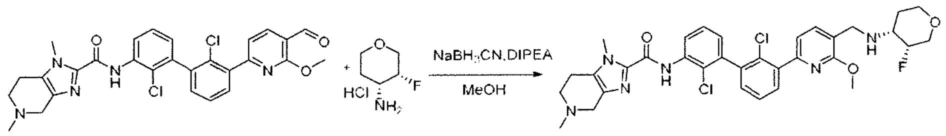


*N*-(2,2'-Dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (50 mg, 0.091 mmol) and (3*R*,4*R*)-3-fluorotetrahydro-2*H*-pyran-4-amine hydrochloride (42 mg, 0.272 mmol) were dissolved in methanol (4 mL), after the reaction mixture was neutralized with DIPEA, 3 drops of acetic acid were added, and the reaction mixture was stirred at room temperature for 3 hours. After sodium cyanoborohydride (11 mg, 0.181 mmol) was added, the reaction mixture was continued to stir at room temperature overnight. After the reaction was completed, the reaction mixture was concentrated, and the residue was purified by reverse phase to obtain the title compound (10.3 mg, 18 %).

MS *m/z* (ESI): 653.1 $[M+H]^+$.

$^1$H NMR (400 MHz, DMSO-$d_6$) δ 9.91 (s, 1H), 8.35 (d, J = 8.2 Hz, 1H), 7.86 (d, J = 7.5 Hz, 1H), 7.68 (d, J = 7.8 Hz, 1H), 7.52 (dt, J = 25.0, 7.9 Hz, 2H), 7.41 (d, J = 7.6 Hz, 1H), 7.28 (d, J = 7.3 Hz, 1H), 7.19 (d, J = 7.6 Hz, 1H), 4.45 (s, 1H), 4.33 (s, 1H), 3.91 (d, J = 8.7 Hz, 6H), 3.79 (d, J = 16.8 Hz, 3H), 3.37 (s, 4H), 2.83 (d, J = 10.3 Hz, 1H), 2.69 (s, 4H), 2.38 (s, 3H), 1.98 (s, 1H), 1.43 (d, J = 11.8 Hz, 1H).

**Embodiment 116**

*N*-(2,2'-Dichloro-3'-(5-(((*trans*-4-hydroxytetrahydrofuran-3-yl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

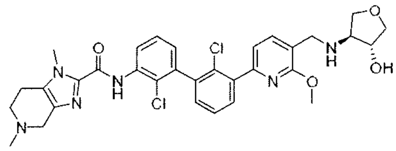

Preparation of *N*-(2,2'-dichloro-3'-(5-(((*trans*-4-hydroxytetrahydrofuran-3-yl)amino)methyl)-6-methoxypyridin-2-yl)-[1,1'-biphenyl]-3-yl)-1 ,5-dimethyl-4,5,6,7-tetrahydro-1*H*-imidazo[4,5-*c*]pyridine-2-carboxamide was referred to Embodiment 114.

MS *m/z* (ESI): 637.2 $[M+H]^+$.

**Embodiment 117**

*N*-(2,2'-Dichloro-3'-(5-((((3-fluorooxetan-3-yl)methyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

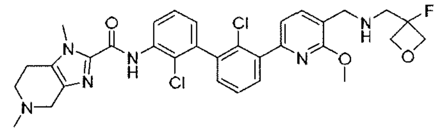

Preparation of *N*-(2,2'-dichloro-3'-(5-((((3-fluorooxetan-3-yl)methyl)amino)methyl)-6-methoxypyridin-2-yl)-[1,1'-biphenyl]-3-yl)-1,5-dimethyl-4,5,6,7-tetrahydro-1H-imidazo[4,5-*c*]pyridine-2-carboxamide was referred to Embodiment 114.

MS *m*/*z* (ESI): 639.2 $[M+H]^+$.

**Embodiment 118**

*N*-(2,2'-Dichloro-3'-(5-((((4-fluorotetrahydro-2*H*-pyran-4-yl)methyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

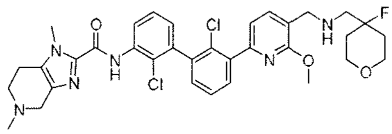

Preparation of *N*-(2,2'-dichloro-3'-(5-((((4-fluorotetrahydro-2*H*-pyran-4-yl)methyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m*/*z* (ESI): 667.2 $[M+H]^+$.

**Embodiment 119**

*N*-(2,2'-Dichloro-3'-(5-(((3-(fluoromethyl)oxetan-3-yl)methyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

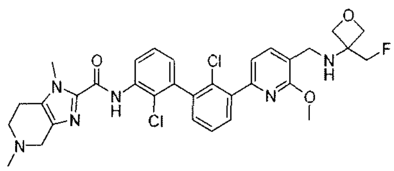

Preparation of *N*-(2,2'-dichloro-3'-(5-(((3-(fluoromethyl)oxetan-3-yl)methyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m*/*z* (ESI): 639.2 $[M+H]^+$.

**Embodiment 120**

*N*-(2,2'-Dichloro-3'-(5-((3-fluoroazetidin-1-yl)methyt)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

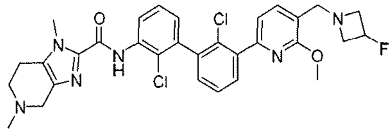

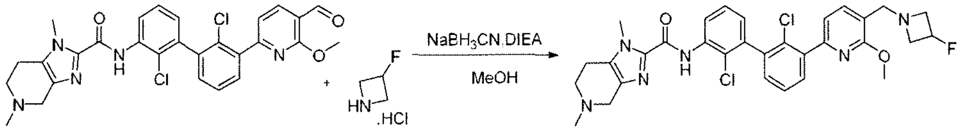


*N*-(2,2'-Dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (55.1 mg, 0.1 mmol) and 3-fluoroazetidine hydrochloride (33.3 mg, 0.3 mmol) were dissolved in methanol (3 mL), then DIPEA was added for neutralization; 3 drops of acetic acid were added, and the mixture was stirred at room temperature for 3 hours. After sodium cyanoborohydride (18.6 mg, 0.3 mmol) was added, the reaction mixture was continued to stir at room temperature overnight. After the reaction was completed, the reaction mixture was concentrated, and the residue was purified by reverse phase to obtain the title compound (14 mg, 23 %).

MS *m/z* (ESI): 609.3 $[M+H]^+$.

$^1H$ NMR (400 MHz, DMSO-$d_6$) δ 9.91 (s, 1H), 8.35 (d, J = 8.4 Hz, 1H), 7.69 (dd, J = 12.5, 7.5 Hz, 2H), 7.51 (dt, J = 23.9, 7.9 Hz, 2H), 7.41 (d, J = 7.6 Hz, 1H), 7.27 (d, J = 7.5 Hz, 1H), 7.18 (d, J = 7.6 Hz, 1H), 5.27 (d, J = 7.3 Hz, 1H), 5.12 (d, J = 7.1 Hz, 1H), 3.94 - 3.85 (m, 5H), 3.66 (d, J = 8.0 Hz, 4H), 3.39 (s, 2H), 3.30 - 3.22 (m, 1H), 3.20 (s, 1H), 2.70 (s, 4H), 2.39 (s, 3H).

**Embodiment 121**

*N*-(2,2'-Dichloro-3'-(5-((3-fluoro-3-methylazetidin-1-yl)methyl)-6-methoxypyridin-2-yl)-[1,1'-biphenyl]-3-yl)-1,5-dimethyl-4,5,6,7-tetrahydro-1*H*-imidazo[4,5-c]pyridine-2-carboxamide

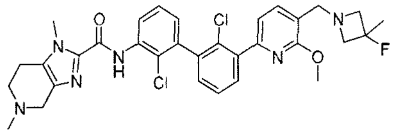

Preparation of *N*-(2,2'-dichloro-3'-(5-((3-fluoro-3-methylazetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 623.2 $[M+H]^+$.

**Embodiment 122**

1-((6-(2,2'-Dichloro-3'-(1,5-dimethyl-4,5,6imidazo[4,5-c]pyridine-2-carboxamido)-[1,1'-biphenyl]-3-yl)-2-methoxypyridin-3-yl)methyl)azetidin-3-yl acetate

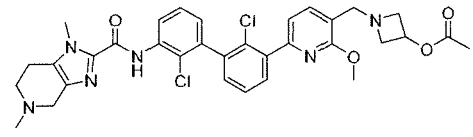

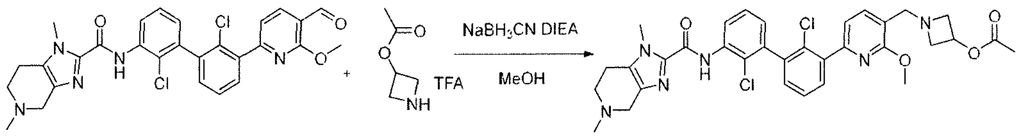


*N*-(2,2'-Dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (73 mg, 0.106 mmol) and azetidin-3-yl acetate trifluoroacetate (61 mg, 0.266 mmol) were dissolved in methanol (4 mL), then DIPEA was added for neutralization; 4 drops of acetic acid were added, and the mixture was stirred at room temperature for 3 hours. After sodium cyanoborohydride (16 mg, 0.266 mmol) was added, the reaction mixture was continued to stir at room temperature overnight. After the reaction was completed, the reaction mixture was concentrated, and the residue was purified by flash silica gel column chromatography (DCM: MeOH=10: 1) to obtain a crude product, which was purified by reverse phase to obtain the title compound (9.9 mg, 15 %).

MS *m*/*z* (ESI): 649.3 [M+H]+.

$^1$H NMR (400 MHz, DMSO-$d_6$) δ 9.91 (s, 1H), 8.38 - 8.31 (m, 1H), 7.74 - 7.64 (m, 2H), 7.54 (t, J = 7.6 Hz, 1H), 7.48 (t, J = 7.9 Hz, 1H), 7.41 (dd, J = 7.5, 1.7 Hz, 1H), 7.26 (d, J = 7.5 Hz, 1H), 7.18 (dd, J = 7.6, 1.6 Hz, 1H), 4.96 (t, J = 5.8 Hz, 1H), 3.91 (d, J = 3.9 Hz, 6H), 3.70 - 3.60 (m, 4H), 3.37 (s, 2H), 3.11 (dd, J = 8.5, 5.4 Hz, 2H), 2.69 (s, 4H), 2.38 (s, 3H), 2.03 (s, 3H).

**Embodiment 123**

*N*-(3'-(5-((6-Acetyl-2,6-diazaspiro[3.3]heptan-2-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

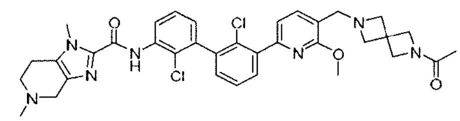

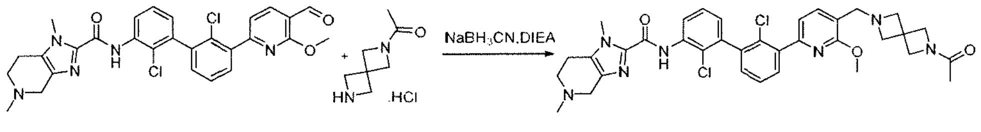


*N*-(2,2'-Dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (115 mg, 0.208 mmol) and 1-(2,6-diazaspiro[3.3]heptan-2-yl)ethan-1-one hydrochloride (110 mg, 0.625 mmol) were dissolved in methanol (4 mL), then DIPEA was added for neutralization; 8 drops of acetic acid were added, and the mixture was stirred at room temperature for 3 hours. After sodium cyanoborohydride (38.8 mg, 0.625 mmol) was added, the reaction mixture was continued to stir at room temperature overnight. After the reaction was completed, the reaction mixture was concentrated, and the residue was purified by reverse phase to obtain the title compound (23.4 mg, 17 %).

MS *m*/*z* (ESI): 674.3 [M+H]+.

$^1$H NMR (400 MHz, DMSO-$d_6$) δ 9.91 (s, 1H), 8.34 (dd, *J* = 8.3, 1.5 Hz, 1H), 7.75-7.64 (m, 2H), 7.52 (dt, *J* = 24.3, 7.8 Hz, 2H), 7.41 (dd, *J* = 7.6, 1.7 Hz, 1H), 7.28 (d, *J* = 7.4 Hz, 1H), 7.18 (dd, *J* = 7.6, 1.6 Hz, 1H), 4.19 (s, 2H), 3.91 (d, *J* = 4.1 Hz, 7H), 3.53 (d, *J* = 65.9 Hz, 9H), 2.74 (d, *J* = 20.1 Hz, 4H), 2.43 (s, 3H), 1.72 (s, 3H).

**Embodiment 124**

*N*-(3'-(5-((2-Oxa-6-azaspiro[3.3]heptan-6-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

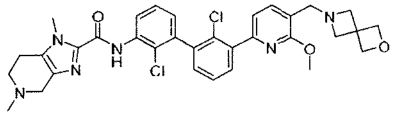

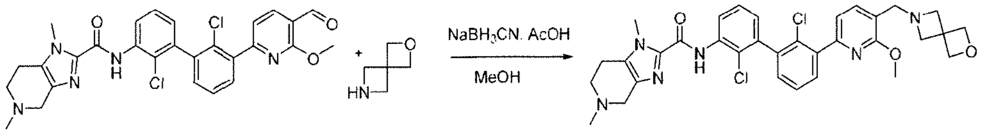


*N*-(2,2'-Dichloro-3'-(5-formyl-6-methoxypyridin-2-yl)-[1,1'-biphenyl]-3-y1)-1,5-dimethyl-4,5,6,7-tetrahydro-1*H*-imidazo[4,5-*c*]pyridine-2-carboxamide (50 mg, 0.091 mmol) and 2-oxa-6-azaspiro[3.3]heptane (27 mg, 0.272 mmol) were dissolved in methanol (3 mL), then 2 drops of acetic acid were added, and the mixture was stirred at room temperature for 2 hours. After sodium cyanoborohydride (14 mg, 0.227 mmol) was added, the reaction mixture was continued to stir at room temperature overnight. After the reaction was completed, the reaction mixture was concentrated, and the residue was purified by reverse phase to obtain the title compound (10 mg, 18 %).

MS *m/z* (ESI): 633.3 [M+H]$^+$.

$^1$H NMR (400 MHz, DMSO-$d_6$) $\delta$ 9.91 (s, 1H), 8.35 (d, J = 8.2 Hz, 1H), 7.67 (t, J = 5.8 Hz, 2H), 7.51 (dt, J = 23.1, 7.8 Hz, 2H), 7.40 (d, J = 7.5 Hz, 1H), 7.25 (d, J = 7.5 Hz, 1H), 7.18 (d, J = 7.7 Hz, 1H), 4.62 (s, 4H), 3.90 (s, 6H), 3.51 (s, 2H), 3.38 (d, J = 6.6 Hz, 6H), 2.69 (s, 4H), 2.38 (s, 3H).

**Embodiment 125**

*N*-(3'-(5-((5-Oxa-2-azaspiro[3.4]octan-2-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

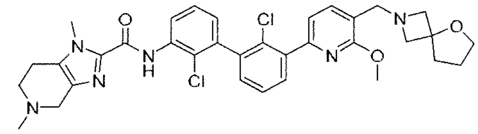

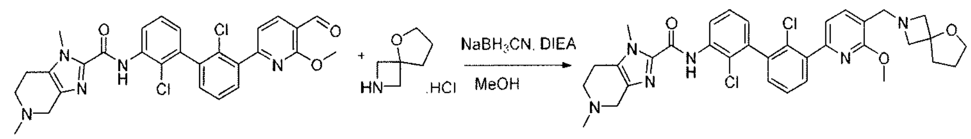


*N*-(2,2'-Dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (76 mg, 0.134 mmol) and 5-oxa-2-azaspiro[3.4]octane hydrochloride (31 mg, 0.206 mmol) were dissolved in methanol (4 mL), then DIPEA was added for neutralization; 4 drops of acetic acid were added, and the mixture was stirred at room temperature for 2 hours. After sodium cyanoborohydride (17 mg, 0.274 mmol) was added, the reaction mixture was continued to stir at room temperature overnight. After the reaction was completed, the reaction mixture was concentrated, and the residue was purified by reverse phase to obtain the title compound (6.9 mg, 8 %).

MS *m/z* (ESI): 647.3 [M+H]$^+$.

$^1$H NMR (400 MHz, DMSO-$d_6$) $\delta$ 9.91 (s, 1H), 8.35 (d, J = 8.2 Hz, 1H), 7.69 (dd, J = 15.2, 7.6 Hz, 2H), 7.51 (dt, J = 23.6, 7.8 Hz, 2H), 7.40 (d, J = 7.5 Hz, 1H), 7.26 (d, J = 7.5 Hz, 1H), 7.18 (d, J = 7.6 Hz, 1H), 3.90 (s, 6H), 3.67

(t, J = 6.8 Hz, 2H), 3.59 (s, 2H), 3.37 (s, 4H), 3.09 (d, J = 6.9 Hz, 2H), 2.69 (s, 4H), 2.38 (s, 3H), 2.04 (t, J = 7.2 Hz, 2H), 1.82 (q, J = 6.7 Hz, 2H).

**Embodiment 126**

*N*-(2,2'-Dichloro-3'-(6-methoxy-5-((6-carbonyl-2,5-diazaspiro[3.4]octan-2-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

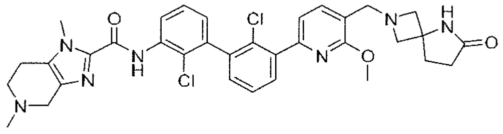

Preparation of *N*-(2,2'-dichloro-3'-(6-methoxy-5-((6-carbonyl-2,5-diazaspiro[3.4]octan-2-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m*/*z* (ESI): 660.2 $[M+H]^+$.

**Embodiment 127**

(*S*)-*N*-(2,2'-Dichloro-3'-(5-(((2-hydroxypropyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

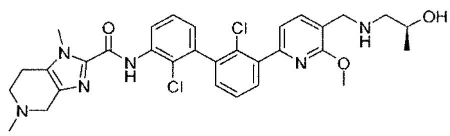

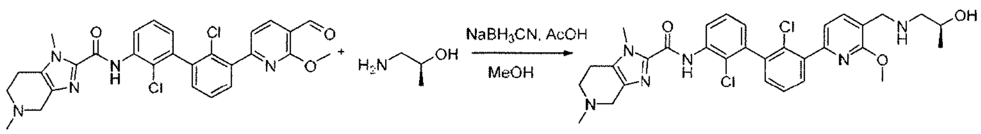


*N*-(2,2'-Dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (50 mg, 0.091 mmol) and (*S*)-1-aminopropan-2-ol (20 mg, 0.272 mmol) were dissolved in methanol (4 mL), then 2 drops of acetic acid were added, and the mixture was stirred at room temperature for 2 hours. After sodium cyanoborohydride (11 mg, 0.181 mmol) was added, the reaction mixture was continued to stir at room temperature overnight. After the reaction was completed, the reaction mixture was concentrated, and the residue was purified by reverse phase to obtain the title compound (4.4 mg, 8 %).

MS *m*/*z* (ESI): 609.4 $[M+H]^+$.

$^1$H NMR (400 MHz, DMSO-$d_6$) $\delta$ 9.91 (s, 1H), 8.35 (d, J = 8.3 Hz, 1H), 7.83 (d, J = 7.2 Hz, 1H), 7.68 (d, J = 7.3 Hz, 1H), 7.55 (t, J = 7.6 Hz, 1H), 7.49 (t, J = 8.2 Hz, 1H), 7.41 (d, J = 7.6 Hz, 1H), 7.29 (d, J = 7.4 Hz, 1H), 7.19 (d, J = 7.6 Hz, 1H), 3.92 (d, J = 11.7 Hz, 6H), 3.80 (s, 2H), 3.37 (s, 2H), 2.69 (s, 4H), 2.55 (s, 3H), 2.39 (s, 3H), 1.07 (d, J = 6.0 Hz, 3H).

**Embodiment 128**

(*R*)-*N*-(2,2'-Dichloro-3'-(5-(((2-hydroxypropyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

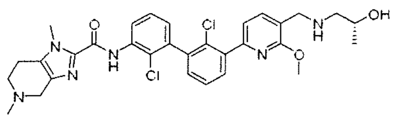

Preparation of (*R*)-*N*-(2,2'-dichloro-3'-(5-(((2-hydroxypropyl)amino)methyl)-6-methoxypyridin-2-yl)-[1,1'-biphenyl]-3-yl)-1,5-dimethyl-4,5,6,7-tetrahydro-1*H*-imidazo[4,5-c]pyridine-2-carboxamide was referred to Embodiment 114.
MS *m/z* (ESI): 609.2 [M+H]+.

## Embodiment 129

*N*-(2,2'-Dichloro-3'-(5-((3-hydroxyazetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

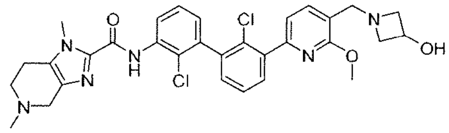

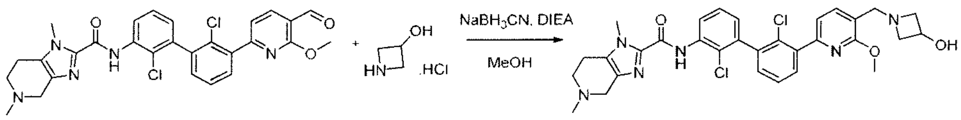


*N*-(2,2'-Dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (30 mg, 0.055 mmol) and azetidin-3-ol hydrochloride (24 mg, 0.217 mmol) were dissolved in methanol (3 mL), then DIPEA was added for neutralization; 3 drops of acetic acid were added, and the mixture was stirred at room temperature for 3 hours. After sodium cyanoborohydride (6.7 mg, 0.109 mmol) was added, the reaction mixture was continued to stir at room temperature overnight. After the reaction was completed, the reaction mixture was concentrated, and the residue was purified by reverse phase to obtain the title compound (5.3 mg, 16 %).
MS *m/z* (ESI): 607.2 [M+H]+.
$^1$H NMR (400 MHz, DMSO-$d_6$) $\delta$ 9.91 (s, 1H), 8.35 (d, J = 8.3 Hz, 1H), 7.68 (t, J = 7.1 Hz, 2H), 7.51 (dt, J = 23.4, 7.8 Hz, 2H), 7.40 (d, J = 7.8 Hz, 1H), 7.26 (d, J = 7.5 Hz, 1H), 7.18 (d, J = 7.7 Hz, 1H), 5.33 (s, 1H), 4.26 - 4.18 (m, 1H), 3.90 (d, J = 3.1 Hz, 5H), 3.58 (d, J = 11.1 Hz, 4H), 3.37 (s, 2H), 2.84 (t, J = 6.7 Hz, 2H), 2.69 (s, 4H), 2.38 (s, 3H), 2.05 - 1.97 (m, 1H).

## Embodiment 130

*N*-(2,2'-Dichloro-3'-(5-((3-hydroxy-3-methylazetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

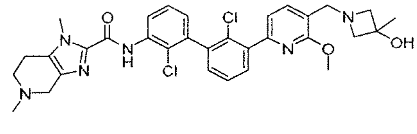

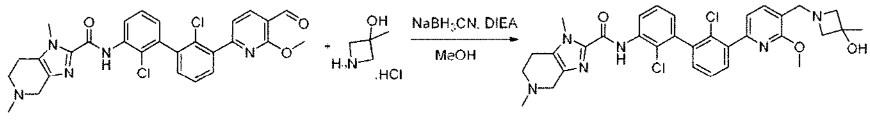


*N*-(2,2'-Dichloro-3'-(5-formyl-6-methoxypyridin-2-yl)-[1,1'-biphenyl]-3-yl)-1,5-dimethyl-4,5,6,7-tetrahydro-

1*H*-imidazo[4,5-*c*]pyridine-2-carboxamide (30 mg, 0.055 mmol) and 3-methylazetidin-3-ol hydrochloride (27 mg, 0.218 mmol) were dissolved in methanol (3 mL), then DIPEA was added for neutralization; 3 drops of acetic acid were added, and the mixture was stirred at room temperature for 3 hours. After sodium cyanoborohydride (6.8 mg, 0.109 mmol) was added, the reaction mixture was continued to stir at room temperature overnight. After the reaction was completed, the reaction mixture was concentrated, and the residue was purified by reverse phase to obtain the title compound (6.4 mg, 19 %).

MS m/z (ESI): 621.2 $[M+H]^+$.

$^1H$ NMR (400 MHz, DMSO-$d_6$) $\delta$ 9.91 (s, 1H), 8.35 (d, J = 8.4 Hz, 1H), 7.69 (t, J = 9.3 Hz, 2H), 7.51 (dt, J = 23.5, 7.8 Hz, 2H), 7.40 (d, J = 7.6 Hz, 1H), 7.27 (d, J = 7.4 Hz, 1H), 7.18 (d, J = 7.7 Hz, 1H), 3.93 - 3.86 (m, 6H), 3.59 (s, 2H), 3.37 (s, 2H), 3.29 (d, J = 6.6 Hz, 2H), 2.97 (d, J = 6.6 Hz, 2H), 2.69 (s, 4H), 2.39 (d, J = 3.4 Hz, 3H), 1.39 (s, 3H).

**Embodiment 131**

*N*-(2,2'-Dichloro-3'-(5-((3-hydroxy-3-(trifluoromethyl)azetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

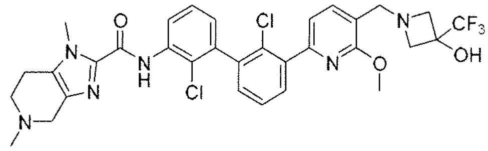

Preparation of *N*-(2,2'-dichloro-3'-(5-((3-hydroxy-3-(trifluoromethyl)azetidin-1-yl)methyl)-6-methoxypyridin-2-yl)-[1,1'-bipheny)]-3-yl)-1,5-dimethyl-4,5,6,7-tetrahydro-1*H*-imidazo[4,5-*c*]pyridine-2-carboxamide was referred to Embodiment 114.

MS *m/z* (ESI): 675.2 $[M+H]^+$.

**Embodiment 132**

*N*-(2,2'-Dichloro-3'-(5-((3-(fluoromethyl)-3-hydroxyazetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

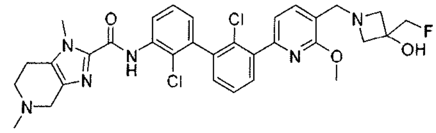

Preparation of *N*-(2,2'-dichloro-3'-(5-((3-(fluoromethyl)-3-hydroxyazetidin-1-yl)methyl)-6-methoxypyridin-2-yl)-[1,1'-biphenyl]-3-yl)-1 ,5-dimethyl-4,5,6,7-tetrahydro-1*H*-imidazo[4,5-*c*]pyridine-2-carboxamide was referred to Embodiment 114.

MS *m/z* (ESI): 639.2 $[M+H]^+$.

**Embodiment 133**

*N*-(2,2'-Dichloro-3'-(5-((3-fluoro-3-(hydroxymethyl)azetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

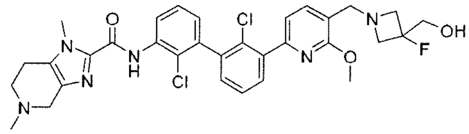

Preparation of *N*-(2,2'-dichloro-3'-(5-((3-fluoro-3-(hydroxymethyl)azetidin-1-yl)methyl)-6-methoxypyridin-2-yl)-[1,1'-biplienyl]-3-yl)-1,5-dimethyl-4,5,6,7-tetrahydro-1*H*-imidazo[4,5-c]pyridine-2-carboxamide was referred to Embodiment 114.

MS *m*/*z* (ESI): 639.2 [M+H]+.

**Embodiment 134**

1-((6-(2,2'-Dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-3-fluoroazetidine-3-carboxylic acid

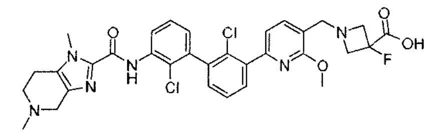

Preparation of 1-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-3-fluoroazetidine-3-carboxylic acid was referred to Embodiment 114.

MS m/z (ESI): 653.2 [M+H]+.

**Embodiment 135**

1-((6-(2,2'-Dichloro-3'-(1,5-dimethyl-4,5,6,7-tetrahydro-1*H*-imidazo[4,5-c]pyridine-2-carboxamido)-[1,1'-biphenyl]-3-yl)-2-methoxypyridin-3-yl)methyl)azetidine-3-carboxylic acid

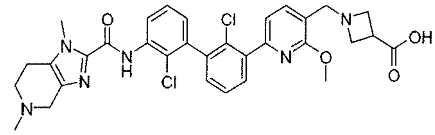

Preparation of 1-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)azetidine-3-carboxylic acid was referred to Embodiment 114.

MS *m*/*z* (ESI): 635.2 [M+H]+.

**Embodiment 136**

((6-(2,2'-Dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-*L*-proline

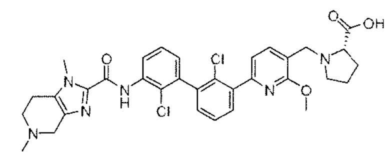

Preparation of ((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-*L*-proline was referred to Embodiment 114.
MS *m*/*z* (ESI): 649.2 [M+H]+.

## Embodiment 137

(*S*)-1-((6-(2,2'-Dichloro-3'-(1,5-dimethyl-4,5,6,7-tetrahydro-1*H*-imidazo[4,5-c]pyridine-2-carboxamido)-[1,1'-biphenyl]-3-yl)-2-methoxypyridin-3-yl)methyl)piperidme-2-carboxylic acid

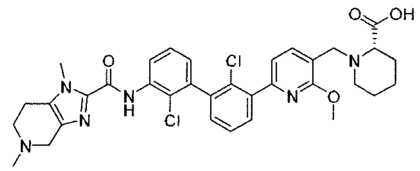

Preparation of (*S*)-1-((6-(2,2'-dichloro-3'-(1,5-dimethyl-4,5,6,7-tetrahydro-1*H*-imidazo[4,5-*c*]pyridine-2-carboxamido)-[1,1 '-biphenyl]-3-yl)-2-methoxypyridin-3-yl)methyl)piperidine-2-carboxylic acid was referred to Embodiment 114.
MS *m*/*z* (ESI): 663.2 [M+H]+.

## Embodiment 138

((6-(2,2'-Dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)glycine

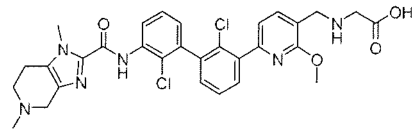

Preparation of ((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)glycine was referred to Embodiment 114.
MS *m*/*z* (ESI): 609.2 [M+H]+.

## Embodiment 139

((6-(2,2'-Dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-*L*-alanine

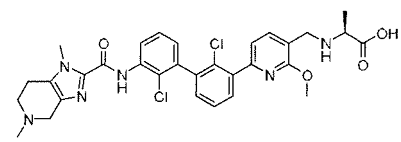

Preparation of ((6-(2,2'-dichloro-3'-(1,5-dimethyl-4,5,6,7-tetrahydro-1*H*-imidazo[4,5-c]pyridine-2-carboxamido)-[1,1-biphenyl]-3-yl)-2-methoxypyridin-3-yl)methyl)-*L*-alanine was referred to Embodiment 114.
MS *m*/*z* (ESI): 623.2 [M+H]+.

**Embodiment 140**

*N*-(2,2'-Dichloro-3'-(6-methoxy-5-(((2-(methylamino)-2-carbonylethyl)amino)methyl)pyridin-2-yl)-[1,1'-bipbenyl]-3-yl)-1,5-dimetbyl-4,5,6,7-tetrahydro-1*H*-imidazo[4,5-c]pyridine-2-carboxamide

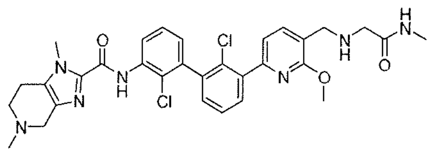

Preparation of *N*-(2,2'-dichloro-3'-(6-methoxy-5-(((2-(methylamino)-2-carbonylethyl)amino)methyl)pyridin-2-yl)-[1,1'-biphenyl]-3-yl)-1,5-dimethyl-4,5,6,7-tetrahydro-1*H*-imidazo[4,5-c]pyridine-2-carboxamide was referred to Embodiment 114.
MS *m/z* (ESI): 622.2 $[M+H]^+$.

**Embodiment 141**

Ethyl ((6-(2,2'-dichloro-3'-(1,5-dimethyl-4,5,6,7-tetrahydro-1*H*-imidazo[4,5-c]pyridine-2-carboxamido)-[1,1'-biphenyl]-3-yl)-2-methoxypyridin-3-yl)methyl)glycinate

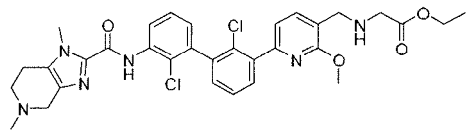

Preparation of ethyl ((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)glycinate was referred to Embodiment 114.
MS *m/z* (ESI): 637.2 $[M+H]^+$.

**Embodiment 142**

Ethyl ((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-L-alaninate

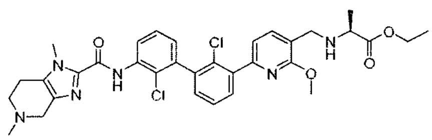

Preparation of ethyl ((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-L-alaninate was referred to Embodiment 114.
MS *m/z* (ESI): 651.2 $[M+H]^+$.

**Embodiment 143**

Ethyl 1-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)azetidine-3-carboxylate

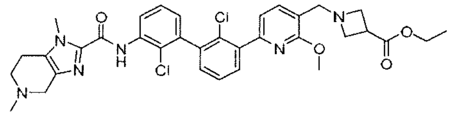

Preparation of ethyl 1-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)azetidine-3-carboxylate was referred to Embodiment 114.
MS *m/z* (ESI): 663.2 [M+H]+.

## Embodiment 144

*N*-(2-Chloro-2'-fluoro-3'-(6-methoxy-5-((oxetan-3-ylamino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

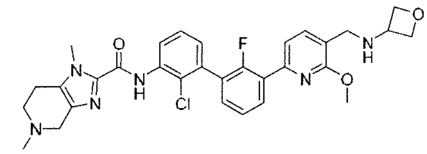

Preparation of *N*-(2-chloro-2'-fluoro-3'-(6-methoxy-5-((oxetan-3-ylamino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.
MS *m/z* (ESI): 591.2 [M+H]+.

## Embodiment 145

*N*-(3'-(5-((6-Acetyl-2,6-diazaspiro[3.3]heptan-2-yl)methyl)-6-methoxypyridin-2-yl)-2-chloro-2'-fluoro-[1,1'-biphenyl]-3-yl)-1,5-dimethyl-4,5,6,7-tetrahydro-1*H*-imidazo[4,5-*c*]pyridine-2-carboxamide

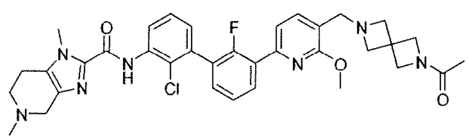

Preparation of *N*-(3'-(5-((6-acetyl-2,6-diazaspiro[3.3]heptan-2-yl)methyl)-6-methoxypyridin-2-yl)-2-chloro-2'-fluoro-[1,1'-biphenyl]-3-yl)-1,5-dimethyl-4,5,6,7-tetrahydro-1*H*-imidazo[4,5-c]pyridine-2-carboxamide was referred to Embodiment 114.
MS *m/z* (ESI): 658.2 [M+H]+.

## Embodiment 146

*N*-(2-Chloro-2'-fluoro-3'-(5-((3-fluoroazetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

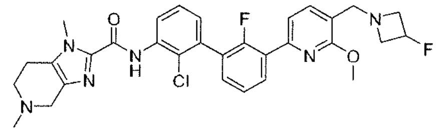

Preparation of *N*-(2-chloro-2'-fluoro-3'-(5-((3-fluoroazetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 593.2 [M+H]+.

**Embodiment 147**

*N*-(2,2'-Dichloro-3'-(6-methoxy-4-methyl-5-((oxetan-3-ylamino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

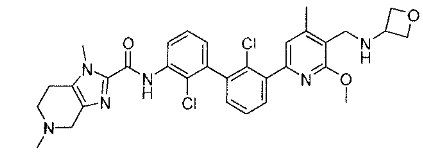

Preparation of *N*-(2,2'-dichloro-3'-(6-methoxy-4-methyl-5-((oxetan-3-ylamino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 621.2 [M+H]+.

**Embodiment 148**

*N*-(3'-(5-((6-Acetyl-2,6-diazaspiro[3.3]heptan-2-yl)methyl)-6-methoxy-4-methylpyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

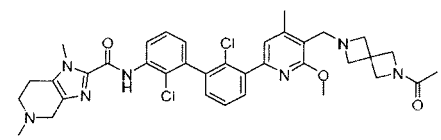

Preparation of *N*-(3'-(5-((6-acetyl-2,6-diazaspiro[3.3]heptan-2-yl)methyl)-6-methoxy-4-methylpyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 688.2 [M+H]+.

**Embodiment 149**

*N*-(2,2'-Dichloro-3'-(5-((3-fluoroazctidin-1-yl)methyl)-6-methoxy-4-methylpyridin-2-yl)-[1,1'-biphenyl]-3-yl)-1,5-dimethyl-4,5,6,7-tetrahydro-1*H*-imidazo[4,5-*c*]pyridine-2-carboxamide

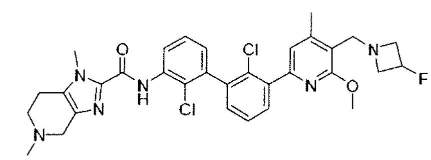

Preparation of *N*-(2,2'-dichloro-3'-(5-((3-fluoroazetidin-1-yl)methyl)-6-methoxy-4-methylpyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 623.2 [M+H]+.

**Embodiment 150**

*N*-(3'-(5-((6-Acetyl-2,6-diazaspiro[3.3]heptan-2-yl)methyl)-6-chloro-4-methylpyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

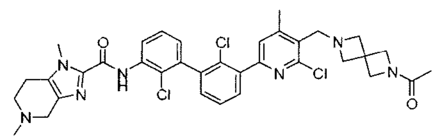

Preparation of *N*-(3'-(5-((6-acetyl-2,6-diazaspiro[3.3]heptan-2-yl)methyl)-6-chloro-4-methylpyridin-2-yl)-2,2'-dichloro-[1,1'-biphenyl]-3-yl)-1,5-dimethyl-4,5,6,7-tetrahydro-1*H*-imidazo[4,5-c]pyridine-2-carboxamide was referred to Embodiment 114.

MS *m/z* (ESI): 692.2 $[M+H]^+$.

**Embodiment 151**

*N*-(3'-(5-((6-Acetyl-2,6-diazaspiro[3.3]heptan-2-yl)methyl)-6-chloropyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

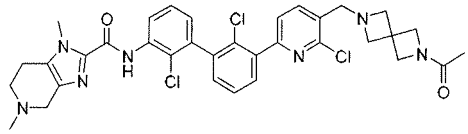

Preparation of *N*-(3'-(5-((6-acetyl-2,6-diazaspiro[3.3]heptan-2-yl)methyl)-6-chleropyridin-2-yl)-2,2'-dich]oro-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 678.2 $[M+H]^+$.

**Embodiment 152**

*N*-(3'-(5-((6-Acetyl-2,6-diazaspiro[3.3]heptan-2-yl)methyl)-6-methoxypyrazin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

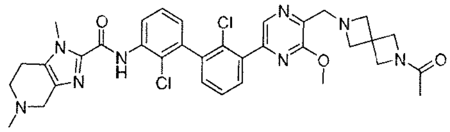

Preparation of *N*-(3'-(5-((6-acetyl-2,6-diazaspiro[3.3]heptan-2-yl)methyl)-6-methoxypyrazin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 114, and the specific synthetic route was as follows:

Step 1: Preparation of 5-bromo-3-methoxypyrazin-2-amine

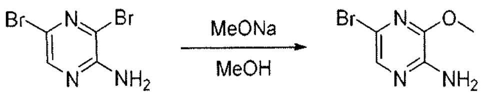


MeONa (11.74 g, 217.48 mmol) was added to MeOH (260 mL) at room temperature, then the mixture was stirred until dissolved, then 3,5-dibromopyrazin-2-amine (50 g, 197.71 mmol) was added, and the system was heated and refluxed for 3 hours. The reaction mixture was cooled to room temperature, filtered, and the filter cake was dried under reduced pressure to obtain the title compound as a brown solid (39.5 g, 93 %).

MS *m/z* (ESI): 204.0 $[M+H]^+$.

Step 2: Preparation of 5-bromo-2-iodo-3-methoxypyrazine

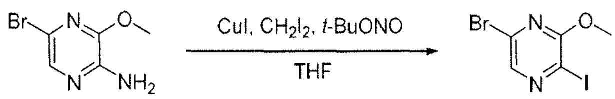


At room temperature, *t*-BuONO (16.15 g, 156.84 mmol) was added dropwise a solution of 5-bromo-3-methoxypyrazin-2-amine (10 g, 49.01 mmol), $CH_2I_2$ (14.45 g, 53.92 mmol) and CuI (10.27 g, 53.92 mmol) in THF (100 mL), then the reaction system was heated to reflux (internal temperature was 66 °C) for 3 hours. After the reaction mixture was cooled, the reaction mixture was filtered through a pad of celite, and the filtrate was concentrated under reduced pressure. The residue was dissolved in EA, then washed with saturated aqueous sodium thiosulfate solution and saturated brine respectively, dried over anhydrous sodium sulfate; the mixture was filtered, then the filtrate was concentrated under reduced pressure, and the residue was purified by silica gel column chromatography (PE/EA=10/1 to 5/1) to obtain the title compound as a brown solid (10.3 g, 67 %).

MS *m/z* (ESI): 315.0 $[M+H]^+$.

Step 3: Preparation of 5-bromo-3-methoxy-2-vinylpyrazine

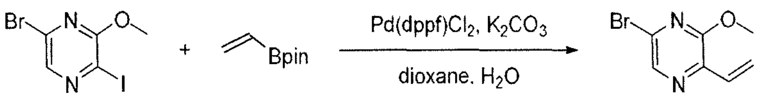


$Pd(dppf)Cl_2$ (1.78 g, 2.43 mmol), $K_2CO_3$ (20.11 g, 145.76 mmol) were added to a solution of 5-bromo-2-iodo-3-methoxypyrazine (15.3 g, 48.59 mmol) and pinacol vinylboronate (8.98 g, 58.30 mmol, 9.89 mL) in a mixed solvent of 1,4-dioxane (150 mL) and water (15 mL), after the reaction system was replaced with nitrogen, the reaction system was stirred at 60 °C for 8 hours. The reaction mixture was cooled and filtered, and the filtrate was concentrated under reduced pressure, and the residue was separated and purified by silica gel column chromatography (PE/EA=5/1 to 2/1) to obtain the title compound as a brown oil (9.83 g, 94 %).

MS *m/z* (ESI): 215.0 $[M+H]^+$.

Step 4: Preparation of 5-bromo-3-methoxypyrazine-2-carbaldehyde

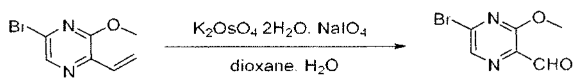


At 0 °C, $NaIO_4$ (25.42 g, 118.85 mmol) was added to a solution of 5-bromo-3-methoxy-2-vinylpyrazine (9.83 g, 45.71 mmol) and $K_2OsO_4.2H_2O$ (336.43 mg, 914.22 μmol) in a mixed solvent of 1,4-dioxane (180 mL) and $H_2O$ (60 mL) in batches. After the addition was completed, the mixture was wanned to room temperature and stirred for 1.5 hours. The reaction mixture was filtered, and the filtrate was concentrated under reduced pressure; the residue was diluted

with DCM, washed with saturated aqueous sodium bicarbonate solution, dried over anhydrous sodium sulfate, then the mixture was concentrated under reduced pressure, and then purified by silica gel column chromatography (PE/EA=100/1 to 3/ 1) to obtain the title compound as a light brown solid (2.83 g, 29 %).

MS *m/z* (ESI): 217.0 [M+H]+.

Step 5: Preparation of 5-(3-bromo-2-chlorophenyl)-3-methoxypyrazine-2-carbaldehyde

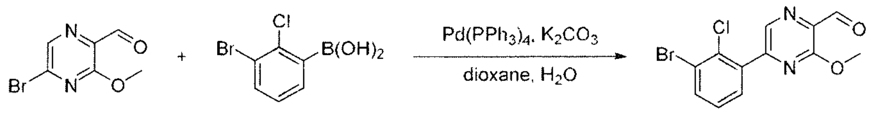


$Pd(PPh_3)_4$ (407.14 mg, 352.50 μmol), $K_2CO_3$ (1.17 g, 8.46 mmol) were added to a solution of (3-bromo-2-chlorophenyl)boronic acid (995.20 mg, 4.23 mmol)and 5-bromo-3-methoxypyrazine-2-carbaldehyde (0.765 g, 3.53 mmol) in a mixed solvent of 1,4-dioxane (15 mL) and water (1.5 mL), then the reaction system was replaced with nitrogen, and stirred at 95 °C for 6 hours. After the reaction mixture was cooled, the reaction mixture was concentrated under reduced pressure. The residue was partitioned between DCM (50 mL) and water (20 mL), and the organic phase was concentrated under reduced pressure, and then subjected to column chromatography (PE/EA=5/1 to 2/1) to obtain the title compound as a light brown solid (430 mg, 37 %).

MS *m/z* (ESI): 327.0 [M+H]+.

Step 6: Preparation of 5-(2-chloro-3-(4,4,5,5-tetramethyl-1,3,2-dioxaborolan-2-yl)phenyl)-3-methoxypyrazine-2-carbaldehyde

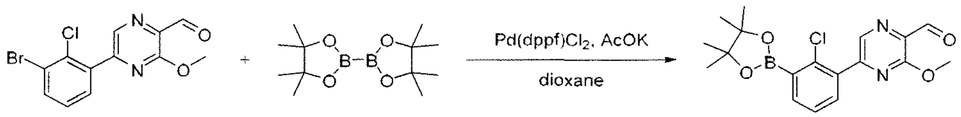


$Pd(dppf)Cl_2$ (189.69 mg, 259.49 μmol) was added to a solution of bis(pinacolato)diboron (790.74 mg, 3.11 mmol), 5-(3-bromo-2-chlorophenyl)-3-methoxylpyrazine-2-carbaldehyde (850 mg, 2.59 mmol) and KOAc (762.91 mg, 7.78 mmol) in 1,4-dioxane (40 mL) at room temperature, then the reaction system was replaced with nitrogen and then heated to 95 °C and stirred for 2.5 hours. The reaction mixture was concentrated under reduced pressure, and the residue was diluted with dichloromethane, then the mixture was filtered; the filtrates were combined, then concentrated under reduced pressure, and the residue was purified by silica gel column chromatography (PE/EA=100/1 to 3/1) to obtain the title compound as a brown solid (630 mg, 65 %).

Step 7: Preparation of *N*-(2,2'-dichloro-3'-(5-formyl-6-methoxypyrazin-2-yl)-[1,1'-biphenyl]-3-yl)-1,5-dimethyl-4,5,6,7-tetrahydro-1*H*-imidazo[4,5-*c*]pyridine-2-carboxamide

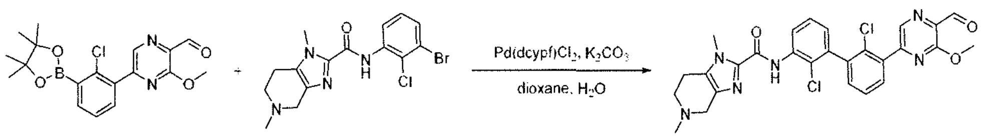


$Pd(dcypf)Cl_2$ (115.58 mg, 152.88 μmol) was added to a solution of *N*-(3-bromo-2-chlorophenyl)-1,5-dimethyl-4,5,6,7-tetrahydro-1*H*-imidazo[4,5-*c*]pyridine-2-carboxamide (586.55 mg, 1.53 mmol), 5-(2-chloro-3-(4,4,5,5-tetramethyl-1,3,2-dioxaborolan-2-yl)phenyl)-3-methoxypyrazine-2-carbaldehyde (630 mg, 1.68 mmol) and $K_2CO_3$ (506.34 mg, 3.67 mmol) in a mixed solvent of 1,4-dioxane (10 mL) and $H_2O$ (1 mL), then the reaction system was replaced with nitrogen three times, and heated to 95 °C and the reaction was carried out for 6 hours. After the reaction mixture was cooled, the layers were separated, and the organic layer was concentrated under reduced pressure; the residue was

diluted with DCM, washed with water and saturated brine, dried over sodium sulfate, and the mixture was filtered; the filtrate was concentrated under reduced pressure, and then subjected to column chromatography (DCM/MeOH=50/1 to 10/1) to obtain the title compound as a light yellow solid (260 mg, 31 %).

MS *m/z* (ESI): 551.2 [M+H]+.

Step 8: Preparation of *N*-(3'-(5-((6-acetyl-2,6-diazaspiro[3.3]heptan-2-yl)methyl)-6-methoxypyrazin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

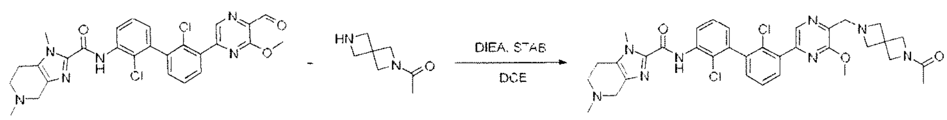


At room temperature, DIPEA (305.16 mg, 2.36 mmol, 411.26 μL) was added to a solution of *N*-(2,2'-dichloro-3'-(5-formyl-6-inethoxypyrazin-2-yl)-[1,1'-biphenyl]-3-yl)-1,5-dimethyl-4,5,6,7-tetrahydro-1*H*-imidazo[4,5-*c*]pyridine-2-carboxamide (260 mg, 472 μmol) and 1-(2,6-diazaspiro[3.3]heptan-2-yl)ethanone trifluoroformate (357.76 mg, 708.35 μmol, 3.2 TFA) in DCE (10 mL), and the reaction mixture was stirred at room temperature for 1 hour, then STAB (298.92 mg, 1.42 mmol) was added, and the reaction mixture was stirred at room temperature for 1 hour. The reaction mixture was partitioned between saturated aqueous sodium bicarbonate solution and DCM. The organic layer was concentrated under reduced pressure, and then separated and purified by silica gel column chromatography and prep-HPLC successively to obtain the title compound as an off-white solid (70 mg, 20 %).

MS *m/z* (ESI): 675.2 [M+H]+.

$^1$H NMR (400 MHz, DMSO-$d_6$) δ 9.91 (s, 1H), 8.42 (s, 1H), 8.35 (dd, J = 8.3, 1.5 Hz, 1H), 7.77-7.69 (m, 1H), 7.64-7.55 (m, 1H), 7.52-7.45 (m, 2H), 7.23-7.16 (m, 1H), 4.16 (s, 2H), 3.96 (s, 3H), 3.90 (s, 3H), 3.88 (s, 2H), 3.70 (s, 2H), 3.41 (s, 4H), 3.36 (s, 2H), 2.72-2.65 (m, 4H), 2.38 (s, 3H), 1.71 (s, 3H).

**Embodiment 153**

*N*-(3'-(5-((6-Acetyl-2,6-diazaspiro[3.3]heptan-2-yl)methyl)-6-cyanopyridin-2-yl)-2,2'-dichloro-[1,1'-biphenyl]-3-yl)-1,5-dimethyl-4,5,6,7-tetrahydro-1*H*-imidazo[4,5-c]pyridine-2-carboxamide

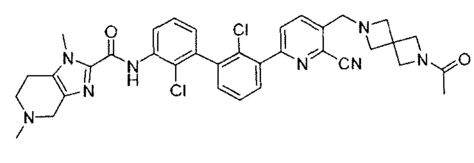

Preparation of *N*-(3'-(5-((6-acetyl-2,6-diazaspiro[3.3]heptan-2-yl)methyl)-6-cyanopyridin-2-yl)-2,2'-dichloro-[1,1'-biphenyl]-3-yl)-1,5-dimethyl-4,5,6,7-tetrahydro-1*H*imidazo[4,5-*c*]pyridine-2-carboxamidee was referred to Embodiment 114.

MS *m/z* (ESI): 669.2 [M+H]+.

**Embodiment 154**

*N*-(3'-(5-((6-Acetyl-2,6-diazaspiro[3.3]heptan-2-yl)methyl)-6-(trifluoromethyl)pyridin-2-yl)-2,2'-dichloro-[1,1'-biphenyl]-3-yl)-1,5-dimetliyl-4,5,6,7-tetrahydro-1*H*-imidazo[4,5-*c*]pyridine-2-carboxamide

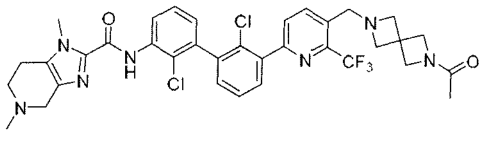

Preparation of *N*-(3'-(5-((6-acetyl-2,6-diazaspiro[3.3]heptan-2-yl)methyl)-6-(trifluoromethyl)pyridin-2-yl)-2,2'-dichloro-[1,1'-biphenyl]-3-yl)-1,5-dimethyl-4,5,6,7-tetrahydro-1*H*-imidazo[4,5-c]pyridine-2-carboxamide was referred to Embodiment 114.

MS *m/z* (ESI): 712.2 [M+H]+.

**Embodiment 155**

*N*-(2,2'-Dichloro-3'-(5-((3-fluoroazetidin-1-yl)methyl)-6-methylpyridin-2-yl)-[1,1'-biphenyl]-3-yl)-1,5-dimethyl-4,5,6,7-tetrahydro-1*H*-imidazo[4,5-*c*]pyridine-2-carboxamide

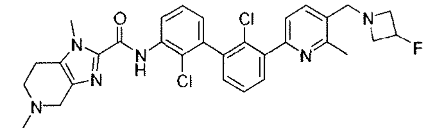

Preparation of *N*-(2,2'-dichloro-3'-(5-((3-fluoroazetidin-1-yl)methyl)-6-methylpyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 593.2 [M+H]+.

**Embodiment 156**

*N*-(2,2'-Dichloro-4"-((3-fluoroazetidin-1-y])methyl)-3"-methoxy-[1,1':3',1"-terphenyl]-3-yl)-1,5-dimethyl-4,5,6,7-tetrahydro-1*H*-imidazo[4,5-*c*]pyridine-2-carboxamide

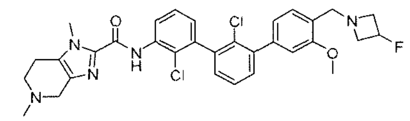

Preparation of *N*-(2,2'-dichloro-4"-((3-fluoroazetidin-1-yl)methyl)-3"-methoxy-[1,1':3',1"-terphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 608.2 [M+H]+.

**Embodiment 157**

*N*-(2,2'-Dichloro-4"-(((2-hydroxyethyl)amino)methyl)-3"-methoxy-[1,1':3',1"-terphenyl]-3-yl)-1,5-dimethyl-4,5,6,7-tetrahydro-1*H*-imidazo[4,5-*c*]pyridine-2-carboxamide

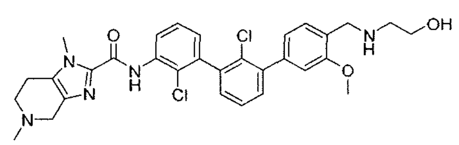

Preparation of *N*-(2,2'-dichloro-4"-(((2-hydroxyethyl)amino)methyl)-3"-methoxy-[1,1':3',1"-terphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 594.2 [M+H]+.

**Embodiment 158**

(*R*)-*N*-(2,2'-Dichloro-4"-(((2-hydroxypropyl)amino)methyl)-3"-methoxy-[1,1':3',1"-terphenyl]-3-yl)-1,5-dimethyl-4,5,6,7-tetrahydro-1*H*-imidazo[4,5-*c*]pyridine-2-carboxamide

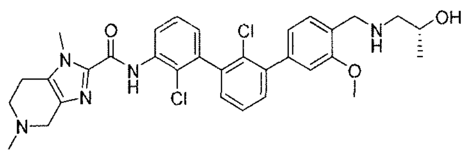

Preparation of (*R*)-*N*-(2,2'-dichloro-4"-(((2-hydroxypropyl)amino)methyl)-3"-methoxy-[1,1':3',1"-terphenyl]-3-yl)-1,5-dimethyl-4,5,6,7-tetrahydro-1*H*-imidazo[4,5-*c*]pyridine-2-carboxamide was referred to Embodiment 114.
MS *m/z* (ESI): 608.2 $[M+H]^+$.

**Embodiment 159**

((2',2"-Dichtoro-3"-(1,5-dimethyl-4,5,6,7-tetrahydro-1*H*-imidazo[4,5-*c*]pyridine-2-carboxamido)-3-methoxy-[1,1':3',1"-terphenyl]-4-yl)methyl)glycine

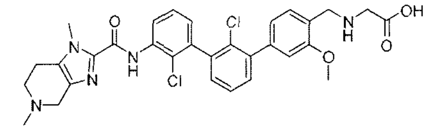

Preparation of ((2',2"-dichloro-3"-(1,5-dimethyl-4,5,6,7-tetrahydro-1*H*-imidazo[4,5-*c*]pyridine-2-carboxamido)-3-methoxy-[1,1':3',1"-terphenyl]-4-yl)methyl)glycine was referred to Embodiment 114.
MS *m/z* (ESI): 608.2 $[M+H]^+$.

**Embodiment 160**

1,5-Dimethyl-*N*-(2,2',3"-trichloro-4"-((3-fluoroazetidin-1-yl)methyl)-[1,1':3',1"-terphenyl]-3-yl)-4,5,6,7-tetrahydro-1*H*-imidazo[4,5-*c*]pyridine-2-carboxamide

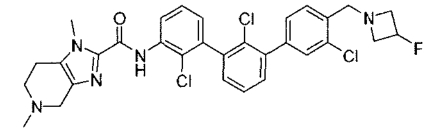

Preparation of 1,5-dimethyl-*N*-(2,2',3"-trichloro-4"-((3-fluoroazetidin-1-yl)methyl)-[1,1': 3',1"-terphenyl]-3-yl)-4,5,6,7-tetrahydro-1*H*-imidazo[4,5-*c*]pyridine-2-carboxamide was referred to Embodiment 114.
MS *m/z* (ESI): 612.2 $[M+H]^+$.

**Embodiment 161**

*N*-(2,2'-Dichloro-4"-((3-fluoroazetidin-1-yl)methyl)-3"-methyl-[1,1':3',1"-terphenyl]-3-yl)-1,5-dimethyl-4,5,6,7-tetrahydro-1*H*-imidazo[4,5-*c*]pyridine-2-carboxamide

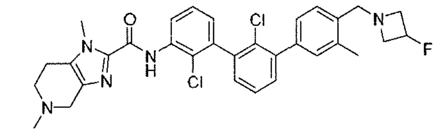

Preparation of N-(2,2'-dichloro-4"-((3-fluoroazetidin-1-yl)methyl)-3"-methyl-[1,1':3',1"-terphenyl]-3-yl)-1,5-dimethyl-4,5,6,7-tetrahydro-1H-imidazo[4,5-c]pyridine-2-carboxamide was referred to Embodiment 114.
MS m/z (ESI): 592.2 [M+H]+.

**Embodiment 162**

N-(2,2'-Dichloro-3'-(5-methoxy-6-((oxetan-3-ylamino)methyl)pyridin-3-yl)-[1,1'-biphenyl]-3-yl)-1,5-dimethyl-4,5,6,7-tetrahydro-1H-imidazo[4,5-c]pyridine-2-carboxamide

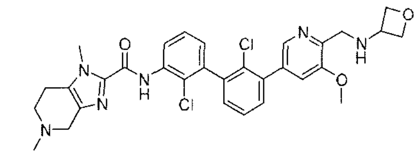

Preparation of N-(2,2'-dichloro-3'-(5-methoxy-6-((oxetan-3-ylamino)methyl)pyridin-3-yl)-[1,1'-biphenyl]-3-yl)-1,5-dimethyl-4,5,6,7-tetrahydro-1H-imidazo[4,5-c]pyridine-2-carboxamide was referred to Embodiment 114.
MS m/z (ESI): 607.2 [M+H]+.

**Embodiment 163**

N-(2,2'-Dichloro-3'-(6-((3-fluoroazetidin-1-yl)methyl)-5-methoxypyridin-3-yl)-[1,1'-biphenyl]-3-yl)-1,5-dimethyl-4,5,6,7-tetrahydro-1H-imidazo[4,5-c]pyridine-2-carboxamide

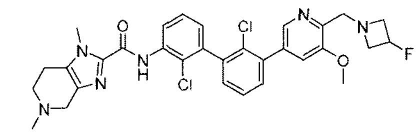

Preparation of N-(2,2'-dichloro-3'-(6-((3-fluoroazetidin-1-yl)methyl)-5-methoxypyridin-3-yl)-[1,1'-biphenyl]-3-yl)-1,5-dimethyl-4,5,6,7-tetrahydro-1H-imidazo[4,5-c]pyridine-2-carboxamide was referred to Embodiment 114.
MS m/z (ESI): 609.2 [M+H]+.

**Embodiment 164**

N-(2,2'-Dichloro-3'-(6-((3-fluoroazetidin-1-yl)methyl)-5-(trifluoromethyl)pyridin-3-yl)-[1,1'-biphenyt]-3-yl)-1,5-dimethyl-4,5,6,7-tetrahydro-1H-imidazo[4,5-c]pyridine-2-carboxamide

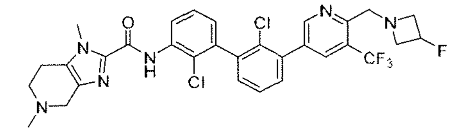

Preparation of N-(2,2'-dichloro-3'-(6-((3-fluoroazetidin-1-yl)methyl)-5-(trifluoromethyl)pyridin-3-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 647.2 [M+H]+.

**Embodiment 165**

*N*-(2,2'-Dichloro-3'-(6-((3-fluoroazetidin-1-yl)methyl)-5-methylpyridin-3-yl)-[1,1'-biphenyl]-3-yl)-1,5-dimethyl-4,5,6,7-tetrahydro-1*H*-imidazo[4,5-*c*]pyridine-2-carboxamide

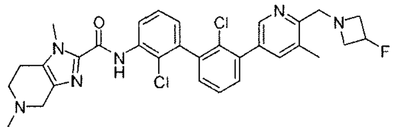

Preparation of *N*-(2,2'-dichloro-3'-(6-((3-fluoroazetidin-1-yl)methyl)-5-methylpyridin-3-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 593.2 [M+H]+.

**Embodiment 166**

*N*-(2,2'-Dichloro-3'-(5-fluoro-6-((3-fluoroazetidin-1-yl)methyl)pyridin-3-yl)-[1,1'-biphenyl]-3-yl)-1,5-dimethyl-4,5,6,7-tetrahydro-1*H*-imidazo[4,5-*c*]pyridine-2-carboxamide

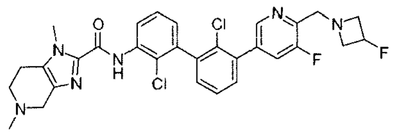

Preparation of *N*-(2,2'-dichloro-3'-(5-fluoro-6-((3-fluoroazetidin-1-yl)methyl)pyridin-3-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 597.2 [M+H]+.

**Embodiment 167**

*N*-(2,2'-Dichloro-3'-(6-((3-fluoroazetidin-1-yl)methyl)-2-methoxypyridin-3-yl)-[1,1'-biphenyl]-3-yl)-1,5-dimethyl-4,5,6,7-tetrahydro-1*H*-imidazo[4,5-c]pyridine-2-carboxamide

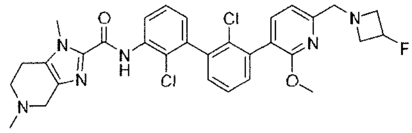

Preparation of *N*-(2,2'-dichloro-3'-(6-((3-fluoroazetidin-1-yl)methyl)-2-methoxypyridin-3-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 609.2 [M+H]+.

**Embodiment 168**

*N*-(2-Chloro-3'-(4-((3-fluoroazetidin-1-yl)methyl)-5-methylthioazol-2-yl)-2'-methyl-[1,1'-biphenyl]-3-yl)-1,5-dimethyl-4,5,6,7-tetrahydro-1*H*-imidazo[4,5-*c*]pyridine-2-carboxamide

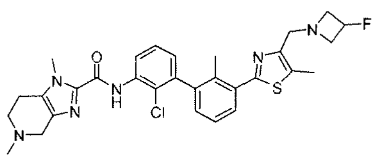

Step 1: Preparation of methyl 2-(3-amino-2-methylphenyl)-5-methylthioazole-4-carboxylate

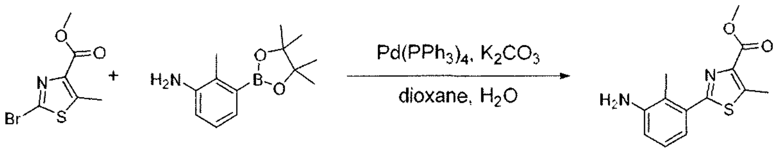


Methyl 2-bromo-5-methylthiazole-4-carboxylate (754 mg, 3.20 mmol), 2-methyl-3-(4,4,5,5-tetramethyl-1,3,2-dioxaboran-2-yl)aniline (782 mg, 3.36 mmol), tetrakis(triphenylphosphine)palladium (369 mg, 0.320 mmol), potassium carbonate (882 mg, 6.39 mmol) were added to a mixed solvent of 1,4-dioxane (10 mL) and water (1 mL), then the reaction system was replaced by nitrogen, and the reaction mixture was heated to 100 °C and the reaction was carried out for 4 hours. The layers of reaction mixture was separated, then the organic layer was concentrated under reduced pressure, and the residue was separated by silica gel column chromatography to obtain the title compound as a brown oil (472 mg, 56 %).
MS *m/z* (ESI): 263.1 $[M+H]^+$.

Step 2: Preparation of methyl 2-(3-bromo-2-methylphenyl)-5-methylthioazole-4-carboxylate

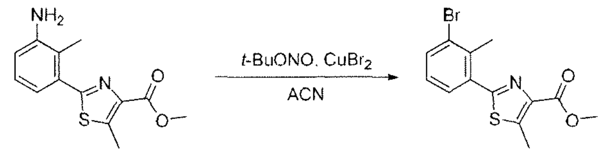


*tert-Butyl* nitrite (371 mg, 3.60 mmol) was added dropwise to a solution of cuprous bromide (611 mg, 2.70 mmol) and methyl 2-(3-amino-2-methylphenyl)-5-methylthioazole-4-carboxylate (472 mg, 1.80 mmol) in acetonitrile (6 mL) at room temperature, and the reaction mixture was heated to 60 °C and the reaction was carried out for 20 minutes. The reaction mixture was concentrated under reduced pressure, and the residue was separated by silica gel column chromatography to obtain the title compound as a brown solid (374 mg, 64 %).
$^1H$ NMR (400 MHz, $CDCl_3$) $\delta$ 7.68 (d, *J* = 7.9 Hz, 1H), 7.54 (d, *J* = 7.7 Hz, 1H), 7.14 (t, *J* = 7.8 Hz, 1H), 3.91 (d, *J* = 2.2 Hz, 3H), 2.81 (s, 3H), 2.60 (s, 3H).
MS *m/z* (ESI): 326.0 $[M+H]^+$.

Step 3: Preparation of (2-(3-bromo-2-methylphenyl)-5-methylthioxazol-4-yl)methanol

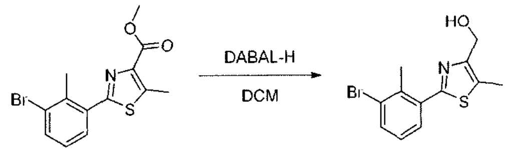


DABAL-H (0.825 mL, 1.5 M solution in toluene) was added dropwise to a solution of methyl 2-(3-bromo-2-methylphenyl)-5-methylthioazole-4-carboxylate (134 mg, 0.412 mmol) in dichloromethane (4 mL) at -78 °C. After the dropwise addition was completed, the temperature was naturally raised to room temperature and the reaction mixture was stirred for 15 minutes. The reaction mixture was cooled to 0 °C, and methanol (1 mL) was added dropwise to quench the reaction, then saturated aqueous sodium tartrate (5 mL) was added, then the reaction mixture was vigorously stirred at room temperature for 1 hour; the layers were separated, and the organic layer was dried over anhydrous sodium sulfate, and the mixture was filtered; then the filtrate was concentrated under reduced pressure, and the residue was separated and purified by silica gel column chromatography to obtain the title compound as a brown solid (35 mg, 28 %).

MS *m/z* (ESI): 298.0 [M+H]+.

Step 4: Preparation of *N*-(2-chloro-3-(4,4,5,5-tetramethyl-1,3,2-dioxaborolan-2-yl)phenyl)-1,5-dimethyl-4,5,6,7-tetrahydro-1*H*-imidazo[4,5-*c*]pyridine-2-carboxamide

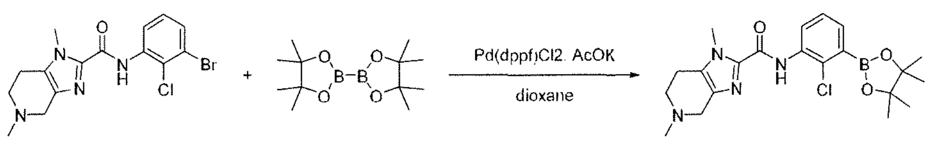


*N*-(3-Bromo-2-chlorophenyl)-1,5-dimethyl-4,5,6,7-tetrahydro-1*H*-imidazo[4,5-*c*]pyridine-2-carboxamide (300 mg, 0.783 mmol), bis(pinacolato)diboron (219 mg, 0.861 mmol), Pd(dppf)$Cl_2$ (57 mg, 0.0783 mmol), AcOK (230 mg, 2.35 mmol) were added to dioxane (5 mL) respectively, then under the protection of nitrogen, the reaction system was microwave heated to 120 °C and the reaction mixture was stirred for 2 days. After the reaction mixture was concentrated, the residue was separated and purified by silica gel column chromatography to obtain the title compound as a brown oil (85 mg, 25 %).

MS *m/z* (ESI): 431.2 [M+H]+.

Step 5: Preparation of *N*-(2-chloro-3'-(4-(hydroxymethyl)-5-methylthioazol-2-yl)-2'-methyl-[1,1'-biphenyl]-3-yl)-1,5-dimethyl-4,5,6,7-tetrahydro-1*H*-imidazo[4,5-*c*]pyridine-2-carboxamide

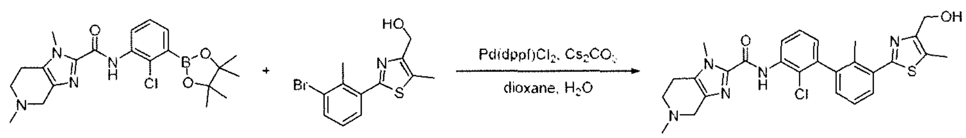


*N*-(2-Chloro-3-(4,4,5,5-tetramethyl-1,3,2-dioxaborolan-2-yl)phenyl)-1,5-dimethyl-4,5,6,7-tetrahydro-1*H*-imidazo[4,5-*c*]pyridine-2-carboxamide (50 mg, 0.116 mmol), (2-(3-bromo-2-methylphenyl)-5-methylthioazol-4-yl)methanol (40 mg, 0.116 mmol), Pd(dppf)$Cl_2$ (18 mg, 0.023 mmol), $Cs_2CO_3$ (75 mg, 0.23 mmol) were added to a mixed solvent of 1,4-dioxane (1.5 mL) and water (0.3 mL) respectively, under the protection of nitrogen, the reaction system was heated to 100 °C and stirred for 1.5 hours. The layers of reaction mixture was separated, and the organic layer was concentrated under reduced pressure, and the residue was separated by silica gel column chromatography to obtain the title compound as a brown oil (35 mg, 57 %).

MS *m/z* (ESI): 522.2 [M+H]+.

Step 6: Preparation of *N*-(2-chloro-3'-(4-formyl-5-methylthioazol-2-yl)-2'-methyl-[1,1'-biphenyl]-3-yl)-1,5-dimethyl-4,5,6,7-tetrahydro-1*H*-imidazo[4,5-*c*]pyridine-2-carboxamide

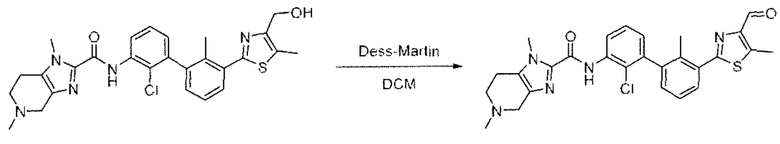


Dess-Martin reagent (43 mg, 0.101 mmol) was added to a solution of *N*-(2-chloro-3'-(4-(hydroxymethyl)-5-methylthioazol-2-yl)-2'-methyl-[1,1'-biphenyl]-3-yl)-1,5-dimethyl-4,5,6,7-tetrahydro-1*H*-imidazo[4,5-*c*]pyridine-2-carboxamide (35 mg, 0.0672 mmol) in DCM (2 mL) at room temperature, then the reaction mixture was stirred at room temperature for 1.5 hours. The reaction mixture was quenched with saturated aqueous $NaHCO_3$ solution (2 mL), then extracted with DCM (5 mL); the organic layer was dried over anhydrous sodium sulfate, and the mixture was filtered; the filtrate was concentrated under reduced pressure, and the residue was separated and purified by silica gel column chromatography to obtain the title compound as a brown solid (35 mg, 100 %).

MS *m*/*z* (ESI): 520.2 $[M+H]^+$.

Step 7: Preparation of *N*-(2-chloro-3'-(4-((3-fluoroazetidin-1-yl)methyl)-5-methylthioazol-2-yl)-2'-methyl-[1,1'-biphenyl]-3-yl)-1,5-dimethyl-4,5,6,7-tetrahydro-1*H*-imidazo[4,5-*c*]pyridine-2-carboxamide

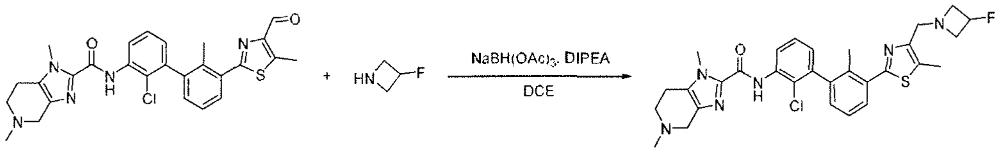


DIPEA (33 μL, 0.202 mmol) was added to a solution of *N*-(2-chloro-3'-(4-formyl-5-methylthioazol-2-yl)-2'-methyl-[1,1'-biphenyl]-3-yl)-1,5-dimethyl-4,5,6,7-tetrahydro-1*H*-imidazo[4,5-c]pyridine-2-carboxamide (35 mg, 0.0673 mmol) and 3-fluoroazetidine hydrochloride (22 mg, 0.202 mmol) in dichloroethane (2 mL), and the reaction mixture was stirred at room temperature for 10 minutes, and then sodium triacetoxyborohydride (71 mg, 0.337 mmol) was added and the reaction mixture was stirred at room temperature for 0.5 hours. The reaction mixture was diluted with DCM (10 mL), then the reaction mixture was washed with saturated aqueous sodium bicarbonate solution (5 mL); the organic layer was concentrated under reduced pressure, and the residue was separated and purified by prep-HPLC to obtain the title compound as a white solid (4 mg, 9 %).

MS *m*/*z* (ESI): 579.2 $[M+H]^+$.

Embodiment 169

*N*-(2-Chloro-3'-(((3-chloro-6-methoxy-5-((oxetan-3-ylamino)methyl)pyridin-2-yl)amino)methyl)-2'-methyl-[1,1'-biphenyl]-3-yl)-1,5-dimethyl-4,5,6,7-tetrahydro-1*H*-imidazo[4,5-*c*]pyridine-2-carboxamide

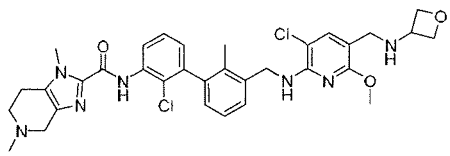

Step 1: Preparation of methyl 6-((3-bromo-2-methylbenzyl)amino)-2-methoxynicotinate

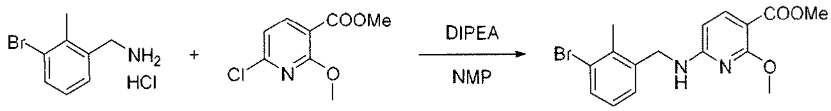


DIPEA (1.51 g, 11.7 mmol) was added to a solution of 3-bromo-2-methylbenzylamine hydrochloride (1.23 g, 5.20 mmol) and methyl 6-chloro-2-methoxynicotinate (871 mg, 4.33 mmol) in NMP (10 mL), then the reaction mixture was microwave heated to 100 °C and the reaction mixture was carried out for 10 hours. The reaction mixture was diluted with ice water (50 mL), then extracted with ethyl acetate (50 mL x 2). The organic layers were combined, then washed with saturated brine (20 mL), dried over anhydrous sodium sulfate, and the mixture was filtered, and the filtrate was concentrated under reduced pressure. The residue was separated and purified by silica gel column chromatography to obtain the title compound as a white solid (330 mg, 37 %).

MS *m/z* (ESI): 365.1 $[M+H]^+$.

Step 2: Preparation of methyl 6-((3-bromo-2-methylbenzyl)amino)-5-chloro-2-methoxynicotinate

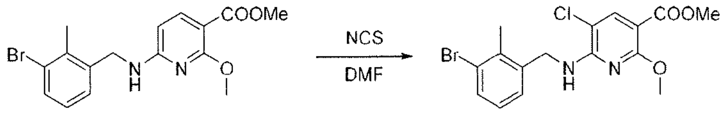


NCS (152 mg, 1.15 mmol) was added to a solution of methyl 6-((3-bromo-2-methylbenzyl)amino)-2-methoxynicotinate (380 mg, 1.04 mmol) in DMF (5 mL), then the reaction mixture was heated to 80 °C and the reaction was carried out for 5 hours. The reaction mixture was diluted with saturated aqueous sodium bicarbonate solution (30 mL), then washed with ethyl acetate (30 mL x 2); the organic layers were combined, then washed with saturated brine (20 mL), and dried over anhydrous sodium sulfate, then the mixture was filtered; the filtrate was concentrated under reduced pressure, and the residue was separated and purified by silica gel column chromatography to obtain the title compound as a brown solid (318 mg, 77 %).

MS *m/z* (ESI): 399.1 $[M+H]^+$.

Step 3: Preparation of (6-((3-bromo-2-methylbenzyl)amino)-5-chloro-2-methoxypyridin-3-yl)methanol

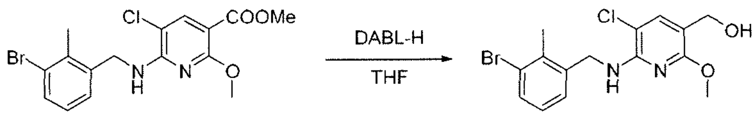


DABAL-H (1.60 mL, 1.5 M in toluene) was added dropwise to a solution of methyl 6-((3-bromo-2-methylbenzyl)amino)-5-chloro-2-methoxylnicotinate (318 mg, 0.799 mmol) in tetrahydrofuran (5 mL) at -78 °C. After the dropwise addition was completed, the temperature was naturally raised to room temperature and the reaction mixture was stirred for 15 minutes. The reaction mixture was cooled to 0 °C, and ethyl acetate (4 mL) was added dropwise to quench the reaction, then saturated aqueous sodium tartrate solution (5 mL) was added, then the reaction mixture was vigorously stirred at room temperature for 1 hour; the layers were separated, and the organic layer was dried over anhydrous sodium sulfate; the mixture was filtered, then the filtrate was concentrated under reduced pressure, and the residue was separated and purified by silica gel column chromatography to obtain the title compound as a white solid (264 mg, 89 %).

MS *m/z* (ESI): 371.1 $[M+H]^+$.

Step 4: Preparation of 6-((3-bromo-2-methylbenzyl)amino)-5-chloro-2-methoxynicotinaldehyde

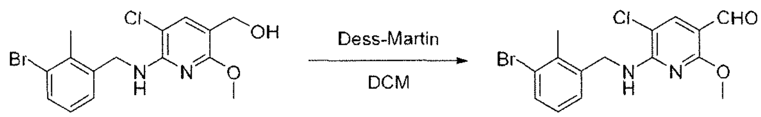


Dess-Martin reagent (454 mg, 1.07 mmol) was added to a solution of (6-((3-bromo-2-methylbenzyl)amino)-5-chloro-2-methoxypyridin-3-yl)methanol (264 mg, 0.713 mmol) in DCM (5 mL) at room temperature, and the reaction mixture was stirred at room temperature for 1 hour. The reaction mixture was quenched with saturated aqueous $NaHCO_3$ solution (5 mL), then extracted with DCM (10 mL); the organic layer was dried over anhydrous sodium sulfate, and the mixture was filtered, then the filtrate was concentrated under reduced pressure, and the residue was separated and purified by silica gel column chromatography to obtain the title compound as a brown solid (83 mg, 32 %).

MS *m/z* (ESI): 369.0 $[M+H]^+$.

Step 5: Preparation of *N*-(3-bromo-2-methylbenzyl)-3-chloro-6-methoxy-5-((oxetan-3-ylamino)methyl)pyridine-2-amine

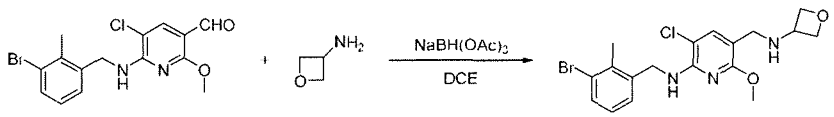


3-Oxetanamine (20 mg, 0.271 mmol) was added to a solution of 6-((3-bromo-2-methylbenzyl)amino)-5-chloro-2-methoxynicotinaldehyde (83 mg, 0.226 mmol) in dichloroethane (2 mL), and the reaction mixture was stirred at room temperature for 1 hour, then sodium triacetoxyborohydride (143 mg, 0.678 mmol) was added, and the reaction mixture was stirred at room temperature for 1 hour. The reaction mixture was quenched with saturated aqueous sodium bicarbonate solution (5 mL), extracted with dichloromethane (10 mL); the organic layer was concentrated under reduced pressure, and the residue was separated and purified by silica gel column chromatography to obtain the title compound as a colorless oil (34 mg, 35 %).

MS *m/z* (ESI): 426.1 $[M+H]^+$.

Step 6: Preparation of *N*-(2-chloro-3'-(((3-chloro-6-methoxy-5-((oxetan-3-ylamino)methyl)pyridin-2-yl)amino)methyl)-2'-methyl-(1,1'-biphenyl]-3-yl)-1,5-dimethyl-4,5,6,7-tetrahydro-1*H*-imidazo[4,5-*c*]pyridine-2-carboxamide

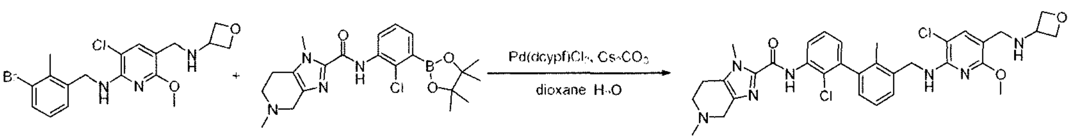


*N*-(3-Bromo-2-methylbenzyl)-3-chloro-6-methoxy-5-((oxetan-3-ylamino)methyl)pyridin-2-amine (35 mg, 0.0827 mmol), *N*-(2-chloro-3-(4,4,5,5-tetramethyl-1,3,2-dioxaborolan-2-yl)phenyl)-1,5-dimethyl-4,5,6,7-tetrahydro-1*H*-imidazo[4,5-*c*]pyridine-2-carboxamide (53 mg, 0.124 mmol), $Pd(dcypf)Cl_2$ (12 mg, 0.0165 mmol) and $Cs_2CO_3$ (54 mg, 0.165 mmol) were added to a mixed solvent of 1,4-dioxane (1.5 mL) and water (0.3 mL) respectively, and the mixture was heated to 100 °C and stirred for 1 hour under the protection of nitrogen. After the reaction mixture was cooled, the layers were separated; the organic phase was concentrated under reduced pressure, and the residue was separated and purified by prep-HPLC to obtain the title compound as a white solid (34 mg, 58 %).

MS *m/z* (ESI): 650.2$[M+H]^+$.

$^1H$ NMR (400 MHz, DMSO-$d_6$) δ 9.91 (s, 1H), 8.28 (dd, J = 8.2, 1.5 Hz, 1H), 7.56 (s, 1H), 7.44 (t, J = 7.9 Hz, 1H), 7.28 (d, J = 7.7 Hz, 1H), 7.20 (t, J = 7.6 Hz, 1H), 7.14 (t, J = 5.8 Hz, 1H), 7.04 (dd, J = 7.6, 1.6 Hz, 1H), 7.00 (d, J = 7.4 Hz, 1H), 4.68-4.52 (m, 4H), 4.36 (t, J = 6.3 Hz, 2H), 4.04-3.97 (m, 1H), 3.91 (s, 3H), 3.68 (s, 3H), 3.57 (s, 2H), 3.53 (s, 2H), 2.88-2.71 (m, 4H), 2.48 (s, 3H), 2.07 (s, 3H).

**Embodiment 170**

(*S*)-1-((2-((2'-Chloro-3'-(1,5-dimethyl-4,5,6,7-tetrahydro-1*H*-imidazo[4,5-*c*]pyridine-2-carboxamido)-2-methyl-[1,1'-biphenyl]-3-yl)methoxy)-4,6-dimethoxypyrimidin-5-yl)methyl)piperidine-2-carboxylic acid

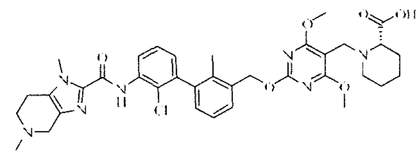

Step 1: Preparation of 2-((3-bromo-2-methylbenzyl)oxy)-4,6-dimethoxypyrimidine

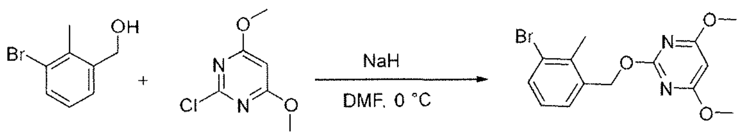


Under ice-water bath conditions, (3-bromo-2-methylphenyl)methanol (2 g, 10 mmol) and 2-chloro-4,6-dimethoxypyrimidine (2.6 g, 15 mmol) were dissolved in anhydrous *N*,*N*-dimethylformamide (50 mL), then sodium hydride (480 mg, 20 mmol) was added under an ice-water bath, and the reaction mixture was stirred and reacted for 1 hour. After the reaction was completed, the reaction mixture was extracted with ethyl acetate (50 mL x 3), washed with saturated aqueous sodium chloride solution (15 mL x 3); the organic phase was collected, dried over anhydrous sodium sulfate. After filtration, the filtrate was concentrated under reduced pressure, then obtained product was separated and purified by silica gel column chromatography to obtain the title compound (3.1 g, 92 %).
MS *m/z* (ESI): 340.9 $[M+H]^+$.

Step 2: Preparation of 2-((3-bromo-2-methylbenzyl)oxy)-4,6-dimethoxypyrimidine-5-carbaldehyde

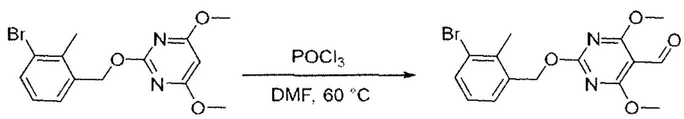


Under the protection of nitrogen, 2-((3-bromo-2-methylbenzyl)oxy)-4,6-dimethoxypyrimidine (3.1 g, 9.14 mmol) was dissolved in anhydrous *N*,*N*-dimethylformamide (50 mL), then phosphorous oxychloride (6.0 mL, 63.98 mmol) was added dropwise under an ice-water bath, and the reaction mixture was slowly heated to 60 °C and stirred overnight. After the reaction was completed, the reaction was quenched with saturated aqueous sodium bicarbonate solution (50 mL x 3), and the reaction mixture was extracted with dichloromethane (50 mL x 3), washed with saturated aqueous sodium chloride solution (15 mL x 3); the organic phase was collected, dried over anhydrous sodium sulfate. After filtration, the filtrate was concentrated under reduced pressure, then obtained product was separated and purified by silica gel column chromatography to obtain the title compound (600 mg, 18 %).
MS *m/z* (ESI): 367.0 $[M+H]^+$.

Step 3: Preparation of 4,6-dimethoxy-2-((2-methyl-3-(4,4,5,5-tetramethyl-1,3,2-dioxaborolan-2-yl)benzyl)oxy)pyrimidine-5-carbaldehyde

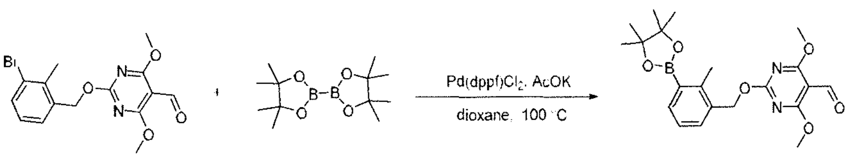


Under the protection of nitrogen, 2-((3-bromo-2-methylbenzyl)oxy)-4,6-dimethoxypyrimidine-5-carbaldehyde (100 mg, 0.27 mmol), bis(pinacolato)diboron (104 mg, 0.41 mmol), [1,1'-bis(diphenylphosphino)ferrocene]dichloride palladium (20 mg, 0.027 mmol) and potassium acetate (53 mg, 0.54 mmol) ) were suspended in anhydrous 1,4-dioxane (10 mL) solution, and the reaction mixture was heated and stirred at 100 °C overnight. After the reaction was completed, the reaction mixture was extracted with dichloromethane (15 mL x 3), washed with saturated aqueous sodium chloride solution (15 mL x 3); the organic phase was collected, dried over anhydrous sodium sulfate. After filtration, the filtrate was concentrated under reduced pressure, then obtained product was separated and purified by silica gel column chromatography to obtain the title compound (102 mg, 90 %).

MS *m/z* (ESI): 415.1 [M+H]$^+$.

Step 4: Preparation of *N*-(2-chloro-3'-(((5-formyl-4,6-dimethoxypyrimidin-2-yl)oxy)methyl)-2'-methyl-[1,1'-biphenyl]-3-yl)-1,5-dimethyl-4,5,6,7-tetrahydro-1*H*-imidazo[4,5-*c*]pyridine-2-carboxamide

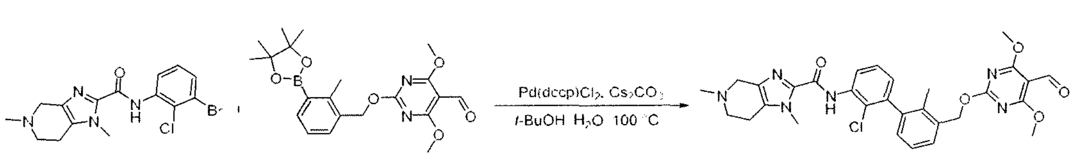


Under the protection of nitrogen, *N*-(3-bromo-2-chlorophenyl)-1,5-dimethyl-4,5,6,7-tetrahydro-1*H*-imidazo[4,5-*c*]pyridine-2-carboxamide (60 mg, 0.156 mmol), 4,6-dimethoxy-2-((2-methyl-3-(4,4,5,5-tetramethyl-1,3,2-dioxaborolan-2-yl)benzyl)oxy)pyrimidine-5-carbaldehyde (102 mg, 0.241 mmol), [1,1'-bis(dicyclohexylphosphino)ferrocene]palladium dichloride (12 mg, 0.016 mmol) and cesium carbonate (102 mg, 0.312 mmol) were suspended in *tert*-butanol (6 mL) and water (6 mL), and the reaction was heated and stirred at 100 °C overnight. After the reaction was completed, the reaction mixture was extracted with dichloromethane (15 mL x 3), washed with saturated aqueous sodium chloride solution (10 mL x 3); the organic phase was collected, dried over anhydrous sodium sulfate. After filtration, the filtrate was concentrated under reduced pressure, then obtained product was separated and purified by silica gel column chromatography to obtain the title compound (35 mg, 38 %).

MS *m/z* (ESI): 591.1 [M+H]$^+$.

Step 5: Preparation of (*S*)-1-((2-((2'-chloro-3'-(1,5-dimethyl-4,5,6,7-tetrahydro-1*H*-imidazo[4,5-*c*]pyridine-2-carboxamido)-2-methyl-[1,1'-biphenyl]-3-yl)methoxy)-4,6-dimethoxypyrimidin-5-yl)methyl)piperidine-2-carboxylic acid

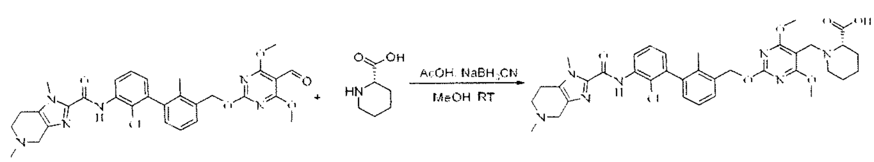


*N*-(2-Chloro-3'-(((5-formyl-4,6-dimethoxypyrimidin-2-yl)oxy)methyl)-2'-methyl-[1,1'-biphenyl]-3-yl)-1,5-dimethyl-4,5,6,7-tetrahydro-1*H*-imidazo[4,5-*c*]pyridine-2-carboxamide (30 mg, 0.05 mmol), (*S*)-piperidine-2-carboxylic acid (19 mg, 0.15 mmol) and a drop of glacial acetic acid were dissolved in methanol (5 mL), and the reaction mixture was stirred at room temperature for 1 hour. After sodium cyanoborohydride (6.3 mg, 0.1 mmol) was added, the reaction mixture was continued to stir at room temperature overnight. After the reaction was completed, the reaction mixture was extracted with dichloromethane (15 mL x 3), washed with saturated aqueous sodium chloride solution (10 mL x 3); the organic phase was collected, dried over anhydrous sodium sulfate. After filtration, the filtrate was concentrated under reduced pressure, then obtained product was separated and purified by prep-HPLC to obtain the title compound (23.9 mg, 68 %).

MS *m/z* (ESI): 704.2 $[M+H]^+$.

$^1H$ NMR (400 MHz, DMSO-$d_6$) δ 9.91 (s, 1H), 8.39 (s, 1H), 7.49 (d, J = 21.6 Hz, 2H), 7.32 (s, 1H), 7.17 (s, 1H), 7.08 (d, J = 6.4 Hz, 1H), 5.47 (s, 2H), 3.89 (d, J = 8.4 Hz, 9H), 3.04 (s, 4H), 2.69 (s, 4H), 2.39 (s, 3H), 2.34 (s, 1H), 2.10 (s, 3H), 1.70 (d, J = 27.6 Hz, 3H), 1.39 (s, 6H).

**Embodiment 171**

(*S*)-1-((6-(2,2'-Dichloro-3'-(l,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)azetidine-2-carboxylic acid

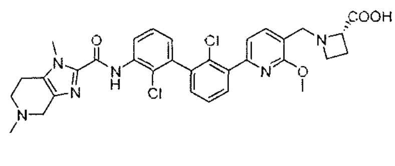

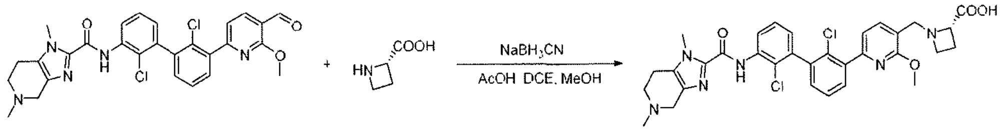


(2S)-Azetidine-2-carboxylic acid (11.02 mg, 109.00 μmol) was added to a mixed solution of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (50 mg, 90.84 μmol) in AcOH (10.00 μL), DMF (0.5 mL) and MeOH (0.5 mL), then the reaction mixture was stirred at room temperature for 2 hours, and then sodium cyanoborohydride (95.83 mg, 454.19 μmol) was added, and the reaction mixture was stirred at room temperature for 16 hours. The reaction mixture was separated and purified by prep-HPLC to obtain the title compound as a white solid (16.5 mg, 26 %).

MS *m/z* (ESI): 635.2 $[M+H]^+$.

$^1H$ NMR (400 MHz, DMSO-$d_6$) δ 9.90 (s, 1H), 8.35 (dd, J = 8.3, 1.6 Hz, 1H), 7.79 (d, J = 7.5 Hz, 1H), 7.68 (dd, J = 7.7, 1.7 Hz, 1H), 7.54 (t, J = 7.6 Hz, 1H), 7.48 (t, J = 8.0 Hz, 1H), 7.41 (dd, J = 7.6, 1.7 Hz, 1H), 7.28 (d, J = 7.5 Hz, 1H), 7.18 (d, J = 7.6 Hz, 1H), 3.94-3.87 (m, 8H), 3.76-3.70 (m, 1H), 3.44-3.41 (m, 1H), 3.37 (s, 2H), 3.13-3.08 (m, 1H), 2.71-2.64 (m, 4H), 2.39 (s, 3H), 2.26-2.16 (m, 2H).

**Embodiment 172**

(*S*)-*N*-(2,2'-Dichloro-3'-(5-((2-(hydroxymethyl)azetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

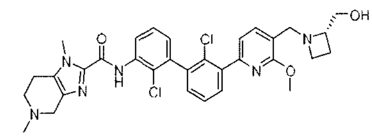

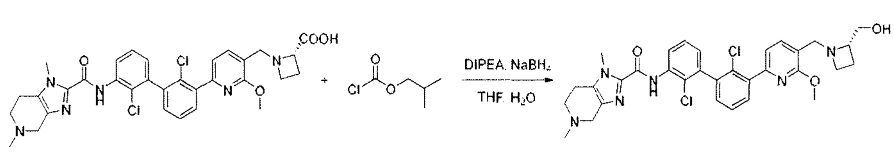


At -20 °C, a solution of isobutyl chloroformate (3.01 mg, 22.01 μmol) in THF (0.1 mL) was added dropwise to a solution of (*S*)-1-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)azetidine-2-carboxylic acid formate (10 mg, 14.67 μmol) and

DIPEA (6.64 mg, 51.35 μmol) in THF (2 mL), then the reaction was carried out at this temperature for 10 minutes, then a solution of $NaBH_4$ (0.83 mg, 22.01 μmol) in water (0.5 mL) was added; the reaction mixture was slowly warm to room temperature and stirred for 1 hour. The reaction mixture was separated and purified by prep-HPLC to obtain the title compound as an off-white solid (2.3 mg, 23 %).

MS *m/z* (ESI): 621.2 $[M+H]^+$.

$^1H$ NMR (400 MHz, DMSO-$d_6$) δ 9.90 (s, 1H), 8.35 (dd, J = 8.3, 1.6 Hz, 1H), 7.75 (d, J = 7.5 Hz, 1H), 7.67 (dd, J = 7.7, 1.8 Hz, 1H), 7.54 (t, J = 7.6 Hz, 1H), 7.48 (t, J = 7.9 Hz, 1H), 7.40 (dd, J = 7.5, 1.7 Hz, 1H), 7.25 (d, J = 7.5 Hz, 1H), 7.18 (d, J = 7.5 Hz, 1H), 3.91 (s, 3H), 3.90 (s, 3H), 3.79-3.74 (m, 1H), 3.53-3.47 (m, 1H), 3.41-3.30 (s, 7H), 2.81-2.75 (m, 1H), 2.72-2.63 (s, 4H), 2.38 (s, 3H), 2.00-1.80 (m, 2H).

**Embodiment 173**

*N*-(2,2'-Dichloro-3'-(5-((3-hydroxy-3-(hydroxymethyl)azetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

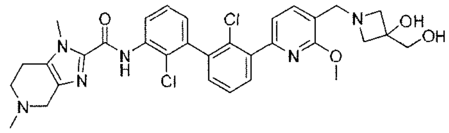

Preparation of *N*-(2,2'-dichloro-3'-(5-((3-hydroxy-3-(hydroxymethyl)azetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 171.

MS *m/z* (ESI): 637.2 $[M+H]^+$.

**Embodiment 174**

*N*-(2,2'-Dichloro-3'-(5-(((1,3-dihydroxypropan-2-yl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

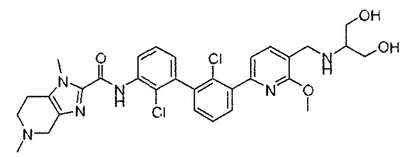

Preparation of *N*-(2,2'-dichloro-3'-(5-(((1,3-dihydroxypropan-2-yl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 171.

MS *m/z* (ESI): 625.2 $[M+H]^+$.

**Embodiment 175**

*N*-(2,2'-Dichloro-3'-(5-((((3,4-*trans*)-3-hydroxytetrahydro-2*H*-pyran-4-yl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

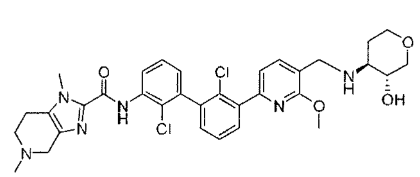

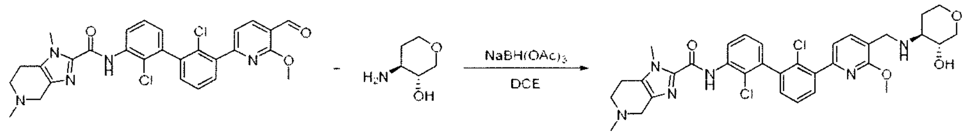


*trans* 4-Amino-tetrahydro-pyran-3-ol (102.16 mg, 872.04 μmol) was added to a solution *of N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (400 mg, 726.70 μmol) in dichloroethane (10 mL), then the reaction mixture was stirred at room temperature for 30 minutes, then sodium triacetoxyborohydride (460.00 mg, 2.18 mmol) was added, and the reaction mixture was stirred at room temperature for 1 hour. The reaction mixture was quenched with saturated aqueous sodium bicarbonate solution (10 mL), then the reaction mixture was extracted with dichloromethane (20 mL); the organic layer was concentrated under reduced pressure, and the residue was separated and purified by prep-HPLC to obtain the title compound as a white solid (234 mg, 46 %).

MS *m*/*z* (ESI): 651.2 [M+H]$^+$.

$^1$H NMR (400 MHz, DMSO-$d_6$) δ 9.91 (s, 1H), 8.35 (dd, J = 8.3, 1.6 Hz, 1H), 7.85 (d, J = 7.5 Hz, 1H), 7.68 (dd, J = 7.7, 1.8 Hz, 1H), 7.55 (t, J = 7.7 Hz, 1H), 7.48 (t, J = 7.9 Hz, 1H), 7.41 (dd, J = 7.6, 1.7 Hz, 1H), 7.27 (d, J = 7.4 Hz, 1H), 7.19 (dd, J = 7.6, 1.6 Hz, 1H), 4.99 (d, J = 5.3 Hz, 1H), 3.92 (s, 3H), 3.90 (s, 3H), 3.83-3.69 (m, 4H), 3.36 (s, 3H), 3.31-3.20 (m, 2H), 2.96 (t, J = 10.3 Hz, 1H), 2.76-2.62 (m, 4H), 2.50-2.41 (m, 1H), 2.38 (s, 3H), 2.00-1.90 (m, 1H), 1.29-1.20 (m, 1H).

## Embodiment 176

*N*-(2,2'-Dichloro-3'-(5-((((1*R*,2*S*)-2-hydroxycyclohexyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

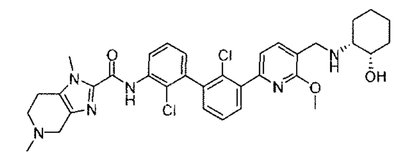

Preparation of *N*-(2,2'-dichloro-3'-(5-((((1*R*,2*S*)-2-hydroxycyclohexyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 171.

MS *m*/*z* (ESI): 649.2 [M+H]$^+$.

## Embodiment 177

(*S*)-*N*-(2,2'-Dichloro-3'-(6-methoxy-5-(((6-carbonylpiperidin-3-yl)amino)methyl)pyridin-2-yl)-[1,1'-biphenyl]-3-yl)-1,5-dimethyl-4,5,6,7-tetraliydro-1*H*-imidazo[4,5-c]pyridine-2-carboxamide

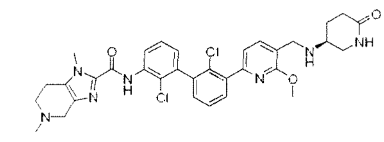

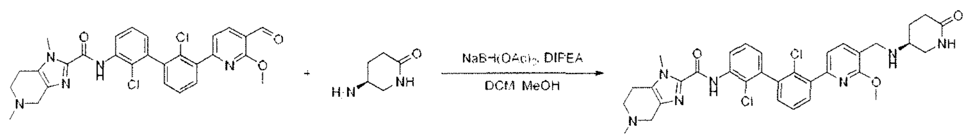

DIPEA (8.45 mg, 65.40 μmol, 11.39 μL) was added to a mixed solution of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-c]pyridine-2-carboxamide (30 mg, 54.50 μmol) and (S)-5-aminopiperidin-2-one hydrochloride (9.85 mg, 65.40 μmol) in dichloromethane (0.5 mL) and methanol (0.5 mL), and the reaction mixture was stirred at room temperature for 1 hour; sodium triacetoxyborohydride (16.90 mg, 272.51 μmol) was added, and the mixture was stirred at room temperature for 1 hour. The reaction mixture was diluted with DCM (10 mL), then the reaction mixture was washed with saturated aqueous sodium bicarbonate solution (5 mL); the organic layer was concentrated under reduced pressure, and the residue was separated and purified by prep-HPLC to obtain the title compound as a white solid (3.2 mg, 9 %).

MS *m/z* (ESI): 648.2 [M+H]+.

**Embodiment 178**

(*S*)-*N*-(2,2'-Dichloro-3'-(6-methoxy-5-(((5-carbonylpyrrolidin-3-yl)amino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

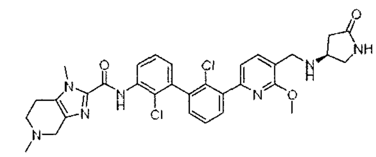

Preparation of (*S*)-*N*-(2,2'-dichloro-3'-(6-methoxy-5-(((5-carbonylpyrrolidin-3-yl)amino)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 171.

MS *m/z* (ESI): 634.2 [M+H]+.

**Embodiment 179**

*N*-(3'-(5-(((1-Acetylazetidin-3-yl)amino)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

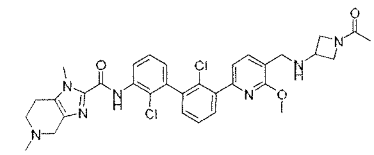

Preparation of *N*-(3'-(5-(((1-acetylazetidin-3-yl)amino)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 171.

MS *m/z* (ESI): 648.2 [M+H]+.

**Embodiment 180**

*N*-(3'-(5-((3-Acetylaminoazetidin-1-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

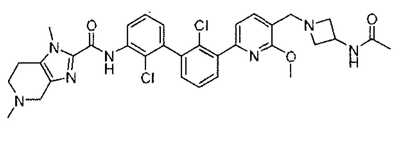

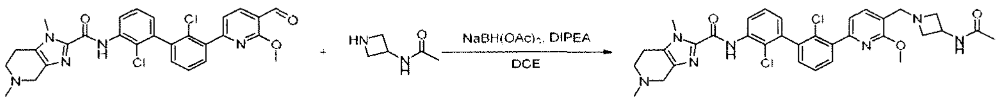


DIPEA (140.88 mg, 1.09 mmol, 189.86 μL) was added to a solution of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-c]pyridine-2-carboxamide (400 mg, 726.70 μmol) and *N*-(azetidin-3-yl)acetamide hydrochloride (164.17 mg, 1.09 mmol) in dichloroethane (5 mL), and the reaction mixture was stirred at room temperature for 1 hour, and then sodium triacetoxyborohydride (462.18 mg, 2.18 mmol) was added, and the reaction mixture was stirred at room temperature for 2 hours. The reaction mixture was quenched with saturated aqueous sodium bicarbonate solution (10 mL), then the reaction mixture was extracted with DCM (20 mL); the organic layer was concentrated under reduced pressure, and the residue was separated and purified by prep-HPLC to obtain the title compound as a white solid (259 mg, 51 %).

MS *m/z* (ESI): 648.2 $[M+H]^+$.

$^1H$ NMR (400 MHz, DMSO-$d_6$) δ 9.90 (s, 1H), 8.38-8.29 (m, 2H), 7.71 (d, J = 7.5 Hz, 1H), 7.68 (dd, J = 7.7, 1.8 Hz, 1H), 7.54 (t, J = 7.6 Hz, 1H), 7.48 (t, J = 7.9 Hz, 1H), 7.40 (dd, J = 7.6, 1.7 Hz, 1H), 7.27 (d, J = 7.5 Hz, 1H), 7.18 (dd, J = 7.6, 1.6 Hz, 1H), 4.30 (q, J = 6.9 Hz, 1H), 3.91 (s, 3H), 3.90 (s, 3H), 3.62-3.56 (m, 4H), 3.39 (s, 2H), 3.00-2.93 (m, 2H), 2.75-2.64 (m, 4H), 2.40 (s, 3H), 1.80 (s, 3H).

## Embodiment 181

*N*-(3'-(5-((3-(Acetylaminomethyl)azetidin-1-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

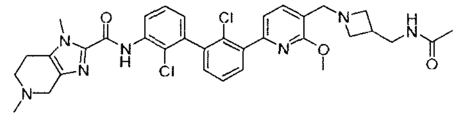

Step 1: Preparation of *tert*-butyl ((1-((6-(2,2'-dichloro-3'-(1,5-dimethyl-4,5,6,7-tetrahydro-1*H*-imidazo[4,5-c]pyridine-2-carboxamido)-[1,1'-biphenyl]-3-yl)-2-methoxypyridin-3-yl)methyl)azetidin-3-yl)methyl)carbamate

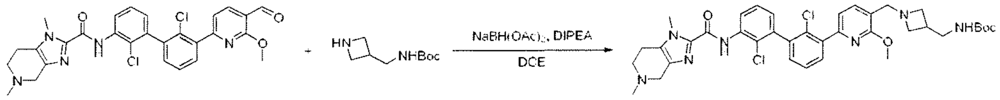


DIPEA (35.22 mg, 272.51 μmol, 47.47 μL) was added to a solution of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-c]pyridine-2-carboxamide (100 mg, 181.67 μmol) and 3-BOC-aminomethylazetidine hydrochloride (60.69 mg, 272.51 μmol) in dichloroethane (2 mL), and the reaction mixture was stirred at room temperature for 0.5 hours, and then sodium triacetoxyborohydride (191.67 mg, 908.37 μmol) was added, and the reaction mixture was stirred at room temperature for 16 hours. The reaction mixture was diluted with DCM (20 mL), then the reaction mixture was washed with saturated aqueous sodium bicarbonate solution (10mL), and the organic layer was concentrated under reduced pressure to obtain the title compound as a brown oil (75 mg, 58 %).

MS *m/z* (ESI): 720.2 $[M+H]^+$.

Step 2: Preparation of *N*-(3'-(5-((3-(aminomethyl)azetidin-1-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

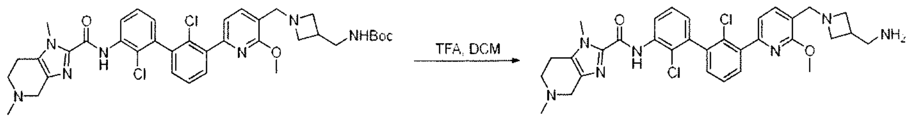


TFA (0.3 mL) was added to a solution of *tert*-butyl ((1-((6-(2,2'-dichloro-3'-(1,5-dimethyl-4,5,6,7-tetrahydro-1*H*-imidazo[4,5-cjpyridine-2-carboxamido)-[1,1'-biphenyl]-3-yl)-2-methoxypyridin-3-yl)methyl)azetidin-3-yl)methyl)carbamate (75 mg, 104.07 μmol) in DCM (0.7 mL), and the reaction mixture was stirred at room temperature for 2 hours. The reaction mixture was concentrated under reduced pressure, and the residue was diluted with DCM (10 mL), and then the mixture was concentrated under reduced pressure; the residue was partitioned between aqueous sodium bicarbonate solution (5 mL) and DCM (10 mL), and the organic layer was dried over anhydrous sodium sulfate; the mixture was filtered and the filtrate was concentrated under reduced pressure to obtain the title compound as a brown oil (60 mg, 93 %).

MS *m*/*z* (ESI): 620.2 $[M+H]^+$.

Step 3: Preparation of *N*-(3'-(5-((3-(acetamidomethyl)azetidin-1-yl)methyl)-6-methoxypyridin-2-yl)-2,2'-dichloro-[1,1'-biphenyl]-3-yl)-1,5-dimethyl-4,5,6,7-tetrahydro-1H-imidazo[4,5-c]pyridine-2-carboxamide

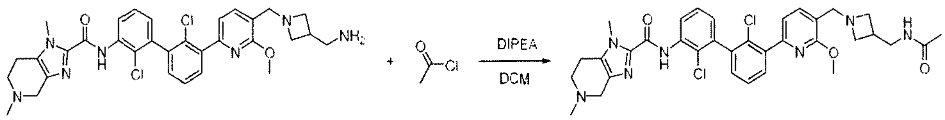


A solution of acetyl chloride (11.38 mg, 145.03 μmol, 10.35 μL) in DCM (0.1 mL) was added dropwise to a solution of *N*-(3'-(5-((3-(aminomethyl)azetidin-1-yl)methyl)-6-methoxypyridin-2-yl)-2,2'-dichloro-[1,1'-biphenyl]-3-yl)-1,5-dimethyl-4,5,6,7-tetrahydro-1H-imidazo[4,5-c]pyridine-2-carboxamide (60 mg, 96.69 μmol) and DIPEA (37.49 mg, 290.06 μmol, 50.52 μL) in DCM (1 mL), then the reaction mixture was warmed to room temperature and the reaction was carried out for 10 minutes. The reaction mixture was concentrated under reduced pressure, and the residue was separated and purified by prep-HPLC to obtain the title compound as a white solid (8.1 mg, 11 %).

MS *m*/*z* (ESI): 662.2 $[M+H]^+$.

$^1$H NMR (400 MHz, DMSO-$d_6$) δ 9.90 (s, 1H), 8.35 (d, J = 8.2 Hz, 1H), 7.91 (s, 1H), 7.72-7.64 (m, 2H), 7.54 (t, J = 7.6 Hz, 1H), 7.48 (t, J = 7.9 Hz, 1H), 7.40 (dd, J = 7.6, 1.7 Hz, 1H), 7.26 (d, J = 7.5 Hz, 1H), 7.18 (d, J = 7.6 Hz, 1H), 3.91 (s, 3H), 3.90 (s, 3H), 3.54 (s, 2H), 3.37 (s, 2H), 3.29 (s, 2H), 3.24 (d, J = 6.4 Hz, 2H), 2.94 (t, J = 6.4 Hz, 2H), 2.73-2.64 (q, 4H), 2.38 (s, 3H), 2.35-2.30 (m, 1H), 1.79 (s, 3H).

**Embodiment 182**

*N*-(2,2'-Dichloro-3'-(5-((3-(cyanomethyl)azetidin-1-yl)methyl)-6-methoxypyridin-2-yl)-[1,1'-biphenyl]-3-yl)-1,5-dimethyl-4,5,6,7-tetrahydro-1H-imidazo[4,5-c]pyridine-2-carboxamide

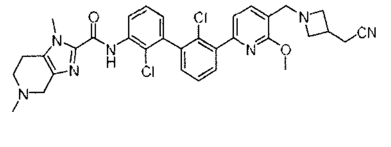

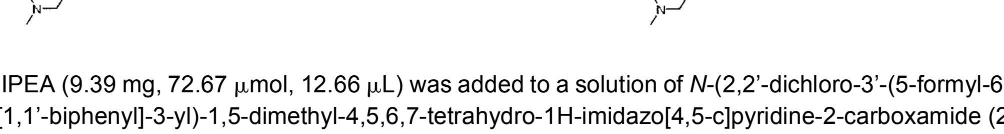


DIPEA (9.39 mg, 72.67 μmol, 12.66 μL) was added to a solution of *N*-(2,2'-dichloro-3'-(5-formyl-6-methoxypyridin-2-yl)-[1,1'-biphenyl]-3-yl)-1,5-dimethyl-4,5,6,7-tetrahydro-1H-imidazo[4,5-c]pyridine-2-carboxamide (20 mg, 36.33 μmol) and 2-(azetidin-3-yl)acetonitrile hydrochloride (9.64 mg, 72.67 μmol) in dichloroethane (0.5 mL), and the reaction mixture was stirred at room temperature for 0.5 hours, and then sodium triacetoxyborohydride (38.33 mg, 181.67 μmol)

was added, and the reaction mixture was stirred at room temperature for 1 hour. The reaction mixture was diluted with DCM (10 mL), then the reaction mixture was washed with saturated aqueous sodium bicarbonate solution (5 mL); the organic layer was concentrated under reduced pressure, and the residue was separated and purified by prep-HPLC to obtain the title compound as a white solid (18.6 mg, 76 %).

MS m/z (ESI): 630.2 $[M+H]^+$.

**Embodiment 183**

*N*-(2,2'-Dichloro-3'-(5-((3-(dimethylcarbamoyl)azetidin-1-yl)methyl)-6-methoxypyridin-2-yl)-[1,1'-biphenyl]-3-yl)-1 ,5-dimethyl-4,5,6,7-tetrahydro-1*H*-imidazo[4,5-c]pyridine-2-carboxamide

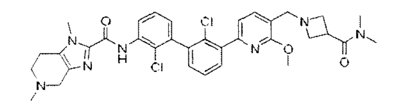

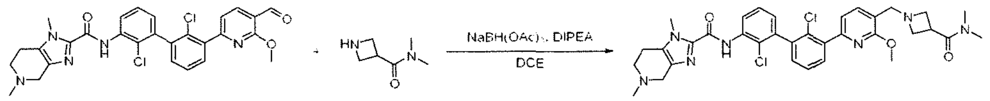


DIPEA (28.18 mg, 218.01 μmol, 37.97 μL) was added to a solution of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-c]pyridine-2-carboxamide (60 mg, 109.00 μmol) and *N,N*-dimethylazetidine-3-carboxamide hydrochloride (27.94 mg, 218.01 μmol) in dichloroethane (2 mL), and the reaction mixture was stirred at room temperature for 0.5 hours, and then sodium triacetoxyborohydride (34.50 mg, 163.51 μmol) was added, and the reaction mixture was stirred at room temperature for 0.5 hours. The reaction mixture was diluted with DCM (10 mL), then the reaction mixture was washed with saturated aqueous sodium bicarbonate solution (5 mL); the organic layer was concentrated under reduced pressure, and the residue was separated and purified by prep-HPLC to obtain the title compound as a white solid (28.3 mg, 36 %).

MS *m/z* (ESI): 662.2 $[M+H]^+$.

$^1H$ NMR (400 MHz, DMSO-$d_6$) δ 9.83 (s, 1H), 8.28 (dd, J = 8.2, 1.5 Hz, 1H), 7.66-7.57 (m, 2H), 7.47 (t, J = 7.6 Hz, 1H), 7.41 (t, J = 8.0 Hz, 1H), 7.33 (dd, J = 7.6, 1.7 Hz, 1H), 7.19 (d, J = 7.4 Hz, 1H), 7.11 (dd, J = 7.6, 1.5 Hz, 1H), 3.84 (s, 3H), 3.83 (s, 3H), 3.47 (s, 2H), 3.47-3.41 (m, 3H), 3.31 (s, 2H), 3.16 (t, J = 3.6 Hz, 2H), 2.76 (s, 3H), 2.74 (s, 3H), 2.67-2.58 (m, 4H), 2.32 (s, 3H).

**Embodiment 184**

*N*-(2,2'-Dichloro-3'-(6-methoxy-5-((3-(metliylcarbamoyl)azetidin-1-yl)methyl)pyridin-2-yl)-[1,1'-biphenyl]-3-yl)-1,5-dimethyl-4,5,6,7-tetrahydro-1*H*-imidazo[4,5-c]pyridine-2-carboxamide

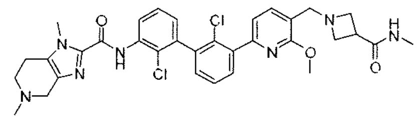

Preparation of *N*-(2,2'-dichloro-3'-(6-methoxy-5-((3-(methylcarbamoyl)azetidin-1-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 171.

MS *m/z* (ESI): 648.2 $[M+H]^+$.

$^1H$ NMR (400 MHz, DMSO-$d_6$) δ 9.90 (s, 1H), 8.35 (dd, J = 8.3, 1.5 Hz, 1H), 7.80-7.74 (m, 1H), 7.73-7.64 (m, 2H), 7.54 (t, J = 7.6 Hz, 1H), 7.48 (t, J = 7.9 Hz, 1H), 7.40 (dd, J = 7.6, 1.7 Hz, 1H), 7.26 (d, J = 7.5 Hz, 1H), 7.18 (dd, J = 7.6, 1.6 Hz, 1H), 3.91 (s, 3H), 3.90 (s, 3H), 3.54 (s, 2H), 3.46-3.40 (m, 2H), 3.36 (s, 2H), 3.24-3.17 (m, 2H), 3.18-3.10 (m, 1H), 2.74-2.66 (m, 4H), 2.58 (d, J = 4.6 Hz, 3H), 2.38 (s, 3H).

**Embodiment 185**

*N*-(2,2'-Dichloro-3'-(5-((6-hydroxy-2-azaspiro[3.3]heptan-2-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

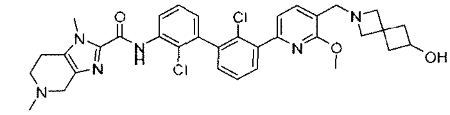

Preparation of *N*-(2,2'-dichloro-3'-(5-((6-hydroxy-2-azaspiro[3.3]heptan-2-yl)methyl)-6-methoxypyridin-2-yl)-[1,1'-biphenyl]-3-yl)-1,5-dimethyl-4,5,6,7-tetrahydro-1H-imidazo [4,5-c]pyridine-2-carboxamide was referred to Embodiment 171.

MS *m*/*z* (ESI): 647.2 $[M+H]^+$.

**Embodiment 186**

*N*-(2,2'-Dichloro-3'-(5-((6-isobutyryl-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1H-imidazo[4,5-c]pyridine-2-carboxamide

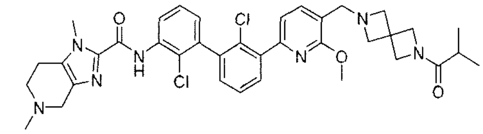

Step 1: Preparation of *tert*-butyl 6-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-2,6-diazaspiro[3.3]heptane-2-carboxylate

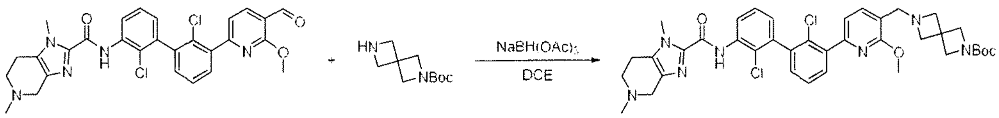


A solution of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide (195 mg, 354 μmol) and *tert*-butyl 2,6-diazaspiro[3.3]heptane-2-carboxylate (117 mg, 590 μmol) in dichloroethane (2 mL) was stirred at room temperature for 0.5 hours, then sodium triacetoxyborohydride (226 mg, 1.06 mmol) was added, then the reaction mixture was stirred at room temperature for 0.5 hours. The reaction mixture was diluted with DCM (20 mL), then washed with saturated aqueous sodium bicarbonate solution (10 mL); the organic layer was concentrated under reduced pressure, and then separated and purified by silica gel column chromatography to obtain the title compound as a brown oil (125 mg, 48 %).

MS *m*/*z* (ESI): 732.2 $[M+H]^+$.

Step 2: Preparation of *N*-(3'-(5-(2,6-diazaspiro[3.3]heptan-2-ylmethyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

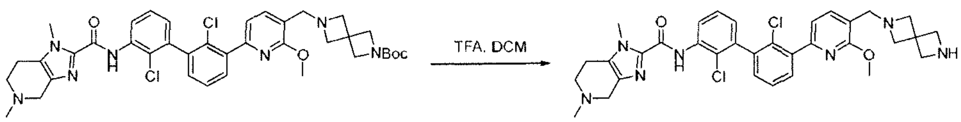

TFA (0.5 mL) was added to a solution of *tert*-butyl 6-(((6-(2,2'-dichloro-3'-(1,5-dimethyl-4,5,6,7-tetrahydro-1H-imidazo[4,5-*c*]pyridine-2-carboxamido)-[1,1'-biphenyl]-3-yl)-2-methoxypyridin-3-yl)methyl)-2,6-diazaspiro[3.3]heptane-2-carboxylate (157 mg, 214 μmol) in DCM (2 mL), then the reaction mixture was stirred at room temperature for 1 hour. The reaction mixture was concentrated under reduced pressure, and the residue was diluted with DCM (10 mL), and then the mixture was concentrated under reduced pressure; the residue was partitioned between aqueous sodium bicarbonate solution (5 mL) and DCM (10 mL), and the organic layer was dried over anhydrous sodium sulfate; the mixture was filtered and the filtrate was concentrated under reduced pressure to obtain the title compound as a brown oil (116 mg, 86%).

MS *m/z* (ESI): 632.2 [M+H]$^+$.

Step 3: Preparation of *N*-(2,2'-dichloro-3'-(5-((6-isobutyryl-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

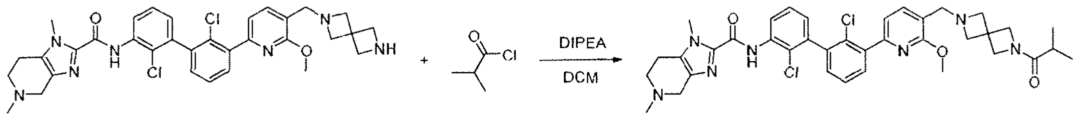


A solution of isobutyryl chloride (8 mg, 75.9 μmol) in DCM (0.1 mL) was added dropwise to a solution of *N*-(3'-(5-(2,6-diazaspiro[3.3]heptan-2-ylmethyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide (40 mg, 63.3 μmol) and DIPEA (24 mg, 190 μmol) in DCM (1 mL) at 0 °C, then the reaction was carried out at 0 °C for 1 hour. The reaction mixture was concentrated under reduced pressure, and the residue was separated and purified by prep-HPLC to obtain the title compound as a white solid (7.8 mg, 17 %).

MS *m/z* (ESI): 702.2 [M+H]$^+$.

**Embodiment 187**

*N*-(2,2'-Dichloro-3'-(5-((6-(cyclopropylcarbonyl)-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1H-imidazo[4,5-*c*]pyridine-2-carboxamide

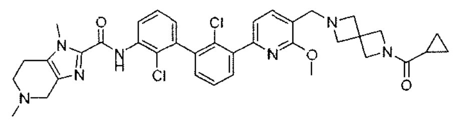

Preparation of *N*-(2,2'-dichloro-3'-(5-((6-(cyclopropylcarbonyl)-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1H-imidazo[4,5-c]pyridine-2-carboxamide was referred to Embodiment 186.

MS *m/z* (ESI): 700.2 [M+H]$^+$.

**Embodiment 188**

*N*-(2,2'-Dichloro-3'-(6-methoxy-5-((6-propionyl-2,6-diazaspiro[3.3]heptan-2-yl)methyl)pyridin-2-yl)-[1,1 '-biphenyl]-3-yl)-1,5-dimethyl-4,5,6,7-tetrahydro-1*H*-imidazo[4,5-*c*]pyridine-2-carboxamide

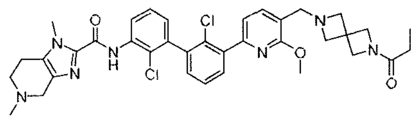

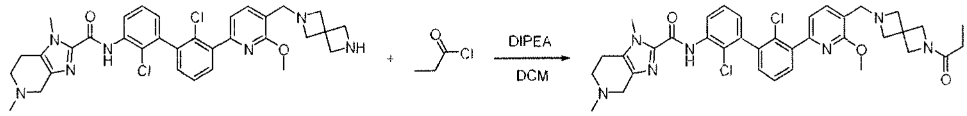


A solution of propionyl chloride (5.48 mg, 59.28 μmol, 5.17 μL) in DCM (0.1 mL) was added dropwise to a solution of *N*-(3'-(5-(2,6-diazaspiro[3.3]heptan-2-ylmethyl)-6-methoxypyridin-2-yl)-2,2'-dichloro-[1,1'-biphenyl]-3-yl)-1,5-dimethyl-4,5,6,7-tetrahydro-1H-imidazo[4,5-c]pyridine-2-carboxamide (25 mg, 39.52 μmol) and DIPEA (25.54 mg, 197.60 μmol, 34.42 μL) in DCM (1 mL) at 0 °C, then the reaction was carried out at room temperature for 0.5 hours. The reaction mixture was concentrated under reduced pressure, and the residue was separated and purified by prep-HPLC to obtain the title compound as a white solid (8.9 mg, 31 %).

MS *m/z* (ESI): 688.2 $[M+H]^+$.

**Embodiment 189**

*N*-(2,2'-Dichloro-3'-(6-methoxy-5-((6-(2,2,2-trifluoroacetyl)-2,6-diazaspiro[3.3]heptan-2-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

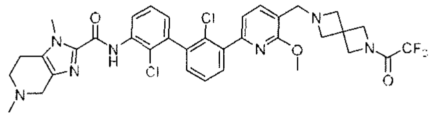

Preparation of *N*-(2,2'-dichloro-3'-(6-methoxy-5-((6-(2,2,2-trifluoroacetyl)-2,6-diazaspiro[3.3]heptan-2-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 186.

MS *m/z* (ESI): 728.2 $[M+H]^+$.

**Embodiment 190**

*N*-(2,2'-Dichloro-3'-(5-((6-(2,2-difluoroacetyl)-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

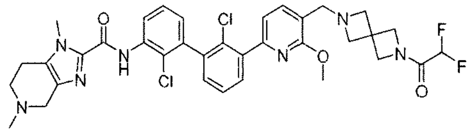

Preparation of *N*-(2,2'-dichloro-3'-(5-((6-(2,2-difluoroacetyl)-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1*H*-imidazo[4,5-c]pyridine-2-carboxamide was referred to Embodiment 186.

MS *m/z* (ESI): 710.2 $[M+H]^+$.

**Embodiment 191**

*N*-(2,2'-Dichloro-3'-(5-((6-(2-cyanoacetyl)-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

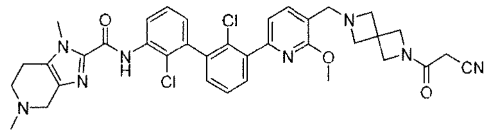

Preparation of *N*-(2,2'-dichloro-3'-(5-((6-(2-cyanoacetyl)-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1H-imidazo[4,5-c]pyridine-2-carboxamide was referred to Embodiment 186.

MS *m/z* (ESI): 699.2 $[M+H]^+$.

**Embodiment 192**

*N*-(2,2'-Dichloro-3'-(6-methoxy-5-((6-(methylsulfonyl)-2,6-diazaspiro[3.3]heptan-2-yl))methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

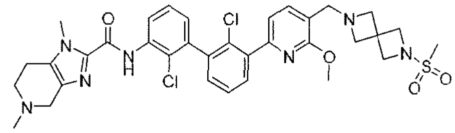

Preparation of *N*-(2,2'-dichloro-3'-(6-methoxy-5-((6-(methylsulfonyl)-2,6-diazaspiro[3.3]heptan-2-yl))methyl)pyridin-2-yl)-[1,1'-biphenyl]-3-yl)-1,5-dimethyl-4,5,6,7-tetrahydro-1H-imidazo[4,5-c]pyridine-2-carboxamide was referred to Embodiment 186.

MS *m/z* (ESI): 710.2 $[M+H]^+$.

**Embodiment 193**

*N*-(2,2'-Dichloro-3'-(5-((6-formyl-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

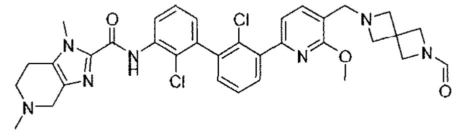

Preparation of *N*-(2,2'-dichloro-3'-(5-((6-formyl-2,6-diazaspiro[3.3]heptan-2-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 186.

MS m/z (ESI): 660.2 $[M+H]^+$.

**Embodiment 194**

*N*-(3'-(5-((7-Acetyl-2,7-diazaspiro[3.5]nonan-2-yl)methyl)-6-methoxypyridin-2-yl)-2,2'-dichloro-[1,1'-biphenyl]-3-yl)-1,5-dimethyl-4,5,6,7-tetrahydro-1H-imidazo[4,5-c]pyridine-2-carboxamide

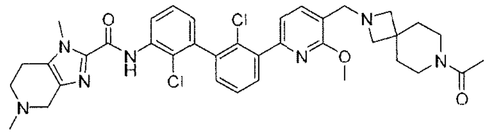

Preparation of *N*-(3'-(5-((7-acetyl-2,7-diazaspiro[3.5]nonan-2-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m*/*z* (ESI): 702.2 $[M+H]^+$.

**Embodiment 195**

*N*-(3'-(5-((6-Acetyl-2,6-diazaspiro[3.4]octan-2-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

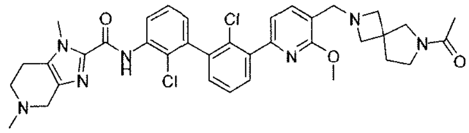

Preparation of *N*-(3'-(5-((6-acetyl-2,6-diazaspiro[3.4]octan-2-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m*/*z* (ESI): 688.2 $[M+H]^+$.

**Embodiment 196**

*N*-(2,2'-Dichloro-3'-(5-((6-isobutyryl-2,6-diazaspiro[3.4]octan-2-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

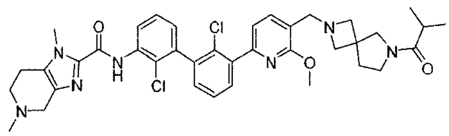

Preparation of *N*-(2,2'-dichloro-3'-(5-((6-isobutyryl-2,6-diazaspiro[3.4]octan-2-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m*/*z* (ESI): 716.2 $[M+H]^+$.

**Embodiment 197**

*N*-(3'-(5-((2-Acetyl-2,7-diazaspiro[3.5]nonan-7-yl)methyl)-6-methoxypyridin-2-yl)-2,2'-dichloro-[1,1'-biphenyl]-3-yl)-1,5-dimethyl-4,5,6,7-tetrahydro-1H-imidazo[4,5-c]pyridine-2-carboxamide

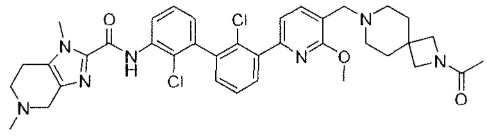

Preparation of *N*-(3'-(5-((2-acetyl-2,7-diazaspiro[3.5]nonan-7-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 702.2 [M+H]+.

### Embodiment 198

*N*-(2,2'-Dichloro-3'-(5-((4-(2-cyanoacetyl)piperazin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

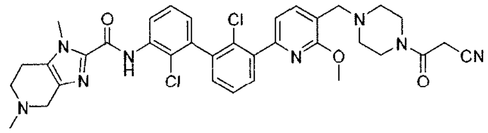

Step 1: Preparation of *tert*-butyl 4-((6-(2,2'-dichloro-3'-(1,5-dimethyl-4,5,6,7-tetrahydro-1H-imidazo)[4,5-*c*]pyridine-2-carboxamido)-[1,1'-biphenyl]-3-yl)-2-methoxypyridin-3-yl)methyl)piperazine-1-carboxylate

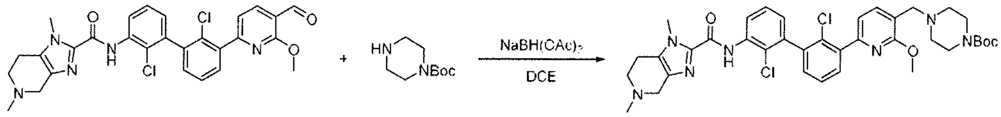


1-(*tert*-Butoxycarbonyl)piperazine (101.51 mg, 545.02 μmol) was added to a solution of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-c]pyridine-2-carboxamide (200 mg, 363.35 μmol) in dichloroethane (2 mL), then the reaction mixture was stirred at room temperature for 30 minutes, then sodium triacetoxyborohydride (230.00 mg, 1.09 mmol) was added, and the reaction mixture was stirred at room temperature for 16 hours. The reaction mixture was quenched with saturated aqueous sodium bicarbonate solution (10 mL), extracted with dichloromethane (20 mL); the organic layer was concentrated under reduced pressure, and the residue was purified by silica gel column chromatography (DCM/MeOH=100/1 to 10/1)to obtain the title compound as a brown oil (166 mg, 63 %).

MS *m/z* (ESI): 720.2 [M+H]+.

Step 2: Preparation of *N*-(2,2'-dichloro-3'-(6-methoxy-5-(piperazin-1-ylmethyl)pyridin-2-yl)-[1,1'-biphenyl]-3-yl)-1 ,5-dimethyl-4,5,6,7-tetrahydro-1*H*-imidazo[4,5-*c*]pyridine-2-carboxamide

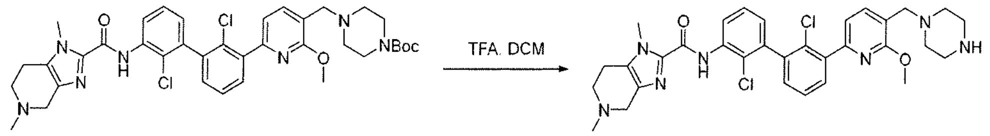


TFA (0.5 mL) was added to a solution of *tert*-butyl 4-(((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)piperazine-1-carboxylate (166 mg, 230.34 μmol) in DCM (1.5 mL), then the reaction mixture was stirred at room temperature for 4 hours. The

reaction mixture was concentrated under reduced pressure, and the residue was diluted with DCM (10 mL), and then the mixture was concentrated under reduced pressure; the residue was partitioned between aqueous sodium bicarbonate solution (5 mL) and DCM (10 mL), and the organic layer was dried over anhydrous sodium sulfate; the mixture was filtered and the filtrate was concentrated under reduced pressure to obtain the title compound as a brown oil (124 mg, 87 %).

MS *m/z* (ESI): 620.2 $[M+H]^+$.

Step 3: Preparation of *N*-(2,2'-dichloro-3'-(5-((4-(2-cyanoacetyl)piperazin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

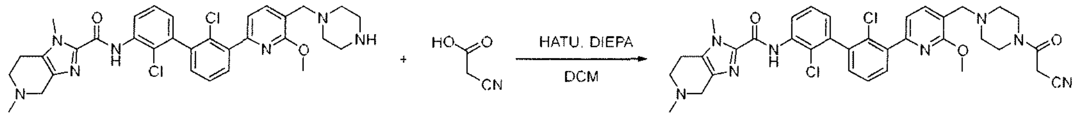


HATU (36.48 mg, 96.69 μmol) was added to a solution of *N*-(2,2'-dichloro-3'-(6-methoxy-5-(piperazin-1-ylmethyl)pyridin-2-yl)-[1,1'-biphenyl]-3-yl)-1,5-dimethyl-4,5,6,7-tetrahydro-1*H*-imidazo[4,5-c]pyridine-2-carboxamide (20 mg, 32.23 μmol), cyanoacetic acid (8.22 mg, 96.69 μmol) and DIPEA (20.83 mg, 161.14 μmol, 28.07 μL) in DCM (1 mL) at room temperature, and the reaction mixture was stirred at room temperature for 1 hour. The reaction mixture was partitioned between saturated aqueous sodium bicarbonate solution (5 mL) and DCM (10 mL), and the organic layer was concentrated under reduced pressure, and then separated and purified by prep-HPLC to obtain the title compound as a white solid (3.4 mg, 13%).

MS *m/z* (ESI): 687.2 $[M+H]^+$.

**Embodiment 199**

*N*-(2,2'-Dichloro-3'-(5-((4-isobutyrylpiperazin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

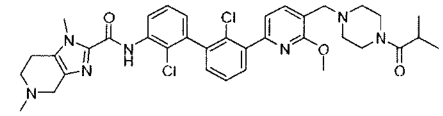

Preparation of *N*-(2,2'-dichloro-3'-(5-((4-isobutyrylpiperazin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 198.

MS *m/z* (ESI): 690.2 $[M+H]^+$.

**Embodiment 200**

*N*-(2,2'-Dichloro-3'-(5-((4-(cyclopropylcarbonyl)piperazin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

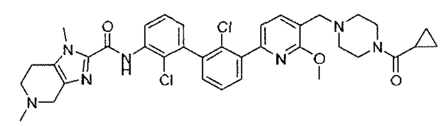

Preparation of *N*-(2,2'-dichloro-3'-(5-((4-(cyclopropylcarbonyl)piperazin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 198.

MS *m/z* (ESI): 688.2 $[M+H]^+$.

**Embodiment 201**

*N*-(2,2'-Dichloro-3'-(6-methoxy-5-((4-propionylpiperazin-1-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

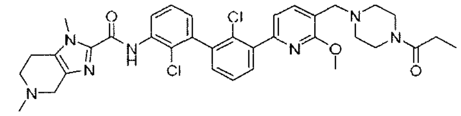

Preparation of *N*-(2,2'-dichloro-3'-(6-methoxy-5-((4-propionylpiperazin-1-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 198.

MS *m/z* (ESI): 676.2 $[M+H]^+$.

**Embodiment 202**

*N*-(2,2'-Dichloro-3'-(6-methoxy-5-((4-(2,2,2-trifluoroacetyl)piperazin-1-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

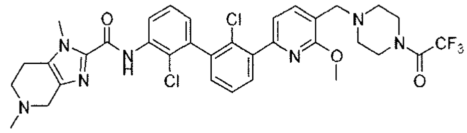

Preparation of *N*-(2,2'-dichloro-3'-(6-methoxy-5-((4-(2,2,2-trifluoroacetyl)piperazin-1-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 198.

MS *m/z* (ESI): 716.2 $[M+H]^+$.

**Embodiment 203**

*N*-(3'-(5-1-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

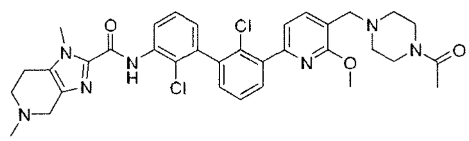

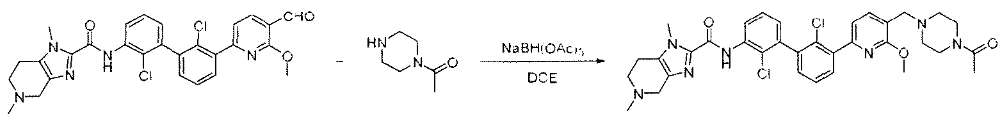


1-Acetylpiperazine (6.99 mg, 54.50 μmol) was added to a solution of *N*-(2,2'-dichloro-3'-(5-formyl-6-methoxypyridin-2-yl)-[1,1'-biphenyl]-3-yl)-1,5-dimethyl-4,5,6,7-tetrahydro-1*H*-imidazo[4,5-c]pyridine-2-carboxamide (20 mg, 36.33 μmol) in dichloroethane (0.5 mL), then the reaction mixture was stirred at room temperature for 1 hour, then sodium triacetoxyborohydride (57.77 mg, 273.80 μmol) was added, and the reaction mixture was stirred at room temperature for I hour. The reaction mixture was quenched with saturated aqueous sodium bicarbonate solution (5 mL), then the reaction mixture was extracted with dichloromethane (10 mL); the organic layer was concentrated under reduced

pressure, and the residue was separated and purified by prep-HPLC to obtain the title compound as a white solid (9.5 mg, 37 %).

MS *m/z* (ESI): 662.2 [M+H]+.

**Embodiment 204**

*N*-(2,2'-Dichloro-3'-(6-methoxy-5-((4-(methylsulfonyl)piperazin-1-yl)methyl)pyridin-2-yl)-[1,1'-biphenyl]-3-yl)-1,5-dimethyl-4,5,6,7-tetrahydro-1*H*-imidazo[4,5-*c*]pyridine-2-carboxamide

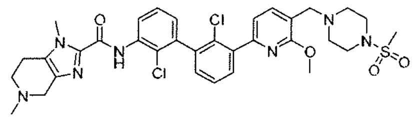

Preparation of *N*-(2,2'-dichloro-3'-(6-methoxy-5-((4-(methylsulfonyl)piperazin-1-yl)methyl)pyridin-2-yl)-[1,1'-biphenyl]-3-yl)-1,5-dimethyl-4,5,6,7-tetrahydro-1*H*-imidazo[4,5-c]pyridine-2-carboxamide was referred to Embodiment 198.

MS *m/z* (ESI): 698.2 [M+H]+.

**Embodiment 205**

*N*-(3'-(5-(((1*S*,4*S*)-5-Acetyl-2,5-diazabicyclo[2.2.1]heptan-2-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

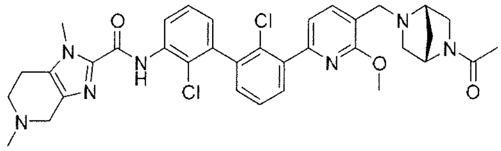

Preparation of *N*-(3'-(5-(((1*S*,4*S*)-5-acetyl-2,5-diazabicyclo[2.2.1]heptan-2-yl)methyl)-6-methoxypyridin-2-yl)-2,2'-dichloro-[1,1'-biphenyl]-3-yl)-1,5-dimethyl-4,5,6,7-tetrahydro-1H-imidazo[4,5-c]pyridine-2-carboxamide was referred to Embodiment 198.

MS *m/z* (ESI): 674.2 [M+H]+.

**Embodiment 206**

*N*-(3'-(5-((6-Acetyl-3,6-diazabicyclo[3.1.1]heptan-3-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

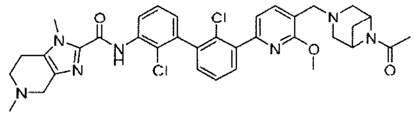

Preparation of *N*-(3'-(5-((6-acetyl-3,6-diazabicyclo[3.1.1]heptan-3-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 198.

MS *m/z* (ESI): 674.2 [M+H]+.

**Embodiment 207**

*N*-(2,2'-Dichloro-3'-(5-((6-isobutyryl-3,6-diazabicyclo[3.1.1]heptan-3-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

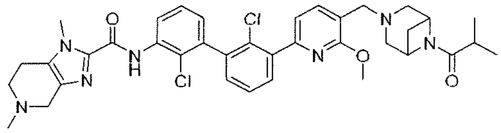

Preparation of *N*-(2,2'-dichloro-3'-(5-((6-isobutyryl-3,6-diazabicyclo[3.1.1]heptan-3-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 198.

MS m/z (ESI): 702.2 $[M+H]^+$.

**Embodiment 208**

*N*-(2,2'-Dichloro-3'-(5-((6-(2-cyanoacetyl)-3,6-diazabicyclo[3.1.1]heptan-3-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

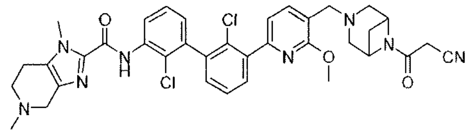

Preparation of *N*-(2,2'-dichloro-3'-(5-((6-(2-cyanoacetyl)-3,6-diazabicyclo[3.1.1]heptan-3-yl)methyl)-6-methoxypyridin-2-yl)-[1,1'-biphenyl]-3-yl)-1,5-dimethyl-4,5,6,7-tetrahydro-1*H*-imidazo[4,5-c]pyridine-2-carboxamide was referred to Embodiment 198.

MS *m/z* (ESI): 699.2 $[M+H]^+$.

**Embodiment 209**

*N*-(3'-(5-((3-Acetyl-3,6-diazabicyclo[3.1.1]heptan-6-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

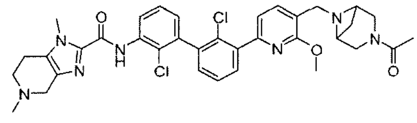

Preparation of *N*-(3'-(5-((3-acetyl-3,6-diazabicyclo[3.1.1]heptan-6-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 198.

MS *m/z* (ESI): 674.2 $[M+H]^+$.

**Embodiment 210**

*N*-(2,2'-Dichloro-3'-(5-((3-isobutyryl-3,6-diazabicydo[3.1.1]heptan-6-yl)methyl)-6-methoxypyridin-2-yl)-[1,1'-biphenyl]-3-yl)-1 ,5-dimethyl-4,5,6,7-tetrahydro-1*H*-imidazo[4,5-*c*]pyridine-2-carboxamide

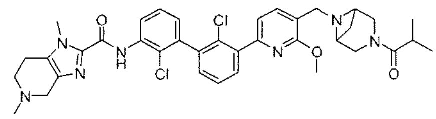

Preparation of *N*-(2,2'-dichloro-3'-(5-((3-isobutyryl-3,6-diazabicyclo[3.1.1]heptan-6-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 198.

MS *m*/*z* (ESI): 702.2 $[M+H]^+$.

**Embodiment 211**

*N*-(2,2'-Dichloro-3'-(5-((4-cyanopiperidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

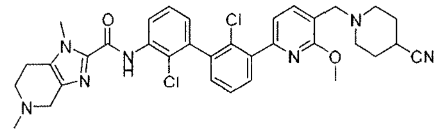

Preparation of *N*-(2,2'-dichloro-3'-(5-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m*/*z* (ESI): 644.2 $[M+H]^+$.

**Embodiment 212**

*N*-(2,2'-Dichloro-3'-(5-((4-cyano-4-methylpiperidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

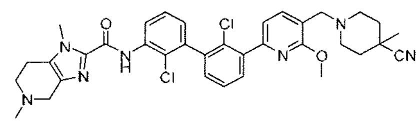

Preparation of *N*-(2,2'-dichloro-3'-(5-((4-cyano-4-methylpiperidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m*/*z* (ESI): 658.2 $[M+H]^+$.

**Embodiment 213**

*N*-(2,2'-Dichloro-3'-(5-((((1-cyanocyclopropyl)methyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

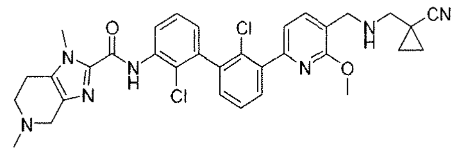

Preparation of *N*-(2,2'-dichloro-3'-(5-((((1-cyanocyclopropyl)methyl)amino)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 630.2 [M+H]+.

**Embodiment 214**

4-(((6-(2,2'-Dichloro-3'-(1,5-dimethyl-4,5,6,7-tetrahydro-1*H*-imidazo[4,5-*c*]pyridin-2-carboxamido)-[1,1'-biphenyl]-3-yl)-2-methoxypyridin-3-yl)methyl)amino)tetrahydro-2*H*-pyran-3-carboxylic acid

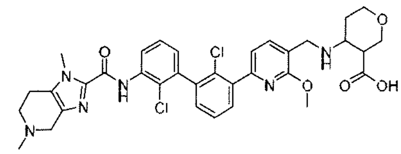

Preparation of 4-(((6-(2,2'-dichloro-3'-(1,5-dimethyl-4,5,6,7-tetrahydro-1*H*-imidazo[4,5-*c*]pyridin-2-carboxamido)-[1,1'-biphenyl]-3-yl)-2-methoxypyridin-3-yl)methyl)amino)tetrahydro-2*H*-pyran-3-carboxylic acid was referred to Embodiment 171.

MS *m/z* (ESI): 679.2 [M+H]+.

**Embodiment 215**

*N*-(2,2'-Dichloro-3'-(5-((3-(hydroxymethyl)azetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide

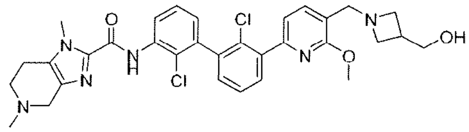

Preparation of *N*-(2,2'-dichloro-3'-(5-((3-(hydroxymethyl)azetidin-1-yl)methyl)-6-methoxypyridin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 171.

MS *m/z* (ESI): 621.2 [M+H]+.

$^1$H NMR (400 MHz, DMSO-$d_6$) δ 9.90 (s, 1H), 8.35 (dd, J = 8.3, 1.6 Hz, 1H), 7.72-7.64 (m, 2H), 7.54 (t, J = 7.6 Hz, 1H), 7.48 (t, J = 7.9 Hz, 1H), 7.40 (dd, J = 7.6, 1.8 Hz, 1H), 7.25 (d, J = 7.4 Hz, 1H), 7.18 (dd, J = 7.6, 1.6 Hz, 1H), 4.57 (s, 1H), 3.91 (s, 3H), 3.90 (s, 3H), 3.56-3.49 (m, 4H), 3.36 (s, 2H), 3.31-3.26 (m, 2H), 3.00-2.93 (m, 2H), 2.73-2.63 (m, 4H), 2.50-2.45 (m, 1H), 2.38 (s, 3H).

**Embodiment 216**

*N*-(2,2'-Dichloro-3'-(6-methoxy-5-((oxetan-3-ylamino)methyl)pyrazin-2-yl)-[1,1'-biphenyl]-3-yl)-1,5-dimethyl-4,5,6,7-tetrahydro-1*H*-imidazo[4,5-*c*]pyridine-2-carboxamide

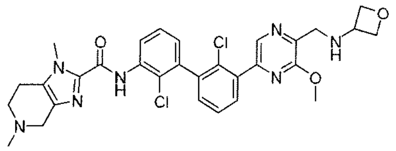

Preparation of *N*-(2,2'-dichloro-3'-(6-methoxy-5-((oxetan-3-ylamino)methyl)pyrazin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 152.
MS *m*/*z* (ESI): 608.2 $[M+H]^+$.

**Embodiment 217**

*N*-(2,2'-Dichloro-3'-(5-((3-fluoroazetidin-1-yl)methyl)-6-methoxypyrazin-2-yl)-[1,1'-biphenyl]-3-yl)-1,5-dimethyl-4,5,6,7-tetrahydro-1*H*-imidazo[4,5-*c*]pyridine-2-carboxamide

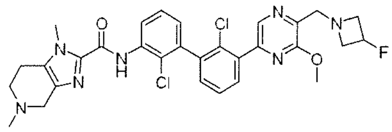

Preparation of *N*-(2,2'-dichloro-3'-(5-((3-fluoroazetidin-1-yl)methyl)-6-methoxypyrazin-2-yl)-[1,1'-biphenyl]-3-yl)-1,5-dimethyl-4,5,6,7-tetrahydo-1*H*-imidazo[4,5-*c*]pyridine-2-carboxamide was referred to Embodiment 152.
MS *m*/*z* (ESI): 610.2 $[M+H]^+$.

**Embodiment 218**

*N*-(3'-(5-((3-Acetylaminoazetidin-1-yl)methyl)-6-methoxypyrazin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

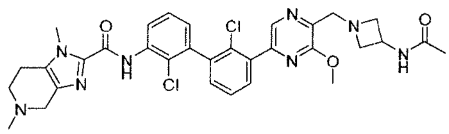

Preparation of *N*-(3'-(5-((3-acetylaminoazetidin-1-yl)methyl)-6-methoxypyrazin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 152.
MS *m*/*z* (ESI): 649.2 $[M+H]^+$.

**Embodiment 219**

*N*-(2,2'-Dichloro-3'-(5-((3-(hydroxymethyl)azetidin-1-yl)methyl)-6-methoxypyrazin-2-yl)-[1,1'-biphenyl]-3-yl)-1,5-dimethyl-4,5,6,7-tetrahydro-1*H*-imidazo[4,5-*c*]pyridine-2-carboxamide

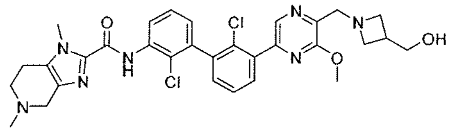

Preparation of *N*-(2,2'-dichloro-3'-(5-((3-(hydroxymethyl)azetidin-1-yl)methyl)-6-methoxypyrazin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 152.

MS *m*/*z* (ESI): 622.2 [M+H]$^+$.

**Embodiment 220**

(*S*)-*N*-(2,2'-Dichloro-3'-(6-methoxy-5-(((6-carbonylpiperidin-3-yl)amino)methyl)pyrazin-2-yl)-[1,1'-biphenyl]-3-yl)-1,5-dimethyl-4,5,6,7-tetrahydro-1*H*-imidazo[4,5-*c*]pyridine-2-carboxamide

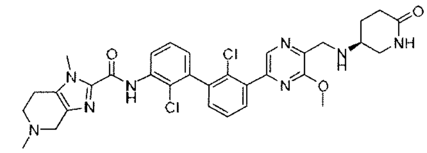

Preparation of (*S*)-*N*-(2,2'-dichloro-3'-(6-methoxy-5-(((6-carbonylpiperidin-3-yl)amino)methyl)pyrazin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 152.

MS *m*/*z* (ESI): 649.2 [M+H]$^+$.

**Embodiment 221**

*N*-(2,2'-Dichloro-3'-(5-((((*trans*)-3-hydroxytetrahydro-2*H*-pyran-4-yl)amino)methyl)-6-methoxypyrazin-2-yl)-[1,1'-biphenyl]-3-yl)-1,5-dimethyl-4,5,6,7-tetrahydro-1*H*-imidazo[4,5-*c*]pyridine-2-carboxamide

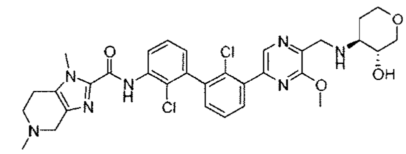

Preparation of *N*-(2,2'-dichloro-3'-(5-((((*trans*)-3-hydroxytetrahydro-2*H*-pyran-4-yl)amino)methyl)-6-methoxypyrazin-2-yl)-[1,1'-biphenyl]-3-yl)-1,5-dimethyl-4,5,6,7-tetrahydro-1H-imidazo[4,5-c]pyridine-2-carboxamide was referred to Embodiment 152.

MS *m*/*z* (ESI): 652.2 [M+H]$^+$.

**Embodiment 222**

*N*-(3'-(5-((6-Acetyl-2,6-diazaspiro[3.3]heptan-2-yl)methyl)-4-methoxypyrimidin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

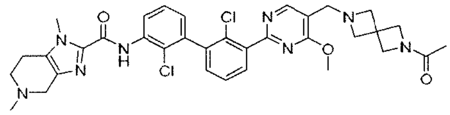

Step 1: Preparation of 2-chloro-4-methoxy-5-vinylpyrimidine

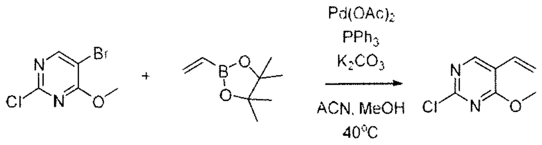


5-Bromo-2-chloro-4-methoxy-pyrimidine (10 g, 44.75 mmol), 4,4,5,5-tetramethyl-2-vinyl-1,3,2-dioxaborolane (8.27 g, 53.70 mmol), palladium acetate (1.00 g, 4.48 mmol), triphenylphosphine (2.35 g, 8.95 mmol) and potassium carbonate (12.37 g, 89.50 mmol) were added to a mixed solvent of acetonitrile (40 mL) and methanol (20 mL); the reaction system was protected with dry nitrogen, then the reaction system was heated to 40 °C and the reaction was stirred for 3.5 hours; the reaction mixture was concentrated under reduced pressure, then silica gel was added, and the residue was separated by silica gel column chromatography to obtain the title compound (5.37 g, 70 %).

MS *m/z* (ESI): 171.1 $[M+H]^+$.

Step 2: Preparation of 2-chloro-4-methoxypyrimidine-5-carbaldehyde

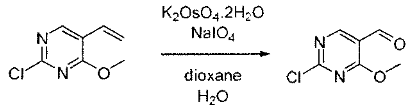


2-Chloro-4-methoxy-5-vinylpyrimidine (5.37 g, 31.48 mmol) was dissolved in a mixed solvent of dioxane (300 mL) and water (120 mL), and potassium osmate dihydrate (116 mg, 314.78 μmol) and sodium periodate (40.4 g, 188.87 mmol) were added sequentially under stirring at room temperature; the reaction mixture was stirred and reacted at room temperature for 1.5 hours, then the reaction mixture was concentrated under reduced pressure to remove most of the organic solvent, and the residue was extracted with ethyl acetate; the ethyl acetate layer was washed successively with saturated aqueous sodium thiosulfate solution and saturated aqueous sodium chloride solution, dried over anhydrous sodium sulfate, and the mixture was filtered; the filtrate was concentrated, and the residue was separated by silica gel column chromatography to obtain the title compound (3.27 g, 60 %).

MS *m/z* (ESI): 173.1 $[M+H]^+$.

Step 3: Preparation of 2-(3-bromo-2-chlorophenyl)-4-methoxypyrimidine-5-carbaldehyde

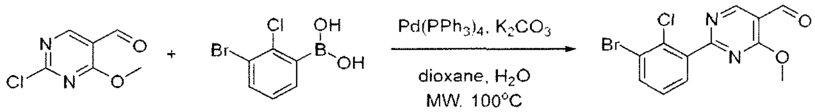


(3-Bromo-2-chloro-phenyl)boronic acid (2.08 g, 8.84 mmol), 2-chloro-4-methoxy-pyrimidine-5-carbaldehyde (1.98 g, 11.49 mmol), $Pd(PPh_3)_4$ (1.02 g, 884.09 μmol) and $K_2CO_3$ (2.44 g, 17.68 mmol) were added to a mixed solvent of dioxane (40 mL) and water (4 mL); the reaction mixture was saturated with nitrogen, and then heated to 100 °C by a microwave synthesizer, and the reaction mixture was stirred and reacted for 5.5 hours, then concentrated under reduced pressure to remove the solvent; the residue was dispersed in ethyl acetate, and washed with saturated aqueous sodium chloride solution, dried over anhydrous sodium sulfate; the mixture was filtered, then the filtrate was concentrated, and the residue was separated by silica gel column chromatography to obtain the title compound (2.0 g, 69 %).

MS *m/z* (ESI): 327.0 $[M+H]^+$.

Step 4: Preparation of 2-(2-chloro-3-(4,4,5,5-tetramethyl-1,3,2-dioxaborolan-2-yl)phenyl)-4-methoxypyrimidine-5-carbaldehyde

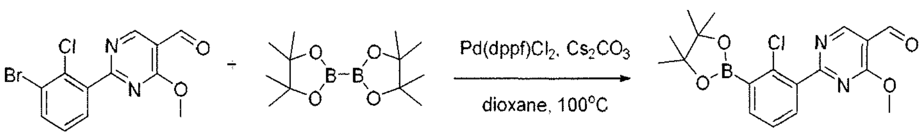


2-(3-Bromo-2-chloro-phenyl)-4-methoxy-pyrimidine-5-carbaldehyde (2.59 g, 7.89 mmol), 4,4,5,5-tetramethyl-2-(4,4,5,5-tetramethyl-1,3,2-dioxaborolan-2-yl)-1,3,2-dioxaborolane (3.01 g, 11.84 mmol), Pd (dppf)$Cl_2$ (644.46 mg, 789.17 μmol) and potassium acetate (1.55 g, 15.78 mmol) were added to dioxane (100 mL); the reaction system was protected with dry nitrogen, and the reaction system was heated to 100 °C, stirred and reactedfor 4 hours. The reaction mixture was concentrated under reduced pressure to remove the solvent, and the residue was separated by silica gel column chromatography to obtain the title compound (1.55 g, 52 %).

MS *m/z* (ESI): 375.2 $[M+H]^+$.

Step 5: Preparation of *N*-(2,2'-dichloro-3'-(5-formyl-4-methoxypyrimidin-2-yl)-[1,1'-biphenyl]-3-yl)-1,5-dimethyl-4,5,6,7-tetrahydro-1*H*-imidazo[4,5-*c*]pyridine-2-carboxamide

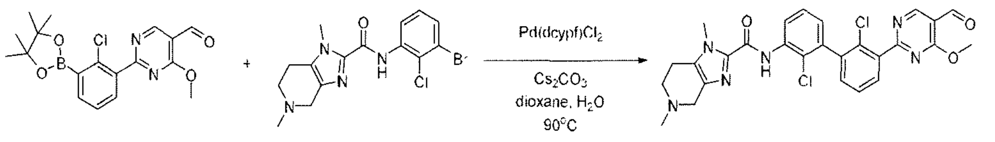


2-[2-Chloro-3-(4,4,5,5-tetramethyl-1,3,2-dioxaborolan-2-yl)phenyl]-4-methoxy-pyrimidine-5-carboxaldehyde (1.55 g, 4.12 mmol), *N*-(3-bromo-2-chloro-phenyl)-1,5-dimethyl-6,7-dihydro-4*H*-imidazo[4,5-c]pyridine-2-carboxamide (1.0 g, 2.61 mmol), bis(dicyclohexylphosphinoferrocene)palladium dichloride (212.99 mg, 257.86 μmol) and cesium carbonate (1.70 g, 5.22 mmol) were added to a mixed solvent of dioxane (40 mL) and water (4 mL); the reaction system was protected with nitrogen, and the reaction mixture was heated to 100 °C, stirred and reacted for 4 hours. The reaction mixture was concentrated under reduced pressure to remove the solvent, and the residue was dispersed in dichloromethane, then washed with saturated aqueous sodium chloride solution, dried over anhydrous sodium sulfate; the mixture was filtered, and the filtrate was concentrated, and the residue was separated by silica gel column chromatography to obtain the title compound (773 mg, 54 %).

MS *m/z* (ESI): 551.1 $[M+H]^+$.

Step 6: Preparation of *N*-(3'-(5-((6-acetyl-2,6-diazaspiro[3.3]heptan-2-yl)methyl)-4-methoxypyrimidin-2-yl)-2,2'-dichloro-[1,1'-biphenyl]-3-yl)-1,5-dimethyl-4,5,6,7-tetrahydro-1*H*-imidazo[4, 5-*c*]pyridine-2-carboxamide

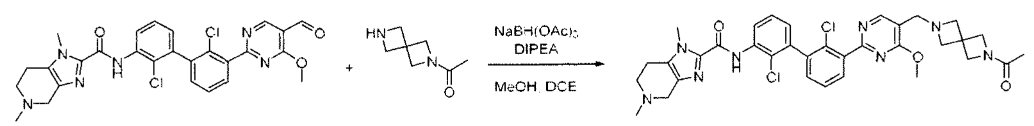


*N*-[2-Chloro-3-[2-chloro-3-(5-formyl-4-methoxy-pyrimidin-2-yl)phenyl]phenyl]-1,5-dimethyl-6,7-dihydro-4*H*-imidazo[4,5-*c*]pyridine-2-carboxamide (100 mg, 181.35 μmol) and 1-(2,6-diazaspiro[3.3]heptan-2-yl)ethan-1-one trifluoroacetate (46.10 mg, 181.35 μmol) were dissolved in a mixed solvent of methanol (6 mL) and 1,2-dichloroethane (2 mL), and DIPEA (117.19 mg, 906.75 μmol, 157.93 μL) was added; the reaction mixture was stirred at room temperature for 2 hours, and sodium triacetoxyborohydride (384.35 mg, 1.81 mmol) was added, then the reaction mixture was continued to stir at room temperature for 30 minutes, then the reaction mixture was concentrated under reduced pressure to remove the solvent, and the residue was dissolved in dichloromethane, then washed successively with saturated aqueous sodium bicarbonate solution and saturated aqueous sodium chloride solution, dried over anhydrous sodium sulfate; the mixture was filtered, and the filtrate was concentrated, and the residue was successively separated by preparative TLC and reverse phase HPLC to obtain the title product (8.3 mg, 7 %).

MS *m/z* (ESI): 675.2 $[M+H]^+$.

$^1H$ NMR (400 MHz, DMSO-$d_6$) δ 9.89 (s, 1H), 8.52 (s, 1H), 8.36 (s, 1H), 7.78 (dd, J = 7.8, 1.1 Hz, 1H), 7.56 (t,

J = 7.7 Hz, 1H), 7.52-7.39 (m, 2H), 7.17 (d, J = 7.5 Hz, 1H), 4.18 (s, 2H), 3.98 (s, 3H), 3.90 (s, 6H), 3.54 (s, 3H), 3.51 (s, 4H), 2.69 (s, 4H), 2.38 (s, 3H), 1.71 (s, 3H).

**Embodiment 223**

*N*-(2,2'-Dichloro-3'-(5-((3-1-yl)methyl)-4-methoxypyrimidin-2-yl)-[1, 1'-biphenyl]-3-yl)-1,5-dimethyl-4,5,6,7-tetrahydro-1*H*-imidazo[4,5-*c*]pyridine-2-carboxamide

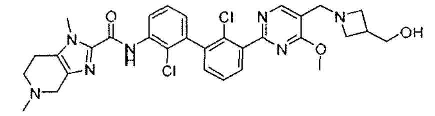

Preparation of *N*-(2,2'-dichloro-3'-(5-((3-(hydroxymethyl)azetidin-1-yl)methyl)-4-methoxypyrimidin-2-yl)-[1,1'-biphenyl]-3-yl)-1,5-dimethyl-4,5,6,7-tetrahydro-1*H*-imidazo[4,5-*c*]pyridine-2-carboxamide was referred to Embodiment 222.

MS *m/z* (ESI): 622.2 $[M+H]^+$.

$^1H$ NMR (400 MHz, $CD_3OD$) δ 8.55-8.43 (m, 2H), 7.72 (dd, J = 7.7, 1.4 Hz, 1H), 7.52 (t, J = 7.6 Hz, 1H), 7.43 (t, J = 7.9 Hz, 2H), 7.13 (dd, J = 7.5, 1.0 Hz, 1H), 4.08 (s, 3H), 3.97 (s, 3H), 3.71 (s, 2H), 3.67 (d, J = 6.3 Hz, 2H), 3.52 (t, J = 7.7 Hz, 4H), 3.21 (t, J = 7.2 Hz, 2H), 2.86 (t, J = 5.4 Hz, 2H), 2.79 (t, J = 5.2 Hz, 2H), 2.70 (dt, J = 13.8, 7.1 Hz, 1H), 2.52 (s, 3H).

**Embodiment 224**

*N*-(3'-(5-((3-Acetylaminoazetidin-1-yl)methyl)-4-methoxypyrimidin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

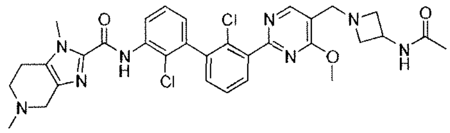

*N*-[2-Chloro-3-[2-chloro-3-(5-formyl-4-methoxy-pyrimidin-2-yl)phenyl]phenyl]-1,5-dimethyl-6,7-dihydro-4*H*-imidazo[4,5-*c*]pyridine-2-carboxamide (100 mg, 181.35 μmol) and *N*-(azetidin-3-yl)acetamide (27.31 mg, 181.35 μmol, CL) were dissolved in a mixed solvent of methanol (6 mL) and 1,2-dichloroethane (2 mL), then DIPEA (117.19 mg, 906.75 μmol, 157.93 μL) was added, and the reaction mixture was stirred at room temperature for 2 hours; sodium triacetoxyborohydride (384.35 mg, 1.81 mmol) was added, then the reaction mixture was continued to stir for 30 minutes at room temperature, then the reaction mixture was concentrated under reduced pressure to remove the solvent; the residue was dissolved in dichloromethane, washed with saturated aqueous sodium bicarbonate solution and saturated aqueous sodium chloride solution successively, dried over anhydrous sodium sulfate; the mixture was filtered, and the filtrate was concentrated, and the residue was separated by preparative TLC and reverse-phase HPLC successively to obtain the title compound (10.6 mg, 9 %).

MS *m/z* (ESI): 649.2 $[M+H]^+$.

$^1H$ NMR (400 MHz, DMSO-$d_6$) δ 9.89 (s, 1H), 8.53 (s, 1H), 8.36 (s, 1H), 8.32 (s, 1H), 7.79 (d, J = 7.8 Hz, 1H), 7.56 (t, J = 7.6 Hz, 1H), 7.48 (dd, J = 13.4, 7.1 Hz, 2H), 7.17 (d, J = 7.5 Hz, 1H), 4.28 (dd, J = 13.4, 7.0 Hz, 1H), 3.98 (s, 3H), 3.90 (s, 3H), 3.58 (s, 2H), 3.57 (s, 1H), 3.55 (s, 1H), 3.36 (s, 2H), 2.96 (t, J = 6.6 Hz, 2H), 2.69 (s, 4H), 2.38 (s, 3H), 1.79 (s, 3H).

**Embodiment 225**

*N*-(3'-(5-((3-(Acetylaminomethyl)azetidin-1-yl)methyl)-4-methoxypyrimidin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

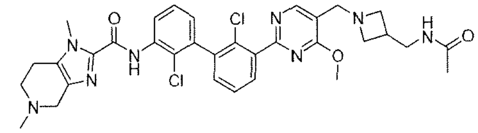

Preparation of *N*-(3'-(5-((3-(acetylaminomethyl)azetidin-1-yl)methyl)-4-methoxypyrimidin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 222.

MS m/z (ESI): 663.2 $[M+H]^+$.

**Embodiment 226**

*N*-(2,2'-Dichloro-3'-(4-methoxy-5-((3-(methylcarbamoyl)azetidin-1-yl)methyl)pyrimidin-2-yl)-[1,1'-biphenyl]-3-yl)-1,5-dimethyl-4,5,6,7-tetrahydro-1*H*-imidazo[4,5-*c*]pyridine-2-carboxamide

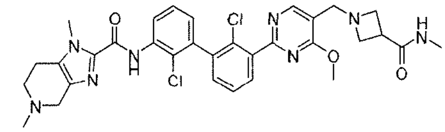

Preparation of *N*-(2,2'-dichloro-3'-(4-methoxy-5-((3-(methylcarbamoyl)azetidin-1-yl)methyl)pyrimidin-2-yl)-[1,1'-biphenyl]-3-yl)-1,5-dimethyl-4,5,6,7-tetrahydro-1*H*-imidazo[4,5-c]pyridine-2-carboxamide was referred to Embodiment 222.

MS *m/z* (ESI): 649.2 $[M+H]^+$.

**Embodiment 227**

Methyl ((2-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-4-methoxypyrimidin-5-yl)methyl)-*L*-serinate

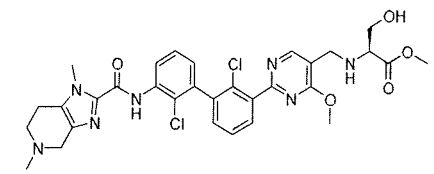

*N*-[2-Chloro-3-[2-chloro-3-(5-formyl-4-methoxy-pyrimidin-2-yl)phenyl]phenyl]-1,5-dimethyl-6,7-dihydro-4*H*-imidazo[4,5-*c*]pyridine-2-carboxamide (100 mg, 181.35 μmol) and methyl L-serinate (21.60 mg, 181.35 μmol) were dissolved in a mixed solvent of methanol (6 mL) and 1,2-dichloroethane (2 mL), then DIPEA (117.19 mg, 906.75 μmol, 157.93 μL) was added, and the reaction mixture was stirred at room temperature for 2 hours; sodium triacetoxyborohydride (384.35 mg, 1.81 mmol) was added, then the reaction mixture was continued to stir for 30 minutes at room temperature, then the reaction mixture was concentrated under reduced pressure to remove the solvent; the residue was dissolved in dichloromethane, washed with saturated aqueous sodium bicarbonate solution and saturated aqueous sodium chloride solution successively, dried over anhydrous sodium sulfate; the mixture was filtered, and the filtrate

was concentrated, and the residue was separated by preparative TLC and reverse-phase HPLC successively to obtain the title compound (5.6 mg, 5 %).

MS *m/z* (ESI): 654.2 $[M+H]^+$.

$^1H$ NMR (400 MHz, DMSO-$d_6$) δ 9.89 (s, 1H), 8.61 (s, 1H), 8.34 (d, J = 8.5 Hz, 1H), 7.77 (d, J = 7.5 Hz, 1H), 7.56 (t, J = 7.2 Hz, 1H), 7.48 (dd, J = 14.5, 7.2 Hz, 2H), 7.17 (d, J = 7.4 Hz, 1H), 3.98 (s, 3H), 3.90 (s, 3H), 3.80 (d, J = 14.7 Hz, 1H), 3.67 (d, J = 15.7 Hz, 1H), 3.60 (d, J = 5.0 Hz, 5H), 3.56-3.50 (m, 3H), 2.68 (s, 4H), 2.38 (s, 3H).

**Embodiment 228**

*N*-(2,2'-Dichloro-3'-(5-(((*trans*-3-hydroxytetrahydro-2*H*-pyran-4-yl)amino)methyl)-4-methoxypyrimidin-2-yl)-[1,1'-biphenyl]-3-yl)-1,5-dimethyl-4,5,6,7-tetrahydro-1*H*-imidazo[4,5-*c*]pyridine-2-carboxamide

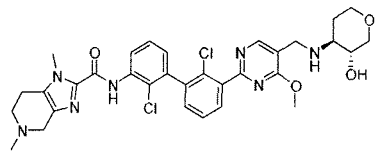

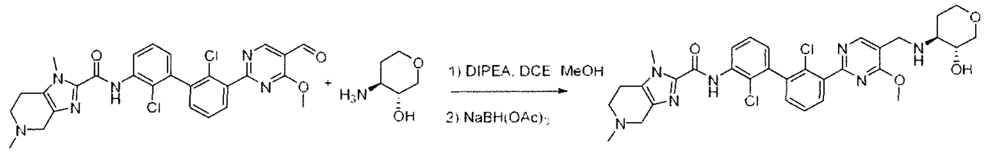


*N*-[2-Chloro-3-[2-chloro-3-(5-formyl-4-methoxy-pyrimidin-2-yl)phenyl]phenyl]-1,5-dimethyl-6,7-dihydro-4*H*-imidazo[4,5-*c*]pyridine-2-carboxamide (100 mg, 181.35 μmol) and *trans*-4-aminotetrahydropyran-3-ol (42.49 mg, 362.70 μmol) were dissolved in a mixed solvent of 1,2-dichloroethane (10 mL) and methanol (1 mL), then diisopropylethylamine (70.31 mg, 544.05 μmol, 94.76 μL) was added, and the reaction mixture was stirred and reacted at room temperature for 14 hours; sodium triacetoxyborohydride (115.31 mg, 544.05 μmol) was added, and the reaction mixture was continued to stir at room temperature for 30 minutes, then the reaction mixture was concentrated under reduced pressure to remove the solvent, and the residue was separated by HPLC to obtain the title compound (21.8 mg, 18 %).

MS *m/z* (ESI): 652.2 $[M+H]^+$.

$^1H$ NMR (400 MHz, $CD_3OD$) δ 8.58 (s, 1H), 8.45 (dd, *J* = 8.3, 1.3 Hz, 1H), 7.73 (dd, *J* = 7.6, 1.5 Hz, 1H), 7.53 (t, *J* = 7.6 Hz, 1H), 7.43 (dd, *J*= 11.0,4.6 Hz, 2H), 7.13 (dd, *J* = 7.6, 1.1 Hz, 1H), 4.11 (s, 3H), 4.01-3.82 (m, 7H), 3.55 (s, 2H), 3.46-3.38 (m, 2H), 3.07 (t, *J*= 10.4 Hz, 1H), 2.89 (t, *J*= 5.8 Hz, 2H), 2.80 (t, *J*= 5.5 Hz, 2H), 2.63-2.48 (m, 4H), 2.15-2.05 (m, 1H), 1.48 (ddd, *J*= 24.7, 12.3, 4.4 Hz, 1H).

**Embodiment 229**

*N*-(2,2'-Dichloro-3'-(5-(((2-hydroxyethyl)amino)methyl)-4-methoxypyrimidin-2-yl)-[1,1'-biphenyl]-3-yl)-1,5-dimethyl-4,5,6,7-tetrahydro-1*H*-imidazo[4,5-*c*]pyridine-2-carboxamide

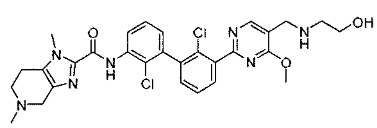

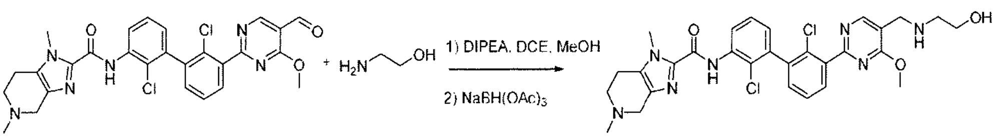


*N*-[2-Chloro-3-[2-chloro-3-(5-formyl-4-methoxy-pyrimidin-2-yl)phenyl]phenyl]-1,5-dimethyl-6,7-dihydro-4*H*-imidazo[4,5-*c*]pyridine-2-carboxamide (100 mg, 181.35 μmol) and 2-aminoethanol (22.15 mg, 362.70 μmol) were dissolved in a mixed solvent of 1,2-dichloroethane (10 mL) and methanol (1 mL), then DIPEA (46.88 mg, 362.70 μmol, 63.17 μL) was added, and the reaction mixture was stirred at room temperature for 14 hours, and sodium triacetoxyborohydride (115.31 mg, 544.05 μmol) was added, then the reaction mixture was continued to stir and react at room temperature for 30 minutes; the reaction mixture was concentrated under reduced pressure to remove the solvent, and the residue was separated by HPLC to obtain the title compound (11.8 mg, 11 %).

MS *m/z* (ESI): 596.2 $[M+H]^+$.

$^1H$ NMR (400 MHz, $CD_3OD$) δ 8.57 (s, 1H), 8.45 (dd, *J*= 8.3, 1.1 Hz, 1H), 7.73 (dd, *J* = 7.7, 1.4 Hz, 1H), 7.53 (t, *J*= 7.6 Hz, 1H), 7.48-7.38 (m, 2H), 7.13 (dd, *J*= 7.6, 1.2 Hz, 1H), 4.11 (s, 3H), 3.97 (s, 3H), 3.92 (s, 2H), 3.71 (t, *J*= 5.4 Hz, 2H), 3.52 (s, 2H), 2.83 (tt, *J* = 10.8, 5.7 Hz, 6H), 2.52 (s, 3H).

**Embodiment 230**

(*S*)-1-((6-(2,2'-Dichloro-3'-(1,5-dimethyl-4,5,6,7-tetrahydro-1*H*-imidazo[4,5-c]pyridine-2-carboxamido)-[1,1'-biphenyl]-3-yl)-2-methoxypyridin-3-yl)methyl)piperidine-2-carboxylic acid

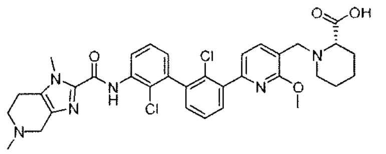

Preparation of (*S*)-1-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)piperidine-2-carboxylic acid was referred to Embodiment 114.

MS *m/z* (ESI): 663.2 $[M+H]^+$.

$^1H$ NMR (400 MHz, DMSO-$d_6$) δ 9.91 (s, 1H), 8.35 (dd, *J* = 8.3, 1.5 Hz, 1H), 7.86 (d, J= 7.5 Hz, 1H), 7.68 (dd, *J* = 7.6, 1.7 Hz, 1H), 7.54 (t, *J* = 7.7 Hz, 1H), 7.48 (t, *J* = 8.0 Hz, 1H), 7.41 (dd, *J* = 7.5, 1.7 Hz, 1H), 7.30 (d, *J* = 7.5 Hz, 1H), 7.19 (dd, *J* = 7.6, 1.6 Hz, 1H), 3.94-3.86 (m, 6H), 3.77 (d, *J* = 15.7 Hz, 1H), 3.59 (d, *J* = 15.7 Hz, 1H), 3.36 (s, 2H), 3.21-3.16 (m, 1H), 2.98-2.91 (m, 1H), 2.74-2.66 (m, 4H), 2.38 (s, 3H), 2.30-2.23 (m, 1H), 1.85-1.72 (m, 2H), 1.59-1.31 (m, 4H).

**Embodiment 231**

Methyl (*S*)-1-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)piperidine-2-carboxylate

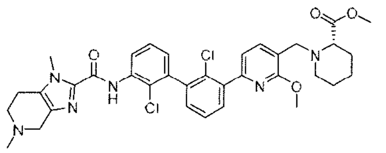

Preparation of methyl (*S*)-1-((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)piperidine-2-carboxylate was referred to Embodiment 114.

MS *m/z* (ESI): 677.2 $[M+H]^+$.

$^1H$ NMR (400 MHz, DMSO-$d_6$) δ 9.91 (s, 1H), 8.36-8.31 (m, 1H), 7.81 (d, *J*= 7.5 Hz, 1H), 7.68 (dd, *J* = 7.7, 1.7 Hz, 1H), 7.54 (t, *J* = 7.7 Hz, 1H), 7.48 (t, *J* = 7.9 Hz, 1H), 7.41 (dd, *J* = 7.5, 1.8 Hz, 1H), 7.29 (d, *J* = 7.5 Hz, 1H), 7.22-7.15 (m, 1H), 3.90 (s, 3H), 3.89 (s, 3H), 3.69-3.55 (m, 5H), 3.37-3.34 (m, 3H), 2.97-2.85 (m, 1H), 2.73-2.64 (m, 4H), 2.38 (s, 3H), 2.34-2.25 (m, 1H), 1.83-1.72 (m, 2H), 1.57-1.49 (m, 2H), 1.47-1.35 (m, 2H).

**Embodiment 232**

((6-(2,2'-Dichloro-3'-(1,5-dimethyl-4,5,6,7-tetrahydro-1*H*-imidazo[4,5-c]pyridine-2-carboxamido)- [1,1'-biphenyl]-3-yl)-2-methoxypyridin-3-yl)methyl)-*L*-serine

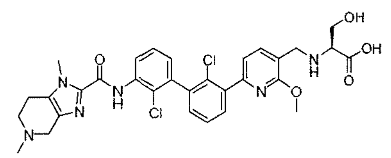

Preparation of ((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-L-serine was referred to Embodiment 114.

MS *m/z* (ESI): 639.2 [M+H]$^+$.

$^1$H NMR (400 MHz, DMSO-$d_6$) $\delta$ 9.91 (s, 1H), 8.35 (d, *J*= 8.1 Hz, 1H), 7.86 (d, *J* = 7.5 Hz, 1H), 7.68 (d, *J* = 7.8 Hz, 1H), 7.55 (t, *J* = 7.6 Hz, 1H), 7.48 (t, *J* = 7.9 Hz, 1H), 7.44-7.38 (m, 1H), 7.29 (d, *J* = 7.4 Hz, 1H), 7.19 (d, *J*= 7.5 Hz, 1H), 3.91 (d, *J* = 11.1 Hz, 7H), 3.84-3.78 (m, 1H), 3.61 (d, *J* = 5.5 Hz, 2H), 3.36 (s, 2H), 3.14-3.10 (m, 1H), 2.74-2.64 (m, 4H), 2.62-2.57 (m, 1H), 2.38 (s, 3H).

**Embodiment 233**

Methyl ((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-*L*-serinate

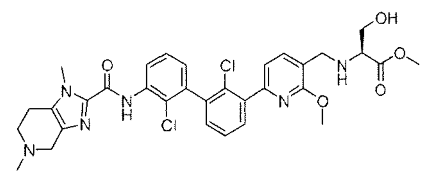

Preparation of methyl ((6-(2,2'-dichloro-3'-(1,5-dimethyl-4,5,6,7-tetrahydro-1*H*-imidazo[4,5-*c*]pyridine-2-carboxamido)-[1,1'-biphenyl]-3-yl)-2-methoxypyridin-3-yl)methyl)-*L*-serinate was referred to Embodiment 114.

MS *m/z* (ESI): 653.2 [M+H]$^+$

$^1$H NMR (400 MHz, DMSO-$d_6$) $\delta$ 9.91 (s, 1H), 8.38-8.31 (m, 1H), 7.81 (d, *J*= 7.5 Hz, 1H), 7.67 (dd, *J* = 7.7, 1.7 Hz, 1H), 7.54 (t, J= 7.6 Hz, 1H), 7.48 (t, *J* = 7.9 Hz, 1H), 7.41 (dd, *J* = 7.6, 1.7 Hz, 1H), 7.27 (d, J= 7.5 Hz, 1H), 7.18 (dd, *J*= 7.6, 1.5 Hz, 1H), 4.87 (s, 1H), 3.91 (s, 3H), 3.90 (s, 3H), 3.78 (d, *J*= 15.1 Hz, 1H), 3.67-3.59 (m, 6H), 3.36 (s, 2H), 3.33 (t, *J* = 5.2 Hz, 1H), 2.76-2.65 (m, 4H), 2.54 (s, 1H), 2.38 (s, 3H).

**Embodiment 234**

*N*-(3'-(5-((6-Acetylamino-2-azaspiro[3.3]heptan-2-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide

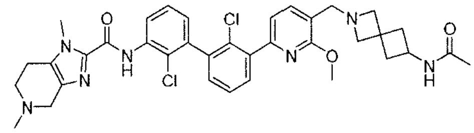

Preparation of *N*-(3'-(5-((6-acetylamino-2-azaspiro[3.3]heptan-2-yl)methyl)-6-methoxypyridin-2-yl)-2,2'-dichloro-[1,1'-biphenyl]-3-yl)-1,5-dimethyl-4,5,6,7-tetrahydro-1*H*-imidazo[4,5-*c*]pyridine-2-carboxamide was referred to Embodiment 114.

MS *m/z* (ESI): 688.2 $[M+H]^+$.

$^1H$ NMR (400 MHz, DMSO-$d_6$) $\delta$ 9.90 (s, 1H), 8.35 (dd, *J* = 8.3, 1.6 Hz, 1H), 8.03 (d, *J* = 7.5 Hz, 1H), 7.72-7.65 (m, 2H), 7.54 (t, *J* = 7.7 Hz, 1H), 7.48 (t, *J* = 8.0 Hz, 1H), 7.40 (dd, *J* = 7.6, 1.7 Hz, 1H), 7.25 (d, *J* = 7.5 Hz, 1H), 7.18 (dd, *J* = 7.6, 1.6 Hz, 1H), 4.08-3.97 (m, 1H), 3.93-3.88 (m, 6H), 3.52 (s, 2H), 3.37 (s, 2H), 3.27 (s, 2H), 3.16 (s, 2H), 2.71-2.64 (m, 4H), 2.43-2.32 (m, 5H), 2.01-1.94 (m, 2H), 1.74 (s, 3H).

**Embodiment 235**

*N*-(2,2'-Dichloro-3'-(5-(((*trans*-3-hydroxytetrahydro-2*H*-pyran-4-yl)amino)methyl)-6-methoxypyridin-2-yl)-[1,1'-biphenyl]-3-yl)-1-ethyl-5-methyl-4,5,6,7-tetrahydro-1*H*-imidazo[4,5-*c*]pyridine-2-carboxamide

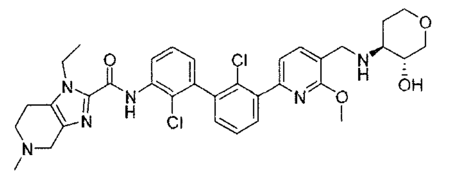

Step 1: Preparation of 4-ethylamino-3-nitropyridine

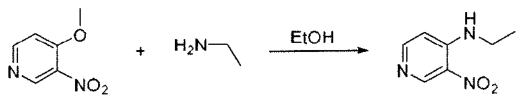


A solution of ethylamine in THF (13 mL, 2 M) was added to a solution of 3-nitro-4-methoxypyridine (1.0 g, 6.49 mmol) in EtOH (20 mL), and the reaction mixture was heated and refluxed for 16 hours. The reaction mixture was concentrated under reduced pressure to obtain the title compound as a yellow powder (1.08 g, 100 %).

MS *m/z* (ESI): 168.1 $[M+H]^+$.

Step 2: Preparation of $N^4$-ethylpyridine-3,4-diamine

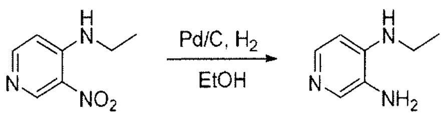


Pd/C (100 mg, 50 % wet, 10 % Pd) was added to a solution of 4-ethylamino-3-nitropyridine (1.08 g, 6.46 mmol) in EtOH (20 mL), and the reaction system was replaced with hydrogen atmosphere by a hydrogen balloon, then the reaction mixture was stirred at room temperature for 2 hours. The reaction mixture was filtered through celite, and the filtrate was concentrated under reduced pressure to obtain the title compound as a gray solid (812 mg, 92 %).

MS *m/z* (ESI): 138.1 $[M+H]^+$.

Step 3: Preparation of methyl 1-ethyl-1*H*-imidazo[4,5-*c*]pyridine-2-carboxylate

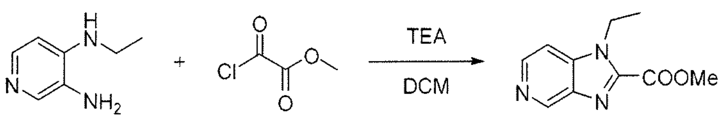

Methyl 2-chloro-2-oxoacetate (951 mg, 7.76 mmol) was added dropwise to a solution of $N^4$-ethylpyridine-3,4-diamine (484 mg, 3.53 mmol) and TEA (857 mg, 8.47 mmol, 1.18 mL) in DCM (10 mL) at 0 °C, after the addition was completed, the reaction mixture was warmed to room temperature, and the mixture was stirred for 1 hour. The reaction mixture was partitioned in saturated aqueous sodium bicarbonate solution (10 mL) and DCM (10 mL); the organic layer was concentrated under reduced pressure, and the residue was separated and purified by silica gel column chromatography (DCM: MeOH = 100:1 to 10:1) to obtain the title compound a milky white solid (327 mg, 45 %).

MS *m/z* (ESI): 206.1 $[M+H]^+$.

Step 4: Preparation of methyl 1-ethyl-5-methyl-4,5,6,7-tetrahydro-1*H*-imidazo[4,5-*c*]pyridine-2-carboxylate

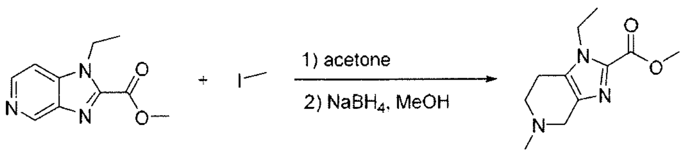


Iodomethane (236 mg, 1.66 mmol) was added to a solution of methyl 1-ethyl-1*H*-imidazo[4,5-c]pyridine-2-carboxylate (325 mg, 1.58 mmol) in acetone (3.0 mL), and the reaction mixture was heated to 56 °C and stirred for 3 hours. The reaction mixture was concentrated under reduced pressure, and the residue was dissolved in MeOH (3 mL), then sodium borohydride (120 mg, 3.17 mmol) was added in portions at room temperature, and the mixture was stirred at room temperature for 0.5 hours. The reaction mixture was diluted with acetone (5 mL), then concentrated under reduced pressure, and the residue was separated and purified by silica gel column chromatography (DCM: MeOH = 100:1 to 10:1) to obtain the title compound as a milky white solid (338 mg, 96 %).

MS *m/z* (ESI): 224.1 $[M+H]^+$.

$^1H$ NMR (400 MHz, $CDCl_3$) $\delta$ 4.35 (q, *J* = 7.2 Hz, 2H), 3.99-3.89 (m, 5H), 3.24 (t, *J* = 5.9 Hz, 2H), 3.05 (t, *J* = 6.0 Hz, 2H), 2.78 (s, 3H), 1.39 (t, *J* = 7.2 Hz, 3H).

Step 5: Preparation of *N*-(3-bromo-2-chlorophenyl)-1-ethyl-5-methyl-4,5,6,7-tetrahydro-1*H*-imidazo[4,5-*c*]pyridine-2-carboxamide

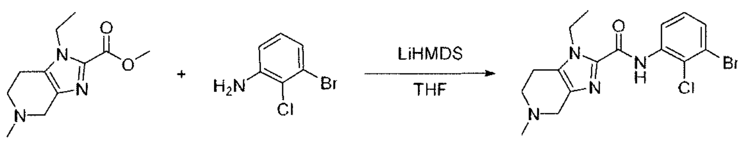


LiHMDS (3.03 mL, 1 M in THF) was added dropwise to a solution of methyl 1-ethyl-5-methyl-4,5,6,7-tetrahydro-1*H*-imidazo[4,5-*c*]pyridine-2-carboxylate (338 mg, 1.51 mmol) and 3-bromo-2-chloroaniline (344 mg, 1.67 mmol) in THF (3 mL) at room temperature and the reaction mixture was stirred at room temperature for 0.5 hours. The reaction mixture was quenched with half-saturated aqueous ammonium chloride solution (3 mL), then extracted with ethyl acetate (10 mL); the organic layer was dried over anhydrous sodium sulfate, and the mixture was filtered, then the filtrate was concentrated under reduced pressure, and the residue was separated and purified by silica gel column chromatography (DCM: MeOH = 100:1 to 10:1) to obtain the title compound as an off-white solid (168 mg, 28 %).

MS *m/z* (ESI): 397.1 $[M+H]^+$.

Step 6: Preparation of *N*-(2,2'-dichloro-3'-(5-formyl-6-methoxypyridin-2-yl)-[1,1'-biphenyl]-3-yl)-1-ethyl-5-methyl-4,5,6,7-tetrahydro-1*H*-imidazo[4,5-*c*]pyridine-2-carboxamide

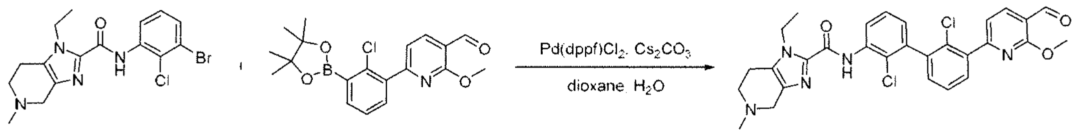


*N*-(3-Bromo-2-chlorophenyl)-1-ethyl-5-methyl-4,5,6,7-tetrahydro-1*H*-imidazo[4,5-*c*]pyridine-2-carboxamide (60 mg, 0.151 mmol), 6-(2-chloro-3-(4,4,5,5-tetramethyl-1,3,2-dioxaborolan-2-yl)phenyl)-2-methoxynicotinaldehyde (62 mg, 0.166 mmol), Pd(dppf)$Cl_2$ (11 mg, 0.016 mmol), $Cs_2CO_3$ (148 mg, 0.453 mmol) were added to a mixed solvent of 1,4-dioxane (1.0 mL) and water (0.2 mL) respectively, then the reaction mixture was heated to 95 °C and stirred for 16 hours under the protection of nitrogen. The layers of the reaction mixture were separated, and the organic layer was concentrated under reduced pressure, and the residue was separated and purified by silica gel column chromatography (DCM: MeOH = 100:1 to 10:1) to obtain the title compound as a brown oil (70 mg, 82 %).

MS *m/z* (ESI): 564.2 $[M+H]^+$.

Step 7: Preparation of *N*-(2,2'-dichloro-3'-(5-((((3,4-*trans*)-3-hydroxytetrahydro-2*H*-pyran-4-yl)amino)methyl)-6-methoxypyridine-[1,1'-biphenyl]-3-yl)-1-ethyl-5-methyl-4,5,6,7-tetrahydro-1*H*-imidazo[4,5-*c*]pyridine-2-carboxamide

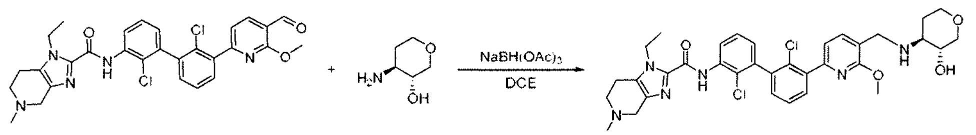


Sodium triacetoxyborohydride (22 mg, 0.106 mmol) was added to a solution of *N*-(2,2'-dichloro-3'-(5-formyl-6-methoxypyridin-2-yl)-[1,1-biphenyl]-3-yl)-1-ethyl-5-methyl-4,5,6,7-tetrahydro-1*H*-imidazo[4,5-*c*]pyridine-2-carboxamide (20 mg, 0.035 mmol) and *trans*-3-amino-4-hydroxy-tetrahydropyran (6 mg, 0.053 mmol) in dichloroethane (0.5 mL) at room temperature, then the reaction mixture was stirred at room temperature for 1 hour. The reaction mixture was partitioned between saturated aqueous sodium bicarbonate solution (2 mL) and DCM (5 mL); the organic phase was dried over anhydrous sodium sulfate, and the mixture was filtered, then the filtrate was concentrated under reduced pressure, and the residue was separated by prep-HPLC to obtain the title compound as a white solid (7 mg, 29 %).

MS *m/z* (ESI): 665.2 $[M+H]^+$.

$^1$H NMR (400 MHz, DMSO-$d_6$) $\delta$ 9.93 (t, 1H), 8.36 (d, *J* = 8.2 Hz, 1H), 7.85 (d, *J* = 7.5 Hz, 1H), 7.68 (dd, *J* = 7.7, 1.8 Hz, 1H), 7.55 (t, *J* = 7.6 Hz, 1H), 7.48 (t, *J* = 8.0 Hz, 1H), 7.44-7.37 (m, 1H), 7.27 (d, *J* = 7.4 Hz, 1H), 7.19 (d, *J* = 7.6 Hz, 1H), 4.38 (q, *J* = 7.1 Hz, 2H), 3.92 (s, 3H), 3.82-3.68 (m, 4H), 3.58-3.55 (m, 1H), 3.37 (s, 2H), 3.28-3.20 (m, 2H), 2.96 (t, *J* = 10.2 Hz, 1H), 2.73-2.66 (m, 4H), 2.38 (s, 3H), 1.99-1.91 (m, 1H), 1.32 (t, *J* = 7.1 Hz, 3H), 1.27-1.19 (m, 1H).

## Embodiment 236

*N*-(2,2'-Dichloro-3'-(6-methoxy-5-((oxetan-3-ylamino)methyl)pyridin-2-yl)-[1,1'-biphenyl]-3-yl)-1-ethyl-5-methyl-4,5,6,7-tetrahydro-1*H*-imidazo[4,5-*c*]pyridine-2-carboxamide

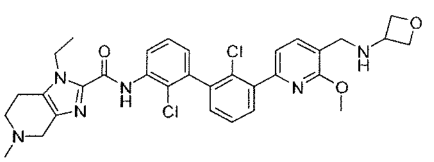

Preparation of *N*-(2,2'-dichloro-3'-(6-methoxy-5-((oxetan-3-ylamino)methyl)pyridin-2-yl)-[1,1'-biphenyl]-3-yl)-1-ethyl-5-methyl-4,5,6,7-tetrahydro-1*H*-imidazo[4,5-*c*]pyridine-2-carboxamide was referred to Embodiment 235.

MS *m/z* (ESI): 621.2 $[M+H]^+$.

$^1$H NMR (400 MHz, DMSO-$d_6$) $\delta$ 9.93 (s, 1H), 8.39-8.33 (m, 1H), 7.78 (d, *J* = 7.5 Hz, 1H), 7.68-7.64 (m, 1H), 7.54 (t, *J* = 7.7 Hz, 1H), 7.48 (t, *J* = 7.9 Hz, 1H), 7.41 (dd, *J* = 7.6, 1.7 Hz, 1H), 7.26 (d, *J* = 7.4 Hz, 1H), 7.18 (dd, *J* = 7.7, 1.6 Hz, 1H), 4.62-4.56 (m, 2H), 4.42-4.35 (m, 2H), 4.35-4.29 (m, 2H), 3.97-3.87 (m, 4H), 3.63 (s, 2H), 3.42 (s, 2H),

2.74-2.65 (m, 4H), 2.63-2.55 (m, 1H), 2.38 (s, 3H), 1.32 (t, *J* = 7.1 Hz, 3H).

**Embodiment 237**

*N*-(3'-(5-((2-Oxa-6-azaspiro[3.3]heptan-6-yl)methyl)-6-methoxypyridin-2-yl)-2,2'-dichloro-[1,1'-biphenyl]-3-yl)-1-ethyl-5-methyl-4,5,6,7-tetrahydro-1*H*-imidazo[4,5-*c*]pyridine-2-carboxamide

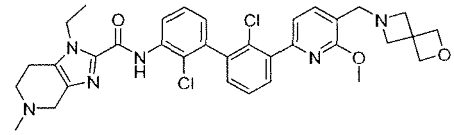

Preparation of *N*-(3'-(5-((2-oxa-6-azaspiro[3.3]heptan-6-yl)methyl)-6-methoxypyridin-2-yl)-2,2'-dichloro-[1,1'-biphenyl]-3-yl)-1-ethyl-5-methyl-4,5,6,7-tetrahydro-1*H*-imidazo[4,5-*c*]pyridine-2-carboxamide was referred to Embodiment 235.
MS *m/z* (ESI): 647.2 $[M+H]^+$.
$^1$H NMR (400 MHz, DMSO-$d_6$) $\delta$ 9.93 (s, 1H), 8.39-8.32 (m, 1H), 7.71-7.64 (m, 2H), 7.54 (t, *J* = 7.6 Hz, 1H), 7.48 (t, *J* = 7.9 Hz, 1H), 7.40 (dd, *J* = 7.5, 1.7 Hz, 1H), 7.25 (d, *J* = 7.5 Hz, 1H), 7.18 (dd, *J* = 7.6, 1.6 Hz, 1H), 4.67-4.57 (m, 4H), 4.38 (q, *J* = 7.1 Hz, 2H), 3.90 (s, 3H), 3.50 (s, 2H), 3.39-3.37 (m, 4H), 3.37 (s, 2H), 2.75-2.65 (m, 4H), 2.38 (s, 3H), 1.31 (t, *J* = 7.1 Hz, 3H).

**Embodiment 238**

*N*-(3'-(5-((6-Acetyl-2,6-diazaspiro[3.3]heptan-2-yl)methyl)-6-methoxypyridin-2-yl)-2,2'-dichloro-[1,1'-biphenyl]-3-yl)-1-ethyl-5-methyl-4,5,6,7-tetrahydro-1*H*-imidazo[4,5-*c*]pyridine-2-carboxamide

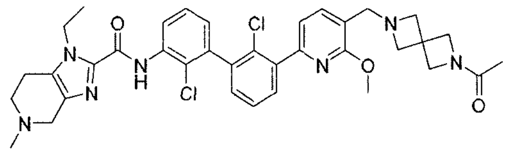

Preparation of *N*-(3'-(5-((6-acetyl-2,6-diazaspiro[3.3]heptan-2-yl)methyl)-6-methoxypyridin-2-yl)-2,2'-dichloro-[1,1'-biphenyl]-3-yl)-1-ethyl-5-methyl-4,5,6,7-tetrahydro-1*H*-imidazo[4,5-*c*]pyridine-2-carboxamide was referred to Embodiment 235.
MS *m/z* (ESI): 688.2 $[M+H]^+$.
$^1$H NMR (400 MHz, DMSO-$d_6$) $\delta$ 9.93 (s, 1H), 8.36-8.32 (m, 1H), 7.76-7.72 (m, 1H), 7.68 (dd, *J* = 7.7, 1.8 Hz, 1H), 7.55 (t, *J* = 7.6 Hz, 1H), 7.49 (t, *J* = 7.9 Hz, 1H), 7.41 (d, *J* = 7.5 Hz, 1H), 7.29 (d, *J* = 7.6 Hz, 1H), 7.21-7.17 (m, 1H), 4.45-4.35 (m, 2H), 4.19 (s, 2H), 3.96-3.88 (m, 5H), 3.58-3.47 (m, 2H), 3.47-3.35 (m, 6H), 2.90-2.71 (m, 4H), 2.47 (s, 3H), 1.72 (s, 3H), 1.32 (t, *J* = 7.1 Hz, 3H).

**Embodiment 239**

*N*-(2,2'-Dichloro-3'-(5-((3-(hydroxymethyl)azetidin-1-yl)methyl)-6-methoxypyridin-2-yl)-[1,1'-biphenyl]-3-yl)-1-ethyl-5-methyl-4,5,6,7-tetrahydro-1*H*-imidazo[4,5-*c*]pyridine-2-carboxamide

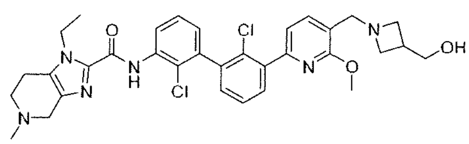

Preparation of *N*-(2,2'-dichloro-3'-(5-((3-(hydroxymethyl)azetidin-1-yl)methyl)-6-methoxypyridin-2-yl)-[1,1'-biphenyl]-3-yl)-1-ethyl-5-methyl-4,5,6,7-tetrahydro-1*H*-imidazo[4,5-c]pyridine-2-carboxamide was referred to Embodiment 235.

MS *m/z* (ESI): 635.2 $[M+H]^+$.

$^1H$ NMR (400 MHz, DMSO-$d_6$) $\delta$ 9.93 (s, 1H), 8.35 (d, *J* = 8.1 Hz, 1H), 7.68 (t, *J* = 8.1 Hz, 2H), 7.54 (t, *J* = 7.7 Hz, 1H), 7.48 (t, *J* = 7.9 Hz, 1H), 7.43-7.37 (m, 1H), 7.26 (d, *J* = 7.5 Hz, 1H), 7.18 (d, *J* = 7.5 Hz, 1H), 4.38 (q, *J* = 7.1 Hz, 2H), 3.91 (s, 3H), 3.56 (s, 2H), 3.51 (s, 2H), 3.37 (s, 2H), 3.34-3.28 (m, 2H), 3.02-3.95 (m, 2H), 2.74-2.67 (m, 4H), 2.54-2.51 (m, 1H), 2.38 (s, 3H), 1.32 (t, *J* = 7.0 Hz, 3H).

## Embodiment 240

1-((6-(2,2'-Dichloro-3'-(1-ethyl-5-methyl-4,5,6,7-tetrahydro-1*H*-imidazo[4,5-*c*]pyridine-2-carboxamido)-[1,1'-biphenyl]-3-yl)-2-methoxypyridin-3-yl)methyl)azetidine-3-carboxylic acid

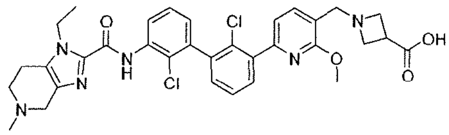

Preparation of 1-((6-(2,2'-dichloro-3'-(1-ethyl-5-methyl-4,5,6,7-tetrahydro-1*H*-imidazo[4,5-*c*]pyridine-2-carboxamido)-[1,1'-biphenyl]-3-yl)-2-methoxypyridin-3-yl)methyl)azetidine-3-carboxylic acid was referred to Embodiment 235.

MS *m/z* (ESI): 649.2 $[M+H]^+$.

$^1H$ NMR (400 MHz, DMSO-$d_6$) $\delta$ 9.93 (s, 1H), 8.36 (d, *J* = 8.4 Hz, 1H), 7.72-7.64 (m, 2H), 7.58-7.51 (m, 1H), 7.48 (t, *J* = 7.9 Hz, 1H), 7.40 (d, *J* = 7.7 Hz, 1H), 7.26 (d, *J* = 7.4 Hz, 1H), 7.18 (d, *J* = 7.5 Hz, 1H), 4.38 (q, *J* = 7.1 Hz, 2H), 3.91 (s, 3H), 3.54 (s, 2H), 3.48-3.41 (m, 2H), 3.37 (s, 2H), 3.29-3.22 (m, 2H), 2.73-2.66 (m, 4H), 2.63-2.57 (m, 1H), 2.38 (s, 3H), 1.31 (t, *J* = 7.1 Hz, 3H).

## Embodiment 241

*N*-(2,2'-Dichloro-3'-(5-((3-(hydroxymethyl)azetidin-1-yl)methyl)-4-methoxypyrimidin-2-yl)-[1,1'-biphenyl]-3-yl)-1-ethyl-5-methyl-4,5,6,7-tetrahydro-1*H*-imidazo[4,5-*c*]pyridine-2-carboxamide

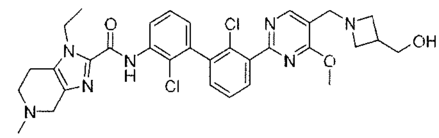

Step 1: Preparation of *N*-(2,2'-dichloro-3'-(5-formyl-4-methoxypyrimidin-2-yl)-[1,1'-biphenyl]-3-yl)-1-ethyl-5-methyl-4,5,6,7-tetrahydro-1*H*-imidazo[4,5-*c*]pyridine-2-carboxamide

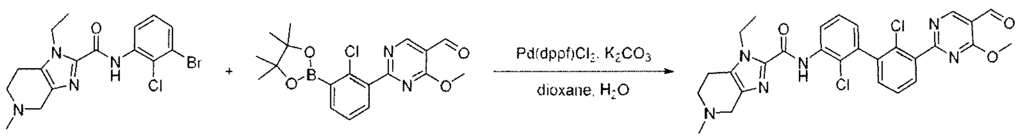


Pd(dppf)$Cl_2$ (21 mg, 0.028 mmol) was added to a solution of *N*-(3-bromo-2-chlorophenyl)-1-ethyl-5-methyl-4,5,6,7-tetrahydro-1*H*-imidazo[4,5-c]pyridine-2-carboxamide (113 mg, 0.284 mmol), 2-(2-chloro-3-(4,4,5,5-tetramethyl-1,3,2-dioxaborolan-2-yl)phenyl)-4-methoxypyrimidine-5-carbaldehyde (213 mg, 0.568 mmol), $K_2CO_3$ (94 mg, 0.682 mmol) in a mixed solvent of 1,4-dioxane (2 mL) and water (0.2 mL), then the reaction system was replaced with nitrogen

three times, and the reaction mixture was heated to 80 °C and the reaction was carried out for 16 hours. The reaction mixture was concentrated under reduced pressure, and the residue was diluted with DCM (5 mL), then washed with saturated brine (5 mL), dried over anhydrous sodium sulfate; the mixture was filtered, and the filtrate was concentrated under reduced pressure, and the residue was separated and purified by silica gel column chromatography (DCM: MeOH = 50: 1 to 5: 1) to obtain the title compound as a light yellow solid (160 mg, 98 %).

MS *m/z* (ESI): 565.2 [M+H]+.

Step 2: Preparation of *N*-(2,2'-dichloro-3'-(5-((3-(hydroxymethyl)azetidin-1-yl)methyl)-4-methoxypyrimidin-2-yl)-[1,1'-biphenyl]-3-yl)-1-ethyl-5-methyl-4,5,6,7-tetrahydro-1*H*-imidazo[4,5-*c*]pyridine-2-carboxamide

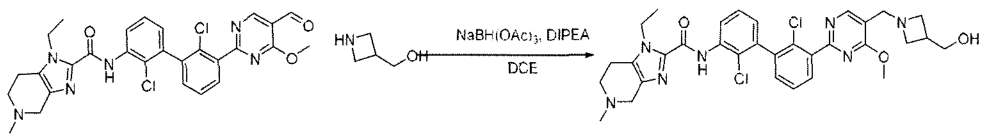


3-Methylhydroxyazetidine hydrochloride (16.42 mg, 0.133 mmol) was added to a solution of *N*-(2,2'-dichloro-3'-(5-formyl-4-methoxypyrimidin-2-yl)-[1,1-biphenyl]-3-yl)-1-ethyl-5-methyl-4,5,6,7-tetrahydro-1*H*-imidazo[4,5-*c*]pyridine-2-carboxamide (50 mg, 0.088 mmol) and DIEA (17 mg, 0.133 mmol) in DCE (0.5 mL), and the reaction mixture was stirred at room temperature for 1 hour, then sodium triacetoxyborohydride (56 mg, 0.265 mmol) was added. The reaction mixture was stirred at room temperature for 16 hours. The reaction mixture was partitioned between saturated aqueous sodium bicarbonate solution (5 mL) and DCM (10 mL), and the organic layer was concentrated under reduced pressure, and then the residue was separated and purified by prep-HPLC to obtain the title compound as a brown solid (6 mg, 9 %).

MS *m/z* (ESI): 636.2 [M+H]+.

$^1$H NMR (400 MHz, DMSO-$d_6$) $\delta$ 9.93 (s, 1H), 8.51 (s, 1H), 8.36 (dd, *J* = 8.3, 1.6 Hz, 1H), 7.78 (dd, *J* = 7.7, 1.8 Hz, 1H), 7.56 (t, *J* = 7.6 Hz, 1H), 7.51-7.44 (m, 2H), 7.18 (dd, *J* = 7.7, 1.6 Hz, 1H), 4.38 (t, *J* = 7.1 Hz, 2H), 3.98 (s, 3H), 3.55 (s, 2H), 3.51 (d, *J* = 6.6 Hz, 2H), 3.37 (s, 2H), 3.31 (t, *J* = 7.2 Hz, 2H), 2.98 (t, *J* = 6.5 Hz, 2H), 2.75-2.65 (m, 4H), 2.48-2.44 (m, 1H), 2.38 (s, 3H), 1.31 (t, *J* = 7.1 Hz, 3H).

**Embodiment 242**

*N*-(2,2'-Dichloro-3'-(5-(((*trans*-3-hydroxytetrahydro-2*H*-pyran-4-yl)amino)methyl)-4-methoxypyrimidin-2-yl)-[1,1'-biphenyl]-3-yl)-1-ethyl-5-methyl-4,5,6,7-tetrahydro-1*H*-imidazo[4,5-*c*]pyridine-2-carboxamide

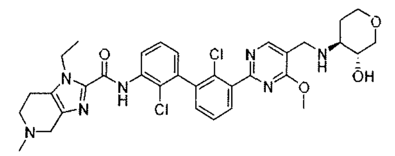

Preparation of *N*-(2,2'-dichloro-3'-(5-(((*trans*-3-hydroxytetrahydro-2*H*-pyran-4-yl)amino)methyl)-4-methoxypyrimidin-2-yl)-[1,1'-biphenyl]-3-yl)-1-ethyl-5-methyl-4,5,6,7-tetrahydro-1*H*-imidazo[4,5-*c*]pyridine-2-carboxamide was referred to Embodiment 241.

MS *m/z* (ESI): 666.2 [M+H]+.

$^1$H NMR (400 MHz, DMSO-$d_6$) $\delta$ 9.93 (s, 1H), 8.68 (s, 1H), 8.39-8.32 (m, 1H), 7.79 (dd, *J* = 7.7, 1.8 Hz, 1H), 7.56 (t, *J* = 7.6 Hz, 1H), 7.52-7.43 (m, 2H), 7.18 (dd, *J* = 7.6, 1.6 Hz, 1H), 4.38 (q, *J* = 7.1 Hz, 2H), 3.99 (s, 3H), 3.83-3.70 (m, 5H), 3.37 (s, 2H), 3.24 (dd, *J* = 9.2, 3.7 Hz, 2H), 2.95 (t, *J*= 10.3 Hz, 1H), 2.73-2.64 (m, 4H), 2.44-2.41 (m, 1H), 2.38 (s, 3H), 1.99-1.92 (m, 1H), 1.36-1.22 (m, 4H), 1.02 (t, *J* = 7.1 Hz, 1H).

**Embodiment 243**

*N*-(3'-(5-((6-Acetyl-2,6-diazaspiro[3.3]heptan-2-yl)methyl)-6-methoxypyridin-2-yl)-2,2'-dichloro-[1,1'-biphenyl]-3-yl)-1-cyclopropyl-5-methyl-4,5,6,7-tetrahydro-1*H*-imidazo[4,5-*c*]pyridine-2-carboxamide

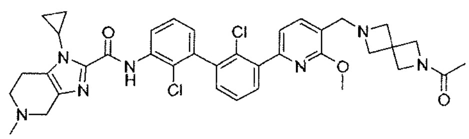

Step 1: Preparation of *N*-cyclopropyl-3-nitropyridin-4-amine

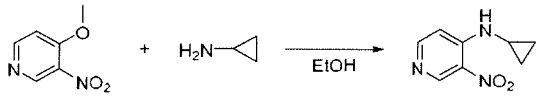


Cyclopropanamine (1.85 g, 32.4 mmol) was added to a solution of 3-nitro-4-methoxypyridine (2.0 g, 13.0 mmol) in EtOH (80 mL), and the reaction mixture was heated and refluxed for 16 hours. The reaction mixture was concentrated to 10 mL under reduced pressure, then slurried at 0 °C; the mixture ws filtered, and the filter cake was washed with cold ethanol (5 mL) and dried under reduced pressure to obtain the title compound as a yellow powder (1.98 g, 85 %).
MS *m/z* (ESI): 180.1 $[M+H]^+$.

Step 2: Preparation of $N^4$-cyclopropylpyridine-3,4-diamine

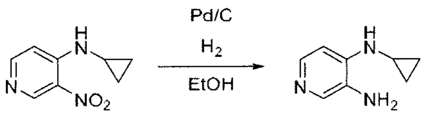


Pd/C (400 mg, 50 % wet, 10 % Pd) was added to a solution of *N*-cyclopropyl-3-nitropyridin-4-amine (1.98 g, 11.1 mmol) in EtOH (100 mL), then the reaction system was replaced with hydrogen atmosphere with a hydrogen balloon, then the reaction mixture was stirred at room temperature for 5 hours. The reaction mixture was filtered through celite, and the filtrate was concentrated under reduced pressure to obtain the title compound as a gray solid (1.57 g, 95 %).
MS m/z (ESI): 150.1 [M+H]+.

Step 3: Preparation of methyl 1-cyclopropyl-1*H*-imidazo[4,5-*c*]pyridine-2-carboxylate

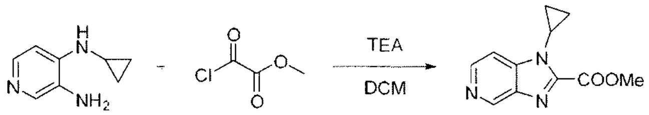


Methyl 2-chloro-2-oxoacetate (2.84 g, 23.2 mmol) was added dropwise to a solution of $N^4$-cyclopropylpyridine-3,4-diamine (1.57 g, 10.5 mmol) and TEA (2.65 g, 26.2 mmol) in DCM (10 mL) at 0 °C, after the addition was completed, the reaction mixture was heated to room temperature, and the mixture was stirred for 1 hour. The reaction mixture was partitioned in saturated aqueous sodium bicarbonate solution (10 mL) and DCM (10 mL), and the organic layer was concentrated under reduced pressure, and the residue was separated and purified by silica gel column chromatography (DCM: MeOH = 100: 1 to 10: 1) to obtain the title compound as a milky white solid (458 mg, 20 %).

MS *m/z* (ESI): 218.1 [M+H]+.

Step 4: Preparation of methyl 1-cyclopropyl-5-methyl-4,5,6,7-tetrahydro-1*H*-imidazo[4,5-*c*]pyridine-2-carboxylate

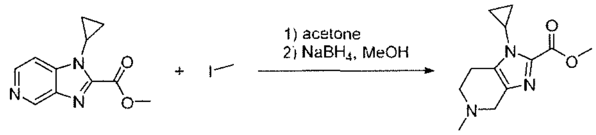


Iodomethane (314 mg, 2.21 mmol) was added to a solution of methyl 1-cyclopropyl-1*H*-imidazo[4,5-c]pyridine-2-carboxylate (458 mg, 2.11 mmol) in acetone (5.0 mL), then the reaction mixture was heated to 56 °C and stirred for 16 hours. The reaction mixture was concentrated under reduced pressure, and the residue was dissolved in MeOH (3 mL); sodium borohydride (160 mg, 4.22 mmol) was added in portions at room temperature, and the mixture was stirred at room temperature for 0.5 hours. The reaction mixture was diluted with acetone (5 mL), then concentrated under reduced pressure, and the residue was separated and purified by silica gel column chromatography (DCM: MeOH = 100: 1 to 10: 1) to obtain the title compound as a milky white solid (397 mg, 80 %).

MS *m/z* (ESI): 236.1 [M+H]+.

Step 5: Preparation of *N*-(3-bromo-2-chlorophenyl)-1-cyclopropyl-5-methyl-4,5,6,7-tetrahydro-1*H*-imidazo[4,5-*c*]pyridine-2-carboxamide

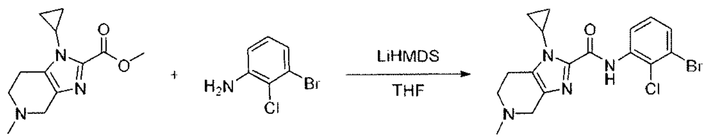


LiHMDS (4.25 mL, 1 M in THF) was added dropwise to a solution of methyl 1-cyclopropyl-5-methyl-4,5,6,7-tetrahydro-1*H*-imidazo[4,5-*c*]pyridine-2-carboxylate (500 mg, 2.13 mmol) and 3-bromo-2-chloroaniline (483 mg, 2.34 mmol) in THF (10 mL) at room temperature, and the reaction mixture was stirred at room temperature for 0.5 hours. The reaction mixture was quenched with half-saturated aqueous ammonium chloride solution (10 mL), then extracted with ethyl acetate (20 mL); the organic layer was dried over anhydrous sodium sulfate, and the mixture was filtered, then the filtrate was concentrated under reduced pressure, and the residue was separated and purified by silica gel column chromatography (DCM: MeOH = 100: 1 to 10: 1) to obtain the title compound as an off-white solid (577 mg, 66 %).

MS *m/z* (ESI): 409.1 [M+H]+.

Step 6: Preparation of 1-cyclopropyl-*N*-(2,2'-dichloro-3'-(5-formyl-6-methoxypyridin-2-yl)-[1,1'-biphenyl]-3-yl)-5-methyl-4,5,6,7-tetrahydro-1*H*-imidazo[4,5-*c*]pyridine-2-carboxamide

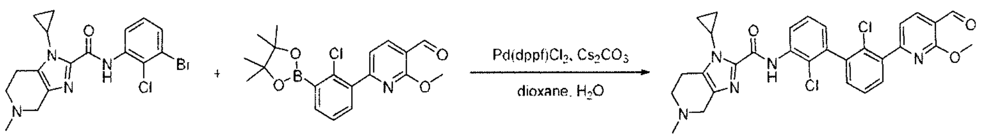


*N*-(3-Bromo-2-chlorophenyl)-1-cyclopropyl-5-methyl-4,5,6,7-tetrahydro-1*H*-imidazo[4,5-c]pyridine-2-carboxamide (264 mg, 0.645 mmol), 6-(2-chloro-3-(4,4,5,5-tetramethyl-1,3,2-dioxaborolan-2-yl)phenyl)-2-methoxynicotinaldehyde (265 mg, 0.709 mmol), $Pd(dppf)Cl_2$ (47 mg, 0.064 mmol), $Cs_2CO_3$ (631 mg, 1.93 mmol) were added to a mixed solvent of 1 ,4-dioxane (5.0 mL) and water (1.0 mL) respectively, then the reaction mixture was heated to 95 °C and stirred for 16 hours under the protection of nitrogen. The layers of the reaction mixture were separated, and the organic layer was concentrated under reduced pressure, and the residue was separated and purified by silica gel column

chromatography (DCM: MeOH = 100: 1 to 10: 1) to obtain the title compound as a brown oil (286 mg, 77 %).

MS *m/z* (ESI): 576.1 [M+H]$^+$.

Step 7: Preparation of *N*-(3'-(5-((6-acetyl-2,6-diazaspiro[3.3]heptan-2-yl)methyl)-6-methoxypyridin-2-yl)-2,2'-dichloro-[1,1'-biphenyl]-3-yl)-1-cyclopropyl-5-methyl-4,5,6,7-tetrahydro-1*H*-imidazo[4,5-*c*]pyridine-2-carboxamide

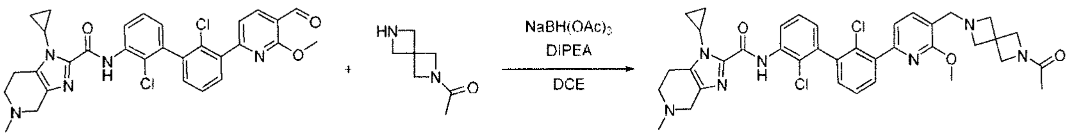


DIPEA (51 mg, 0.395 mmol) was added to a solution of 1-cyclopropyl-*N*-(2,2'-dichloro-3'-(5-formyl-6-methoxypyridin-2-yl)-[1,1'-biphenyl]-3-yl)-5-methyl-4,5,6,7-tetrahydro-1*H*-imidazo[4,5-c]pyridine-2-carboxamide (60 mg, 0.104 mmol) and 1-(2,6-diazaspiro[3.3]heptan-2-yl)ethanone trifluoroacetate (60 mg, 0.125 mmol) in DCE (0.6 mL) at room temperature, then the reaction mixture was stirred at room temperature for 1 hour, then sodium triacetoxyborohydride (33 mg, 0.156 mmol) was added, and the mixture was stirred at room temperature for 16 hours. The reaction mixture was partitioned between saturated aqueous sodium bicarbonate solution (2 mL) and DCM (5 mL); the organic phase was dried over anhydrous sodium sulfate, and the mixture was filtered, then the filtrate was concentrated under reduced pressure, and the residue was separated by prep-HPLC to obtain the title compound as white solid (17 mg, 22 %).

MS *m/z* (ESI): 700.2 [M+H]$^+$.

$^1$H NMR (400 MHz, DMSO-$d_6$) $\delta$ 9.86 (s, 1H), 8.39 (dd, *J* = 8.3, 1.6 Hz, 1H), 7.73 -7.64 (m, 2H), 7.54 (t, *J* = 7.6 Hz, 1H), 7.48 (t, *J* = 7.9 Hz, 1H), 7.40 (dd, *J* = 7.5, 1.7 Hz, 1H), 7.26 (d, *J* = 7.4 Hz, 1H), 7.17 (dd, *J* = 7.6, 1.6 Hz, 1H), 4.18 (s, 2H), 3.94-3.86 (m, 5H), 3.53 (s, 2H), 3.45-3.41 (m, 1H), 3.39-3.30 (m, 6H), 2.81-2.73 (m, 2H), 2.69-2.62 (m, 2H), 2.37 (s, 3H), 1.72 (s, 3H), 1.12-1.06 (m, 2H), 0.97-0.89 (m, 2H).

**Embodiment 244**

1-Cyclopropyl-*N*-(2,2'-dichloro-3'-(5-(((*trans*-3-hydroxytetrahydro-2*H*-pyran-4-yl)amino)methyl)-6-methoxypyridin-2-yl)-[1,1'-biphenyl]-3-yl)-5-methyl-4,5,6,7-tetrahydro-1*H*-imidazo[4,5-*c*]pyridine-2-carboxamide

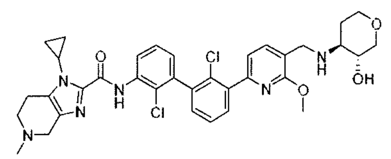

Preparation of 1-cyclopropyl-*N*-(2,2'-dichloro-3'-(5-(((*trans*-3-hydroxytetrahydro-2*H*-pyran-4-yl)amino)methyl)-6-methoxypyridin-2-yl)-[1,1'-biphenyl]-3-yl)-5-methyl-4,5,6,7-tetrahydro-1*H*-imidazo[4,5-c]pyridine-2-carboxamide was referred to Embodiment 243.

MS *m/z* (ESI): 677.2 [M+H]$^+$.

$^1$H NMR (400 MHz, DMSO-$d_6$) $\delta$ 9.93 (d, *J* = 4.3 Hz, 1H), 8.30-8.23 (m, 1H), 8.09 (d, *J* = 7.6 Hz, 1H), 7.69 (dd, *J* = 7.7, 1.7 Hz, 1H), 7.60-7.54 (m, 1H), 7.50 (d, *J* = 7.9 Hz, 1H), 7.44 (d, *J* = 7.5 Hz, 1H), 7.36 (d, *J* = 7.5 Hz, 1H), 7.23 (d, *J* = 7.5 Hz, 1H), 4.39-4.25 (m, 3H), 4.21-4.11 (m, 2H), 3.97 (s, 3H), 3.70-3.65 (m, 2H), 3.52-3.43 (m, 1H), 3.42-3.37 (m, 1H), 3.35-3.26 (m, 1H), 3.23-3.08 (m, 4H), 3.08-2.97 (m, 2H), 2.95-2.82 (m, 4H), 2.23-2.14 (m, 1H), 1.84-1.74 (m, 1H), 1.16-0.87 (m, 4H).

**Embodiment 245**

1-Cyclopropyl-*N*-(2,2'-dichloro-3'-(5-((3-(hydroxymethyl)azetidin-1-yl)methyl)-6-methoxypyridin-2-yl)-[1,1'-biphenyl]-3-yl)-5-methyl-4,5,6,7-tetrahydro-1*H*-imidazo[4,5-*c*]pyridine-2-carboxamide

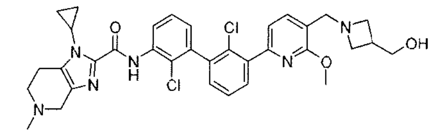

Preparation of 1-cyclopropyl-*N*-(2,2'-dichloro-3'-(5-((3-(hydroxymethyl)azetidin-1-yl)methyl)-6-methoxypyridin-2-yl)-[1,1'-biphenyl]-3-yl)-5-methyl-4,5,6,7-tetrahydro-1*H*-imidazo[4,5-*c*]pyridine-2-carboxamide was referred to Embodiment 243.

MS *m/z* (ESI): 647.2 [M+H]+.

$^1$H NMR (400 MHz, DMSO-$d_6$) $\delta$ 9.86 (s, 1H), 8.39 (dd, *J* = 8.2, 1.6 Hz, 1H), 7.73-7.64 (m, 2H), 7.54 (t, *J* = 7.6 Hz, 1H), 7.48 (t, *J* = 8.0 Hz, 1H), 7.40 (dd, *J* = 7.5, 1.7 Hz, 1H), 7.26 (d, *J* = 7.5 Hz, 1H), 7.17 (dd, *J* = 7.6, 1.6 Hz, 1H), 3.91 (s, 3H), 3.56 (s, 2H), 3.52 (d, *J* = 6.6 Hz, 2H), 3.47-3.39 (m, 1H), 3.35 (s, 2H), 3.34-3.30 (m, 2H), 3.03-2.96 (m, 2H), 2.81-2.74 (m, 2H), 2.70-2.62 (m, 2H), 2.53-2.51 (m, 1H), 2.37 (s, 3H), 1.12-1.06 (m, 2H), 0.98-0.92 (m, 2H).

**Embodiment 246**

*N*-(3'-(5-((6-Acetylamino-2-azaspiro[3.3]heptan-2-yl)methyl)-6-methoxypyridin-2-yl)-2,2'-dichloro-[1,1'-biphenyl]-3-yl)-1-cyclopropyl-5-methyl-4,5,6,7-tetrahydro-1*H*-imidazo[4,5-*c*]pyridine-2-carboxamide

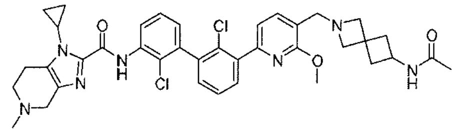

Preparation of *N*-(3'-(5-((6-acetylamino-2-azaspiro[3.3]heptan-2-yl)methyl)-6-methoxypyridin-2-yl)-2,2'-dichloro-[1,1'-biphenyl]-3-yl)-1-cyclopropyl-5-methyl-4,5,6,7-tetrahydro-1*H*-imidazo[4,5-c]pyridine-2-carboxamide was referred to Embodiment 243.

MS *m/z* (ESI): 714.2 [M+H]+.

$^1$H NMR (400 MHz, DMSO-$d_6$) $\delta$ 9.92 (s, 1H), 8.28 (dd, *J* = 8.3, 1.5 Hz, 1H), 8.18-8.12 (m, 1H), 8.00 (d, *J* = 7.6 Hz, 1H), 7.69 (dd, *J* = 7.7, 1.7 Hz, 1H), 7.58 (t, *J* = 7.6 Hz, 1H), 7.51 (t, *J* = 7.9 Hz, 1H), 7.47-7.41 (m, 1H), 7.37 (d, *J* = 7.5 Hz, 1H), 7.23 (dd, *J* = 7.6, 1.6 Hz, 1H), 4.41-4.31 (m, 4H), 4.20-4.14 (m, 1H), 4.12-4.09 (m, 2H), 4.08-3.99 (m, 2H), 3.98 (s, 3H), 3.71-3.63 (m, 1H), 3.52-3.42 (m, 1H), 3.41-3.31 (m, 1H), 3.19-3.04 (m, 2H), 2.90 (d, *J* = 4.5 Hz, 3H), 2.63-2.54 (m, 1H), 2.50-2.42 (m, 1H), 2.17-2.03 (m, 2H), 1.75 (s, 3H), 1.17-1.11 (m, 2H), 1.03-0.98 (m, 1H), 0.93-0.85 (m, 1H).

## Biological Test Evaluation

The present disclosure is further described and explained in conjunction with test embodiments, but the scope of the present disclosure is not limited by these embodiments.

**Test Embodiment 1: Test of inhibitory effect of the compounds of the present disclosure on human PD-1/PD-L1 binding**

Experimental purpose: The purpose of this test embodiment was to measure the inhibitory activity of the compounds on human PD-1/PD-L1 binding.

Experimental equipment: centrifuge (5810R) was purchased from Eppendorf Company, pipette was purchased from Eppendorf or Rainin Company, microplate reader was purchased from American BioTek Company and the model was H1MFD full-function microplate reader.

Experimental reagents: Binding Domain diluent buffer was purchased from Cisbio Company, Cat. No.: 62DLBDDF; MAb Anti-6HIS Eu cryptate Gold was purchased from Cisbio Company with the article number of 61HI2KLA; PAb Anti Human IgG-XL665 was purchased from Cisbio Company with the article number of 61HFCXLB; PD-L1-His protein was purchased from Abcam Company with the article number of ab167713; PD-1-Fc protein was purchased from R&D Company with the article number of 1086-PD; Detection buffer was purchased from Cisbio Company with the article number of 62SDBRDD; 384-well plate was purchased from PerkinElmer Company with the article number of

6007290.

Experimental method: In order to test the inhibitory activity of the compounds on human PD-1/PD-L1 binding, the method of time-resolved fluorescence resonance energy transfer (TR-FRET) was used in this experiment to test the inhibition of the compounds on human PD-1/PD-L1 binding, and the half inhibitory concentration $IC_{50}$ of inhibitory activity of the compounds on human PD-1/PD-L1 binding was obtained.

The specific experimental operations were as follows:

This experiment was performed in a 384-well plate with a total reaction volume of 20 μL. Compounds were diluted to different concentrations (10 μM or 1 μM starting, 3-fold dilution, 11 doses) using assay buffer Binding Domain diluent buffer (Cisbio # 62DLBDDF), then PD-L1-His protein (19 to 238 amino acids) (Abcam # ab167713) was diluted to 10 nM with assay buffer, and PD-1-Fc protein (25 to 167 amino acids) (R&D # 1086-PD) was diluted to 20 nM with assay buffer; the compound, PD-L1 protein and PD-1 protein were added to a 384-well plate respectively, and the total volume was 10 μL, then centrifuged at 1000 rpm for 1 minute to mix well, and incubated for 60 minutes at room temperature. Then 10 μL of a mixed solution of MAb Anti-6HIS Eu cryptate Gold (Cisbio# 61HI2KLA) and PAb Anti Human IgG-XL665 (Cisbio# 61HFCXLB) diluted by HTRF Detection buffer was added to each well, and centrifuged at 1000 rpm for 1 minute to mix well. The reaction was carried out at room temperature for 3 hours or at 4 °C overnight, then a microplate reader (BioTek Synergy H1) was put into for reading; the excitation wavelength was 340 nm, and the emission wavelengths were 620 nm and 665 nm.

Experimental data processing method:

The ratio of fluorescence readings at 620 nm and 665 nm (665 nm/620 nm) was calculated, and the inhibition rate was calculated. The concentration of compound and inhibition rate were fitted by nonlinear regression using Graphpad Prism software to obtain $IC_{50}$ values, as shown in Table 1 below.

Table 1 $IC_{50}$ values of compounds on human PD-1/PD-L1 binding

| Embodiment number | PD-1/PD-L1 binding $IC_{50}$ (nM) |
|---|---|
| 1 | 6.9 |
| 2 | 5.0 |
| 11 | 5.1 |
| 18 | 2.4 |
| 22 | 5.9 |
| 30 | 5.7 |
| 35 | 3.6 |
| 36 | 5.0 |
| 37 | 1.5 |
| 38 | 2.8 |
| 39 | 5.0 |
| 40 | 2.0 |
| 41 | 4.0 |
| 43 | 4.1 |
| 49 | 5.1 |
| 50 | 9.0 |
| 51 | 5.5 |
| 56 | 2.1 |
| 57 | 3.0 |
| 58 | 1.5 |
| 59 | 4.7 |
| 60 | 6.9 |
| 70 | 10.0 |

(continued)

| Embodiment number | PD-1/PD-L1 binding $IC_{50}$ (nM) |
|---|---|
| 81 | 2.4 |
| 82 | 3.9 |
| 83 | 2.7 |
| 88 | 9.0 |
| 95 | 2.4 |
| 96 | 2.3 |
| 97 | 5.0 |
| 98 | 6.3 |
| 99 | 4.4 |
| 100 | 6.0 |
| 102 | 1.0 |
| 104 | 9.1 |
| 105 | 8.5 |
| 106 | 6.5 |
| 108 | 8.4 |
| 109 | 4.7 |
| 112 | 9.4 |
| 114 | 4.3 |
| 120 | 7.4 |
| 122 | 3.5 |
| 123 | 1.6 |
| 124 | 3.8 |
| 125 | 8.4 |
| 126 | 2.0 |
| 127 | 4.6 |
| 128 | 6.5 |
| 129 | 2.9 |
| 130 | 4.8 |
| 135 | 1.2 |
| 138 | 0.8 |
| 140 | 9.1 |
| 152 | 1.3 |
| 156 | 10.0 |
| 157 | 4.0 |
| 158 | 6.4 |
| 159 | 2.2 |
| 163 | 7.7 |
| 170 | 4.1 |

(continued)

| Embodiment number | PD-1/PD-L1 binding $IC_{50}$ (nM) |
|---|---|
| 171 | 0.9 |
| 172 | 6.3 |
| 173 | 3.0 |
| 174 | 3.0 |
| 175 | 4.2 |
| 179 | 7.9 |
| 180 | 3.8 |
| 181 | 3.4 |
| 183 | 4.7 |
| 184 | 7.0 |
| 185 | 5.3 |
| 186 | 8.1 |
| 188 | 6.3 |
| 190 | 8.5 |
| 191 | 4.8 |
| 192 | 9.0 |
| 193 | 6.4 |
| 194 | 7.9 |
| 195 | 3.6 |
| 196 | 10.0 |
| 197 | 4.3 |
| 198 | 9.6 |
| 205 | 8.8 |
| 208 | 9.0 |
| 209 | 7.1 |
| 214 | 5.3 |
| 215 | 5.0 |
| 218 | 3.7 |
| 222 | 5.2 |
| 223 | 2.4 |
| 224 | 4.0 |
| 225 | 4.5 |
| 228 | 4.2 |
| 229 | 3.2 |
| 230 | 1.8 |
| 232 | 1.7 |
| 234 | 2.5 |
| 235 | 6.4 |

(continued)

| Embodiment number | PD-1/PD-L1 binding $IC_{50}$ (nM) |
|---|---|
| 237 | 5.2 |
| 238 | 4.1 |
| 239 | 4.9 |
| 240 | 2.8 |
| 243 | 8.4 |
| 245 | 4.4 |
| 246 | 5.4 |

Experimental conclusion: The embodiment compounds of the present disclosure show good binding inhibitory activity in the PD-1/PD-L1 inhibition test.

**Test Embodiment 2. Test of the activity of compounds of the present disclosure on inducing the endocytosis of PD-L1 protein on the surface of tumor cells**

Experimental purpose: The purpose of this test embodiment was to test the activity of the compound on inducing the endocytosis of PD-L1 on the surface of tumor cells.

Experimental equipment: Centrifuge (5702R) was purchased from Eppendorf Company, pipette was purchased from Eppendorf or Rainin Company, flow cytometer was purchased from Beckman Coulter with the model of DxFlex.

Experimental reagents: PE Mouse Anti-Human CD274 antibody was purchased from BD Pharmingen Companywith the article number of 557924; BSA was purchased from Sigma Company, Cat. No.: B2064-100G; PBS was purchased from Gibco Company, Cat. No.: 10010049; 24-well plate was purchased from Corning Company, Cat. No.: 3526.

Experimental method: In order to test the activity of the compound on inducing the endocytosis of PD-L1, in this experiment, the activity of the compound on inducing the endocytosis of PD-L1 was tested by detecting the change degree of PD-L1 on the tumor cell surface with fluorescently labeled anti-PD-L1 antibody, and the $IC_{50}$ of activity of the compound on inducing the endocytosis of PD-L1 was obtained.

The specific experimental operations were as follows:

The mouse colon cancer cells MC38-hPDL1 with high expression of hPD-L1 were collected, and adjusted to a suitable density and plated in a 24-well plate, and placed in a 37 °C, 5 % $CO_2$ incubator to adhere overnight. Compounds were prepared into different concentrations by medium and added to a 24-well plate, and a vehicle control well was set up. After the cells were incubated in a 37 °C, 5 % $CO_2$ incubator for 16 hours, the 24-well plate was taken out, and MC38-hPDL1 cells with different treatments in the plate were collected. After the cells were washed once with FACS buffer (PBS containing 0.5 % BSA), and the cells were prepared to an appropriate density with FACS buffer. PE Mouse Anti-Human CD274 antibody (BD Pharmingen#557924) was added, and the cells were shaken at room temperature and incubated for 30 minutes without light, then the cells were washed twice with FACS buffer, resuspended in 100 μL of PBS, and a flow cytometer was used to detect the fluorescent signal on the cell surface, and the isotype control was set as a negative control.

Experimental data processing method:

The endocytosis rate of the compounds was calculated from the fluorescence signals of different treatment groups, and the compound concentration and endocytosis rate were fitted by nonlinear regression using GraphPad Prism software, and $IC_{50}$ values were obtained, as shown in Table 2.

Table 2 Maximum endocytosis rate and $IC_{50}$ value of compounds on the surface of tumor cells

| PD-L1 | | |
|---|---|---|
| **Embodiment number** | **MC38 maximum endocytosis rate** | **MC38 $IC_{50}$ (nM)** |
| 1 | 88 % | 3.4 |
| 2 | 89% | 4.6 |
| 6 | 93 % | 1.1 |

(continued)

| PD-L1 | | |
|---|---|---|
| **Embodiment number** | **MC38 maximum endocytosis rate** | **MC38 $IC_{50}$ (nM)** |
| 11 | 85 % | 8.1 |
| 12 | 84% | 6.5 |
| 13 | 87% | 6.6 |
| 18 | 91 % | 1.5 |
| 19 | 85 % | 7.0 |
| 22 | 92% | 14.5 |
| 25 | 95 % | 0.9 |
| 30 | 94% | 3.7 |
| 35 | 96% | 1.3 |
| 36 | 94% | 3.1 |
| 37 | 97 % | 0.6 |
| 38 | 96% | 0.6 |
| 39 | 97% | 1.9 |
| 40 | 97% | 1.4 |
| 41 | 90% | 1.9 |
| 44 | 88% | 4.2 |
| 49 | 93% | 10.7 |
| 51 | 91 % | 11.8 |
| 56 | 91 % | 12.2 |
| 58 | 94% | 5.1 |
| 59 | 85% | 9.6 |
| 70 | 81 % | 4.0 |
| 72 | 88% | 6.5 |
| 81 | 86% | 5.0 |
| 82 | 85% | 12.0 |
| 90 | 80% | 9.9 |
| 91 | 81 % | 14.0 |
| 92 | 83 % | 9.0 |
| 95 | 94% | 2.3 |
| 98 | 93 % | 5.6 |
| 99 | 91 % | 3.9 |
| 112 | 87% | 11.0 |
| 114 | 95% | 1.2 |
| 115 | 93 % | 5.3 |
| 120 | 95 % | 1.2 |
| 122 | 97% | 0.4 |
| 123 | 97% | 0.4 |

(continued)

| PD-L1 | | |
|---|---|---|
| **Embodiment number** | **MC38 maximum endocytosis rate** | **MC38 $IC_{50}$ (nM)** |
| 124 | 96% | 0.4 |
| 125 | 96% | 0.7 |
| 126 | 97% | 1.0 |
| 127 | 97% | 0.7 |
| 128 | 98% | 0.6 |
| 129 | 95 % | 1.0 |
| 130 | 97% | 1.2 |
| 135 | 98% | 3.1 |
| 138 | 97% | 4.5 |
| 152 | 97% | 0.6 |
| 157 | 95 % | 1.5 |
| 158 | 95% | 1.7 |
| 171 | 93 % | NA |
| 172 | 94% | NA |
| 173 | 97% | 0.9 |
| 174 | 97% | 1 |
| 175 | 98 % | 0.4 |
| 176 | 97% | 1.2 |
| 177 | 97% | NA |
| 179 | 96% | NA |
| 180 | 96% | 0.6 |
| 181 | 97% | NA |
| 182 | 97% | NA |
| 183 | 95% | NA |
| 184 | 96% | NA |
| 185 | 97% | 0.7 |
| 186 | 96% | 0.7 |
| 187 | 95 % | 1.1 |
| 188 | 97% | 0.4 |
| 189 | 96% | NA |
| 190 | 97% | 0.8 |
| 191 | 97% | NA |
| 192 | 97% | 0.3 |
| 193 | 96% | NA |
| 194 | 96% | NA |
| 195 | 96% | NA |
| 196 | 96% | NA |

(continued)

| PD-L1 | | |
|---|---|---|
| **Embodiment number** | **MC38 maximum endocytosis rate** | **MC38 $IC_{50}$ (nM)** |
| 197 | 93 % | NA |
| 198 | 94% | NA |
| 199 | 93 % | NA |
| 200 | 95 % | NA |
| 201 | 94% | NA |
| 202 | 89% | NA |
| 203 | 95 % | NA |
| 204 | 89% | NA |
| 205 | 96% | NA |
| 206 | 90% | NA |
| 208 | 81 % | NA |
| 209 | 90% | NA |
| 210 | 88% | NA |
| 211 | 95% | 3.1 |
| 212 | 94% | 11.6 |
| 213 | 96% | 4.1 |
| 214 | 93% | NA |
| 215 | 96% | NA |
| 217 | 96% | 1.4 |
| 218 | 95% | NA |
| 222 | 92 % | NA |
| 223 | 94% | NA |
| 224 | 90% | NA |
| 225 | 93 % | NA |
| 228 | 95 % | NA |
| 229 | 95% | NA |
| 230 | 95 % | NA |
| 232 | 94% | NA |
| 234 | 97% | NA |
| 235 | 93 % | NA |
| 236 | 90% | NA |
| 237 | 94% | NA |
| 238 | 95 % | NA |
| 239 | 95 % | NA |
| 240 | 97% | NA |
| 243 | 91 % | NA |
| 244 | 94% | NA |

(continued)

| PD-L1 | | |
|---|---|---|
| **Embodiment number** | **MC38 maximum endocytosis rate** | **MC38 $IC_{50}$ (nM)** |
| 245 | 93 % | NA |
| 246 | 94% | NA |

Experimental conclusion: The compounds shown in the present disclosure can induce the endocytosis of PD-L1 on the surface of tumor cells well.

**Test Embodiment 3. Inhibitory effect of the compounds of the present disclosure on CHO-PDL1/Jurkat-PD1 cell pathway**

Experimental purpose: The purpose of this test embodiment was to test the inhibitory effect of compounds on the CHO-PDL1/Jurkat-PD1 cell pathway.

Experimental equipment: Centrifuge (5810R) was purchased from Eppendorf Company, pipette was purchased from Eppendorf or Rainin Company, microplate reader was purchased from American BioTek Company and the model was HIMFD full-function microplate reader.

Experimental reagents: CHO-PDL1 cells and NFAT-luc2/PD1 Jurkat cells were obtained from Promega Company, Cat. No.: J1252; RPMI 1640 was purchased from Gibco Company, Cat. No.: 22400089; FBS was purchased from Gibco Company, Cat. No.: 10091148; One-Glo reagent was purchased from Promega Company, Cat. No.: E6120; the 96-well plate was purchased from Corning Company, Cat. No.: 3610.

Experimental method: Two stably transfected cell lines were included in this experiment, CHO-K1 cells stably transfected with PD-L1 and Jurkat cells stably transfected with PD-1 and NFAT luciferase reporter genes. Compounds were incubated with the two cell lines. Then, due to the inhibitory effect on the PD-1/PD-L1 pathway, the activation degree of Jurkat cells changed, so that the level of the reporter gene NFAT in the downstream of Jurkat cells changed, and the level of NFAT was characterized by detecting the signal value of luciferase, so that the inhibitory effect of the test compound on CHO-PDLl/Jurkat-PDl pathway was characterized.

The specific experimental operations were as follows:

CHO-PDL1 cells (Promega, #J1252) were cultured to a suitable cell density. After digestion, the cells were resuspended by complete medium to become a suitable density of cell suspension. The cell suspension was plated 100 μL per well on a 96-well plate (Corning, #3610), and placed in a 37 °C, 5 % $CO_2$ incubator and incubated overnight, and assay medium (RPMI 1640+2 % FBS, RPMI 1640 Cat. No.: Gibco, #22400089, FBS Cat. No.: Gibco, #10091148) was used to prepare compound solutions of different concentrations. NFAT-luc2/PDl Jurkat cells (Promega, #J1252) were collected and resuspended to the appropriate cell density using assay medium. The supernatant of the CHO-PDL1 cell culture plate was aspirated, and 40 μL of the prepared compound solution and 40 μL of resuspended NFAT-luc2/PD1 Jurkat cells were added, and incubated in a 5 % $CO_2$ incubator at 37 °C for 6 hours, and the cell plate was taken out, then 40 μL of One-Glo reagent (Promega, #E6120) was added to each well, shaked and mixed well, incubated in the dark at room temperature for 10 minutes, then put into a microplate reader and the luminescence program was used to read the luminescence value of the cell plate.

Experimental data processing method:

The $EC_{50}$ value was fitted according to the compound concentration and the luminescence signal value, and the maximum induction fold of each compound relative to the signal value of the non-dosed group was calculated, as shown in Table 3.

Table 3 $EC_{50}$ of the compounds on CHO-PDLl/Jurkat-PDl cells and the maximum induction fold of the compounds relative to the signal value of the non-dosed group

| Embodiment number | CHO-PDL1/Jurkat-PD1 $EC_{50}$ (nM) | Maximum induction fold |
|---|---|---|
| 6 | 60 | 3.3 |
| 26 | 90 | 3.9 |
| 36 | 93 | 3.1 |
| 37 | 63 | 4.8 |
| 38 | 46 | 3.7 |
| 40 | 75 | 4.0 |

(continued)

| Embodiment number | CHO-PDL1/Jurkat-PD1 $EC_{50}$ (nM) | Maximum induction fold |
|---|---|---|
| 58 | 99 | 3.5 |
| 72 | 73 | 3.1 |
| 78 | 68 | 2.8 |
| 90 | 99 | 3.2 |
| 95 | 94 | 3.8 |
| 120 | 70 | 3.2 |
| 123 | 27 | 3.4 |
| 126 | 94 | 3.5 |
| 152 | 66 | 3.4 |
| 159 | 45 | 3.2 |
| 173 | 82 | 3.7 |
| 174 | 66 | 3.5 |
| 175 | 86 | 3.6 |
| 177 | 97 | 3.7 |
| 180 | 47 | 3.1 |
| 181 | 59 | |
| 184 | 62 | 2.8 |
| 186 | 61 | 3.0 |
| 188 | 93 | 3.8 |
| 192 | 85 | 3.8 |
| 193 | 84 | 2.8 |
| 195 | 89 | 3.6 |
| 197 | 67 | 3.5 |
| 218 | 54 | 2.8 |
| 223 | 73 | 3.3 |
| 234 | 78 | 4.7 |
| 243 | 74 | 3.1 |

Experimental conclusion: The embodiment compounds of the present disclosure show better inhibitory activity in the inhibition test of CHO-PDLI/Jurkat-PDI cell pathway.

**Test Embodiment 4. Test of combination of the compounds of the present disclosure to improve the stability (melting temperature) of PD-L1 protein**

Experimental purpose: To test the ability of compounds to enhance the stability of PD-L1 protein and increase the melting temperature of the protein.

Experimental equipment: Quantitative PCR instrument (Quantstudio6 Flex) was purchased from Life Company, and pipettes were purchased from Eppendorf or Rainin Company.

Experimental reagents: Protein Thermal Shift™ Dye Kit was purchased from Thermofisher Company, Cat. No.: 4461146; PD-L1 protein was purchased from Aero Biosystems Company, Cat. No.: PD1-H5229; HEPES, 1M Buffer Solution was purchased from Thermofisher Company, Cat. No.: 15630080; NaCl was purchased from Sinopharm Chemical Reagent Co., Ltd., Cat. No.: 10019318.

Experimental method: In this experiment, the thermal shift method was used to test the change degree in the melting temperature (Tm) of the PD-L1 protein before and after the compound was combined to characterize the ability of the compound to improve the stability of the PD-L1 protein, thereby showing the binding ability of the compound to the PD-L1 protein.

The specific experimental operations were as follows:

A solution containing 10 μM HEPES, SYPRO Orange, and 150 mM NaCl was prepared as experimental buffer, and human PD-L1 protein was added at a final concentration of 2 μM. The above reaction mixture was divided into 8-row PCR tubes, and each tube was 19.5 μL, then 0.5 μL of the test compound or DMSO was added respectively; the total reaction system was 20 μL, and the final compound concentration was 10 μM, and 2.5 % DMSO was set as vehicle control. PCR tube was put into the PCR machine, and the melt curve function was selected to detect the melting temperature of PD-L1 protein in different treatment groups (heated from 25 °C to 95 °C, 0.03 °C/s).

Experimental data processing method:

The experimental data file of the PCR instrument was imported into the thermal shift software to obtain the melting temperature (Tm) of each treatment group, and the Tm of the DMSO vehicle control group was subtracted to obtain the change value (ΔTm) of the melting temperature, as shown in the following table:

Table 4 Change values in melting temperature of compounds

| **Embodiment number** | ΔTm **(°C)** |
|---|---|
| 30 | 11.2 |
| 90 | 11.0 |
| 91 | 10.3 |
| 92 | 10.5 |
| 95 | 14.4 |
| 96 | 10.5 |
| 102 | 10.8 |
| 112 | 12.0 |
| 114 | 17.9 |
| 115 | 12.7 |
| 120 | 15.8 |
| 122 | 14.8 |
| 123 | 16.5 |
| 124 | 16.2 |
| 125 | 15.6 |
| 126 | 17.1 |
| 127 | 18.7 |
| 128 | 16.8 |
| 129 | 13.9 |
| 130 | 18.1 |
| 135 | 16.7 |
| 138 | 14.0 |
| 152 | 16.8 |
| 157 | 11.2 |
| 158 | 10.3 |
| 171 | 10.3 |

(continued)

| Embodiment number | ΔTm (°C) |
|---|---|
| 172 | 13.9 |
| 173 | 13.5 |
| 174 | 16.6 |
| 175 | 18.0 |
| 176 | 24.3 |
| 177 | 15.0 |
| 179 | 12.3 |
| 180 | 20.6 |
| 181 | 15.0 |
| 182 | 16.3 |
| 183 | 21.0 |
| 184 | 16.7 |
| 185 | 20.9 |
| 186 | 21.4 |
| 187 | 21.1 |
| 188 | 16.5 |
| 189 | 19.5 |
| 190 | 14.0 |
| 191 | 11.7 |
| 192 | 14.0 |
| 193 | 12.3 |
| 194 | 13.2 |
| 195 | 14.5 |
| 196 | 12.4 |
| 197 | 16.0 |
| 198 | 12.0 |
| 199 | 11.4 |
| 200 | 12.5 |
| 201 | 12.1 |
| 203 | 13.5 |
| 203 | 10.5 |
| 205 | 13.3 |
| 206 | 11.6 |
| 209 | 10.7 |
| 210 | 10.7 |
| 211 | 14.6 |
| 212 | 14.9 |
| 213 | 14.5 |

(continued)

| Embodiment number | ΔTm (°C) |
|---|---|
| 214 | 10.2 |
| 215 | 17.3 |
| 217 | 13.0 |
| 218 | 13.7 |
| 223 | 12.3 |
| 224 | 11.3 |
| 227 | 11.9 |
| 228 | 11.6 |
| 229 | 12.0 |
| 230 | 11.3 |
| 232 | 11.1 |
| 234 | 15.6 |
| 235 | 11.3 |
| 236 | 10.6 |
| 237 | 11.6 |
| 238 | 10.8 |
| 239 | 11.9 |
| 240 | 13.0 |
| 244 | 11.4 |
| 245 | 10.2 |
| 246 | 11.0 |

Experimental conclusion:

According to the above scheme, it can be concluded that the compounds shown in the present disclosure show the ability to improve the stability of PD-L1 protein in the test of increasing the melting temperature of PD-L1 protein.

**Test Embodiment 5. *In vitro* cytotoxicity of the compounds of the present disclosure on NFAT-luc2/PD1 Jurkat cells and MC38-hPDL1 cells**

Experimental purpose: To test the *in vitro* cytotoxicity of compounds on NFAT-luc2/PDl Jurkat cells and MC38-hPDL1 cells.

Experimental equipment: Centrifuge (5810R) was purchased from Eppendorf Company, pipette was purchased from Eppendorf or Rainin Company, microplate reader was purchased from American BioTek Company and the model was HIMFD full-function microplate reader.

Experimental reagents: RPMI 1640 was purchased from Gibco Company, Cat. No.: 22400089; FBS was purchased from Gibco Company, Cat. No.: 10091148; PBS was purchased from Gibco Company, Cat. No.: 10010049; Cell Titer-Glo was purchased from Promega Company, Cat. No.: G7573; the cell culture plate was purchased from Coming Company, Cat. No.: 3610.

Experimental method:

When the cells were cultured to an appropriate degree of confluency, the cells were collected, and the cells was adjusted in an appropriate cell concentration by complete medium, then the cell suspension was plated in a 96-well plate, 90 μL per well, and put in a 37 °C, 5 % $CO_2$ incubator overnight; compound solutions of different concentrations

were prepared by DMSO and medium, and a vehicle control was set; compound solutions were added to a 96-well plate, 10 μL per well, and put in a 37 °C, 5 % $CO_2$ incubator and cultured for 72 hours. After that, CellTiter-Glo solution was added, the mixture was shaken and mixed evenly, incubated in the dark for 10 minutes, and a BioTek Synergy HI microplate reader was used for reading.

Experimental data processing method:

Luminescence signal value was used to calculate the inhibition rate, and the concentration and inhibition rate were fitted by nonlinear regression curve using Graphpad Prism software to obtain $IC_{50}$ values, as shown in the following table:

Table 5 *In vitro* cytotoxicity of compounds

| Embodiment number | NFAT-luc2/PD1 Jurkat $IC_{50}$ (nM) | Jurkat-PD1 inhibition rate (%) | MC38-hPDL1 $IC_{50}$ (nM) | MC38-hPDL1 inhibition rate (%) |
|---|---|---|---|---|
| 30 | 2953 | 99.0 % | >10000 | 4.0 % |
| 90 | 1402 | 99.0 % | >10000 | 6.8 % |
| 102 | >10000 | -12.0 % | >10000 | -6.7 % |
| 112 | 8627 | 57.0 % | >10000 | 13.0 % |
| 114 | 7626 | 69.0 % | >10000 | 14.0 % |
| 152 | 4459 | 97% | >10000 | 15 % |
| 159 | >10000 | 24% | >10000 | 5% |
| 177 | 5484 | 84% | >10000 | 15 % |
| 183 | 5819 | 71 % | >10000 | 14 % |
| 215 | 4320 | 85 % | 9345 | 51 % |
| 217 | 5372 | 79% | 7149 | 67% |
| 218 | 4320 | 85 % | 9345 | 51 % |
| 222 | >10000 | 36% | >10000 | 0 % |
| 223 | 4766 | 77% | >10000 | 2% |
| 224 | 9501 | 48% | >10000 | 1 % |
| 225 | 9073 | 51 % | >10000 | 1 % |

Experimental conclusion:

From the above results, it can be seen that the compounds shown in the present disclosure have weaker cytotoxicity on NFAT-luc2/PD1 Jurkat and MC38-hPDL1 cells.

**Test Embodiment 6. Pharmacokinetics test in Balb/C mice**

1. Research purposes:

Taking Balb/C mice as the test animals, the pharmacokinetic behavior in the plasma of mice of the following compound embodiments was studied when orally administered at a dose of 5 mg/kg.

2. Experimental protocol

2.1 Experimental drug:

The embodiments of the present disclosure, self-made.

2.2 Experimental animals:

Balb/C Mouse 6/embodiment, male, Shanghai Jisijie Laboratory Animal Co., Ltd., animal production license number (SCXK (Shanghai) 2013-0006 No.311620400001794).

2.3 Drug preparation:

0.5 % HPMC (1 % Tween 80), dissolved by sonication, to prepare a clear solution or a homogeneous suspension.

2.3 Administration:

Balb/C mice, male; p.o. after overnight fasting, respectively, at a dose of 5 mg/kg, administered in a volume of 10 mL/kg.

2.4 Sample collection:

Before and after administration of mice, at 0, 0.5, 1, 2, 4, 6, 8 and 24 hours, 0.1 mL of blood was collected from the orbit, placed in an EDTA-$K_2$ test tube, and centrifuged at 6000 rpm at 4 °C for 6 min to separate the plasma, stored at -80 °C.

2.5 Sample processing:

1) 40 μL of plasma samples were added to 160 μL of acetonitrile for precipitation, mixed and centrifuged at 3500 × g for 5 to 20 minutes.
2) 100 μL of the supernatant solution after treatment was taken for LC/MS/MS to analyze the concentration of the compounds to be tested.

2.6 Liquid phase analysis

- Liquid phase condition: Shimadzu LC-20AD pump
- MS conditions: AB Sciex API 4000 Mass Spectrometer
- Chromatographic column: phenomenex Gemiu 5 μm C18 50 × 4.6 mm
- Mobile phase: A solution was aqueous 0.1 % formic acid solution, B solution was acetonitrile
- Flow rate: 0.8 mL/min
- Elution time: 0 to 4.0 minutes, the eluent was as follows:

| Time/minute | A solution | B solution |
|---|---|---|
| 0.01 | 90% | 10% |
| 0.5 | 90% | 10% |
| 0.8 | 5% | 95 % |
| 2.4 | 5 % | 95% |
| 2.5 | 90% | 10% |
| 4.0 | Stop | |

3. Test results and analysis

The main parameters of pharmacokinetics were calculated with WinNonlin 8.2, and the results of the mice pharmacokinetic experiments were shown in the following table:

Table 6 Results of pharmacokinetic experiments in mice

| Embodiment number | Pharmacokinetic experiment (5mg/kg) | | | | |
|---|---|---|---|---|---|
| | Peak time | Curve area | Drug concentration in plasma | Half life | Average residence time |
| | $t_{max}$(h) | $AUC_{0-t}$ (ng/mL*h) | $C_{max}$(ng/mL) | $t_{1/2}$ (h) | $MRT_{0-\infty}$(h) |
| 114 | 2.0 | 596 | 48 | 9.0 | 11.3 |
| 120 | 2.0 | 500 | 34 | 12.0 | 17.0 |
| 123 | 4.0 | 778 | 59 | 6.7 | 12.8 |
| 124 | 4.0 | 523 | 36 | 20.0 | 25.0 |
| 126 | 2.0 | 677 | 54 | 8.4 | 12.3 |
| 127 | 6.0 | 743 | 37 | 47 | 69.0 |
| 152 | 8.0 | 1138 | 93 | NA | 10.0 |
| 175 | 6.0 | 1634 | 132 | NA | NA |
| 176 | 2.0 | 629 | 55 | 15.2 | 21.6 |
| 180 | 2.0 | 887 | 116 | 9.5 | 12.9 |
| 183 | 6.0 | 1192 | 75 | 10.9 | 16.8 |
| 186 | 2.0 | 537 | 31 | 15.2 | 22.5 |
| 223 | 1.0 | 356 | 24 | 10.5 | 16.0 |
| 225 | 2.0 | 1920 | NA | 9.2 | 10.6 |
| 234 | 2.0 | 273 | 25 | 14.3 | 20.8 |

Experimental conclusion:

It can be seen from the results of the mouse pharmacokinetic experiments in the table that the embodiment compounds of the present disclosure show good metabolic properties, and both the exposure AUC and the maximum drug concentration in plasma $C_{max}$ are good.

4. Experimental conclusion:

It can be seen from the results of the canine pharmacokinetic experiments in the table that the embodiment compounds of the present disclosure show good metabolic properties, and both the exposure AUC and the maximum drug concentration in plasma $C_{max}$ are good.

**Test Embodiment 7. Pharmacokinetic test of plasma and tumor of tumor-bearing nude mice**

1. Research purposes:

Taking tumor-bearing nude mice as the test animals, the pharmacokinetic behavior in the plasma and tumor of tumor-bearing nude mice of the following compound embodiments was studied when orally administered at a dose of 30 mg/kg.

2. Experimental protocol

2.1 Experimental drug:

The embodiments of the present disclosure, self-made.

2.2 Experimental animals:

Tumor-bearing nude mouse 21/embodiment, female, Shanghai B&K Laboratory Animal Co., Ltd., animal production license number (SCXK (Shanghai) 2013-0006 No.311620400001794).

2.3 Drug preparation:

0.5 % HPMC (1 % Tween 80), dissolved by sonication, to prepare a clear solution or a homogeneous suspension.

2.4 Administration:

Tumor-bearing nude mice, female; p.o. after overnight fasting, respectively, at a dose of 30 mg/kg, administered in a volume of 10 mL/kg.

2.5 Sample collection:

Before and after administration of tumor-bearing nude mice, at 0, 1, 2, 4, 6, 8, 16 and 24 hours, 0.1 mL of blood was collected from the orbit, placed in an EDTA-$K_2$ test tube, and centrifuged at 6000 rpm at 4 °C for 6 min to separate the plasma. After dissection, the tumor tissue was weighed and stored at-80 °C.

2.6 Sample processing:

1) Tumor tissue was homogenized with methanol and water at a ratio of 1: 3, and then centrifuged to get the supernatant. 40 μL of tumor homogenate supernatant and 40 μL of plasma sample were added to 160 μL of acetonitrile for precipitation, and after mixing, centrifuged at 3500 × g for 5 to 20 minutes.
2) 100 μL of the supernatant solution after treatment was taken for LC/MS/MS to analyze the concentration of the compounds to be tested.

2.7 Liquid phase analysis

- Liquid phase condition: Shimadzu LC-20AD pump
- MS conditions: AB Sciex API 4000 Mass Spectrometer
- Chromatographic column: phenomenex Gemiu 5 μm C18 50 × 4.6 mm
- Mobile phase: A solution was aqueous 0.1 % formic acid solution, B solution was acetonitrile
- Flow rate: 0.8 mL/min
- Elution time: 0 to 4.0 minutes, the eluent was as follows:

| Time/minute | A solution | B solution |
|---|---|---|
| 0.01 | 90% | 10% |
| 0.5 | 90% | 10% |
| 0.8 | 5% | 95 % |
| 2.4 | 5% | 95 % |
| 2.5 | 90% | 10% |
| 4.0 | Stop | |

3. Test results and analysis

The main parameters of pharmacokinetics were calculated with WinNonlin 8.2, and the results of the tumor-bearing nude mice pharmacokinetic experiments were shown in the following table:

Table 7 Results of concentrations of the embodiment compounds of the present disclosure in the plasma/tumor of tumor-bearing nude mice

| Embodiment number | Drug concentration in plasma (ng/mL) | Drug concentration in tumor (ng/mL) | Tumor plasma ratio |
|---|---|---|---|
| 123 | 821 | 2285 | 2.8 |
| 152 | 153 | 707 | 4.6 |
| 173 | 291 | 628 | 2.2 |
| 174 | 291 | 697 | 2.4 |
| 175 | 485 | 1009 | 2.1 |
| 183 | 309 | 697 | 2.3 |
| 223 | 432 | 1504 | 3.5 |

4. Experimental conclusion:

The above data showes that the drug concentration of the embodiment compounds of the present disclosure in the mouse tumor is higher than that in the plasma.

**Test embodiment 8. Plasma protein binding rate experiment**

1. Experimental purpose:

The purpose of this experimental method was to detect the plasma protein binding of the embodiment compounds in plasma.

2. Experimental equipment and materials:

Ultra-high performance liquid chromatography tandem mass spectrometer, refrigerated centrifuge, vortexer, electrothermal constant temperature oscillating water tank, pipette, continuous liquid dispenser, 96-well plate, tissue homogenizer (used for tissue sample analysis), addition of acetonitrile solution with internal standard, blank matrix (plasma, urine or tissue homogenate, etc.)

3. Experimental steps:

3.1 Preparation of plasma

The frozen plasma was thawed at room temperature or in a water bath at 37 °C, then centrifuged at 3500 rpm for 5 min, and the supernatant was taken.

3.2 Preparation of reaction termination solution

Acetonitrile containing internal standard was used as termination solution and stored in a refrigerator at 2 to 8 °C.

3.3 Preparation of compound working solution

Preparation of compound working solution: Stock solutions were diluted with DMSO to a final concentration of 1 mM.

3.4 Preparation of plasma solutions

2.5 μL of compound working solution was added to 497.5 μL of blank plasma to a final concentration of 5 μM, shaked and mixed well.

3.5 Equilibrium dialysis

1) The equilibrium dialysis device was prepared, and the detection membrane device was put into the equilibrium dialysis 96-well plate;
2) 200 μL of the prepared plasma solution was added to the membrane, n=2;
3) another 5 μL of plasma solution was taken, diluted 10 times with 45 μL of blank plasma of the same species, and 200 μL of acetonitrile termination solution containing internal standard was added, and stored in a -20 °C refrigerator;
4) 350 μL of dialysate (100 mM phosphate buffer) was added outside the membrane;
5) the dialysis plate was sealed and incubated in a 37 °C water bath for 5 hours;
6) after dialysis, 5 μL was taken out from the sample hole in the membrane, and diluted 10 times with 45 μL of blank plasma of the same species; 50 μL of dialysate was taken out from the sample hole outside the membrane, and 200 μL of acetonitrile termination solution containing internal standard was added;
7) the supernatant was taken after sample centrifugation;
8) LC-MS analysis.

4. Chromatographic conditions:

Instrument: Shimadzu LC-30 AD;
Chromatographic column: XBridge® C18 (50*4.6 mm, 5 μm particle size);
Mobile phase: A: 0.1 % formic acid solution, B: methanol;
Wash gradient: 0.2 to 1.6 min 5 %A to 95 %A, 3.0 to 3.1 min 95 %A to 5 %A;
Running time: 4.0 min.

5. Mass spectrometry conditions:

Instrument: API5500 liquid chromatography-mass spectrometer, AB Sciex, USA;
Ion source: electrospray ionization source (ESI);
Dry gas: $N_2$, temperature was 500 °C;
Electrospray voltage: 5000 V;
Detection method: positive ion detection;
Scanning mode: the reaction monitoring (MRM) mode was selected.

6. Experimental results: as shown in Table 5:

Table 8 Results of plasma protein binding rate of embodiment compounds

| Embodiment number | mouse |
|---|---|
| | % Unbound |
| 114 | 0.33 |
| 123 | 0.95 |
| 124 | 0.57 |
| 152 | 1.81 |
| 175 | 0.52 |
| 180 | 0.44 |
| 186 | 0.30 |

(continued)

| Embodiment number | mouse |
|---|---|
| | % Unbound |
| 188 | 0.52 |

7. Experimental conclusion:

The above data showes that the embodiment compounds of the present disclosure show high plasma protein binding rate.

**Test embodiment 9. Inhibitory activity test of hERG potassium channel**

1. Cell Preparation

1.1 CHO-hERG cells were cultured in a 175 $cm^2$ culture flask. When the cell density reached 60 to 80 %, the culture medium was removed, washed with 7 mL of PBS, and then digested with 3 mL of Detachin.

1.2 After the digestion was completed, 7 mL of culture medium was added to neutralize, then centrifuged, and the supernatant was aspirated, and 5 mL of culture medium was added to resuspend to ensure the cell density was 2 to $5\times10^6$/mL.

2. Solution preparation: the composition of intracellular solution and extracellular solution were shown in the following table:

| Reagent | Extracellular solution (mM) | Intracellular solution (mM) |
|---|---|---|
| $CaCl_2$ | 2 | 5.374 |
| $MgCl_2$ | 1 | 1.75 |
| KCl | 4 | 120 |
| NaCl | 145 | - |
| Glucose | 10 | - |
| HEPES | 10 | 10 |
| EGTA | - | 5 |
| Na-ATP | - | 4 |
| pH | 7.40 (adjusted with NaOH), Osmolarity~305 mOsm | 7.25 (adjusted with KOH), Osmolarity~290 mOsm |

3. Electrophysiological recording process

Single-cell high-impedance sealing and whole-cell pattern formation were all performed automatically by the Qpatch instrument. After the whole-cell recording pattern was obtained, the cell was clamped at -80 mV, before a +40 mV depolarizing stimulus for 5 s was given, a pre-voltage of -50 mV for 50 ms was given, then repolarized to -50 mV for 5 s, and then returned to -80 mV. This voltage stimulus was applied every 15 seconds, and recorded for 2 minutes, then the extracellular solution was given and then recorded for 5 minutes. Then the dosing process was started. The compound concentration started from the lowest test concentration, and each test concentration was administered for 2.5 minutes. After all concentrations were continuously administered, the positive control compound 3 μM Cisapride was administered. At least 3 cells were tested at each concentration ($n\geq3$).

4. Compound Preparation

4.1 20 mM compound mother solution was diluted with extracellular solution, and 5 μL of 20 mM compound mother solution was added to 2495 μL extracellular solution, diluted 500-fold to 40 μM, and then 3-fold serial dilutions were made to obtain the final concentration to be tested in the extracellular solution containing 0.2 % DMSO.

4.2 The highest test concentration was 40 μM at a total of 6 concentrations, 40, 13.33, 4.44, 1.48, 0.49, 0.16 μM in order, respectively

4.3 The DMSO content in the final test concentration should not exceed 0.2 %, and this concentration of DMSO had no effect on the hERG potassium channel.

5. Data Analysis

The experimental data was analyzed by XLFit software.

6. Quality Control

Environment: humidity 20 to 50 %, temperature 22 to 25 °C
Reagents: The experimental reagents used were purchased from Sigma, with a purity of >98 %
Experimental data in the report must meet the following criteria:

whole cell sealing impedance > 100 MΩ
tail current amplitude > 400 pA

Pharmacological parameters:

The inhibitory effect of multiple concentrations of Cisapride on hERG channel was set as a positive control.

7. Experimental results:

Table 9: Inhibition results of the embodiment compounds of the present disclosure on hERG current at multiple concentrations

| Embodiment number | hERG (μM) |
|---|---|
| 223 | 14.79 |

8. Experimental conclusion:

Inhibition on cardiac hERG potassium ion channel by drugs is the main reason for drug-induced QT prolongation syndrome. It can be seen from the experimental results that the embodiment compounds of the present disclosure have no obvious inhibitory effect on the cardiac hERG potassium ion channel, and can avoid the cardiotoxicity and side effects at high doses.

**Test Embodiment 10. Tumor inhibition experiment on MC38-hPDL1 xenograft model**

1. Experimental purpose:

To evaluate the antitumor activity of the test compounds against C57 mice subcutaneous xenografts tumor of MC38-hPDL1 cells.

2. Experimental instruments and reagents:

2.1 Instruments:

Ultra-clean workbench (BSC-1300II A2, Shanghai Boxun Industrial Co., Ltd. Medical Equipment Factory);
$CO_2$ incubator (311, Thermo);
Centrifuge (Centrifuge 5720R, Eppendorf);
Automatic cell counter (Countess II, Life);
Pipette (10-20 μL, Eppendorf);
Microscope (TS100, Nikon);
Vernier caliper (500-196, Mitutoyo, Japan);

Cell culture flasks (T25/T75/T225, Corning).

2.2 Reagents:

DMEM (11995-065, Gibco);
Fetal bovine serum (FBS) (10099-141, Gibco);
Phosphate buffer solution (PBS) (10010-023, Gibco).

2.3 Test compounds:

The embodiment compounds of the present disclosure, self-made.

3. Experimental operation:

MC38-hPDL1 cells were taken out from the cell bank, added to DMEM medium (DMEM + 10 % FBS) after recovery, and placed in a $CO_2$ incubator (incubator temperature was 37 °C, $CO_2$ concentration was 5 %) to culture. MC38-hPDLI cells were collected when the number of cells increased to the required number of inoculation *in vivo.* Counted with an automatic cell counter, and resuspend the cells with PBS according to the counting results to prepare a cell suspension (density was $1\times10^6$/mL), and placed in an ice box for use.

6-8-week-old female C57 mice weighing approximately 18-22 grams were used. Mice were housed in SPF animal room, single cage, 4-5 mice per cage. All cages, litter and water were sterilized at high temperature before use, and all animals had free access to food and water. Before the start of the experiment, C57 mice were marked with a disposable ear tag for rats and mice, and the skin of the inoculation site was disinfected with 75 % alcohol before inoculation. Each mouse was subcutaneously inoculated with 0.1 mL (containing $1*10^5$ cells) of MC38-hPDLI cells on the right back. When the tumor volume reached 40-180 $mm^3$, group administration was started, with 8 mice in each group. Each test compound was administered orally twice daily for 14 days. Tumor volume was measured twice a week and mice were weighed twice a week, and tumor TGI (%) was calculated.

4. Data processing:

Tumor volume ($mm^3$), the calculation formula was: V=0.5*D*d*d, wherein D and d were the long and short diameters of the tumor, respectively.

Calculation of TGI( %):

When the tumor did not regress, TGI(%) = [(1-(average tumor volume at the end of administration of a treatment group - average tumor volume at the beginning of administration of the treatment group))/(average tumor volume at the end of treatment of a solvent control group - average tumor volume at the beginning of treatment of the solvent control group)]× 100 %;

When the tumor regressed, TGI(%)=[1-(average tumor volume at the end of administration of a treatment group-average tumor volume at the beginning of administration of this treatment group)/average tumor volume at the beginning of administration of this treatment group]× 100 %.

5. Experimental results:

The test results were shown in Table 10:

Table 10 Pharmacodynamic parameters of the compounds in xenografted tumor mice

| Grouping | Administration dosage (mpk) | Administration Way | Administration frequency | Number of animals per group (n) | Tumor volume ($mm^3$, Mean ± SD) | | ΔT/ΔC (%) | TGI (%) |
|---|---|---|---|---|---|---|---|---|
| | | | | | Day 1 | Day14 | Day 15 | Day 15 |
| 114 | 60 | po | bid | 7 | 69.72±21.92 (69.26±22.37) | 140.27±77.92 (350.57±235.90) | 25.08 % | 74.92 % |
| 123 | 10 | po | bid | 8 | 75.93 ± 26.17 (75.75 ± 24.32) | 644.86 ± 398.92 (1,642.85 ± 1,147.67) | 36.30 % | 63.70 % |
| 123 | 30 | po | qd | 8 | 75.50 ± 25.70 (75.75 ± 24.32) | 582.87 ± 359.11 (1,642.85 ± 1,147.67) | 32.38 % | 67.62 % |

Remarks: The data in parentheses indicates that the tumor volume of Vehicle QD × 2w group (ie, the control group) corresponding to this embodiment at the corresponding time.

6. Experimental conclusion:

The above data showes that the embodiment compounds of the present disclosure have a strong inhibitory effect on the C57 mice subcutaneous xenografted tumor of **MC38-hPDL1** cells.

## Claims

**1.** A compound represented by general formula (Ib), a stereoisomer thereof or a pharmaceutically acceptable salt thereof:

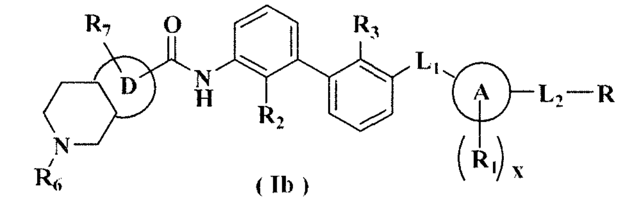

(Ib)

wherein:

$L_1$ is selected from a bond, $-(CH_2)_{n1}-$, $-(CH_2)_{n1}NR_{aa}C(O)(CR_{aa}R_{bb})_{n2}-$, $-(CH_2)_{n1}(CR_{aa}R_{bb})_{n2}-$, $-(CR_{aa}R_{bb})_{n1}O(CH_2)_{n2}-$, $-(CH_2)_{n1}O(CR_{aa}R_{bb})_{n2}-$, $-(CR_{aa}R_{bb})_{n1}S(CH_2)_{n2}-$, $-(CH_2)_{n1}S(CR_{aa}R_{bb})_{n2}-$, $-(CR_{aa}R_{bb})_{n1}(CH_2)_{n2}NR_{cc}-$, $-(CH_2)_{n1}NR_{aa}(CR_{bb}R_{cc})_{n2}-$, $-(CH_2)_{n1}C(O)(CR_{aa}R_{bb})_{n2}-$, $-(CH_2)_{n1}P(O)R_{aa}-$, $-(CH_2)_{n1}S(O)_{n2}-$, $-(CH_2)_{n1}S(O)_{n2}NR_{aa}-$, $-(CH_2)_{n1}NR_{aa}S(O)_{n2}-$ or $-(CH_2)_{n1}C(O)NR_{aa}-$,

$L_2$ is selected from a bond, $-(CH_2)_{n3}-$, $-(CR_{dd}R_{ee})_{n3}-$, $-(CR_{dd}R_{ee})_{n3}(CH_2)_{n4}NR_{ff}-$, $-(CH_2)_{n3}NR_{dd}(CR_{ee}R_{ff})_{n4}-$, $-(CH_2)_{n3}(CR_{dd}R_{ee})_{n4}-$, $-(CR_{dd}R_{ee})_{n3}O(CH_2)_{n4}-$, $-(CH_2)_{n3}O(CR_{dd}R_{ee})_{n4}-$, $-(CR_{aa}R_{bb})_{n3}S(CH_2)_{n4}-$, $-(CH_2)_{n3}S(CR_{aa}R_{bb})_{n4}-$, $-(CH_2)_{n3}C(O)(CR_{dd}R_{ee})_{n4}-$, $-(CH_2)_{n3}NR_{dd}C(O)(CR_{ee}R_{ff})_{n4}-$, $-(CH_2)_{n3}P(O)R_{dd}-$, $-(CH_2)_{n3}S(O)_{n4}-$, $-(CH_2)_{n3}S(O)_{n4}NR_{dd}-$, $-(CH_2)_{n3}NR_{dd}S(O)_{n4}-$ or $-(CH_2)_{n3}C(O)NR_{dd}-$;

$R_{aa}$, $R_{bb}$ and $R_{cc}$ are each independently selected from hydrogen, deuterium, halogen, amino, nitro, hydroxyl, cyano, oxo, thioxo, carboxyl, alkyl, deuterated alkyl, haloalkyl, hydroxyalkyl, alkoxy, haloalkoxy, alkenyl, alkynyl, heterocyclylalkyl, cycloalkyl, heterocyclyl, aryl or heteroaryl, and the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl, and heteroaryl are optionally further substituted;

or, any two of $R_{aa}$, $R_{bb}$ and $R_{cc}$ are connected to form a cycloalkyl, heterocyclyl, aryl or heteroaryl, and the cycloalkyl, heterocyclyl, aryl and heteroaryl are optionally further substituted;

$R_{dd}$, $R_{ee}$ and $R_{ff}$ are each independently selected from hydrogen, deuterium, halogen, amino, nitro, hydroxyl, cyano, oxo, thioxo, carboxyl, alkyl, deuterated alkyl, haloalkyl, hydroxyalkyl, alkoxy, haloalkoxy, alkenyl, alkynyl, heterocyclylalkyl, cycloalkyl, heterocyclyl, aryl or heteroaryl, and the amino, alkyl, deuterated alkyl, haloalkyl, hydroxyalkyl, alkoxy, haloalkoxy, alkenyl, alkynyl, heterocyclylalkyl, cycloalkyl, heterocyclyl, aryl, and heteroaryl are optionally further substituted;

or, any two of $R_{dd}$, $R_{ee}$ and $R_{ff}$ are connected to form a cycloalkyl, heterocyclyl, aryl or heteroaryl, wherein the cycloalkyl, heterocyclyl, aryl and heteroaryl are optionally further substituted;

ring A is selected from cycloalkyl, heterocyclyl, aryl or heteroaryl;

R is selected from hydrogen, deuterium, halogen, amino, hydroxyl, mercapto, cyano, nitro, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloalkyl, alkoxy, alkylthio, haloalkoxy, hydroxyalkyl, cycloalkyl, heterocyclyl, aryl and heteroaryl are optionally further substituted;

$R_1$ is selected from hydrogen, deuterium, halogen, amino, hydroxyl, mercapto, cyano, nitro, oxo, thioxo, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloalkyl, alkoxy, alkylthio, haloalkoxy, hydroxyalkyl, cycloalkyl, heterocyclyl, aryl and heteroaryl are optionally further substituted;

$R_2$ is selected from hydrogen, deuterium, halogen, amino, hydroxyl, mercapto, cyano, nitro, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloaclkyl, alkoxy, alkylthio, haloalkoxy,

hydroxyalkyl, cycloalkyl, heterocyclyl, aryl and heteroaryl are optionally further substituted;

$R_3$ is selected from hydrogen, deuterium, halogen, amino, hydroxyl, mercapto, cyano, nitro, alkyl, alkenyl, alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloaclkyl, alkoxy, alkylthio, haloalkoxy, hydroxyalkyl, cycloalkyl, heterocyclyl, aryl and heteroaryl are optionally further substituted;

ring D is selected from heterocyclyl or heteroaryl, preferably five- or six-membered heteroaryl, more preferably imidazolyl, thiazolyl or pyridyl;

$R_6$ is selected from hydrogen, deuterium, halogen, amino, hydroxyl, mercapto, cyano, nitro, oxo, thioxo, alkyl, alkenyl, alkynyl, deuterated alkyl, haloalkyl, alkoxy, alkylthio, haloalkoxy, hydroxyalkyl, cycloalkyl, heterocyclyl, aryl, heteroaryl, $-(CH_2)_{m3}R_d$, $-(CH_2)_{m3}(CR_dR_e)_{m4}R_f$, $-(CH_2)_{m3}NR_dCHR_eR_f$, $(CH_2)_{m3}(CR_dR_e)_{m4}C(O)OR_f$, $-(CH_2)_{m3}C(O)O(CR_dR_e)_{m4}OC(O)R_f$, $-(CH_2)_{m3}CR_d = CR_eR_f$, $-(CH_2)_{m3}OR_d$,$-(CH_2)_{m3}NR_dR_e$, $-(CH_2)_{m3}SR_d$, $-(CH_2)_{m3}C(O)R_d$, $-(CH_2)_{m3}C(O)OR_d$, $-(CH_2)_{m3}S(O)_{m4}R_d$, $-(CH_2)_{m3}NR_dC(O)R_e$ or $-(CH_2)_{m3}C(O)NR_dR_e$, and the amino, alkyl, alkenyl, alkynyl, deuterated alkyl, haloalkyl, alkoxy, alkylthio, haloalkoxy, hydroxyalkyl, cycloalkyl, heterocyclyl, aryl and heteroaryl are optionally further substituted,

preferably hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl , $C_{3-12}$ cycloalkyl, 3- to 12-membered heterocyclyl, $C_{6-14}$ aryl, 5- to 14-membered heteroaryl, $-(CH_2)_{m3}R_d$, $-(CH_2)_{m3}(CR_dR_e)_{m4}R_f$, $-(CH_2)_{m3}OR_d$, $-(CH_2)_{m3}NR_dR_e$, $-(CH_2)_{m3}NR_dCHR_eR_f$,$-(CH_2)_{m3}C(O)O(CR_dR_e)_{m4}OC(O)R_f$ or $-(CH_2)_{m3}CR_d = CR_eR_f$,

more preferably hydrogen, methyl, ethyl, isopropyl, methoxy, cyclopropyl,

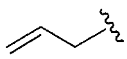, 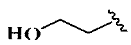,

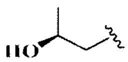, 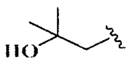, 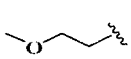, 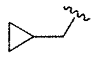, 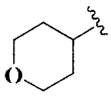, 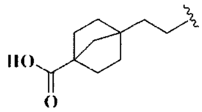, 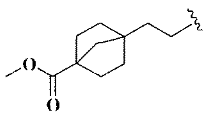,

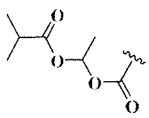, 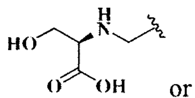 or 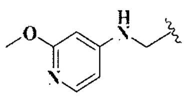;

$R_d$, $R_e$ and $R_f$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl, 5- to 14-membered heteroaryl, $-(CH_2)_{m5}R_h$, $(CH_2)_{m5}C(O)OR_h$, $-(CH_2)_{m5}OR_h$,$-(CH_2)_{m5}NR_hR_i$, $-(CH_2)_{m5}SR_h$, $-(CH_2)_{m5}C(O)R_h$, $-(CH_2)_{m5}NR_hC(O)R_i$ and $-(CH_2)_{m5}C(O)NR_hR_i$;

$R_h$ and $R_i$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

or, any two of $R_d$, $R_e$ and $R_f$ are connected to form a cycloalkyl, heterocyclyl, aryl or heteroaryl, and the cycloalkyl, heterocyclyl, aryl and heteroaryl are optionally further substituted;

$R_7$ is selected from hydrogen, deuterium, halogen, amino, hydroxyl, mercapto, cyano, nitro, alkyl, alkenyl,

alkynyl, deuterated alkyl, haloalkyl, alkoxy, alkylthio, haloalkoxy, hydroxyalkyl, cycloalkyl, heterocyclyl, aryl or heteroaryl, and the amino, alkyl, alkenyl, alkynyl, deuterated alkyl, haloaclkyl, alkoxy, alkylthio, haloalkoxy, hydroxyalkyl, cycloalkyl, heterocyclyl, aryl and heteroaryl are optionally further substituted,
preferably hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl;
more preferably hydrogen, fluorine, chlorine, bromine, methyl, ethyl or isopropyl;
x is an integer from 0 to 6;
n1 is an integer from 0 to 3;
n2 is an integer from 0 to 3;
n3 is an integer from 0 to 3;
n4 is an integer from 0 to 3;
m1 is an integer from 0 to 3;
m2 is an integer from 0 to 3;
m3 is an integer from 0 to 3;
m4 is an integer from 0 to 3; and
m5 is an integer from 0 to 3.

**2.** The compound as claimed in claim 1, the stereoisomer thereof or the pharmaceutically acceptable salt thereof, wherein, the general formula (Ib) is further represented by general formula (I):

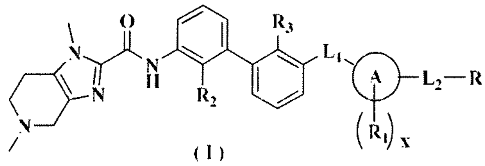

(I)

.

**3.** The compound as claimed in claim 1 or claim 2, the stereoisomer thereof or the pharmaceutically acceptable salt thereof, wherein, $L_1$ is selected from a bond, $-NR_{aa}C(O)-$, $-(CH_2)_{n1}-$, $-C(O)-$, $-O(CH_2)_{n2}-$,$-(CH_2)_{n1}O-$, $-S(CH_2)_{n2}-$, $-(CH_2)_{n1}S-$ or $-(CH_2)_{n1}NR_{aa}-$,

more preferably a bond, -NHC(O)-, $-CH_2-$, -C(O)-, -O-, $-OCH_2-$, $-CH_2O-$, -S-, $-SCH_2-$, $-CH_2S-$, -NH- or $-CH_2NH-$;
$R_{aa}$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n1 is an integer from 0 to 3.

**4.** The compound as claimed in claim 1 or claim 2, the stereoisomer thereof or the pharmaceutically acceptable salt thereof, wherein, $L_2$ is selected from a bond, $-(CH_2)_{n3}-$, $-(CR_{dd}R_{ee})_{n3}-$,$-(CR_{dd}R_{ee})_{n3}(CH_2)_{n4}NR_{ff}-$, $-(CH_2)_{n3}NR_{dd}(CR_{ee}R_{ff})_{n4}-$, $-(CH_2)_{n3}(CR_{dd}R_{ee})_{n4}-$, $-(CR_{dd}R_{ee})_{n3}O(CH_2)_{n4}-$,$-(CH_2)_{n3}O(CR_{dd}R_{ee})_{n4}-$, $-(CR_{aa}R_{bb})_{n3}S(CH_2)_{n4}-$, $-(CH_2)_{n3}S(CR_{aa}R_{bb})_{n4}-$, $-(CH_2)_{n3}C(O)(CR_{dd}R_{ee})_{n4}-$,$-(CH_2)_{n3}NR_{dd}C(O)(CR_{ee}R_{ff})_{n4}-$, $-(CH_2)_{n3}P(O)R_{dd}-$, $-(CH_2)_{n3}S(O)_{n4}-$, $-(CH_2)_{n3}S(O)_{n4}NR_{dd}-$,$-(CH_2)_nNR_{dd}S(O)_{n4}-$ or $-(CH_2)_{n3}C(O)NR_{dd}-$;

preferably a bond, $-(CH_2)_{n3}-$, $-(CR_{dd}R_{ee})_{n3}-$, $-(CR_{dd}R_{ee})_{n3}(CH_2)_{n4}NR_{ff}-$ or $-(CH_2)_{n3}NR_{dd}(CR_{ee}R_{ff})_{n4}-$, more preferably a bond,

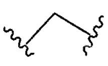 , 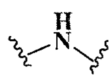 , 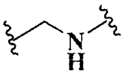 , 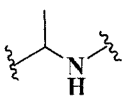 , 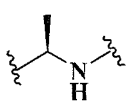 ,

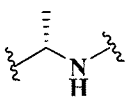, 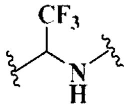, 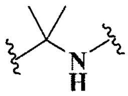, 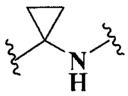, 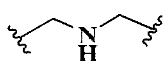, 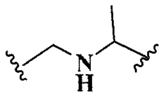, 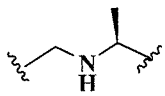,

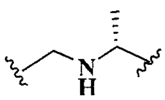, 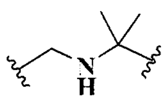, 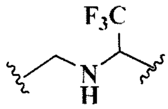, 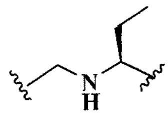, 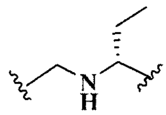, 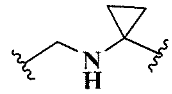,

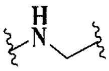 or 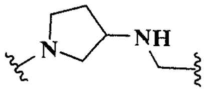;

$R_{dd}$, $R_{ee}$ and $R_{ff}$ are each independently selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

or, any two of $R_{dd}$, $R_{ee}$ and $R_{ff}$ are connected to form a $C_{3-12}$ cycloalkyl, 3- to 12-membered heterocyclyl, $C_{6-14}$ aryl or 5- to 14-membered heteroaryl, and the $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

n3 is an integer from 0 to 3; and

n4 is an integer from 0 to 3;

or, $L_2$ is selected from $-(CR_{dd}R_{ee})_{n3}-$, $-(CH_2)_{n3}NR_{dd}(CR_{ee}R_{ff})_{n4}-$ or $-(CH_2)_{n3}O(CR_{ee}R_{ff})_{n4}-$;

preferably

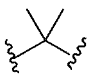, 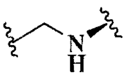, 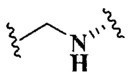, 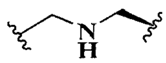, 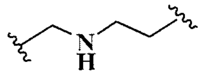, 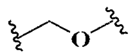,

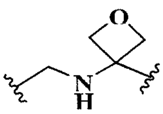 or 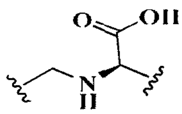;

$R_{dd}$, $R_{ee}$ and $R_{ff}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

or, any two of $R_{dd}$, $R_{ee}$ and $R_{ff}$ are connected to form a $C_{3-12}$ cycloalkyl, 3- to 12-membered heterocyclyl, $C_{6-14}$ aryl or 5- to 14-membered heteroaryl, and the $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-

membered heterocyclyl, $C_{6-14}$ aryl and 5- to 14-membered heteroaryl;
n3 is an integer from 0 to 3; and
n4 is an integer from 0 to 3.

**5.** The compound as claimed in claims 1 to 4, the stereoisomer thereof or the pharmaceutically acceptable salt thereof, wherein, ring A is selected from $C_{3-8}$ cycloalkyl, 3- to 10-membered heterocyclyl, $C_{6-10}$ aryl or 5- to 12-membered heteroaryl,

preferably, 5- to 10-membered nitrogen-containing heteroaryl,
more preferably, 5- to 6-membered nitrogen-containing heteroaryl,
most preferred are the following groups:

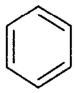, 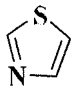, 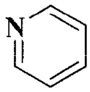, 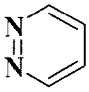, 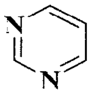 or 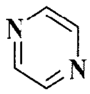.

**6.** The compound as claimed in claim 1 or 2, the stereoisomer thereof or the pharmaceutically acceptable salt thereof, wherein, R is selected from hydrogen, halogen, amino, hydroxyl, cyano, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl or $-(CH_2)_{n5}NR_{gg}C(O)R_{hh}$, and the amino, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl and 5- to 12-membered heteroaryl are optionally further substituted by one or more substituents of deuterium, halogen, amino, hydroxyl, oxo, $C_{1-6}$ alkyl, $C_{1-6}$ haloalkyl, $C_{1-6}$ hydroxyalkyl, $C_{1-6}$ alkoxy, $C_{3-8}$ cycloalkyl, $-(CH_2)_{n6}OR_{ii}$, $-(CH_2)_{n6}C(O)R_{ii}$ or $-(CH_2)_{n6}C(O)OR_{ii}$;

preferably fluorine, chlorine, bromine, hydroxyl, vinyl, ethynyl,

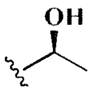, 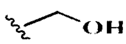, 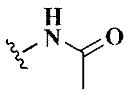,

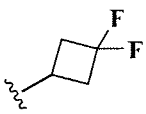, 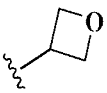, 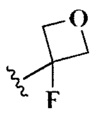, 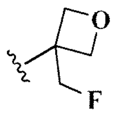, 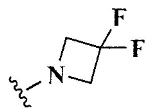, 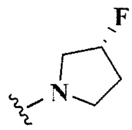, 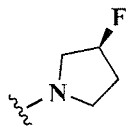,

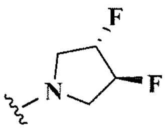, 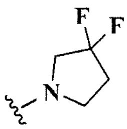, 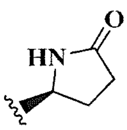, 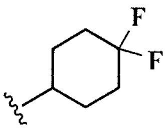, 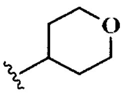, 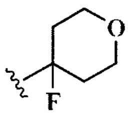, 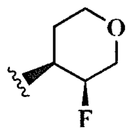,

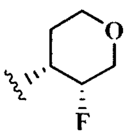, 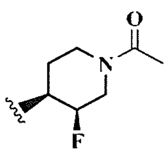, 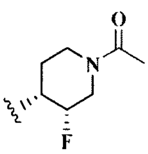, 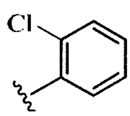, 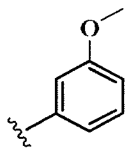, 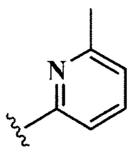, 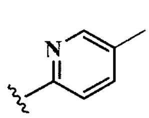,

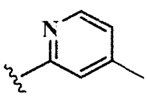, 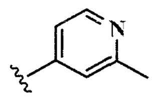, 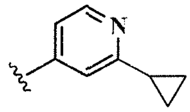, 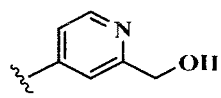, 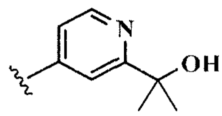,

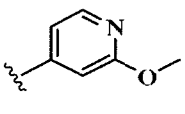, 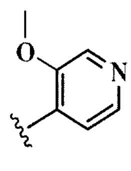, 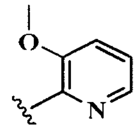, 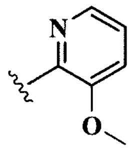, 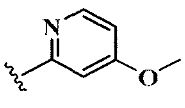, 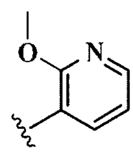,

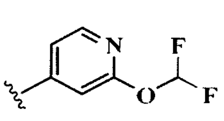, 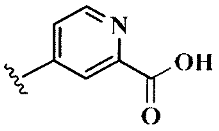, 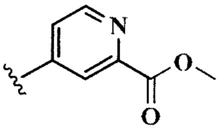, 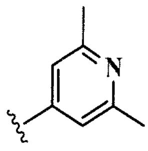, 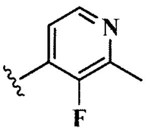,

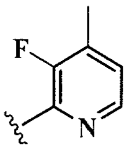, 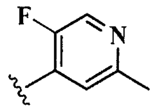, 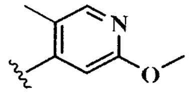, 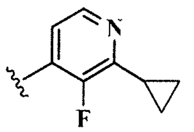, 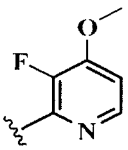, 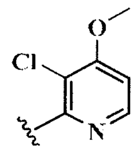,

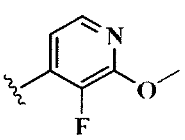, 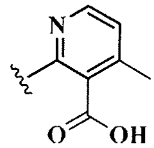, 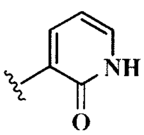, 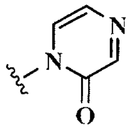, 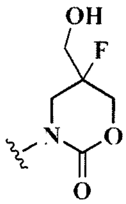, 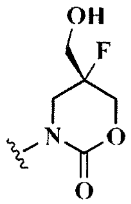, 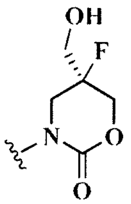 or

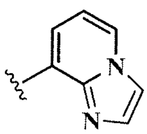;

$R_{gg}$, $R_{hh}$ and $R_{ii}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n5 is an integer from 0 to 3; and
n6 is an integer from 0 to 3;
or, R is selected from hydrogen, halogen, amino, hydroxyl, cyano, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $(CH_2)_{n5}C(O)OR_{gg}$, $(CH_2)_{n5}C(O)NR_{gg}R_{hh}$ or $-(CH_2)_{n5}NR_{gg}C(O)R_{hh}$, and the amino, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl and 5- to 12-membered heteroaryl are optionally further substituted by one or more substituents of hydrogen, deuterium, halogen, amino, hydroxyl, oxo, $C_{1-6}$ alkyl, $C_{1-6}$ haloalkyl, $C_{1-6}$ hydroxyalkyl, $C_{1-6}$ alkoxy, $C_{3-8}$ cycloalkyl, $-(CH_2)_{n6}OR_{ii}$, $-(CH_2)_{n6}C(O)R_{ii}$, $-(CH_2)_{n6}OC(O)R_{ii}$ or $-(CH_2)_{n6}C(O)OR_{ii}$;
preferably methyl, difluoromethyl, $-C(O)OH$, $-C(O)OCH_2CH_3$, $-C(O)NHCH_3$,

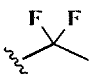,

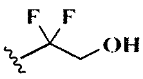, 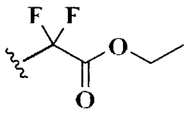, 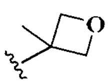, 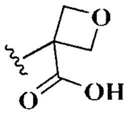, 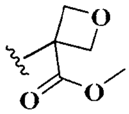, 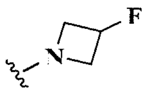, 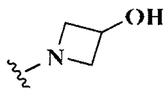,

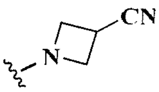, 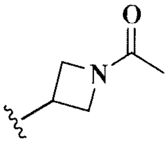, 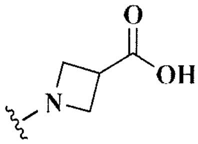, 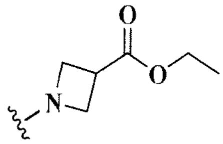, 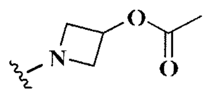,

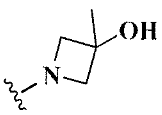, 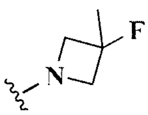, 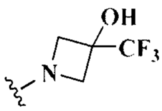, 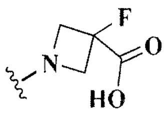, 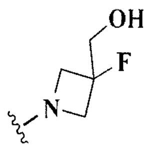, 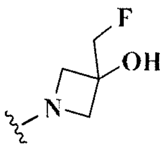,

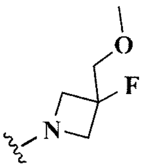, 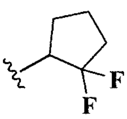, 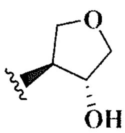, 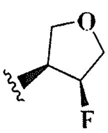, 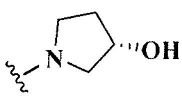, 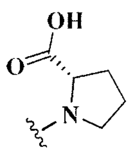, 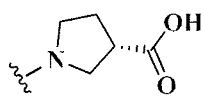,

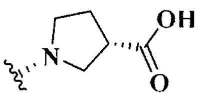, 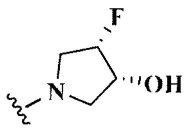, 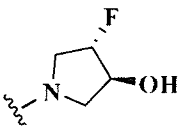, 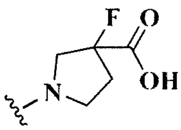, 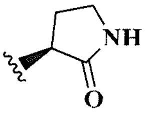,

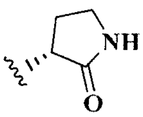, 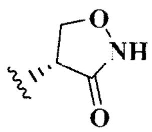, 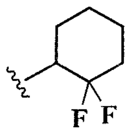, 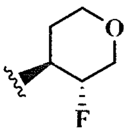, 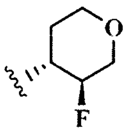, 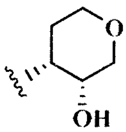, 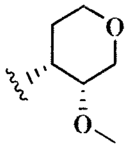,

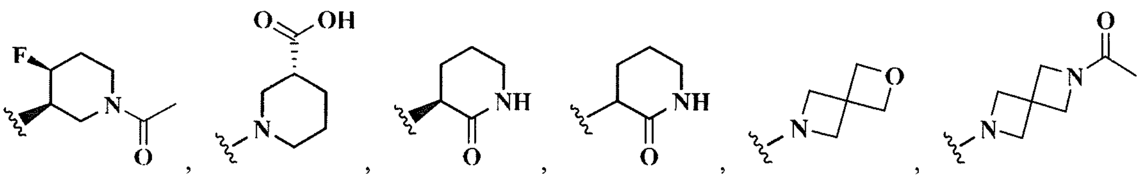 , , , , , ,

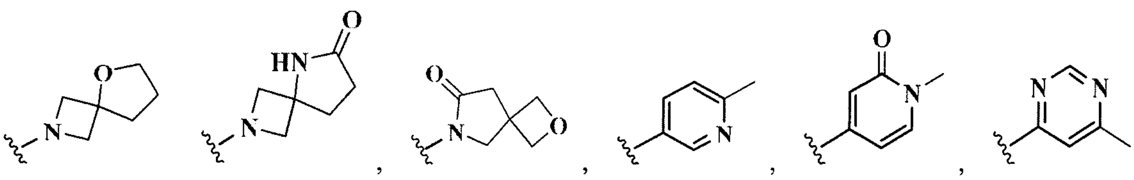 , , , , ,

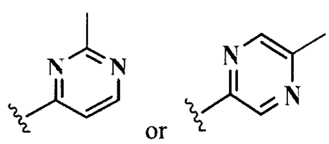 or ;

$R_{gg}$, $R_{hh}$ and $R_{ii}$ are each independently selected from hydrogen, deuterium, halogen, amino, hydroxyl, mercapto,

cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n5 is an integer from 0 to 3; and
n6 is an integer from 0 to 3;
or, R is selected from hydrogen, halogen, amino, hydroxyl, cyano, $C_{1-3}$ alkyl, $C_{1-3}$ haloalkyl, $C_{2-4}$ alkenyl, $C_{2-4}$ alkynyl, $C_{3-8}$ cycloalkyl, 3- to 10-membered heterocyclyl, $C_{6-10}$ aryl, 5- to 12-membered heteroaryl, $(CH_2)_{n5}C(O)OR_{gg}$, $(CH_2)_{n5}C(O)NR_{gg}R_{hh}$ or $-(CH_2)_{n5}NR_{gg}C(O)R_{hh}$, and the amino, $C_{1-3}$ alkyl, $C_{1-3}$ haloalkyl, $C_{2-4}$ alkenyl, $C_{2-4}$ alkynyl, $C_{3-8}$ cycloalkyl, 3- to 10-membered heterocyclyl, $C_{6-10}$ aryl and 5- to 12-membered heteroaryl are optionally further substituted by one or more substituents of deuterium, halogen, amino, hydroxyl, cyano, carboxyl, oxo, $C_{1-6}$ alkyl, $C_{1-6}$ haloalkyl, $C_{1-6}$ hydroxyalkyl, $C_{1-6}$ alkoxy, $C_{3-8}$ cycloalkyl, $(CH_2)_{n6}R_{ii}$, $-(CH_2)_{n6}OR_{ii}$, $-(CH_2)_{n6}C(O)R_{ii}$, $-(CH_2)_{n6}OC(O)R_{ii}$, $-(CH_2)_{n6}C(O)OR_{ii}$, $-(CH_2)_{n6}C(O)NR_{ii}R_i$, $-(CH_2)_{n6}NR_{ii}R_i$, $-(CH_2)_{n6}NR_{ii}C(O)R_i$ or $-(CH_2)_{n6}S(O)_{m6}R_{ii}$;
preferably $-CH(CH_2OH)_2$, $-CH(CH_2OH)(COOCH_3)$,

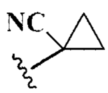, 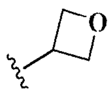, 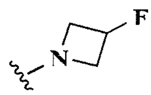,

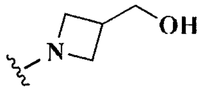, 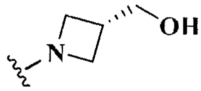, 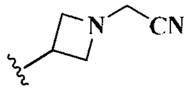, 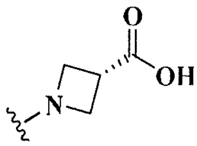, 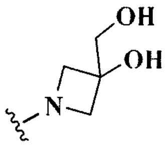,

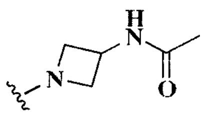, 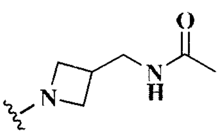, 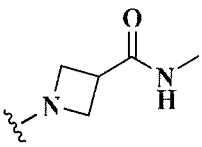, 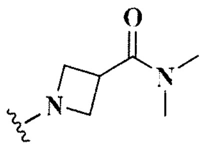, 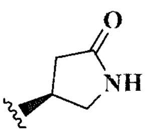,

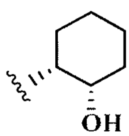, 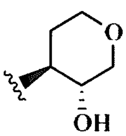, 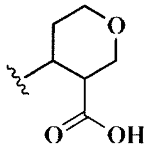, 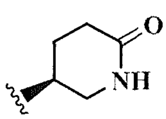, 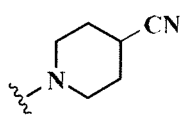, 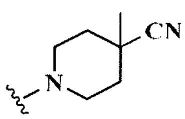,

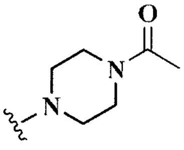, 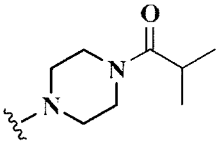, 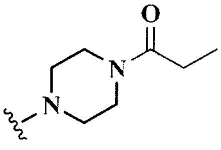, 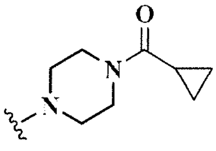, 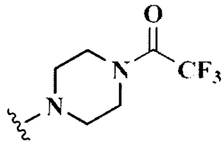,

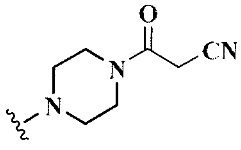, 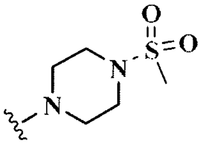, 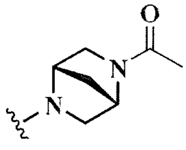, 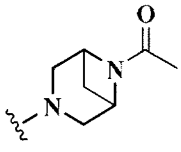, 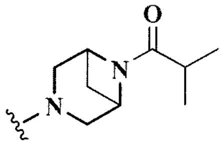,

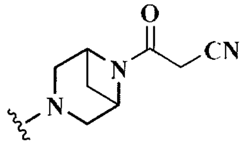, 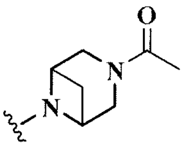, 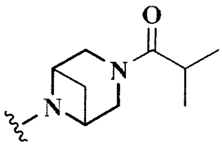, 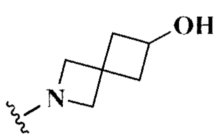, 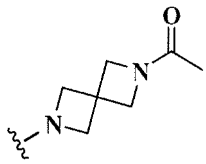,

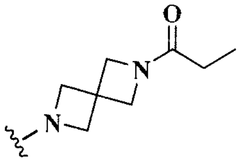, 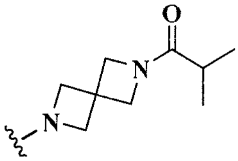, 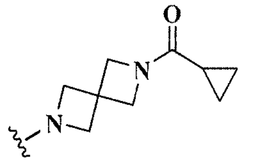, 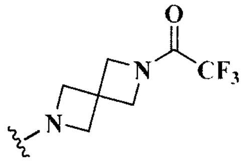,

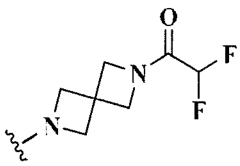, 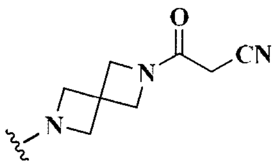, 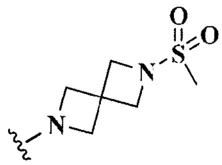, 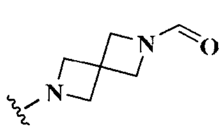,

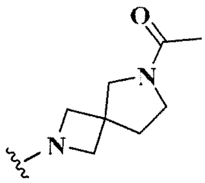, 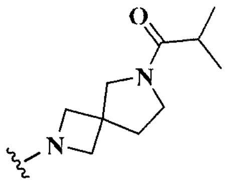, 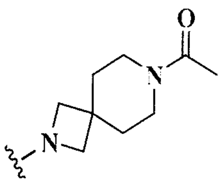 or 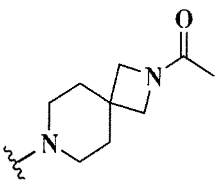;

$R_{gg}$, $R_{hh}$, $R_{ii}$ and $R_i$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n5 is an integer from 0 to 3;
n6 is an integer from 0 to 3; and
m6 is an integer from 0 to 2.

**7.** The compound as claimed in claim 1, the stereoisomer thereof or the pharmaceutically acceptable salt thereof, wherein, $R_1$ is selected from hydrogen, halogen, amino, hydroxyl, cyano, oxo, $C_{1-3}$ alkyl, $C_{1-3}$ haloalkyl, $C_{1-3}$ alkoxy, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $-(CH_2)_{n7}R_{jj}$, $-(CH_2)_{n7}C(O)NR_{jj}R_{pp}$ or $-O(CH_2)_{n7}R_{jj}$;

preferably hydrogen, fluorine, chlorine, bromine, methyl, ethyl, isopropyl, trifluoromethyl, methoxy, cyano, oxo, cyclopropyl, $-OCH_2CN$ or $-C(O)NH_2$;
$R_{jj}$ and $R_{pp}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl; and

n7 is an integer from 0 to 3.

8. The compound as claimed in claim 1, the stereoisomer thereof or the pharmaceutically acceptable salt thereof, wherein, $R_2$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

preferably hydrogen, halogen or $C_{1-3}$ alkyl,
more preferably chlorine or methyl;
or, $R_2$ is selected from hydrogen, fluorine, chlorine, bromine, methyl, ethyl or isopropyl.

9. The compound as claimed in claim 1, the stereoisomer thereof or the pharmaceutically acceptable salt thereof, wherein, $R_3$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

preferably hydrogen, halogen or $C_{1-3}$ alkyl;
more preferably chlorine or methyl;
or, $R_3$ is selected from hydrogen, fluorine, chlorine, bromine, methyl, ethyl or isopropyl.

10. The compound as claimed in claim 2, the stereoisomer thereof or the pharmaceutically acceptable salt thereof, wherein, the general formula (I) is further represented by general formula (II):

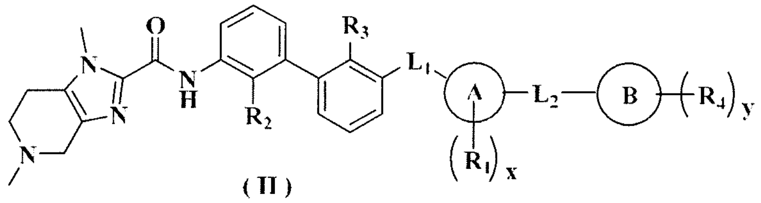

(II)

wherein:

ring B is selected from $C_{3-12}$ cycloalkyl, 3- to 12-membered heterocyclyl, $C_{6-14}$ aryl or 5- to 14-membered heteroaryl;
$R_4$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl,
preferably substituted by one or more substituents of hydrogen, halogen, amino, hydroxyl, oxo, $C_{1-6}$ alkyl, $C_{1-6}$ haloalkyl, $C_{1-6}$ alkoxy, $C_{3-8}$ cycloalkyl;

y is an integer from 0 to 6.

**11.** The compound as claimed in any one of claims 1, 3 to 9, the stereoisomer thereof or the pharmaceutically acceptable salt thereof, wherein, the general formula (Ib) is further represented by general formula (IV-A):

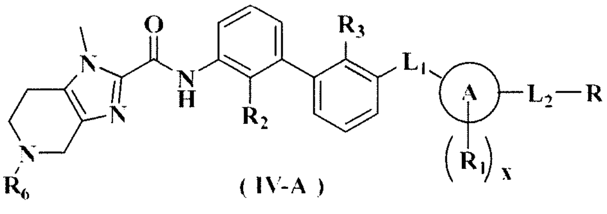

( IV-A )

wherein:

$L_1$ is selected from a bond or -NHC(O)-;
$L_2$ is selected from a bond, $-(CH_2)_{n3}-$, $-(CR_{dd}R_{ee})_{n3}-$, $-(CR_{dd}R_{ee})_{n3}O-$, $-(CR_{dd}R_{ee})_{n3}(CH_2)_{n4}NR_{ff}-$ or $-(CH_2)_{n3}NR_{dd}(CR_{ee}R_{ff})_{n4}-$;
ring A is selected from

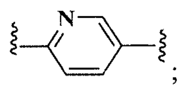
;

$R_1$ is selected from hydrogen, halogen, amino, hydroxyl, cyano, oxo, $C_{1-3}$ alkyl, $C_{1-3}$ haloalkyl, $C_{1-3}$ alkoxy, $C_{1-6}$ hydroxyalkyl, $C_{1-6}$ alkyl, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $-(CH_2)_{n7}OR_{jj}$, $-(CH_2)_{n7}C(O)NR_{jj}R_{pp}$ or $-O(CH_2)_{n7}R_{jj}$;
$R_2$ is selected from hydrogen, halogen or $C_{1-3}$ alkyl, preferably methyl or chlorine;
$R_3$ is selected from hydrogen, halogen or $C_{1-3}$ alkyl, preferably methyl or chlorine;
R is selected from hydrogen, halogen, amino, hydroxyl, cyano, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl, 5- to 12-membered heteroaryl, $(CH_2)_{n5}C(O)OR_{gg}$, $(CH_2)_{n5}C(O)NR_{gg}R_{hh}$ or $-(CH_2)_{n5}NR_{gg}C(O)R_{hh}$, and the amino, $C_{1-3}$ alkyl, $C_{1-3}$ haloalkyl, $C_{2-4}$ alkenyl, $C_{2-4}$ alkynyl, $C_{3-8}$ cycloalkyl, 3- to 8-membered heterocyclyl, $C_{6-10}$ aryl and 5- to 12-membered heteroaryl are optionally further substituted by one or more substituents of hydrogen, deuterium, halogen, amino, hydroxyl, oxo, $C_{1-6}$ alkyl, $C_{1-6}$ haloalkyl, $C_{1-6}$ hydroxyalkyl, $C_{1-6}$ alkoxy, $C_{3-8}$ cycloalkyl, $-(CH_2)_{n6}OR_{ii}$, $-(CH_2)_{n6}C(O)R_{ii}$, $-(CH_2)_{n6}OC(O)R_{ii}$ or $-(CH_2)_{n6}C(O)OR_{ii}$;
$R_6$ is selected from hydrogen, methyl, ethyl, isopropyl, methoxy, cyclopropyl,

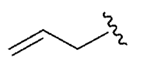
,

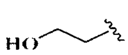
, 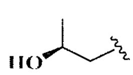
, 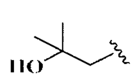
, 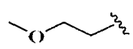
, 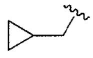
, 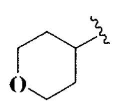
, 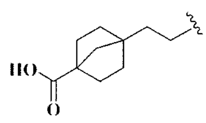
,

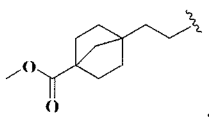
, 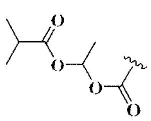
, 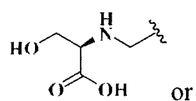
or 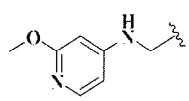

, preferably methyl;
$R_{dd}$, $R_{ee}$, $R_{ff}$, $R_{gg}$, $R_{jj}$, $R_{pp}$, $R_{hh}$ and $R_{ii}$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl,

$C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n3, n4, n6 and n7 are each independently an integer selected from 0 to 3; and
x is an integer from 0 to 6.

**12.** The compound as claimed in claim 1 or 11, the stereoisomer thereof or the pharmaceutically acceptable salt thereof, wherein, the general formula (Ib) is further represented by general formula (IV):

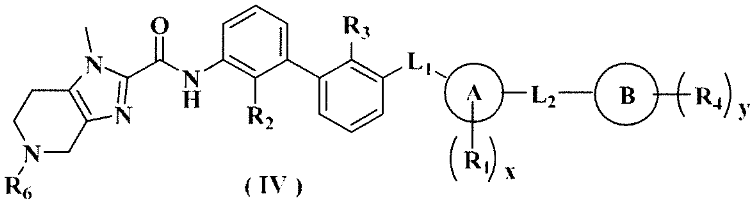

wherein:

$L_1$ is selected from a bond or -NHC(O)-;
ring A is selected from

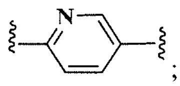
;

ring B is selected from $C_{3-12}$ cycloalkyl, 3- to 12-membered heterocyclyl, $C_{6-14}$ aryl or 5- to 14-membered heteroaryl;
$R_4$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, $-(CH_2)_{n8}R_{kk}$, $-(CH_2)_{n8}OR_{kk}$, $-(CH_2)_{n8}C(O)R_{kk}$ or $-(CH_2)_{n8}C(O)OR_{kk}$, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
$R_{kk}$ is selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
y is an integer from 0 to 6.

**13.** The compound as claimed in claims 10 and 12, the stereoisomer thereof or the pharmaceutically acceptable salt thereof, wherein:

ring B is selected from $C_{3-8}$ cycloalkyl, 3- to 10-membered heterocyclyl, $C_{6-10}$ aryl or 5- to 10-membered heteroaryl;
preferably $C_{6-10}$ aryl, 3- to 8-membered nitrogen-containing heterocyclyl or 5- to 10-membered nitrogen-containing heteroaryl,

more preferred are the following groups:

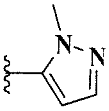 , 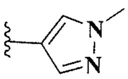 , 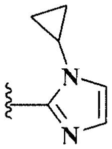 , 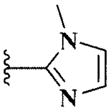 , 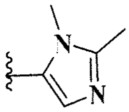 , 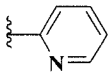 , 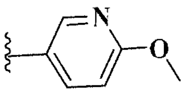 ,

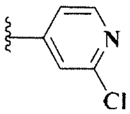 , 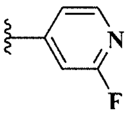 , 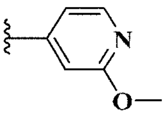 , 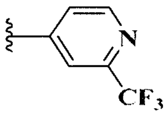 , 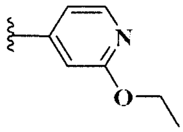 , 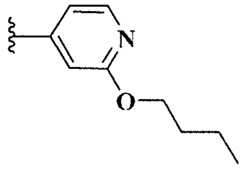 ,

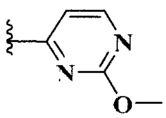 , 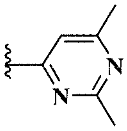 , 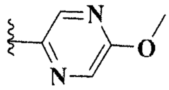 , 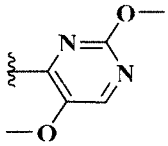 , 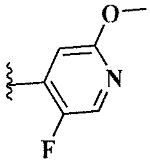 , 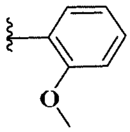 ,

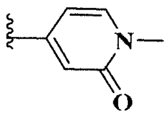 , 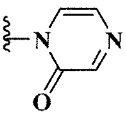 , 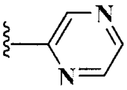 , 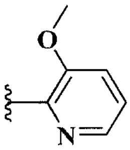 , 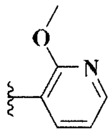 , 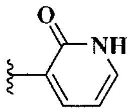 , 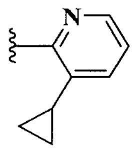 ,

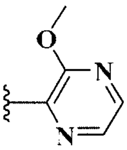 or 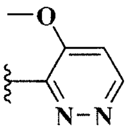 ;

or, ring B is selected from $C_{3-8}$ cycloalkyl, 3- to 8-membered heterocyclyl, $C_{6-10}$ aryl or 5- to 10-membered heteroaryl;

preferably $C_{3-8}$ cycloalkyl, 3- to 8-membered nitrogen-containing heterocyclyl, 3- to 8-membered oxygen-containing heterocyclyl, $C_{6-10}$ aryl or 5- to 10-membered nitrogen-containing heteroaryl,

more preferred are the following groups:

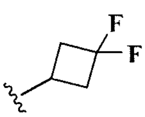 , 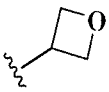 , 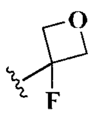 , 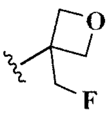 , 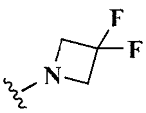 , 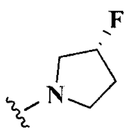 , 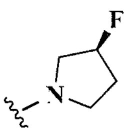 ,

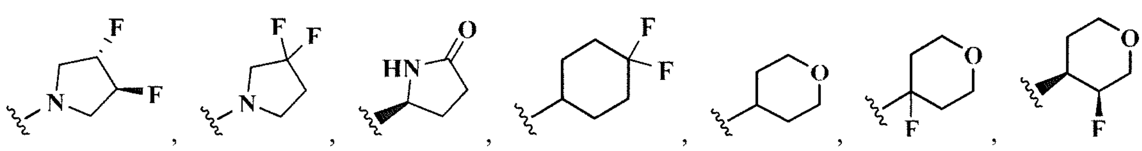
, , , , , , ,

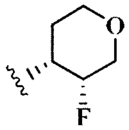, 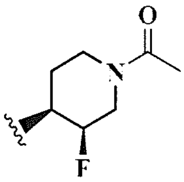, 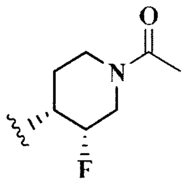, 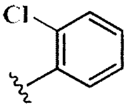, 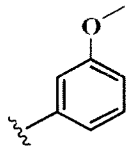, 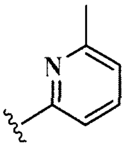, 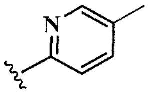,

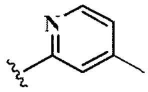, 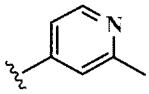, 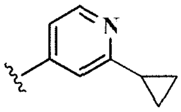, 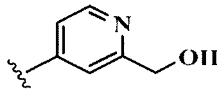, 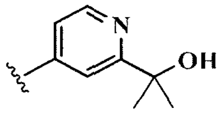,

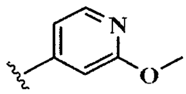, 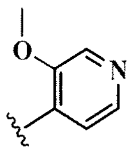, 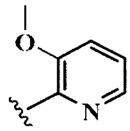, 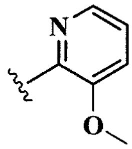, 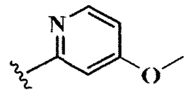, 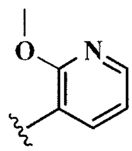,

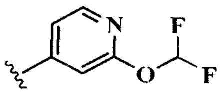, 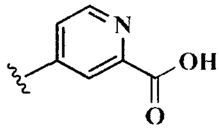, 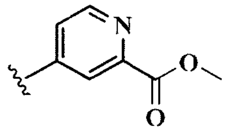, 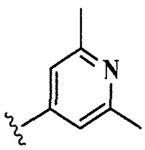, 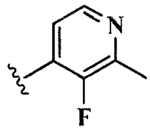,

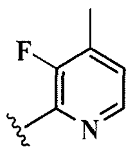, 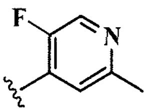, 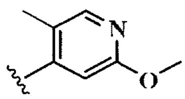, 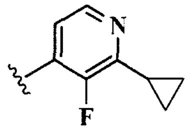, 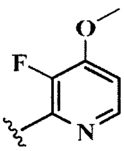, 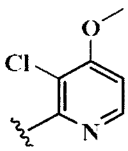,

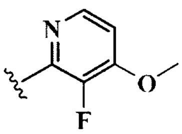, 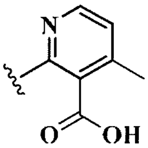, 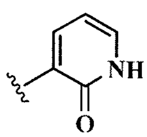, 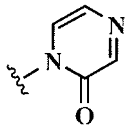, 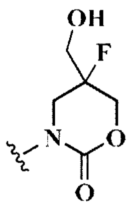, 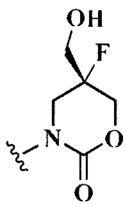, 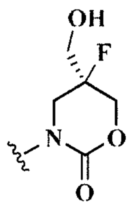

or

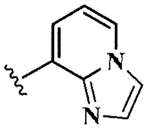.

**14.** The compound as claimed in claims 10 and 12, the stereoisomer thereof or the pharmaceutically acceptable salt thereof, wherein: ring B is selected from following groups:

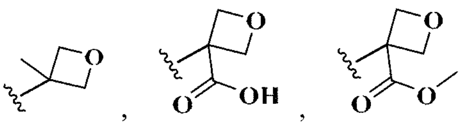, , , 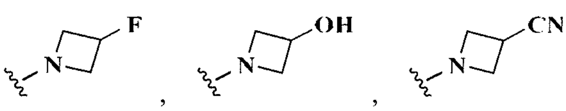, , ,

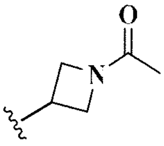, 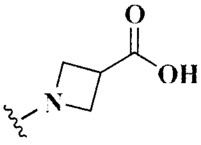, 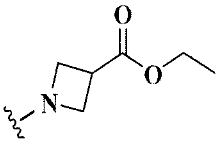, 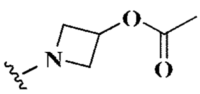, 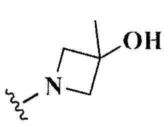,

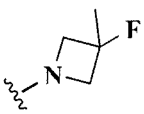, 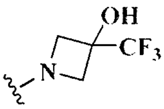, 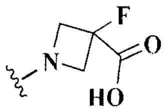, 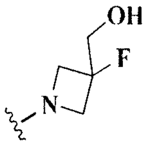, 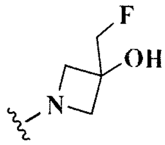, 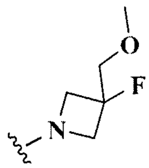,

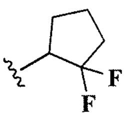, 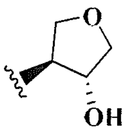, 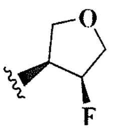, 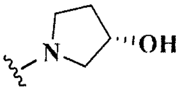, 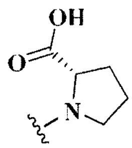, 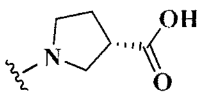,

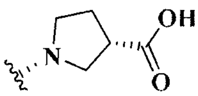, 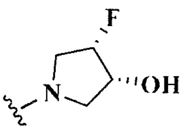, 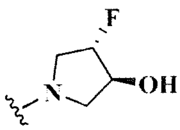, 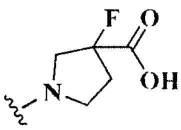, 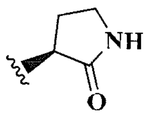, 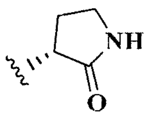,

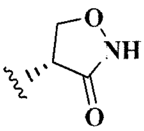, 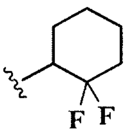, 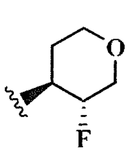, 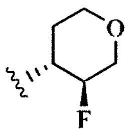, 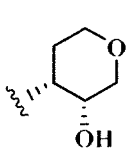, 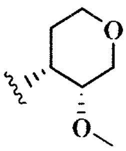, 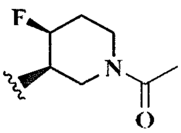,

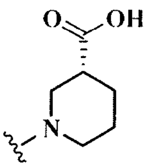, 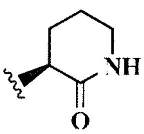, 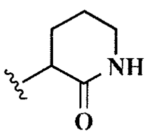, 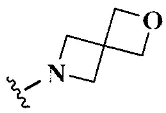, 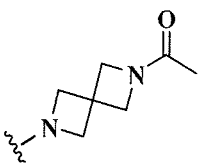, 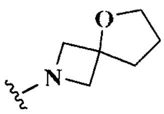,

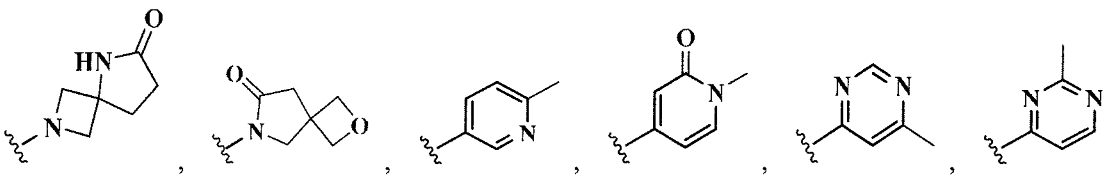

or

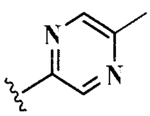.

**15.** The compound as claimed in claims 10 and 12, the stereoisomer thereof or the pharmaceutically acceptable salt thereof, wherein:

ring B is selected from $C_{3-6}$ cycloalkyl, 3- to 6-membered oxygen-containing heterocyclyl or 3- to 10-membered nitrogen-containing heterocyclyl;
more preferred are the following groups:

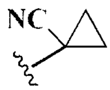, 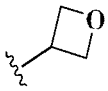, 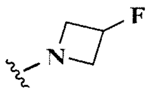, 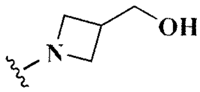, 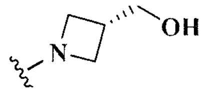, 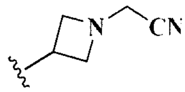,

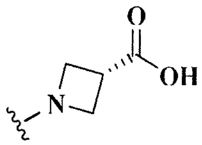, 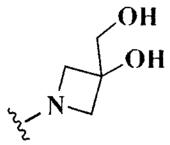, 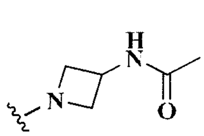, 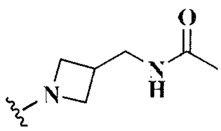, 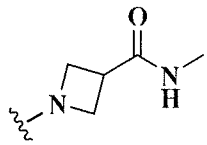,

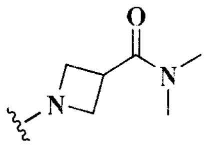, 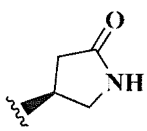, 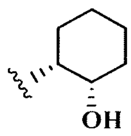, 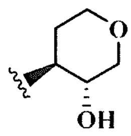, 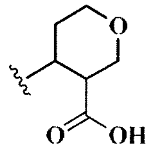, 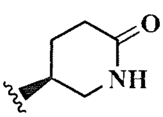,

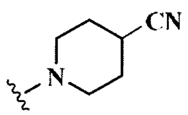, 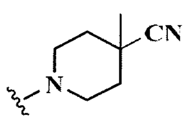, 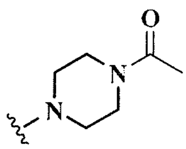, 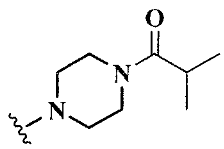, 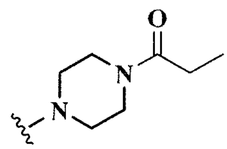,

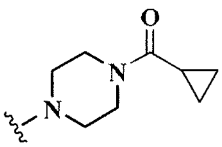, 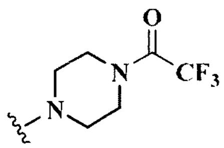, 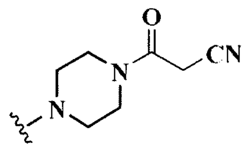, 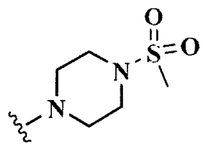, 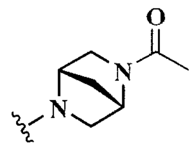,

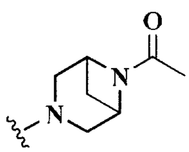, 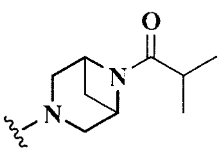, 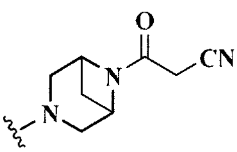, 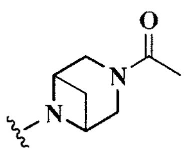, 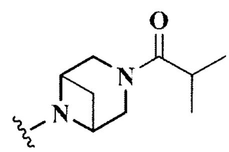,

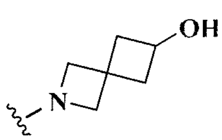, 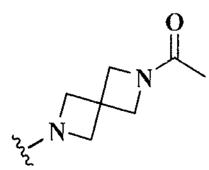, 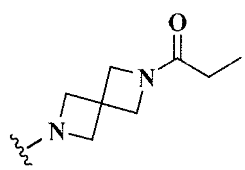, 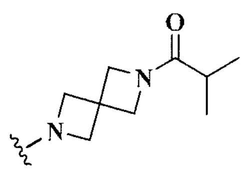,

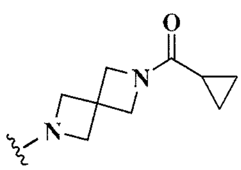, 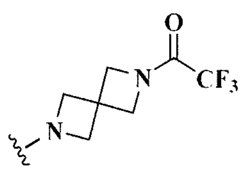, 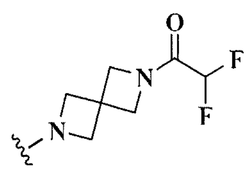, 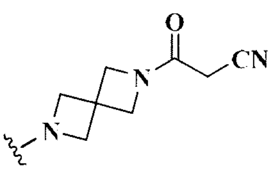,

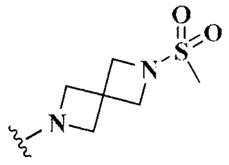, 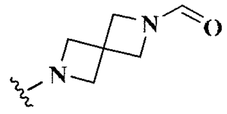, 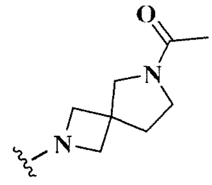, 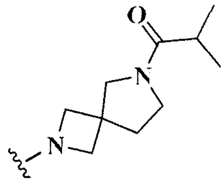,

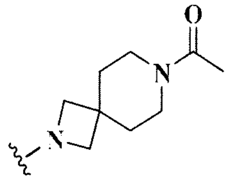 or 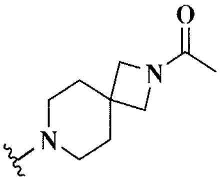.

**16.** The compound as claimed in claims 10 and 12, the stereoisomer thereof or the pharmaceutically acceptable salt thereof, wherein, $R_4$ is selected from hydrogen, halogen, amino, hydroxyl, cyano, carboxyl, oxo, $C_{1-3}$ alkyl, $C_{1-3}$ haloalkyl, $C_{1-3}$ hydroxyalkyl, $C_{1-3}$ alkoxy, $C_{2-4}$ alkenyl, $C_{2-4}$ alkynyl, $C_{3-8}$ cycloalkyl, 3-to 8-membered heterocyclyl, $C_{6-10}$ aryl, 5- to 12-membered heteroaryl, $-(CH_2)_{n8}R_{kk}$, $-(CH_2)_{n8}OR_{kk}$, - $(CH_2)_{n8}OC(O)R_{kk}$, $-(CH_2)_{n8}C(O)R_{kk}$, $-(CH_2)_{n8}C(O)OR_{kk}$, $-(CH_2)_{n8}C(O)NR_{kk}R_k$, $-(CH_2)_{n8}NR_{kk}R_k$, - $(CH_2)_{n8}NR_{kk}C(O)R_k$ or $-(CH_2)_{n8}S(O)_{m8}R_{kk}$;

preferably hydrogen, fluorine, chlorine, bromine, methyl, ethyl, isopropyl, methoxy, hydroxyl, cyano, carboxyl, oxo, difluoromethyl, trifluoromethyl, cyclopropyl, $-CH_2F$, $-CH_2OH$, $-CH_2CN$, $-C(CH_3)_2OH$, - $C(O)CH_3$, $-C(O)OCH_3$, $-OCHF_2$, $-C(CH_3)_2OH$, $-CH_2OCH_3$, $-C(O)OCH_2CH_3$, $-OC(O)CH_3$, $-NHC(O)CH_3$, - $CH_2NHC(O)CH_3$, $-C(O)NHCH_3$, $-C(O)N(CH_3)_2$, $-C(O)CH_2CH_3$, $-C(O)CH(CH_3)_2$, $-C(O)CF_3$, $-C(O)CHF_2$, - $C(O)CH_2CN$, -CH(O)-, $S(O)_2CH_3$ or

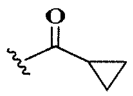;

$R_{kk}$ and $R_k$ are each independently selected from hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
n8 is an integer from 0 to 3; and
m8 is an integer from 0 to 2.

**17.** The compound as claimed in claim 1, the stereoisomer thereof or the pharmaceutically acceptable salt thereof, wherein, the compound is further represented by general formula (V):

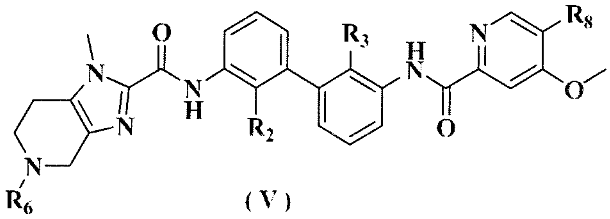

(V)

wherein:

$R_2$ is selected from hydrogen, halogen or $C_{1-6}$ alkyl, preferably halogen or $C_{1-3}$ alkyl, more preferably chlorine or methyl;

$R_3$ is selected from hydrogen, halogen or $C_{1-6}$ alkyl, preferably halogen or $C_{1-3}$ alkyl, more preferably chlorine or methyl;

$R_6$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl, 5- to 14-membered heteroaryl, $-(CH_2)_{m3}R_d$, $-(CH_2)_{m3}(CR_dR_e)_{m4}R_f$, $-(CH_2)_{m3}OR_d$, $-(CH_2)_{m3}NR_dR_e$, $-(CH_2)_{m3}NR_dCHR_eR_f$, $-(CH_2)_{m3}C(O)O(CR_dR_e)_{m4}OC(O)R_f$ or $-(CH_2)_{m3}CR_d=CR_eR_f$,
preferably hydrogen, methyl, ethyl, isopropyl, methoxy, cyclopropyl,

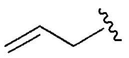, 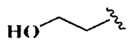,

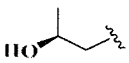, 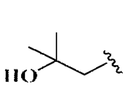, 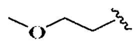, 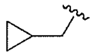, 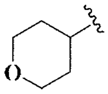, 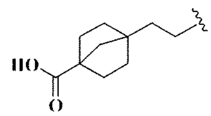, 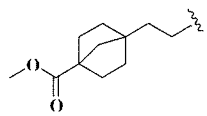,

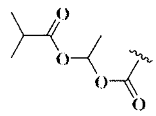, 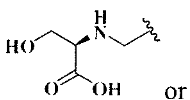 or 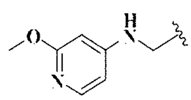;

$R_8$ is selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ haloalkoxy, $C_{1-6}$ alkylthio, $C_{1-6}$ hydroxyalkyl, $C_{3-12}$ cycloalkyl, 3- to 12-membered heterocyclyl, $C_{6-14}$ aryl or 5- to 14-membered heteroaryl, $-(CH_2)_{n9}R_{ll}$, $-(CH_2)_{n9}NR_{11}(CR_{mm}R_{nn})_{n10}R_{oo}$ or $-(CH_2)_{n9}NR_{ll}(CH_2)_{n10}NR_{mm}C(O)R_{nn}$, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
preferably hydrogen, halogen, amino, hydroxyl, $C_{1-6}$ alkyl, $C_{1-6}$ haloalkyl, $C_{1-6}$ alkoxy, $C_{1-6}$ hydroxyalkyl, $C_{2-6}$ alkenyl, $C_{2-6}$ alkynyl, $-(CH_2)_{n9}R_{ll}$, $-(CH_2)_{n9}NR_{ll}(CR_{mm}R_{nn})_{n10}R_{oo}$ or $-(CH_2)_{n9}NR_{ll}(CH_2)_{n10}NR_{mm}C(O)R_{nn}$,
more preferably $-CH_2OH$, $-C(CH_3)_2OH$, $-CH_2NHCH_2CH_2F$, $-CH_2NHCH_2CH_2OH$, $-CH_2NHCH_2CH(OH)CH_3$, $-CH_2NHCH_2CH_2NHC(O)CH_3$, $-CH_2NHCH_2CH=CH_2$ or $-CH_2NHCH_2C\equiv CH$;

$R_{ll}$, $R_{mm}$, $R_{nn}$ and $R_{oo}$ are each independently selected from hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl or 5- to 14-membered heteroaryl, and the amino, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;

n9 is an integer from 0 to 3; and

n10 is an integer from 0 to 3.

**18.** The compound as claimed in claim 1, the stereoisomer thereof or the pharmaceutically acceptable salt thereof, wherein, the compound is further represented by general formula (VI):

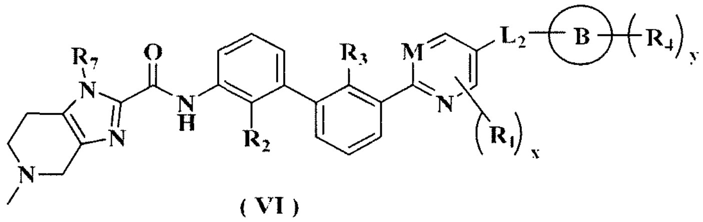

( VI )

wherein:

$R_1$ is selected from hydrogen, halogen, cyano, $C_{1-3}$ alkyl, $C_{1-3}$ haloalkyl or $C_{1-3}$ alkoxy, preferably hydrogen, fluorine, chlorine, cyano, methyl, trifluoromethyl or methoxy;
$R_2$ is selected from hydrogen, halogen or $C_{1-3}$ alkyl, preferably chlorine or methyl;
$R_3$ is selected from hydrogen, halogen or $C_{1-3}$ alkyl, preferably fluorine, chlorine or methyl;
M is -CH- or -N-;
$L_2$ is selected from a bond, $-(CH_2)_{n3}-$ or $-(CH_2)_{n3}NR_{dd}(CR_{ee}R_{ff})_{n4}-$, preferably a bond,

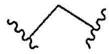 ,

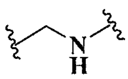 , 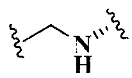 , 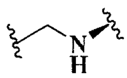 , 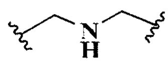 , 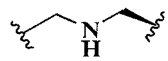 , 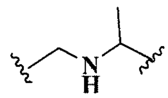

or

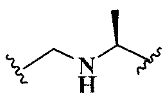 ;

ring B is selected from $C_{3-8}$ cycloalkyl, 3- to 10-membered heterocyclyl, $C_{6-10}$ aryl or 5- to 10-membered heteroaryl, preferably $C_{3-6}$ cycloalkyl, 3- to 10-membered heterocyclyl containing 1 to 3 heteroatoms selected from N or O, or 5- to 6-membered nitrogen-containing heteroaryl, and more preferably the following groups:

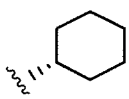 , 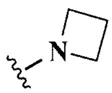 , 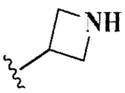 , 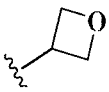 , 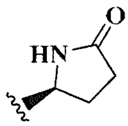 , 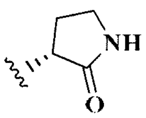 , 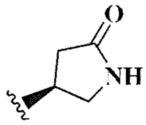 ,

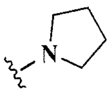 , 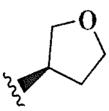 , 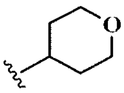 , 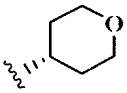 , 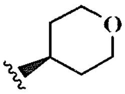 , 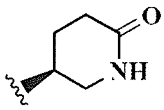 , 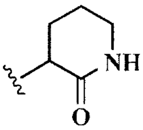 ,

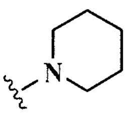 , 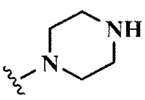 , 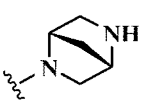 , 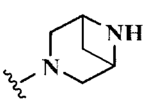 , 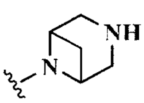 , 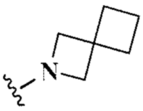 , 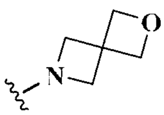 ,

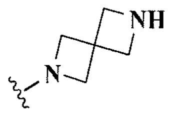, 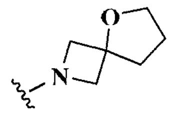, 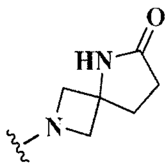, 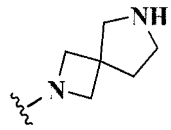, 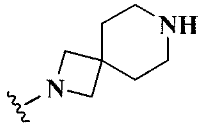,

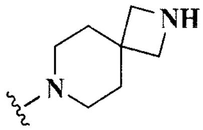 or 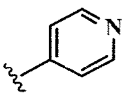;

$R_4$ is selected from hydrogen, deuterium, halogen, hydroxyl, cyano, carboxyl, $C_{1-3}$ alkyl, $C_{1-3}$ deuterated alkyl, $C_{1-3}$ haloalkyl, $C_{1-3}$ alkoxy, $C_{1-3}$ hydroxyalkyl, $-(CH_2)_{n8}R_{kk}$, $-(CH_2)_{n8}OR_{kk}$, $-(CH_2)_{n8}OC(O)R_{kk}$, $-(CH_2)_{n8}C(O)R_{kk}$, $-(CH_2)_{n8}C(O)OR_{kk}$, $-(CH_2)_{n8}C(O)NR_{kk}R_k$, $-(CH_2)_{n8}NR_{kk}C(O)R_k$ or $-(CH_2)_{n8}S(O)_{m8}R_{kk}$, preferably hydrogen, fluorine, chlorine, bromine, methyl, ethyl, propyl, isopropyl, hydroxyl, cyano, carboxyl, oxo, trifluoromethyl, hydroxymethyl, methoxy, $-CH_2F$, $-CH_2OH$, $-CH_2CN$, $-OC(O)CH_3$, $-C(O)OCH_3$, $-C(O)OCH_2CH_3$, $-NHC(O)CH_3$, $-CH_2NHC(O)CH_3$, $-C(O)N(CH_3)_2$, $-C(O)NHCH_3$, $-C(O)CH_3$, $-C(O)CH_2CH_3$, $-C(O)CH(CH_3)_2$, $-C(O)CF_3$, $-C(O)CHF_2$, $-C(O)CH_2CN$, $-CH(O)$, $-S(O)_2CH_3$ or

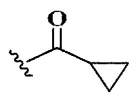;

$R_{dd}$, $R_{ee}$, $R_{ff}$, $R_{kk}$ and $R_k$ are each independently selected from hydrogen, deuterium, halogen, amino, hydroxyl, mercapto, cyano, nitro, carboxyl, $C_{1-3}$ alkyl, $C_{2-4}$ alkenyl, $C_{2-4}$ alkynyl, $C_{1-3}$ deuterated alkyl, $C_{1-3}$ haloalkyl, $C_{1-3}$ alkoxy, $C_{1-3}$ alkylthio, $C_{1-3}$ haloalkoxy, $C_{1-3}$ hydroxyalkyl, $C_{3-12}$ cycloalkyl, 3- to 12-membered heterocyclyl, $C_{6-14}$ aryl or 5- to 14-membered heteroaryl, and the amino, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl are optionally substituted by one or more substituents of hydrogen, deuterium, halogen, amino, hydroxyl, mercapto, cyano, nitro, carboxyl, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ alkoxy, $C_{1-6}$ alkylthio, $C_{1-6}$ haloalkoxy, $C_{1-6}$ hydroxyalkyl, $C_{3-12}$ cycloalkyl, 3- to 12-membered heterocyclyl, $C_{6-14}$ aryl and 5- to 14-membered heteroaryl;
$R_7$ is selected from hydrogen, $C_{1-3}$ alkyl or $C_{3-6}$ cycloalkyl; preferably methyl, ethyl or cyclopropyl;
n3, n4, n8 and m8 are each independently selected from 0, 1 or 2;
x is 1 or 2; and
y is 0, 1, 2 or 3.

**19.** The compound as claimed in any one of claims 1 to 18, the stereoisomer thereof or the pharmaceutically acceptable salt thereof, wherein:

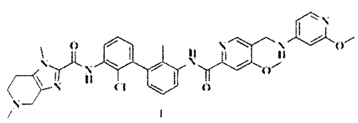
1

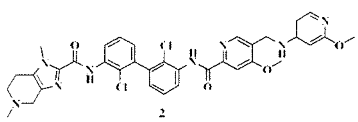
2

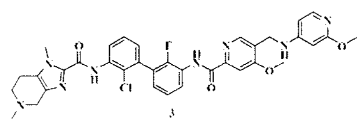
3

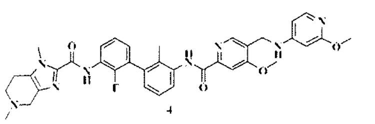
4

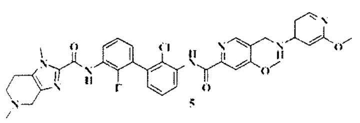
5

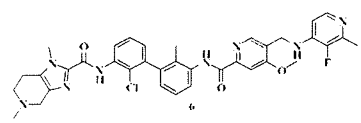
6

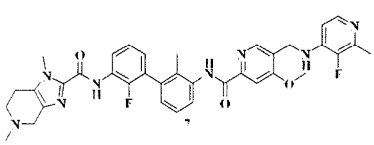
7

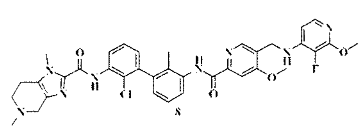
8

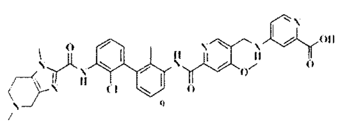
9

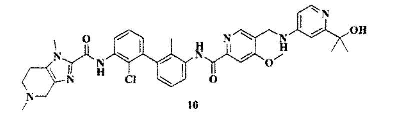
10

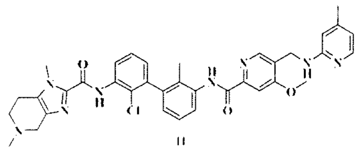
11

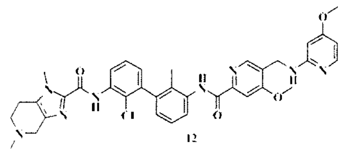
12

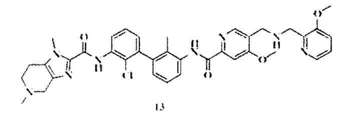
13

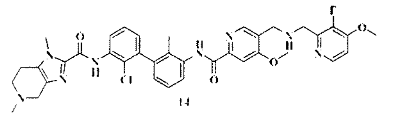
14

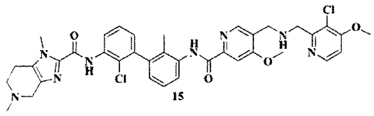
15

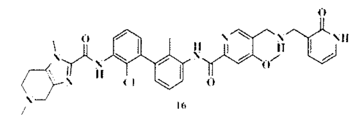
16

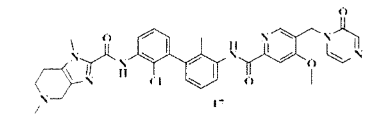
17

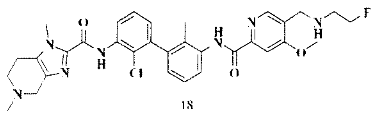
18

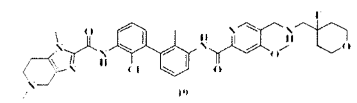
19

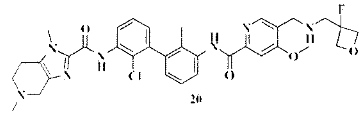
20

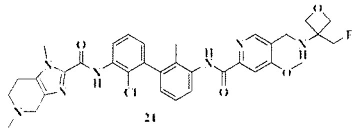
21

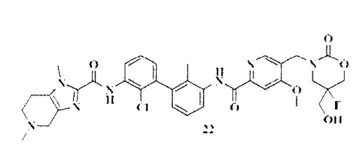
22

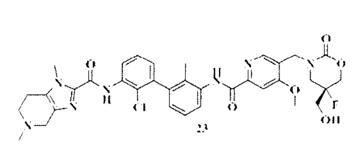
23

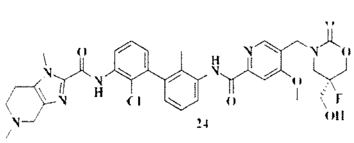
24

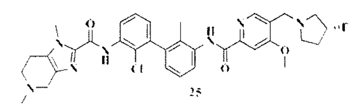
25

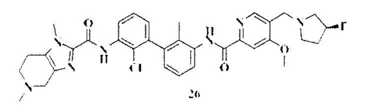
26

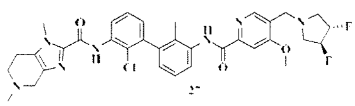
27

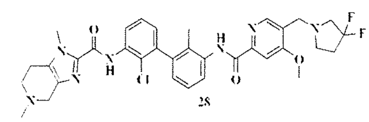
28

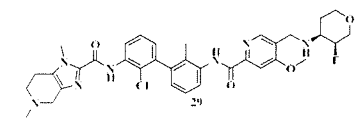
29

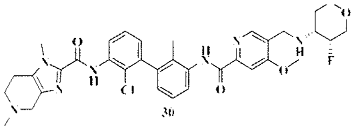
30

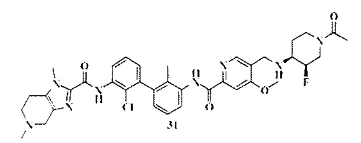
31

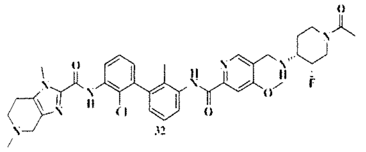
32

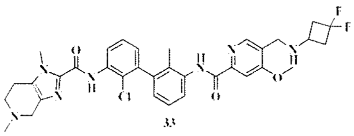
33

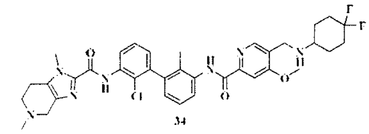
34

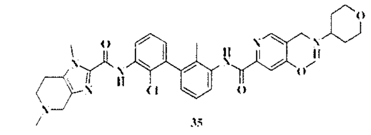
35

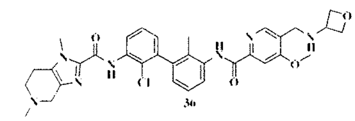
36

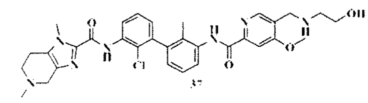
37

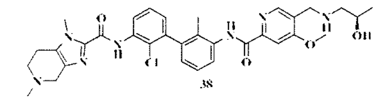
38

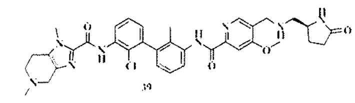
39

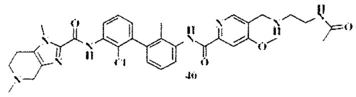
40

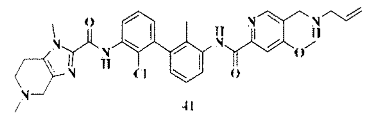
41

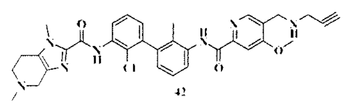
42

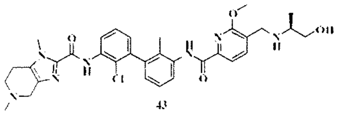
43

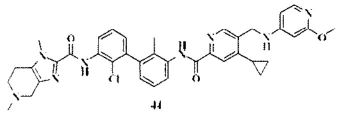
44

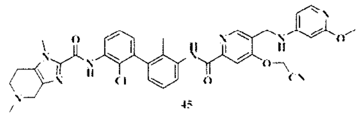
45

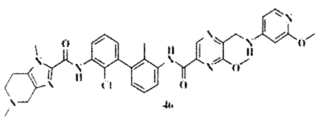
46

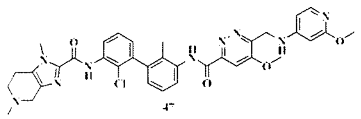
47

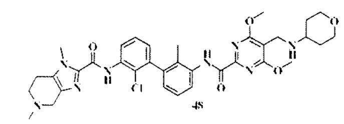
48

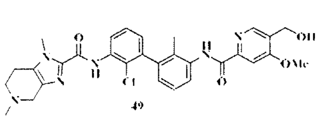
49

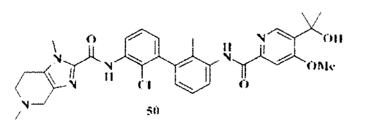
50

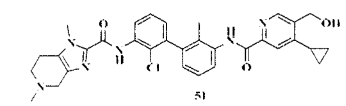
51

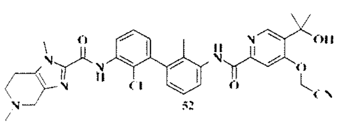
52

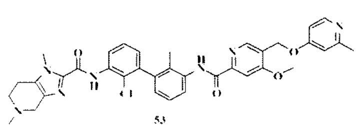
53

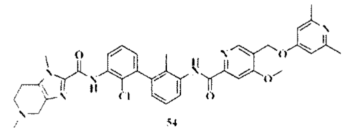
54

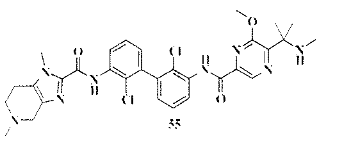
55

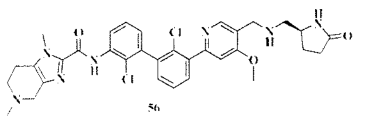
56

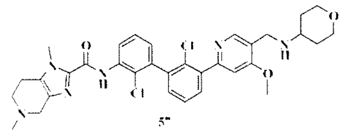
57

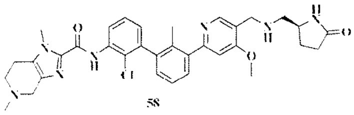
58

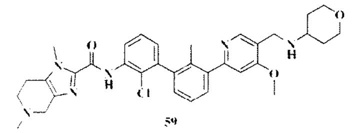
59

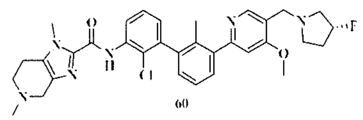
60

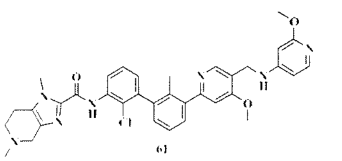
61

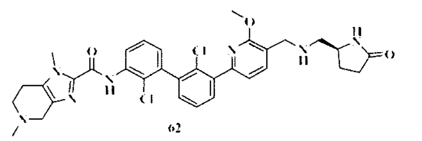
62

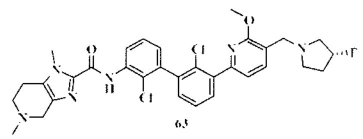
63

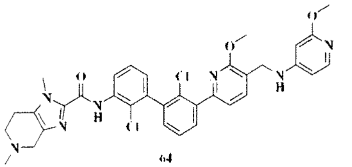
64

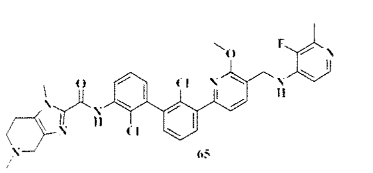
65

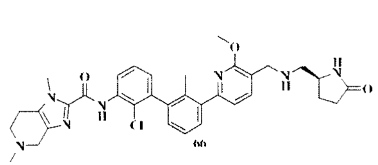
66

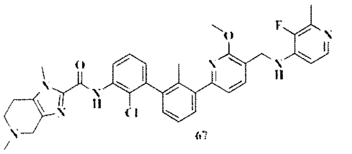
67

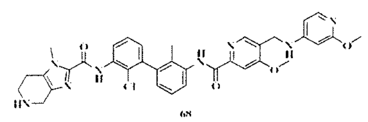
68

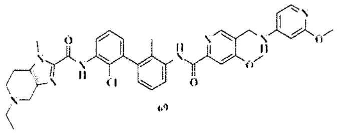
69

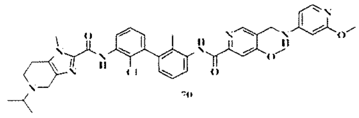
70

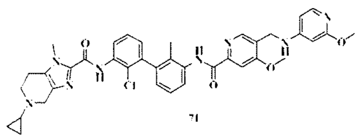
71

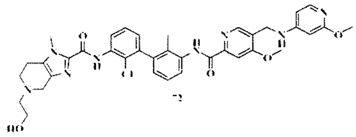
72

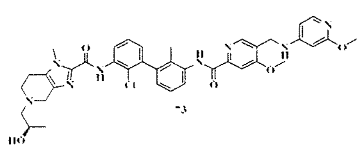
73

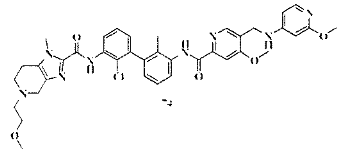
74

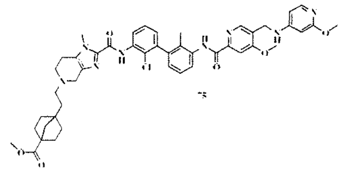
75

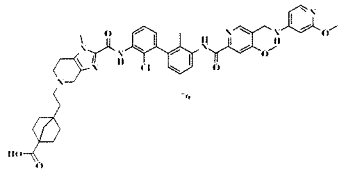
76

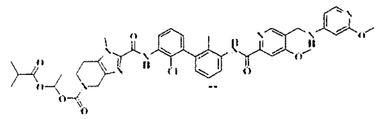
77

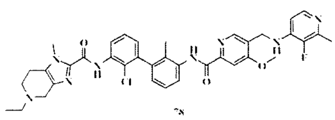
78

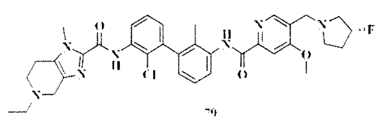
79

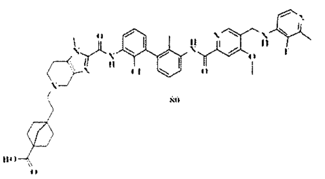
80

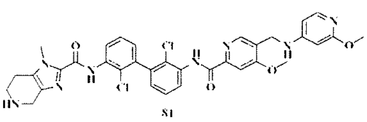
81

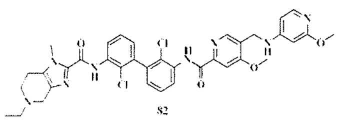
82

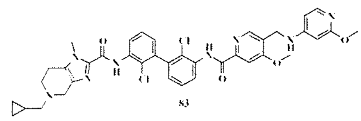
83

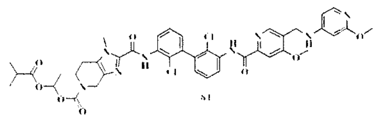
84

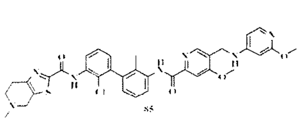
85

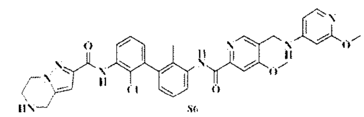
86

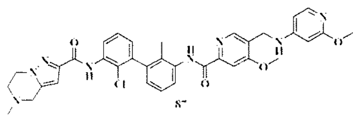
87

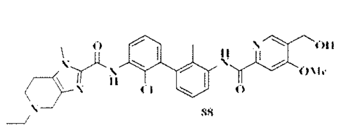
88

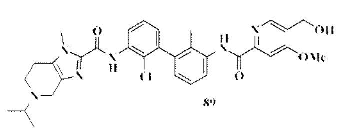
89

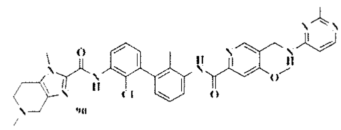
90

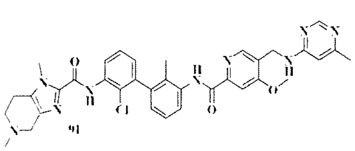
91

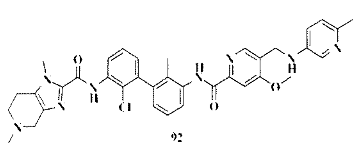
92

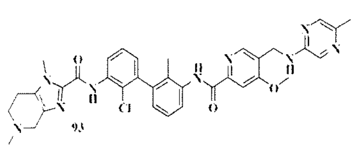
93

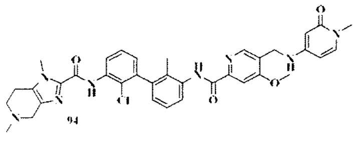
94

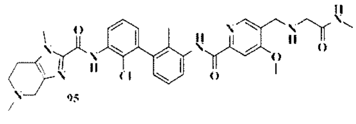
95

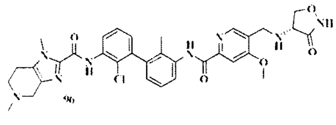
96

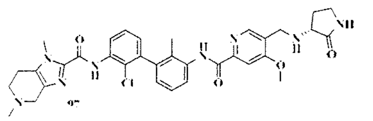
97

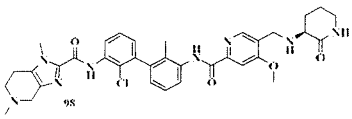
98

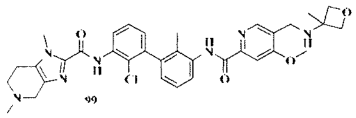
99

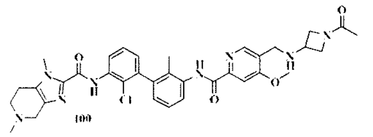
100

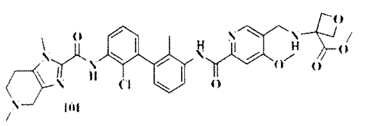
101

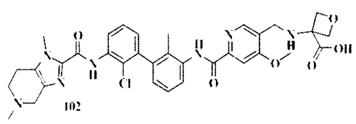
102

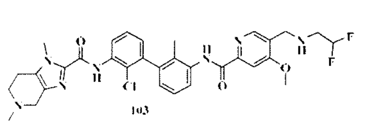
103

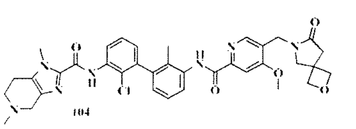
104

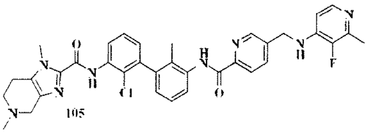
105

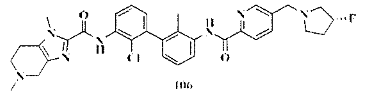
106

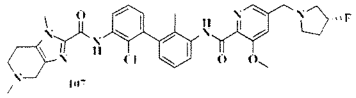
107

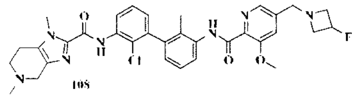
108

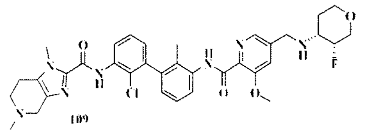
109

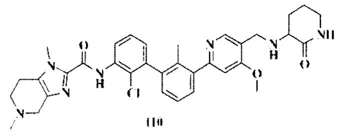
110

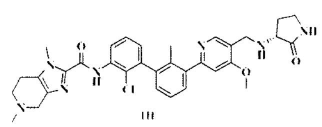
111

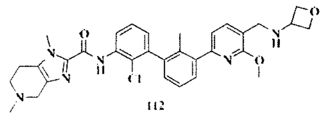
112

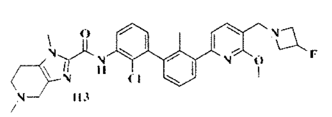
113

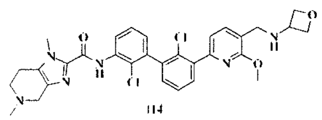
114

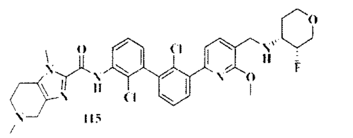
115

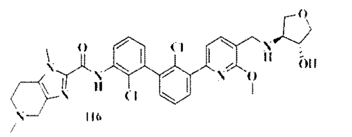
116

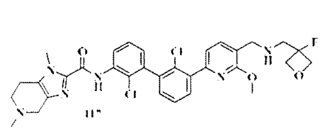
117

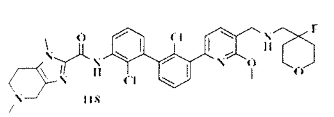
118

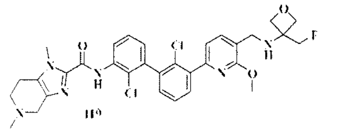
119

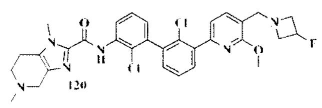
120

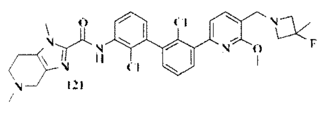
121

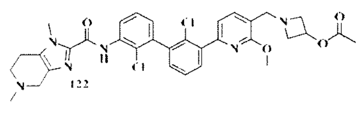
122

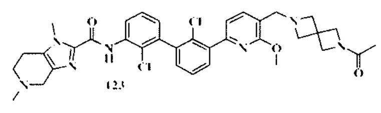
123

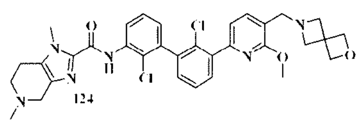
124

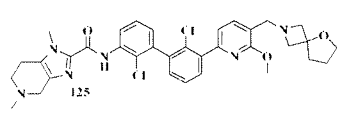
125

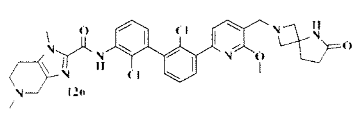
126

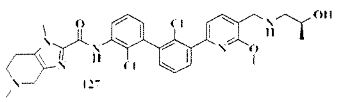
127

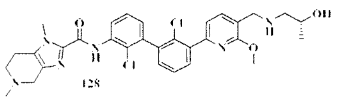
128

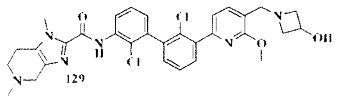
129

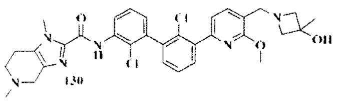
130

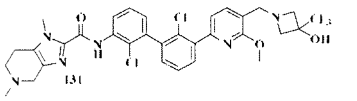
131

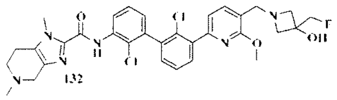
132

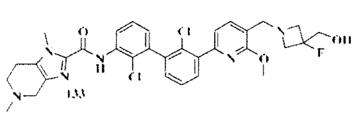
133

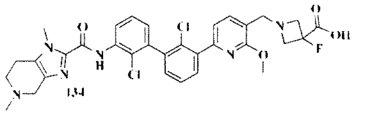
134

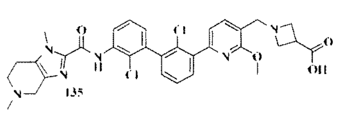
135

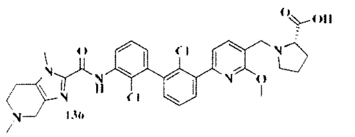
136

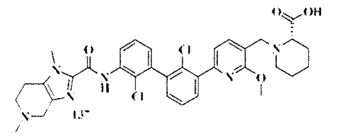
137

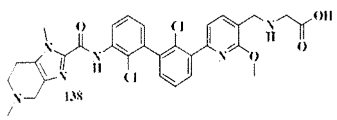
138

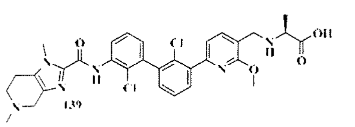
139

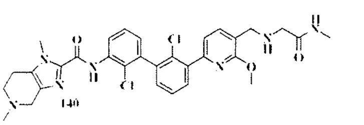
140

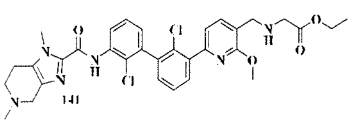
141

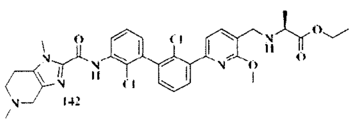
142

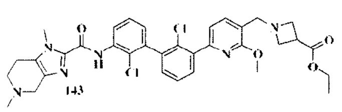
143

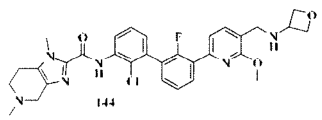
144

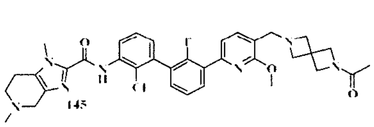
145

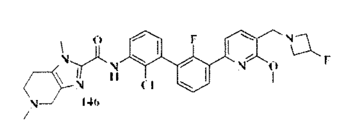
146

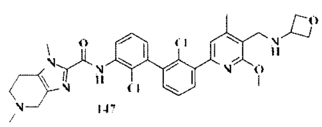
147

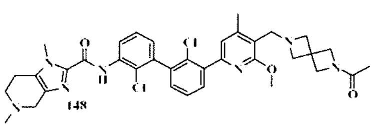
148

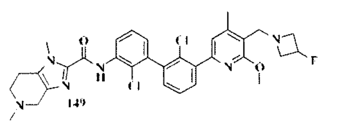
149

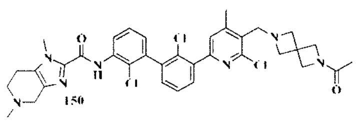
150

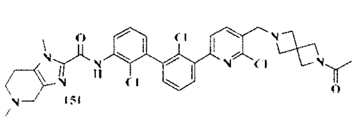
151

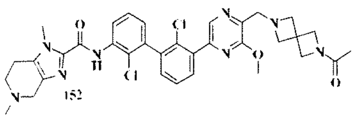
152

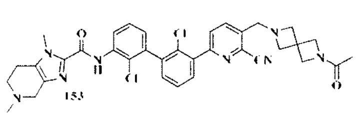
153

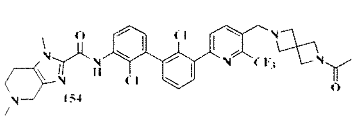
154

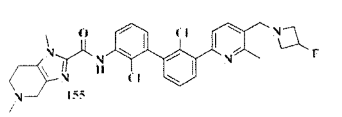
155

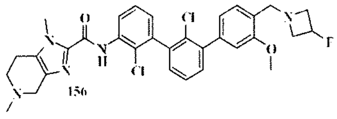
156

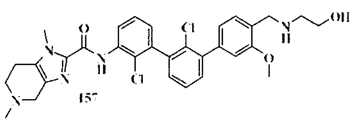
157

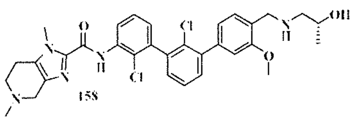
158

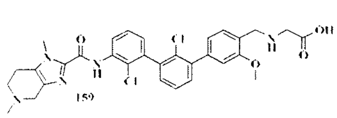
159

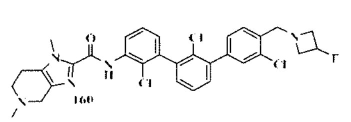
160

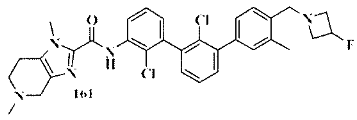
161

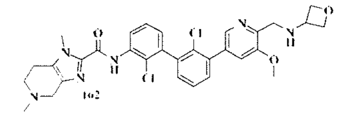
162

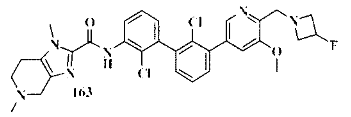
163

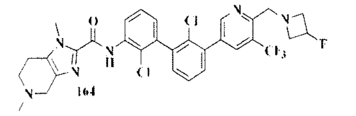
164

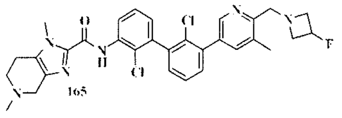
165

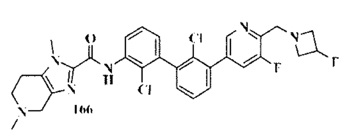
166

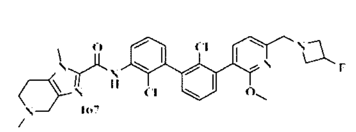
167

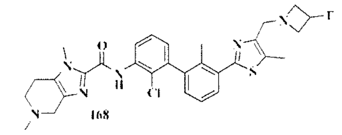
168

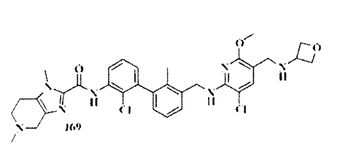
169

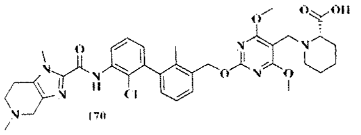
170

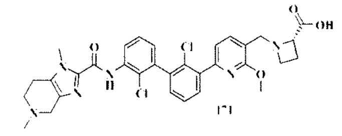
171

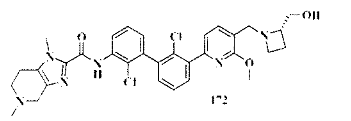
172

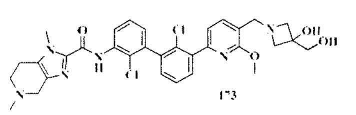
173

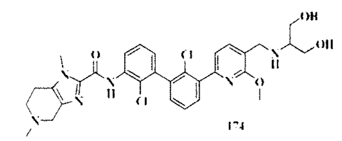
174

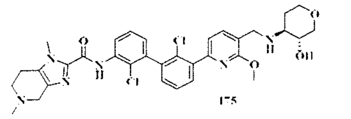
175

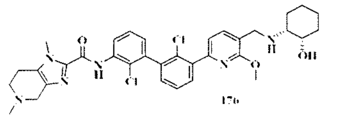
176

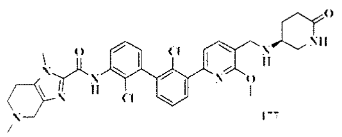
177

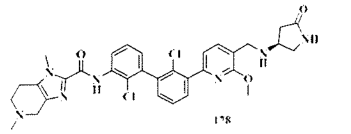
178

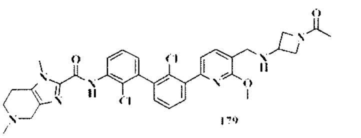
179

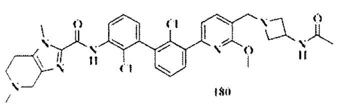
180

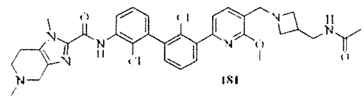
181

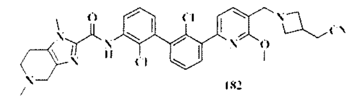
182

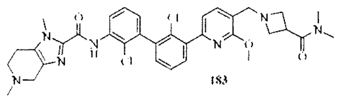
183

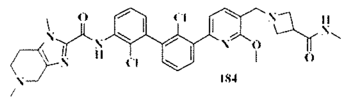
184

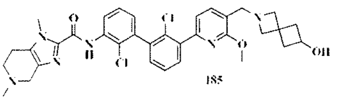
185

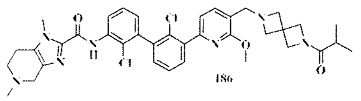
186

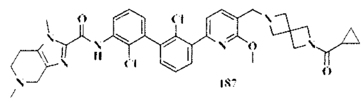
187

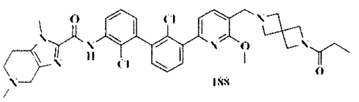
188

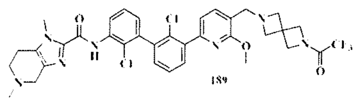
189

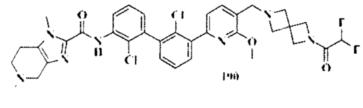
190

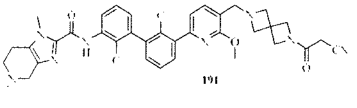
191

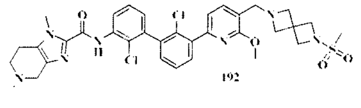
192

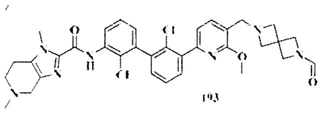
193

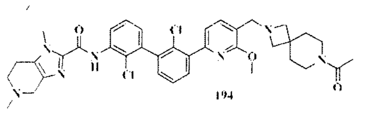
194

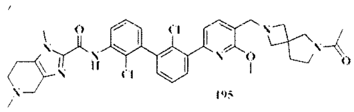
195

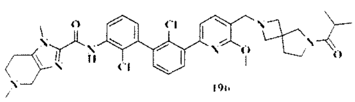
196

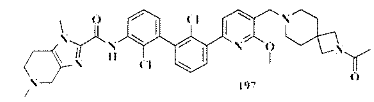
197

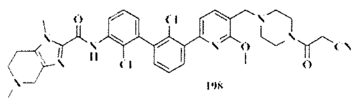
198

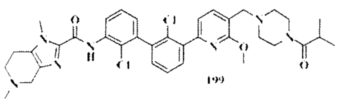
199

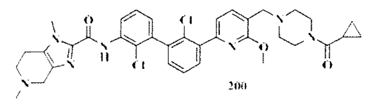
200

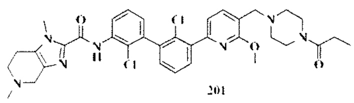
201

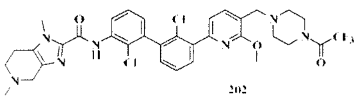
202

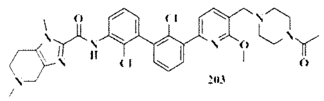
203

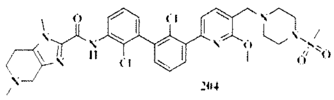
204

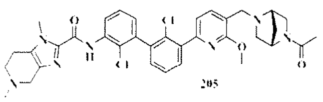
205

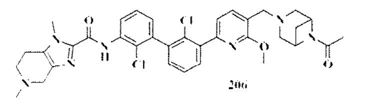
206

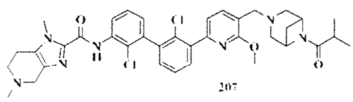
207

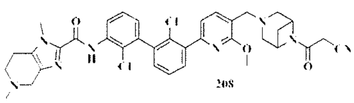
208

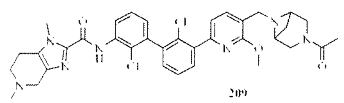
209

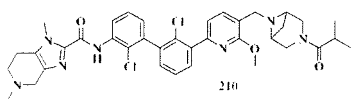
210

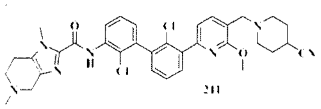
211

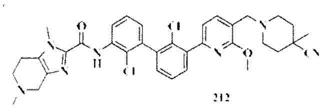
212

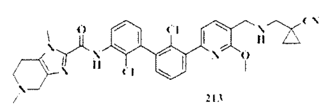
213

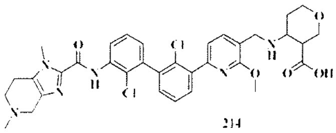
214

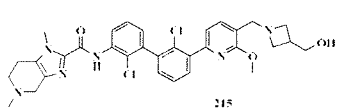
215

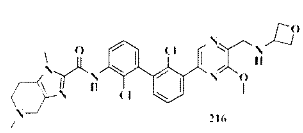
216

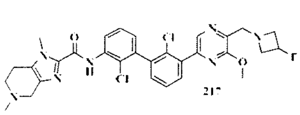
217

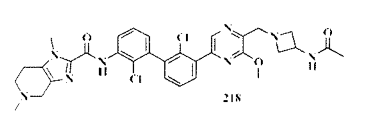
218

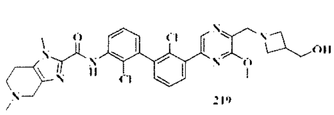
219

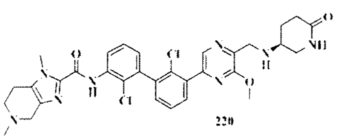
220

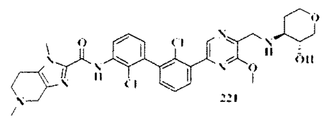
221

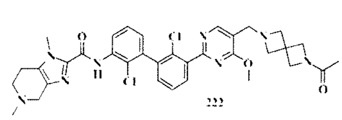
222

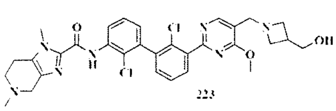
223

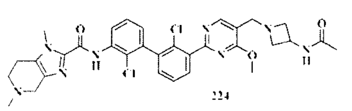
224

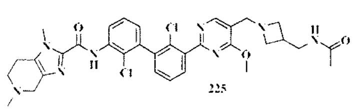
225

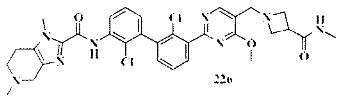
226

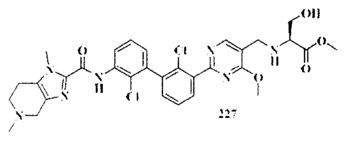
227

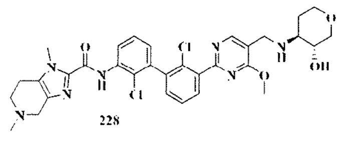
228

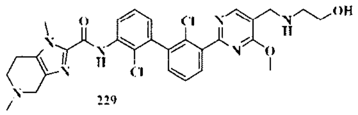
229

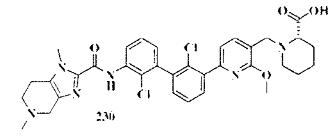
230

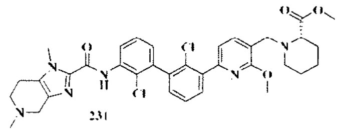
231

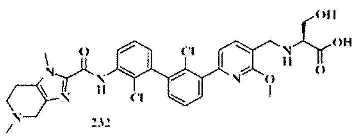
232

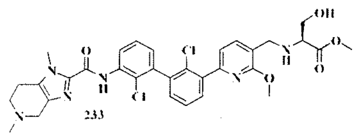
233

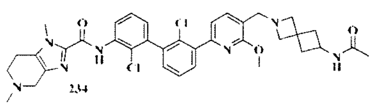
234

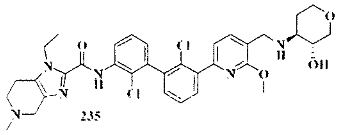
235

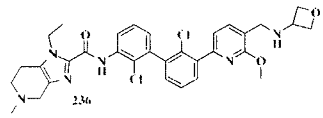
236

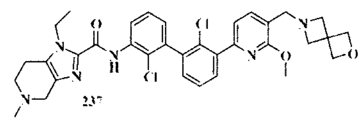
237

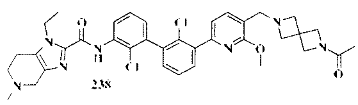
238

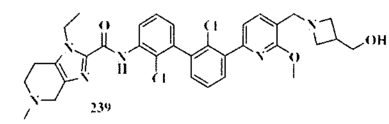
239

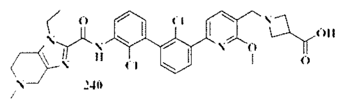
240

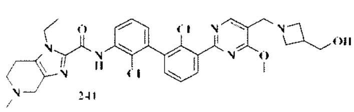
241

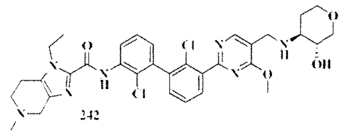
242

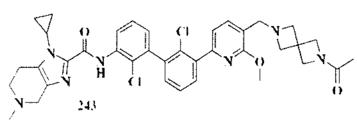
243

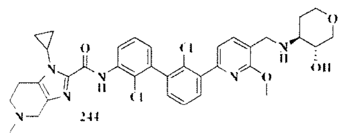
244

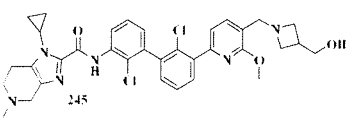
245

or

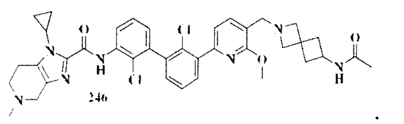
246

.

**20.** A method for preparing the compound represented by general formula (IV-A) as defined in claim 11, the compound represented by general formula (IV) as defined in claim 12 or the compound represented by general formula (VI) as defined in claim 18, the stereoisomer thereof or the pharmaceutically acceptable salt thereof, wherein, the method comprises the following steps:

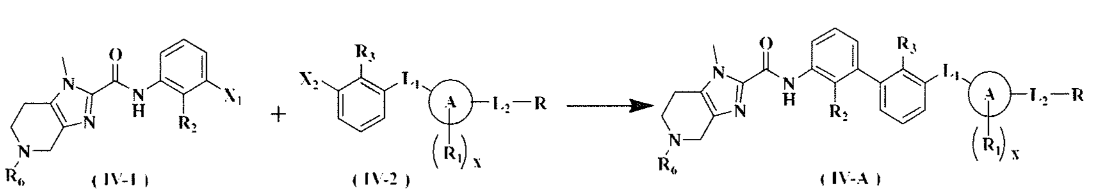

reacting the compound represented by general formula (IV-1) with the compound represented by general formula (IV-2) to obtain the target compound represented by general formula (IV-A);

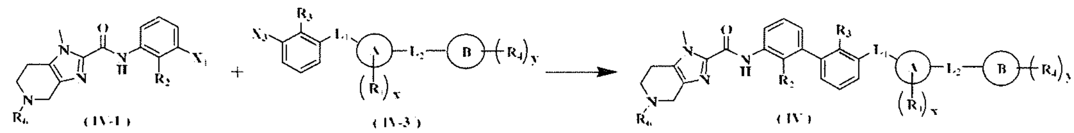

reacting the compound represented by general formula (IV-1) with the compound represented by general formula (IV-3) to obtain the target compound represented by general formula (IV);

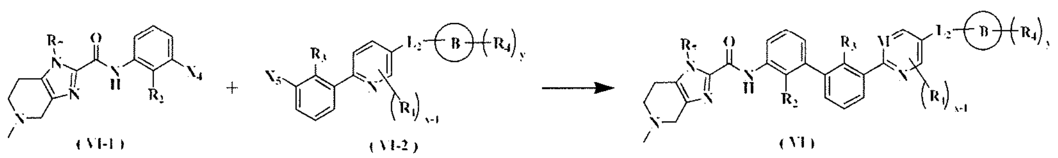

reacting the compound represented by general formula (VI-1) with the compound represented by general formula (VI-2) to obtain the target compound represented by general formula (VI);
wherein:

$X_1$ and $X_4$ are each independently selected from halogen, boronic acid or borate ester; preferably halogen; more preferably bromine;

$X_2$, $X_3$ and $X_5$ are each independently selected from halogen, boronic acid or borate ester; preferably borate ester; more preferably

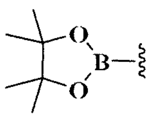

.

**21.** A pharmaceutical composition, comprising a therapeutically effective amount of the compound as defined in any one of claims 1 to 19, and the stereoisomer thereof or the pharmaceutically acceptable salt thereof, and one or more pharmaceutically acceptable carriers or excipients.

**22.** A use of the compound as defined in any one of claims 1 to 19, the stereoisomer thereof or the pharmaceutically acceptable salt thereof, or the pharmaceutical composition as defined in claim 21 in the manufacture of a PD-1/PD-L1 inhibitor medicament.

**23.** A use of the compound as defined in any one of claims 1 to 19, the stereoisomer thereof or the pharmaceutically acceptable salt thereof, or the pharmaceutical composition as defined in claim 21 in the manufacture of a medicament for the treatment of cancer, infectious diseases and autoimmune diseases; preferably the cancer is selected from skin cancer, lung cancer, urological tumor, hematological tumor, breast cancer, glioma, digestive system tumor, reproductive system tumor, lymphoma, neurological tumor, brain tumor, head or neck cancer; the infectious disease is selected from bacterial infection or viral infection; the autoimmune disease is selected from organ-specific autoimmune disease or systemic autoimmune disease, wherein, the organ-specific autoimmune disease comprises chronic lymphocytic thyroiditis, hyperthyroidism, insulin-dependent diabetes mellitus, myasthenia gravis, ulcerative colitis, pernicious anemia with chronic atrophic gastritis, pulmonary hemorrhagic nephritic syndrome, primary biliary cirrhosis, multiple cerebral sclerosis, acute idiopathic polyneuritis, and the systemic autoimmune diseases comprises rheumatoid arthritis, systemic lupus erythematosus, systemic vasculitis, scleroderma, pemphigus, dermatomyositis, mixed connective tissue disease or autoimmune hemolytic anemia.

## INTERNATIONAL SEARCH REPORT

International application No.
**PCT/CN2020/141307**

**A. CLASSIFICATION OF SUBJECT MATTER**

C07D 471/04(2006.01)i; A61P 35/00(2006.01)i; A61P 37/00(2006.01)i; A61K 31/395(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

WPI, EPODOC, CNPAT, CAPLUS(STN), REGISTRY(STN), CNKI, 万方, WANFANG, 百度, BAIDU: 豪森, 翰森生物, PD-1, PD-L1, 抑制剂, 癌症, 免疫性疾病, 联苯, inhibitor, cancer, immune disease, biphenyl, 结构通式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2018/119224 A1 (INCYTE CORP.) 28 June 2018 (2018-06-28) embodiment 1, 10, 12-54, 60-183 and 185, and description, table 1, page 1, lines 1-5, page 3, lines 1-27, page 24, line 12 to page 31, line 19, and page 42, lines 11-16 | 1-23 |
| X | WO 2018/119221 A1 (INCYTE CORP.) 28 June 2018 (2018-06-28) embodiment 11, and description, page 1, lines 1-5, page 3, lines 3-15, and page 9, the last line to page 10, line 1 | 1-17, 19-23 |
| X | WO 2019/192506 A1 (BETTA PHARMACEUTICALS CO., LTD.) 10 October 2019 (2019-10-10) embodiments 1, 2, 4, 8, 10-15, 32, 35, 36, 39, 40, 42, 44, 45, 48-50, 64, 65, 74-82, 89-92, 97, 167, 169, 170, 172-178, 182-188 and 190 | 1-9, 21-23 |
| X | CN 110582493 A (INCYTE CORPORATION) 17 December 2019 (2019-12-17) embodiments 36-42, 50-53, 87, 90-95, 142, 143, 154-158, 240 and 259-271, and description, paragraphs [0001] and [0006]-[0011] | 1-10, 13-16, 20-23 |
| X | WO 2019/217821 A1 (INCYTE CORP.) 14 November 2019 (2019-11-14) embodiments 1-66, and description, page 1, lines 9-12, and page 3, lines 1-17 | 1-10, 13-16, 21-23 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 March 2021** | **25 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/141307**

**C. DOCUMENTS CONSIDERED TO BE RELEVANT**

| Category* | Citation of document, with indication, where appropriate, of the relevant passages | Relevant to claim No. |
|---|---|---|
| X | WO 2018/119286 A1 (INCYTE CORP.) 28 June 2018 (2018-06-28) embodiments 1, 14, 31-36, 45-69 and 99, and description, page 1, lines 1-5, and page 3, lines 3-15 | 1-10, 13-16, 20-23 |
| PX | CN 111925367 A (SHANGHAI HANSOH BIOMEDICAL CO., LTD. et al.) 13 November 2020 (2020-11-13) description, paragraphs [0010]-[0034] and [0087]-[0093], and embodiments 1, 2, 4, 6, 16-18, 30, 37, 46-48, 50, 52 and 53 | 1-23 |
| PX | CN 111039942 A (LONGWOOD BIOPHARMACEUTICALS CO., LTD.) 21 April 2020 (2020-04-21) claims 5-14 | 1-10, 13-16, 20-23 |
| PX | WO 2020/024997 A1 (SHANGHAI ENNOVABIO PHARMACEUTICALS CO., LTD.) 06 February 2020 (2020-02-06) description, page 11, lines 1-5, and page 12, lines 1-30 | 1-10, 13-16, 20-23 |
| PX | WO 2020/232256 A1 (CHEMOCENTRYX INC.) 19 November 2020 (2020-11-19) description, paragraphs [0007], [0008], [0035], [0066], [0067] and [0084], and page 258, compounds 1.398 and 1.400 | 1-10, 13-16, 19-23 |

Form PCT/ISA/210 (second sheet) (January 2015)

**INTERNATIONAL SEARCH REPORT**
**Information on patent family members**

International application No.
**PCT/CN2020/141307**

| Patent document cited in search report | Publication date (day/month/year) | Patent family member(s) | Publication date (day/month/year) |
|---|---|---|---|
| WO 2018/119224 A1 | 28 June 2018 | IL 267458 D0 | 29 August 2019 |
| | | US 10806785 B2 | 20 October 2020 |
| | | CA 3047980 A1 | 28 June 2018 |
| | | EP 3558990 A1 | 30 October 2019 |
| | | AU 2017382258 A1 | 04 July 2019 |
| | | CL 2019001743 A1 | 04 October 2019 |
| | | PH 12019501448 A1 | 01 June 2020 |
| | | US 2018177870 A1 | 28 June 2018 |
| | | PE 20200005 A1 | 06 January 2020 |
| | | CR 20190317 A | 13 September 2019 |
| | | CN 110267953 A | 20 September 2019 |
| | | EC SP19052315 A | 30 September 2019 |
| | | MX 2019007651 A | 07 February 2020 |
| | | JP 2020504739 A | 13 February 2020 |
| | | KR 20190112265 A | 04 October 2019 |
| | | TW 201828940 A | 16 August 2018 |
| | | CO 2019007852 A2 | 18 September 2019 |
| | | BR 112019012957 A2 | 26 November 2019 |
| WO 2018/119221 A1 | 28 June 2018 | US 2018179179 A1 | 28 June 2018 |
| | | EP 3558973 A1 | 30 October 2019 |
| | | US 2020325115 A1 | 15 October 2020 |
| WO 2019/192506 A1 | 10 October 2019 | CN 111936475 A | 13 November 2020 |
| CN 110582493 A | 17 December 2019 | PH 12019501443 A1 | 28 October 2019 |
| | | IL 267461 D0 | 29 August 2019 |
| | | US 2018179197 A1 | 28 June 2018 |
| | | MX 2019007416 A | 11 December 2019 |
| | | CR 20190318 A | 21 October 2019 |
| | | CL 2019001744 A1 | 04 October 2019 |
| | | CO 2019007863 A2 | 01 April 2020 |
| | | AU 2017382870 A1 | 04 July 2019 |
| | | KR 20190111025 A | 01 October 2019 |
| | | EP 3558985 A1 | 30 October 2019 |
| | | US 2019225601 A1 | 25 July 2019 |
| | | EC SP19052302 A | 30 September 2019 |
| | | US 10308644 B2 | 04 June 2019 |
| | | CA 3047986 A1 | 28 June 2018 |
| | | NI 201900070 A | 11 March 2020 |
| | | US 10800768 B2 | 13 October 2020 |
| | | PE 20191532 A1 | 23 October 2019 |
| | | WO 2018119266 A1 | 28 June 2018 |
| | | BR 112019012993 A2 | 03 December 2019 |
| | | TW 201835073 A | 01 October 2018 |
| | | JP 2020504737 A | 13 February 2020 |
| WO 2019/217821 A1 | 14 November 2019 | US 2020277309 A1 | 03 September 2020 |
| | | CA 3099994 A1 | 04 November 2019 |
| | | US 10618916 B2 | 14 April 2020 |
| | | TW 201946626 A | 16 December 2019 |
| | | US 2019345170 A1 | 14 November 2019 |
| WO 2018/119286 A1 | 28 June 2018 | TW 201835049 A | 01 October 2018 |

Form PCT/ISA/210 (patent family annex) (January 2015)

**INTERNATIONAL SEARCH REPORT**
**Information on patent family members**

International application No.
**PCT/CN2020/141307**

| Patent document cited in search report | | | Publication date (day/month/year) | Patent family member(s) | | | Publication date (day/month/year) |
|---|---|---|---|---|---|---|---|
| | | | | US | 2018179202 | A1 | 28 June 2018 |
| | | | | EP | 3558963 | A1 | 30 October 2019 |
| | | | | JP | 2020514272 | A | 21 May 2020 |
| | | | | CA | 3047991 | A1 | 28 June 2018 |
| CN | 111925367 | A | 13 November 2020 | | None | | |
| CN | 111039942 | A | 21 April 2020 | | None | | |
| WO | 2020/024997 | A1 | 06 February 2020 | CN | 110790758 | A | 14 February 2020 |
| WO | 2020/232256 | A1 | 19 November 2020 | US | 2020383979 | A1 | 10 December 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010006873
- CN 202010470293
- CN 202010922161
- WO 2015034820 A
- WO 2015160641 A
- WO 2014151634 A
- WO 2017066227 A
- WO 2017070089 A
- WO 2017106634 A
- WO 2017112730 A
- WO 2017192961 A
- WO 2017222976 A
- WO 2018013789 A
- WO 2018044783 A
- WO 2018119224 A
- WO 2018119236 A
- WO 2018119263 A
- WO 2018119266 A
- WO 2018119286 A
- WO 2011161699 A
- WO 2012168944 A
- WO 2013132317 A
- WO 2013144704 A
- WO 2015033299 A
- WO 2015033301 A
- WO 2015033303 A
- WO 2015036927 A